# EUROPÄISCHE PATENTSCHRIFT

(11) **EP 3 256 462 B1**
(45) Veröffentlichungstag und Bekanntmachung des Hinweises auf die Patenterteilung: **21.08.2019**
(21) Anmeldenummer: 16702955.2
(22) Anmeldetag: 05.02.2016
(51) Int. Cl.: C07D 401/14, A01N 43/50, C07D 401/12, C07D 405/14, C07D 413/14, C07D 409/14

(54) **SUBSTITUIERTE 2-THIOIMIDAZOLYL-CARBOXAMIDE ALS SCHÄDLINGSBEKÄMPFUNGSMITTEL**
SUBSTITUTED 2-THIOIMIDAZOLYL-CARBOXAMIDES AS PESTICIDES
2-THIOIMIDAZOLYL-CARBOXAMIDE SUBSTITUÉ COMME PRODUIT DE LUTTE CONTRE LES PARASITES

(30) Priorität: 09.02.2015 EP 15154252
(43) Veröffentlichungstag der Anmeldung: 20.12.2017
(73) Patentinhaber: Bayer CropScience Aktiengesellschaft, 40789 Monheim am Rhein (DE)
(72) Erfinder: FISCHER, Reiner, 40789 Monheim (DE); HEIL, Markus, 42799 Leichlingen (DE); JANSEN, Johannes-Rudolf, 40789 Monheim (DE); KÜBBELER, Susanne, 40589 Düsseldorf (DE); WILCKE, David, 40219 Düsseldorf (DE); KÖHLER, Adeline, 40764 Langenfeld (DE); WILLOT, Matthieu, 40215 Düsseldorf (DE); EILMUS, Sascha, 42799 Leichlingen (DE); ILG, Kerstin, 50670 Köln (DE); MALSAM, Olga, 51503 Rösrath (DE); LÖSEL, Peter, 51371 Leverkusen (DE); PORTZ, Daniela, 52391 Vettweiss (DE); ANDERSCH, Wolfram, 51469 Bergisch Gladbach (DE)
(74) Vertreter: BIP Patents
(86) Internationale Anmeldenummer: PCT/EP2016/052445
(87) Internationale Veröffentlichungsnummer: WO 2016/128298

(56) Entgegenhaltungen:
- EP-A2- 0 312 960
- WO-A1-2012/084670
- WO-A1-2014/063942
- WO-A2-2011/009804
- D. NORRIS ET. AL: "Synthesis of imidazo[1,5-a]quinoxalin-4(5H)-one template via a novel intramolecularp", TETRAHEDRON LETTERS, Bd. 42, Nr. 26, 25. Juni 2001 (2001-06-25) , Seiten 4297-4299, XP002740775, ISSN: 0040-4039
- M. P. HAY ET. AL.: "Design, Synthesis, and Evaluation of Imidazolylmethyl Carbamate Prodrugs of Alkylating Agents.", TETRAHEDRON, Bd. 56, Nr. 4, 21. Januar 2000 (2000-01-21), Seiten 645-657, XP002740776,
- D. E. BIERER ET. AL.: "Regiospecific Synthesis of the Aminoimiazolquinoxaline (IQx) Mutagens from Cooked Foods.", JOURNAL OF ORGANIC CHEMISTRY, Bd. 57, Nr. 5, 1. Februar 1992 (1992-02-01), Seiten 1390-1405, XP002740777,
- A. JORDAAN ET. AL.: "The Synthesis of 1-Methyl-5-(alpha-indolyl)imidazole and 1-Methyl-2-ethylthiol-5-(alpha-indolyl9-im idazole.", JOURNAL OF HETEROCYCLIC CHEMISTRY, Bd. 5, Nr. 5, 1. Oktober 1968 (1968-10-01) , Seiten 723-725, XP002740778,
- F. HADIZADEH ET. AL.: "Synthesis of Substituted 2-(2-Alkylthio-1-benzyl-5-imidazolyl)-1,3, 4-oxadiazoles.", JOURNAL OF HETEROCYCLIC CHEMISTRY, Bd. 39, Nr. 4, 1. Juli 2002 (2002-07-01), Seiten 841-844, XP002740779,
- R. BOSSIO ET. AL.: "Synthesis and Properties of Some S-Derivatives of 1-Benzyl- and 1-(2-Pyridylmethyl)-5-Substituted 2-Mercaptoimidazoles.", HETEROCYCLES, Bd. 24, Nr. 4, 1. April 1986 (1986-04-01), Seiten 983-989, XP009184745,

## Beschreibung

Die vorliegende Anmeldung betrifft neue heterocyclische Verbindungen, Verfahren und Zwischenprodukte zu Ihrer Herstellung und ihre Verwendung zur Bekämpfung von tierischen Schädlingen.

In WO 2011/009804 A2 sind heterocyclische Verbindungen unter anderem auch Imidazolylcarboxamide beschrieben, die als Insektizide verwendet werden können.

Moderne Insektizide müssen vielen Anforderungen genügen, beispielsweise in Bezug auf Höhe, Dauer und Breite ihrer Wirkung und möglichen Verwendung. Es spielen Fragen der Toxizität, der Nützling- und Bestäuberschonung, der Umwelteigenschaften, der Aufwandmengen, der Kombinierbarkeit mit anderen Wirkstoffen oder Formulierhilfsmitteln eine Rolle sowie die Frage des Aufwands, der für die Synthese eines Wirkstoffs betrieben werden muss, ferner können Resistenzen auftreten, um nur einige Paramenter zu nennen. Schon aus all diesen Gründen kann die Suche nach neuen Pflanzenschutzmitteln nicht als abgeschlossen betrachtet werden und es besteht ständig Bedarf an neuen Verbindungen mit gegenüber den bekannten Verbindungen zumindest in Bezug auf einzelne Aspekte verbesserten Eigenschaften.

Aufgabe der vorliegenden Erfindung war es, Verbindungen bereitzustellen, durch die das Spektrum der Schädlingsbekämpfungsmittel unter verschiedenen Aspekten ergänzt wird.

In Tetrahedron Letters, Band 42, S.4297-4299 (2001), Tetrahedron, Band 56, S.645-57 (2001), Journal of Organic Chemistry, Band 57, S. 1390-1405 (1992), Journal of Heterocyclic Chemistry, Band 5, S. 723-5 (1968), Journloa of Heterocyclic Chemistry, Band 39, S. 841-844 (2002). Heterocycles, Band 24, S. 983-989 (1986) und EP-A-0,312,960 werden Moleküle die den als Zwischenverbindungen bei der Herstellung der Verbindungen der Formel (I) anfallenden Molekülen der Formel (IV), (VIII), (XI), (XXI), und (XXII) ähnlich sind genannt. Sie dienen dort jedoch nicht zur Herstellung von Schädlingsbekämpfungsmittel.

Gelöst wird die Aufgabe, sowie weitere nicht explizit genannte Aufgaben, die aus den hierin diskutierten Zusammenhängen ableitbar oder erschließbar sind, durch die Bereitstellung von Verbindungen der Formel (I) 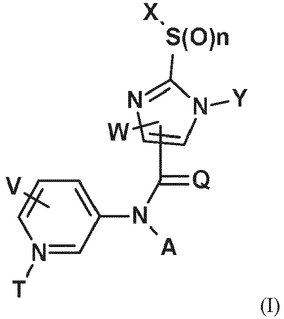 in welcher
- Q: für Sauerstoff oder Schwefel steht,
- V: für einen Rest aus der Reihe Wasserstoff, Halogen, Alkyl, Halogenalkyl, Alkoxy, Halogenalkoxy und Cyano steht,
- W: für einen Rest aus der Reihe Wasserstoff, Halogen, Alkyl, Halogenalkyl, Alkoxy, Halogenalkoxy und Cyano steht,
- X: für einen Rest aus der Reihe gegebenenfalls substituiertes Alkyl, Alkenyl, Alkinyl, gegebenenfalls durch Heteroatome unterbrochenes und gegebenenfalls substituiertes, gesättigtes oder ungesättigtes Cycloalkyl, gegebenenfalls durch Heteroatome unterbrochenes und gegebenenfalls substituiertes, gesättigtes oder ungesättigtes Cycloalkylalkyl, gegebenenfalls substituiertes Aryl, Hetaryl, gegebenenfalls substituiertes Arylalkyl, Hetarylalkyl und Cyano steht,
- Y: für einen Rest aus der Reihe Wasserstoff, gegebenenfalls substituiertes Alkyl, Alkenyl, Alkinyl, gegebenenfalls durch Heteroatome unterbrochenes und gegebenenfalls substituiertes Cycloalkyl, gegebenenfalls durch Heteroatome unterbrochenes und gegebenenfalls substituiertes Cycloalkylalkyl, Arylalkyl, Hetarylalkyl und Cyano steht,
- n: für eine Zahl 0,1 oder 2 steht,
- A: für einen Rest aus der Reihe Wasserstoff, gegebenenfalls substituiertes Alkyl, Alkenyl, Alkinyl und gegebenenfalls durch Heteroatome unterbrochenes und gegebenenfalls substituiertes Cycloalkyl und Cycloalkyl-alkyl steht,
- T: für Sauerstoff oder ein Elektronenpaar steht,
und deren Salze.

Bevorzugte Substituenten bzw. Bereiche der in den Verbindungen der Formel (I) aufgeführten Reste werden im Folgenden erläutert. Ihre Kombination bildet den Vorzugsbereich (1).
- Q: für Sauerstoff oder Schwefel steht,
- V: für einen Rest aus der Reihe Wasserstoff, Halogen, C₁-C₄-Alkyl, C₁-C₄-Halogenalkyl, C₁-C₄-Alkoxy, C₁-C₄-Halogenalkoxy und Cyano steht,
- W: für einen Rest aus der Reihe Wasserstoff, Halogen, C₁-C₄-Alkyl, C₁-C₄-Halogenalkyl, C₁-C₄-Alkoxy, C₁-C₄-Halogenalkoxy und Cyano steht,
- X: für einen Rest aus der Reihe gegebenenfalls einfach bis mehrfach unabhängig voneinander durch Halogen, C₁-C₄-Alkoxy, C₁-C₄-Alkyl-S(O)ₘ-, Cyano, C(O)OR², CONR²R³, C(G)R² substituiertes C₁-C₈-Alkyl, C₃-C₈-Alkenyl, C₃-C₈-Alkinyl, gegebenenfalls einfach bis zweifach unabhängig voneinander durch O, S(O)ₘ, C(G)R², NR⁴ unterbrochenes und gegebenenfalls einfach bis vierfach unabhängig voneinander durch Halogen, C₁-C₄-Alkyl, C₁-C₄-Halogenalkyl, C₁-C₄-Alkoxy, Cyano substituiertes C₃-C₈-Cycloalkyl oder C₅-C₈-Cycloalkenyl, gegebenenfalls einfach bis zweifach unabhängig voneinander durch O, S(O)ₘ, C(G)R², NR⁴ unterbrochenes und gegebenenfalls einfach bis vierfach unabhängig voneinander durch Halogen, C₁-C₄-Alkyl, C₁-C₄-Halogenalkyl, C₁-C₄-Alkoxy, Cyano substituiertes, geradkettiges oder verzweigtes, C₃-C₈-Cycloalkyl-C₁-C₄-Alkyl oder C₅-C₈-Cycloalkenyl-C₁-C₄-Alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₘ-, C₁-C₄-Halogenalkoxy, C₁-C₄-Halogenalkyl-S(O)ₘ-, Nitro und Cyano substituiertes Aryl oder Hetaryl, welches gegebenenfalls durch einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₘ-, C₁-C₄-Halogenalkoxy, C₁-C₄-Halogenalkyl-S(O)ₘ-, Nitro und Cyano substituiertes Aryl oder Hetaryl substituiert sein kann, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₘ- C₁-C₄-Halogenalkoxy, C₁-C₄-Halogenalkyl-S(O)ₘ-, Nitro und Cyano substituiertes, geradkettiges oder verzweigtes Aryl-C₁-C₄-alkyl, Hetaryl-C₁-C₄-alkyl steht,
- G: für O, N-CN, N-OR² steht,
- Y: für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₘ-, Cyano substituiertes C₁-C₆-Alkyl, C₃-C₆-Alkenyl, C₃-C₆-Alkinyl, gegebenenfalls einfach bis zweifach unabhängig voneinander durch Halogen, C₁-C₄-Alkyl, C₁-C₄-Halogenalkyl, C₁-C₄-Alkoxy, Cyano substituiertes C₃-C₈-Cycloalkyl, gegebenenfalls einfach bis zweifach unabhängig voneinander durch O, S(O)ₘ, CO, NR⁴ unterbrochenes und gegebenenfalls einfach bis vierfach unabhängig voneinander durch Halogen, C₁-C₄-Alkyl, C₁-C₄-Halogenalkyl, C₁-C₄-Alkoxy, Cyano substituiertes, geradkettiges oder verzweigtes C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₘ-, C₁-C₄-Halogenalkoxy, C₁-C₄-Halogenalkyl-S(O)ₘ-, Nitro und Cyano substituiertes, Arylalkyl oder Hetarylalkyl und Cyano steht,
- m: für eine Zahl 0,1 oder 2 steht,
- n: für eine Zahl 0,1 oder 2 steht,
- A: für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₘ-, Cyano substituiertes C₁-C₆-Alkyl, C₃-C₆-Alkenyl, C₃-C₆-Alkinyl und gegebenenfalls einfach bis zweifach unabhängig voneinander durch Halogen, C₁-C₄-Alkyl, C₁-C₄-Halogenalkyl, C₁-C₄-Alkoxy, Cyano substituiertes C₃-C₆-Cycloalkyl und gegebenenfalls einfach bis zweifach unabhängig voneinander durch Halogen, C₁-C₄-Alkyl, C₁-C₄-Halogenalkyl, C₁-C₄-Alkoxy, Cyano substituiertes, geradkettiges oder verzweigtes C₃-C₈-Cycloalkyl-C₁-C₄-alkyl steht,
- R²: für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₘ-, substituiertes C₁-C₈-Alkyl, C₃-C₈-Alkenyl, C₃-C₈-Alkinyl, gegebenenfalls einfach durch O, S(O)ₘ, unterbrochenes und gegebenenfalls einfach bis zweifach unabhängig voneinander durch Halogen, C₁-C₄-Alkyl, C₁-C₄-Halogenalkyl, C₁-C₄-Alkoxy, Cyano substituiertes C₃-C₈-Cycloalkyl, gegebenenfalls einfach durch O, S(O)ₙ, unterbrochenes und gegebenenfalls einfach bis zweifach unabhängig voneinander durch Halogen, C₁-C₄-Alkyl, C₁-C₄-Halogenalkyl, C₁-C₄-Alkoxy, Cyano substituiertes, geradkettiges oder verzweigtes C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₘ-, C₁-C₄-Halogenalkoxy, C₁-C₄-Halogenalkyl-S(O)ₘ-, Nitro und Cyano substituiertes Aryl, Hetaryl und gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₘ-, C₁-C₄-Halogenalkoxy, C₁-C₄-Halogenalkyl-S(O)ₘ-, Nitro und Cyano substituiertes, geradkettiges oder verzweigtes Aryl-C₁-C₄-alkyl, Hetaryl-C₁-C₄-alkyl steht,
- R³: für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₘ-, Cyano substituiertes C₁-C₆-Alkyl, C₃-C₆-Alkenyl, C₃-C₆-Alkinyl steht,
- R⁴: für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₘ-, Cyano substituiertes C₁-C₆-Alkyl, C₃-C₆-Alkenyl, C₃-C₆-Alkinyl und für die Reste CONR²R³ und COR² steht, wobei für R² und R³ die vorstehenden Definitionen des Vorzugbereichs (1) gelten,
- T: für Sauerstoff oder ein Elektronenpaar steht
und deren Salze.

Besonders bevorzugte Substituenten bzw. Bereiche der in den Verbindungen der Formel (I) aufgeführten Reste werden im Folgenden erläutert. Ihre Kombination bildet den Vorzugsbereich (2).
- Q: für Sauerstoff steht
- V: für einen Rest aus der Reihe Wasserstoff, Fluor, Chlor, Brom, Methyl, Ethyl, Difluormethyl, Trifluormethyl, Methoxy, Ethoxy, Difluormethoxy, Trifluormethoxy und Cyano steht,
- W: für einen Rest aus der Reihe Wasserstoff, Fluor, Chlor, Brom, Methyl, Ethyl, Difluormethyl, Trifluormethyl, Methoxy, Ethoxy, Difluormethoxy, Trifluormethoxy und Cyano steht,
- X: für einen Rest aus der Reihe gegebenenfalls einfach bis siebenfach unabhängig voneinander durch Fluor, Chlor, Brom, Methoxy, Ethoxy, Methyl-S(O)ₘ-, Ethyl-S(O)ₘ-, Cyano, C(O)OR², CONR²R³, C(G)R² substituiertes C₁-C₆-Alkyl, C₃-C₆-Alkenyl, C₃-C₆-Alkinyl, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Brom, Methyl, Ethyl, Trifluormethyl, Methoxy, Ethoxy, Cyano substituiertes C₃-C₆-Cycloalkyl, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Brom, Methyl, Ethyl, Trifluormethyl, Methoxy, Ethoxy, Cyano substituiertes, geradkettiges oder verzweigtes C₃-C₆-Cycloalkyl-C₁-C₂-alkyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₙ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes Phenyl, Naphtyl Pyridyl, Pyrimidyl, Pyridazinyl, Pyrazinyl, Triazinyl, Furanyl, Thienyl, Oxazolyl, Isoxazolyl, Thiazolyl, Isothiazolyl, Pyrazolyl, Imidazolyl, Oxdiazolyl, Thiadiazolyl, Triazolyl, Benzimidazolyl, Imidazopyridinyl, welches gegebenenfalls durch einfach bis dreifach unabhängig voneinander durch Fluor, Chlor, Methyl, Ethyl, Difluormethyl, Trifluormethyl, Methoxy, Ethoxy, Methyl-S(O)m-, Ethyl-S(O)n-, Difluormethoxy, Trifluormethoxy, Trifluormethyl-S(O)m-, Difluorethyl-S(O)m-, Trifluorethyl-S(O)m-, Nitro und Cyano substituiertes Phenyl substituiert sein kann, gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₘ-, Et-S(O)ₘ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes, geradkettiges oder verzweigtes Phenyl-C₁-C₂-alkyl, Pyridyl-C₁-C₂-alkyl, Pyrimidyl-C₁-C₂-alkyl, Thiazolyl-C₁-C₂-alkyl, Pyrazolyl-C₁-C₂-alkyl und Cyano steht,
- G: für O, N-OR² steht,
- Y: für einen Rest aus der Reihe Wasserstoff, gegebenenfalls einfach bis fünffach unabhängig voneinander durch Fluor, Chlor, Brom, Methoxy, Ethoxy, Methyl-S(O)ₘ-, Ethyl-S(O)ₘ-,Cyano substituiertes C₁-C₄-Alkyl, C₃-C₄-Alkenyl, C₃-C₄-Alkinyl, gegebenenfalls einfach bis zweifach unabhängig voneinander durch Fluor, Chlor, Brom, Methyl, Ethyl, Trifluormethyl, Methoxy, Ethoxy, Cyano substituiertes C₃-C₆-Cycloalkyl, gegebenenfalls einfach bis zweifach unabhängig voneinander durch O, S(O)ₘ, CO, NR⁴ unterbrochenes und gegebenenfalls einfach bis vierfach unabhängig voneinander durch Fluor, Chlor, Brom, Methyl, Ethyl, Trifluormethyl, Methoxy, Ethoxy, Cyano substituiertes, geradkettiges oder verzweigtes C₃-C₆-Cycloalkyl-C₁-C₂-alkyl, gegebenenfalls einfach bis zweifach unabhängig voneinander durch Fluor, Chlor, Brom, Methyl, Ethyl, C₁-C₄-Halogenalkyl, Trifluormethyl, Methoxy, Ethoxy, Cyano, Nitro substituiertes Aryl- C₁-C₂-alkyl oder Hetaryl- C₁-C₂-alkyl und Cyano steht,
- m: für eine Zahl 0,1 oder 2 steht,
- n: für eine Zahl 0,1 oder 2 steht,
- A: für einen Rest aus der Reihe Wasserstoff, gegebenenfalls einfach bis fünffach unabhängig voneinander durch Fluor, Chlor, Brom, Methoxy, Ethoxy, Methyl-S(O)ₘ-, Ethyl-S(O)ₘ-, Cyano substituiertes C₁-C₆-Alkyl, C₃-C₆-Alkenyl, C₃-C₆-Alkinyl und gegebenenfalls einfach bis zweifach unabhängig voneinander durch Fluor, Chlor, Brom, Methyl, Ethyl, Trifluormethyl, Methoxy, Ethoxy, Cyano substituiertes C₃-C₆-Cycloalkyl und gegebenenfalls einfach bis zweifach unabhängig voneinander durch Fluor, Chlor, Brom, Methyl, Ethyl, Trifluormethyl, Methoxy, Ethoxy, Cyano substituiertes, geradkettiges oder verzweigtes C₃-C₆-Cycloalkyl-C₁-C₂-alkyl steht,
- R²: für einen Rest aus der Reihe Wasserstoff, gegebenenfalls einfach bis fünffach unabhängig voneinander durch Fluor, Chlor, Brom, Methoxy, Ethoxy, Methyl-S(O)ₘ-, Ethyl-S(O)ₘ-, substituiertes C₁-C₆-Alkyl, C₃-C₆-Alkenyl, C₃-C₆-Alkinyl, gegebenenfalls einfach durch O, S(O)ₘ, unterbrochenes und gegebenenfalls einfach bis zweifach unabhängig voneinander durch Fluor, Chlor, Brom, Methyl, Ethyl, Trifluormethyl, Methoxy, Ethoxy, Cyano substituiertes C₃-C₆-Cycloalkyl, gegebenenfalls einfach durch O, S(O)ₘ, unterbrochenes und gegebenenfalls einfach bis zweifach unabhängig voneinander durch Fluor, Chlor, Brom, Methyl, Ethyl, Trifluormethyl, Methoxy, Ethoxy, Cyano substituiertes, geradkettiges oder verzweigtes C₃-C₆-Cycloalkyl-C₁-C₂-alkyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₘ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes Phenyl oder Pyridyl und gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₘ-, Difluormethoxy, Trifluormethoxy, Trifluormethyl-S(O)ₙ-, Difluorethyl-S(O)ₙ-, Trifluorethyl-S(O)ₙ-, Nitro und Cyano substituiertes, geradkettiges oder verzweigtes Phenyl-C₁-C₂-alkyl, Pyridyl-C₁-C₂-alkyl, Pyrimidyl-C₁-C₂-alkyl, Thiazolyl-C₁-C₂-alkyl steht,
- R³: für einen Rest aus der Reihe Wasserstoff, gegebenenfalls einfach bis fünffach unabhängig voneinander durch Fluor, Chlor, Brom, Methoxy, Ethoxy, Methyl-S(O)ₘ-, Ethyl-S(O)ₘ-, Cyano substituiertes C₁-C₄-Alkyl, C₃-C₄-Alkenyl, C₃-C₄-Alkinyl steht,
- R⁴: für einen Rest aus der Reihe Wasserstoff, gegebenenfalls einfach bis fünffach unabhängig voneinander durch Fluor, Chlor, Brom, Methoxy, Ethoxy, Methyl-S(O)ₘ-, Ethyl-S(O)ₘ-, Cyano substituiertes C₁-C₄-Alkyl, C₃-C₄-Alkenyl, C₃-C₄-Alkinyl und für die Reste CONR²R³ und COR² steht, wobei für R² und R³ die vorstehenden Definitionen des Vorzugbereichs (2) gelten,
- T: für Sauerstoff oder ein Elektronenpaar steht
und deren Salze.

Ganz besonders bevorzugte Substituenten bzw. Bereiche der in den Verbindungen der Formel (I) aufgeführten Reste werden im Folgenden erläutert. Unter Berücksichtigung der Position der Carboxamidgruppe am Imidazolrest ergibt sich die ganz besonders bevorzugte Struktur (I-A). Ihre Kombination bildet den Vorzugsbereich (3-A). 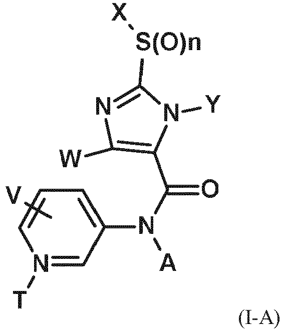
- V: für einen Rest aus der Reihe Wasserstoff, Fluor, Chlor, Methyl und Cyano steht,
- W: für einen Rest aus der Reihe Wasserstoff, Fluor, Chlor, Brom. Methyl, Ethyl und Cyano steht,
- X: für einen Rest aus der Reihe gegebenenfalls einfach, zweifach, dreifach, vierfach, fünffach unabhängig voneinander durch Fluor, Chlor, Brom, Methoxy, Ethoxy, Methyl-S(O)ₘ-, Ethyl-S(O)ₘ-, Cyano substituiertes C₁-C₆-Alkyl, C₃-C₄-Alkenyl, C₃-C₄-Alkinyl, die gegebenenfalls einfach durch die Gruppen C(O)OR², CONR²R³, C(G)R² substituiert sein können, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Methyl, Ethyl, Trifluormethyl, Methoxy, Cyano substituiertes C₃-C₆-Cycloalkyl, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Methyl, Ethyl, Trifluormethyl, Methoxy, Cyano substituiertes C₃-C₆-Cycloalkyl-methyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₘ-, Difluormethoxy, Trifluormethoxy, Trifluorinethyl-S(O)ₘ-, Difluorethyl-S(O)ₘ-, Trifluorethyl-S(O)ₘ-, Nitro und Cyano substituiertes Phenyl, Pyridyl, Pyrimidyl, Thienyl, Thiazolyl, Oxazolyl, Imidazolyl, Oxadiazolyl, Thiadiazolyl, Triazolyl, Benzimidazolyl, Imidazopyridinyl, welches gegebenenfalls durch einfach bis zweifach unabhängig voneinander durch Fluor, Chlor, Methyl, Ethyl, Difluormethyl, Trifluormethyl, Methoxy, Ethoxy, Methyl-S(O)m-, Ethyl-S(O)n-, Difluormethoxy, Trifluormethoxy, Trifluormethyl-S(O)m-, Difluorethyl-S(O)m-, Trifluorethyl-S(O)m-, Nitro und Cyano substituiertes Phenyl substituiert sein kann, gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₘ-, Et-S(O)ₘ-, Difluormethoxy, Trifluormethoxy, Trifluormethyl-S(O)ₙ-, Difluorethyl-S(O)ₘ-, Trifluorethyl-S(O)ₘ-, Nitro und Cyano substituiertes Benzyl, Pyridyl-methyl, Pyrimidyl-methyl, Thiazolylmethyl, Pyrazolyl-C₁-C₂-alkyl und Cyano steht,
- G: für O, N-OR² steht,
- Y: für einen Rest aus der Reihe Wasserstoff, gegebenenfalls einfach bis dreifach unabhängig voneinander durch Fluor, Methoxy, Ethoxy, Cyano substituiertes Methyl, Ethyl, Propyl, Allyl Propargyl und Benzyl steht,
- m: für eine Zahl 0,1 oder 2 steht,
- n: für eine Zahl 0,1 oder 2 steht,
- A: für einen Rest aus der Reihe Wasserstoff, gegebenenfalls einfach bis dreifach unabhängig voneinander durch Fluor, Methoxy, Ethoxy, Cyano substituiertes Methyl, Ethyl, Propyl, Allyl, Propargyl, Cylopropyl oder Cyclopropylmethyl steht,
- R²: für einen Rest aus der Reihe Wasserstoff, gegebenenfalls einfach bis dreifach unabhängig voneinander durch Fluor, Chlor, Methoxy, Ethoxy, Methyl-S(O)ₘ-, Ethyl-S(O)ₘ-, substituiertes C₁-C₆-Alkyl, C₃-C₆-Alkenyl, C₃-C₆-Alkinyl, gegebenenfalls einfach durch O, S(O)ₘ, unterbrochenes und gegebenenfalls einfach bis zweifach unabhängig voneinander durch Fluor, Chlor, Methyl, Ethyl, Trifluormethyl, Methoxy, Cyano substituiertes C₃-C₆-Cycloalkyl, gegebenenfalls einfach durch O, S(O)ₘ, unterbrochenes und gegebenenfalls einfach bis zweifach unabhängig voneinander durch Fluor, Chlor, Methyl, Ethyl, Trifluormethyl, Methoxy, Cyano substituiertes C₃-C₆-Cycloalkyl-methyl gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₘ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes Phenyl oder Pyridyl und gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₘ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes Benzyl, Pyridyl-methyl, Pyrimidyl-methyl, Thiazolyl-methyl steht,
- R³: für einen Rest aus der Reihe Wasserstoff, gegebenenfalls einfach bis dreifach unabhängig voneinander durch Fluor, Chlor, Brom, Methoxy, Ethoxy, Methyl-S(O)ₘ-, Ethyl-S(O)ₘ-, Cyano substituiertes C₁-C₄-Alkyl, C₃-C₄-Alkenyl, C₃-C₄-Alkinyl steht,
- R⁴: für einen Rest aus der Reihe Wasserstoff, gegebenenfalls einfach bis dreifach unabhängig voneinander durch Fluor, Chlor, Brom, Methoxy, Ethoxy, Methyl-S(O)ₘ-, Ethyl-S(O)ₘ-, Cyano substituiertes C₁-C₄-Alkyl, C₃-C₄-Alkenyl, C₃-C₄-Alkinyl und für die Reste CONR²R³ und COR² steht, wobei für R² und R³ die vorstehenden Definitionen des Vorzugbereichs (3A) gelten,
- T: für Sauerstoff oder ein Elektronenpaar steht
und deren Salze.

Ganz besonders bevorzugte Substituenten bzw. Bereiche der in den Verbindungen der Formel (I) aufgeführten Reste werden im Folgenden erläutert. Unter Berücksichtigung der Position der Carboxamidgruppe am Imidazolrest ergibt sich die ganz besonders bevorzugte Struktur (I-B). Ihre Kombination bildet den Vorzugsbereich (3-B). 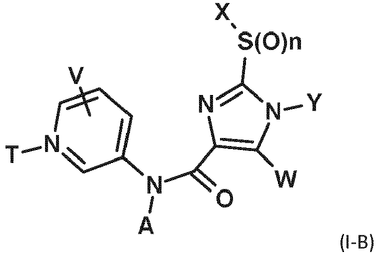
- V: für einen Rest aus der Reihe Wasserstoff, Fluor, Chlor, Methyl und Cyano steht,
- W: für einen Rest aus der Reihe Wasserstoff, Fluor, Chlor, Brom. Methyl, Ethyl und Cyano steht,
- X: für einen Rest aus der Reihe gegebenenfalls einfach bis fünffach unabhängig voneinander durch Fluor, Chlor, Brom, Methoxy, Ethoxy, Methyl-S(O)ₘ-, Ethyl-S(O)ₘ-, Cyano, substituiertes C₁-C₆-Alkyl, C₃-C₄-Alkenyl, C₃-C₄-Alkinyl, die gegebenenfalls einfach durch die Gruppen C(O)OR², CONR²R³, C(G)R² substituiert sein können,, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Methyl, Ethyl, Trifluormethyl, Methoxy, Cyano substituiertes C₃-C₆-Cycloalkyl, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Methyl, Ethyl, Trifluormethyl, Methoxy, Cyano substituiertes C₃-C₆-Cycloalkyl-methyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₘ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes Phenyl, Pyridyl, Pyrimidyl, Thienyl, Thiazolyl, Oxazolyl, Imidazolyl, Oxadiazolyl, Thiadiazolyl, Triazolyl, Benzimidazolyl, Imidazopyridinyl, welches gegebenenfalls durch einfach bis zweifach unabhängig voneinander durch Fluor, Chlor, Methyl, Ethyl, Difluormethyl, Trifluormethyl, Methoxy, Ethoxy, Methyl-S(O)m-, Ethyl-S(O)n-, Difluormethoxy, Trifluormethoxy, Trifluormethyl-S(O)m-, Difluorethyl-S(O)m-, Trifluorethyl-S(O)m-, Nitro und Cyano substituiertes Phenyl substituiert sein kann, gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₘ-, Et-S(O)ₘ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes Benzyl, Pyridyl-methyl, Pyrimidyl-methyl, Thiazolylmethyl, Pyrazolyl-C₁-C₂-alkyl und Cyano steht,
- G: für O, N-OR² steht,
- Y: für einen Rest aus der Reihe Wasserstoff, gegebenenfalls einfach bis dreifach unabhängig voneinander durch Fluor, Methoxy, Ethoxy, Cyano substituiertes Methyl, Ethyl, Propyl, Allyl Propargyl und Benzyl steht,
- m: für eine Zahl 0,1 oder 2 steht,
- n: für eine Zahl 0,1 oder 2 steht,
- A: für einen Rest aus der Reihe Wasserstoff, gegebenenfalls einfach bis dreifach unabhängig voneinander durch Fluor, Methoxy, Ethoxy, Cyano substituiertes Methyl, Ethyl, Propyl, Allyl, Propargyl, Cylopropyl oder Cyclopropyl-methyl steht,
- R²: für einen Rest aus der Reihe Wasserstoff, gegebenenfalls einfach bis dreifach unabhängig voneinander durch Fluor, Chlor, Methoxy, Ethoxy, Methyl-S(O)ₘ-, Ethyl-S(O)ₘ-, substituiertes C₁-C₆-Alkyl, C₃-C₆-Alkenyl, C₃-C₆-Alkinyl, gegebenenfalls einfach durch O, S(O)ₘ, unterbrochenes und gegebenenfalls einfach bis zweifach unabhängig voneinander durch Fluor, Chlor, Methyl, Ethyl, Trifluormethyl, Methoxy, Cyano substituiertes C₃-C₆-Cycloalkyl, gegebenenfalls einfach durch O, S(O)ₘ, unterbrochenes und gegebenenfalls einfach bis zweifach unabhängig voneinander durch Fluor, Chlor, Methyl, Ethyl, Trifluormethyl, Methoxy, Cyano substituiertes C₃-C₆-Cycloalkyl- methyl gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₘ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes Phenyl oder Pyridyl und gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₘ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes Benzyl, Pyridyl-methyl, Pyrimidyl-methyl, Thiazolyl-methyl steht,
- R³: für einen Rest aus der Reihe Wasserstoff, gegebenenfalls einfach bis dreifach unabhängig voneinander durch Fluor, Chlor, Brom, Methoxy, Ethoxy, Methyl-S(O)ₘ-, Ethyl-S(O)ₘ-, Cyano substituiertes C₁-C₄-Alkyl, C₃-C₄-Alkenyl, C₃-C₄-Alkinyl steht,
- R⁴: für einen Rest aus der Reihe Wasserstoff, gegebenenfalls einfach bis dreifach unabhängig voneinander durch Fluor, Chlor, Brom, Methoxy, Ethoxy, Methyl-S(O)ₘ-, Ethyl-S(O)ₘ-, Cyano substituiertes C₁-C₄-Alkyl, C₃-C₄-Alkenyl, C₃-C₄-Alkinyl und für die Reste CONR²R³ und COR² steht, wobei für R² und R³ die vorstehenden Definitionen des Vorzugbereichs (3B) gelten,
- T: für Sauerstoff oder ein Elektronenpaar steht
und deren Salze.

Insbesonders bevorzugte Substituenten bzw. Bereiche der in den Verbindungen der Formel (I) aufgeführten Reste werden im Folgenden erläutert. Unter Berücksichtigung der Position der Carboxamidgruppe am Imidazolrest ergibt sich die insbesonders bevorzugte Struktur (I-A). Ihre Kombination mit den insbesonders bevorzugten Substituenten bildet den Vorzugsbereich (4-A). 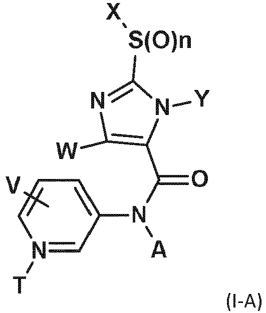
- V: für Wasserstoff und Fluor steht,
- W: für einen Rest aus der Reihe Wasserstoff, Chlor, Brom und Methyl steht,
- X: für einen Rest aus der Reihe gegebenenfalls einfach bis dreifach unabhängig voneinander durch Fluor, Chlor, Brom, Methoxy, Ethoxy, Methylsulfanyl, Ethylsulfanyl, Methylsulfinyl, Ethylsulfinyl, Methylsulfonyl, Ethylsulfonyl, substituiertes Methyl, Ethyl, Propyl, Isopropyl, Butyl, iso-Butyl, sec.-Butyl, tert.-Butyl, Pentyl, Isopentyl, 2.2-dimethyl-propyl, Hexyl, neo-Hexyl, Allyl, Methallyl, 2-Butenyl, Propargyl, 2-Butinyl, die gegebenenfalls einfach durch die Gruppen C(O)OR², CONR²R³, C(G)R² substituiert sein können, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Methyl, Ethyl, Trifluormethyl, Methoxy, Cyano substituiertes Cyclopropyl, Cyclobutyl, Cyclopentyl, Cyclohexyl, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Methyl, Ethyl, Trifluormethyl, Methoxy, Cyano substituiertes Cyclopropylmethyl, Cyclobutylmethyl, Cyclopentylmethyl, Cyclohexyl-methyl, gegebenenfalls einfach bis zweifach unabhängig voneinander durch Fluor, Chlor, Brom, Methyl, Ethyl, Difluormethyl, Trifluormethyl, Methoxy, Ethoxy, Methylsulfanyl, Ethylsulfanyl, Methylsulfinyl, Ethylsulfinyl, Methylsulfonyl, Ethylsulfonyl, Difluormethoxy, Trifluormethoxy, Trifluormethylsulfanyl, Trifluormethylsulfinyl, Trifluormethylsulfonyl, Difluormethylsulfanyl, Difluormethylsulfinyl, Difluormethylsulfonyl, Trifluorethylsulfanyl, Trifluorethylsulfinyl Trifluorethylsulfonyl, Nitro und Cyano substituiertes Phenyl, Pyridyl, Pyrimidyl, Thienyl, Thiazolyl, Oxazolyl, Imidazolyl, Oxadiazolyl, Thiadiazolyl, Triazolyl, Benzimidazolyl, Imidazopyridinyl, welches gegebenenfalls durch Phenyl substituiert sein kann, gegebenenfalls einfach bis zweifach unabhängig voneinander durch Fluor, Chlor, Brom, Methyl, Ethyl, Difluormethyl, Trifluormethyl, Methoxy, Ethoxy, Methylsulfanyl, Ethylsulfanyl, Methylsulfinyl, Ethylsulfinyl, Methylsulfonyl, Ethylsulfonyl, Difluormethoxy, Trifluormethoxy, Trifluormethylsulfanyl, Trifluormethylsulfinyl, Trifluormethylsulfonyl, Difluormethylsulfanyl, Difluormethylsulfinyl, Difluormethylsulfonyl, Trifluorethylsulfanyl, Trifluorethylsulfinyl Trifluorethylsulfonyl, Nitro und Cyano substituiertes Benzyl, Pyridylmethyl, Pyrimidylmethyl, Thiazolylmethyl, Pyrazolyl-C₁-C₂-alkyl und Cyano steht,
- G: für O, N-OR² steht,
- Y: für einen Rest aus der Reihe Wasserstoff, Methyl, Ethyl, Propyl, Difluorethyl, Trifluorethyl, Methoxymethyl, Ethoxymethyl, Cyanomethyl und Benzyl steht,
- m: für eine Zahl 0,1 oder 2 steht,
- n: für eine Zahl 0,1 oder 2 steht,
- A: für einen Rest aus der Reihe Wasserstoff, Methyl, Ethyl, Propyl, Difluorethyl, Trifluorethyl, Methoxymethyl, Ethoxymethyl, Cyanomethyl, Allyl, Propargyl, Cylopropyl oder Cyclopropylmethyl steht,
- R²: für einen Rest aus der Reihe Wasserstoff, gegebenenfalls einfach bis dreifach unabhängig voneinander durch Fluor, Chlor, Methoxy, Ethoxy substituiertes Methyl, Ethyl, Propyl, Isopropyl, n-Butyl, iso-Butyl, sec.-Butyl, tert.-Butyl, Allyl, Methallyl, 2-Butenyl, Propargyl, 2-Butinyl gegebenenfalls einfach durch O, S(O)ₘ, unterbrochenes und gegebenenfalls einfach bis zweifach unabhängig voneinander durch Fluor, Chlor, Methyl, Ethyl, Trifluormethyl, Methoxy, Cyano substituiertes Cyclopropyl, Cyclobutyl, Cyclopentyl, Cyclohexyl,, gegebenenfalls einfach durch O, S(O)ₘ, unterbrochenes und gegebenenfalls einfach bis zweifach unabhängig voneinander durch Fluor, Chlor, Methyl, Ethyl, Trifluormethyl, Methoxy, Cyano substituiertes Cyclopropyl-methyl, Cyclobutyl-methyl, Cyclopentyl-methyl, Cyclohexyl-methyl, gegebenenfalls einfach bis zweifach unabhängig voneinander durch Fluor, Chlor, Brom, Methyl, Ethyl, Difluormethyl, Trifluormethyl, Methoxy, Ethoxy, Methylsulfanyl, Ethylsulfanyl, Methylsulfinyl, Ethylsulfinyl, Methylsulfonyl, Ethylsulfonyl, Difluormethoxy, Trifluormethoxy, Trifluormethylsulfanyl, Trifluormethylsulfinyl, Trifluormethylsulfonyl, Difluormethylsulfanyl, Difluormethylsulfinyl, Difluormethylsulfonyl, Trifluorethylsulfanyl, Trifluorethylsulfinyl Trifluorethylsulfonyl, Nitro und Cyano substituiertes Phenyl oder Pyridyl und gegebenenfalls einfach bis zweifach unabhängig voneinander durch Fluor, Chlor, Brom, Methyl, Ethyl, Difluormethyl, Trifluormethyl, Methoxy, Ethoxy, Difluormethoxy, Trifluormethoxyl, Nitro und Cyano substituiertes Benzyl, Pyridyl-methyl, Pyrimidylmethyl, Thiazolylmethyl steht,
- R³: für einen Rest aus der Reihe Wasserstoff, Methyl, Ethyl, Propyl, Isopropyl, Butyl, iso-Butyl, und Allyl steht,
- R⁴: für einen Rest aus der Reihe Wasserstoff, gegebenenfalls einfach bis dreifach unabhängig voneinander durch Fluor, Methoxy, Ethoxy, Cyano Methyl, Ethyl, Propyl, Isopropyl, Butyl, iso-Butyl, und Allyl und für die Reste CONR²R³ und COR² steht, wobei für R² und R³ vorstehenden Definitionen des Vorzugbereichs (4A) gelten,
- T: für Sauerstoff oder ein Elektronenpaar steht
und deren Salze.

Insbesonders bevorzugte Substituenten bzw. Bereiche der in den Verbindungen der Formel (I) aufgeführten Reste werden im Folgenden erläutert. Unter Berücksichtigung der Position der Carboxamidgruppe am Imidazolrest ergibt sich die insbesonders bevorzugte Struktur (I-B). Ihre Kombination mit den insbesonders bevorzugten Substituenten bildet den Vorzugsbereich (4-B). 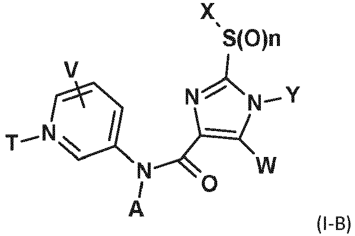
- V: für Wasserstoff und Fluor steht,
- W: für einen Rest aus der Reihe Wasserstoff, Chlor, Brom und Methyl steht,
- X: für einen Rest aus der Reihe gegebenenfalls einfach bis dreifach unabhängig voneinander durch Fluor, Chlor, Brom, Methoxy, Ethoxy, Methylsulfanyl, Ethylsulfanyl, Methylsulfinyl, Ethylsulfinyl, Methylsulfonyl, Ethylsulfonyl, Cyano substituiertes Methyl, Ethyl, Propyl, Isopropyl, n-Butyl, iso-Butyl, sec.-Butyl, tert.-Butyl, Pentyl, Isopentyl, 2.2-dimethyl-propyl, Hexyl, neo-Hexyl, Allyl, Methallyl, 2-Butenyl, Propargyl, 2-Butinyl, die gegebenenfalls einfach durch die Gruppen C(O)OR², CONR²R³, C(G)R² substituiert sein können, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Methyl, Ethyl, Trifluormethyl, Methoxy, Cyano substituiertes Cyclopropyl, Cyclobutyl, Cyclopentyl, Cyclohexyl, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Methyl, Ethyl, Trifluormethyl, Methoxy, Cyano substituiertes Cyclopropylmethyl, Cyclobutylmethyl, Cyclopentylmethyl, Cyclohexylmethyl, gegebenenfalls einfach bis zweifach unabhängig voneinander durch Fluor, Chlor, Brom, Methyl, Ethyl, Difluormethyl, Trifluormethyl, Methoxy, Ethoxy, Methylsulfanyl, Ethylsulfanyl, Methylsulfinyl, Ethylsulfinyl, Methylsulfonyl, Ethylsulfonyl, Difluormethoxy, Trifluormethoxy, Trifluormethylsulfanyl, Trifluormethylsulfinyl, Trifluormethylsulfonyl, Difluormethylsulfanyl, Difluormethylsulfinyl, Difluormethylsulfonyl, Trifluorethylsulfanyl, Trifluorethylsulfinyl Trifluorethylsulfonyl, Nitro und Cyano substituiertes Phenyl, Pyridyl, Pyrimidyl, Thienyl, Thiazolyl, Oxazolyl, Imidazolyl, Oxadiazolyl, Thiadiazolyl, Triazolyl, Benzimidazolyl, Imidazopyridinyl, welches gegebenenfalls durch Phenyl substituiert sein kann, gegebenenfalls einfach bis zweifach unabhängig voneinander durch Fluor, Chlor, Brom, Methyl, Ethyl, Difluormethyl, Trifluormethyl, Methoxy, Ethoxy, Methylsulfanyl, Ethylsulfanyl, Methylsulfinyl, Ethylsulfinyl, Methylsulfonyl, Ethylsulfonyl, Difluormethoxy, Trifluormethoxy, Trifluormethylsulfanyl, Trifluormethylsulfinyl, Trifluormethylsulfonyl, Difluormethylsulfanyl, Difluormethylsulfinyl, Difluormethylsulfonyl, Trifluorethylsulfanyl, Trifluorethylsulfinyl Trifluorethylsulfonyl, Nitro und Cyano substituiertes Benzyl, Pyridylmethyl, Pyrimidylmethyl, Thiazolylmethyl, Pyrazolyl-C₁-C₂-alkyl und Cyano steht,
- G: für O, N-OR² steht,
- Y: für einen Rest aus der Reihe Wasserstoff, Methyl, Ethyl, Propyl, Difluorethyl, Trifluorethyl, Methoxymethyl, Ethoxymethyl, Cyanomethyl und Benzyl steht,
- m: für eine Zahl 0,1 oder 2 steht,
- n: für eine Zahl 0,1 oder 2 steht,
- A: für einen Rest aus der Reihe Wasserstoff, Methyl, Ethyl, Propyl, Difluorethyl, Trifluorethyl, Methoxymethyl, Ethoxymethyl, Cyanomethyl, Allyl, Propargyl, Cylopropyl oder Cyclopropylmethyl steht,
- R²: für einen Rest aus der Reihe Wasserstoff, gegebenenfalls einfach bis dreifach unabhängig voneinander durch Fluor, Chlor, Methoxy, Ethoxy substituiertes Methyl, Ethyl, Propyl, Isopropyl, n-Butyl, iso-Butyl, sec.-Butyl, tert.-Butyl, Allyl, Methallyl, 2-Butenyl, Propargyl, 2-Butinyl gegebenenfalls einfach durch O, S(O)ₘ, unterbrochenes und gegebenenfalls einfach bis zweifach unabhängig voneinander durch Fluor, Chlor, Methyl, Ethyl, Trifluormethyl, Methoxy, Cyano substituiertes Cyclopropyl, Cyclobutyl, Cyclopentyl, Cyclohexyl, gegebenenfalls einfach durch O, S(O)ₘ, unterbrochenes und gegebenenfalls einfach bis zweifach unabhängig voneinander durch Fluor, Chlor, Methyl, Ethyl, Trifluormethyl, Methoxy, Cyano substituiertes Cyclopropylmethyl, Cyclobutylmethyl, Cyclopentylmethyl, Cyclohexylmethyl, gegebenenfalls einfach bis zweifach unabhängig voneinander durch Fluor, Chlor, Brom, Methyl, Ethyl, Difluormethyl, Trifluormethyl, Methoxy, Ethoxy, Methylsulfanyl, Ethylsulfanyl, Methylsulfinyl, Ethylsulfinyl, Methylsulfonyl, Ethylsulfonyl, Difluormethoxy, Trifluormethoxy, Trifluormethylsulfanyl, Trifluormethylsulfinyl, Trifluormethylsulfonyl, Difluormethylsulfanyl, Difluormethylsulfinyl, Difluormetliylsulfonyl, Trifluorethylsulfanyl, Trifluorethylsulfinyl Trifluorethylsulfonyl, Nitro und Cyano substituiertes Phenyl oder Pyridyl und gegebenenfalls einfach bis zweifach unabhängig voneinander durch Fluor, Chlor, Brom, Methyl, Ethyl, Difluormethyl, Trifluormethyl, Methoxy, Ethoxy, Difluormethoxy, Trifluormethoxyl, Nitro und Cyano substituiertes Benzyl, Pyridyl-methyl, Pyrimidyl-methyl, Thiazolyl-methyl steht,
- R³: für einen Rest aus der Reihe Wasserstoff, Methyl, Ethyl, Propyl, Isopropyl, n-Butyl, iso-Butyl, und Allyl steht,
- R⁴: für einen Rest aus der Reihe Wasserstoff, gegebenenfalls einfach bis dreifach unabhängig voneinander durch Fluor, Methoxy, Ethoxy, Cyano Methyl, Ethyl, Propyl, Isopropyl, n-Butyl, iso-Butyl, und Allyl und für die Reste CONR²R³ und COR² steht, wobei für R² und R³ die vorstehenden Definitionen des Vorzugbereichs (4B) gelten,
- T: für Sauerstoff oder ein Elektronenpaar steht
und deren Salze.

Ganz besonders bevorzugte Substituenten bzw. Bereiche der in den Verbindungen der Formel (I) aufgeführten Reste werden im Folgenden erläutert. Ihre Kombination bildet den Vorzugsbereich (5). 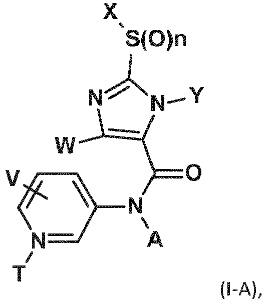
- V: für Wasserstoff steht,
- W: für einen Rest aus der Reihe Wasserstoff, Chlor und Brom und bevorzugt für Wasserstoff steht,
- X: für einen Rest aus der Reihe, Methyl, Ethyl, n-Butyl, n-Pentyl, n-Propyl, iso-Propyl, Allyl, 3,3-Dimethylallyl, Propargyl, Cyclohexyl, Tetrahydropyranyl, Tetrahydrothiopyranyl, 3-Oxetanyl, 5-Oxa-[3.3.0]-bicycloheptanyl, Methoxyethyl, Methoxypropyl, Ethoxyethyl, Ethylthioethyl, Methylthioethyl, Difluormethyl, Trifluormethyl, 2,2,2-Trifluorethyl, 3,3,3-Trifluorpropyl, 3-Chlor-2,2,3,3-tetra-fluorpropyl, 3-Fluorpropyl, 3,3-Difluorpropyl, 2,2,2-Trifluorethylthio-ethyl, Methylcarbonyl-methyl, c-Propylcarbonyl-methyl, tert.-Butylcarbonyl-methyl, Methoxycarbonyl-methyl, Ethoxycarbonyl-methyl, Hydroxycarbonyl-methyl, Carbamoyl-methyl, N-Methylcarbamoyl-methyl, N-c-Propylcarbamoyl-methyl, N,N-Dimethylcarbamoylmethyl, 2-Methoximino-propyl, Cyclopropylmethyl, Phenyl, 4-Methylphenyl, 2-Nitrophenyl, 3-Methylthiophenyl, 4-Chlor-phenyl, 4-Fluor-phenyl, 4-tert.-Butyl-phenyl, 4-Methoxy-phenyl, 4-Nitrophenyl, 4-Dimethylamino-pbenyl, 2-Fluor-phenyl, 2-Methoxy-phenyl, 2-Dimethylaminosulfonyl-phenyl, 2-Dimethylaminocarbamoylphenyl, 3-Nitrophenyl, 3-Trifluormethyl-phenyl, 3-Chlor-phenyl, 2,5-Dichlor-phenyl, 3,5-Dichlor-phenyl, 4-Chlor-3-trifluormethyl-phenyl, 2,4,5-Trichlor-phenyl, 2-Pyridyl, 5-(2-Chlor)pyridyl, 2-(5-Methyl)-pyridyl, 2-(6-Methyl)-pyridyl, 2-(3-Trifluormethyl)-pyridyl, 2-Pyrimidyl, 2-(4-Methyl)-pyrimidyl, 2-(5-Methyl)-pyrimidyl, 2-(4-Methoxy)-pyrimidyl, 2-(5-Fluor)-pyrimidyl, 2-(4-Trifluormethyl)-pyrimidyl, 2-(5-Trifluormethyl)-pyrimidyl, 2-(4,6-Dimethyl)-pyrimidyl, 2-(4,5-Dimethyl)-pyrimidyl, 2-(4,6-Dimethoxy)-pyrimidyl, -CH₂-2-pyrimidyl, -CH₂-2-pyrazinyl, -CH₂-5-(1-methyl)-imidazolyl, -CH₂-3-(1-methyl)-pyrazolyl, -CH₂-4-pyridyl, -CH₂-2-pyridyl, -CH₂-2-(1-methyl)-imidazolyl, -CH₂-3-pyridyl, -CH₂-2-furanyl, -CH₂-5-(2-chlor)-pyridyl, Benzyl, 3,4-Dichlor-benzyl, 2,6-Difluor-benzyl, 2-Fluor-6-methoxy-benzyl, 2,6-Dichlor-benzyl, 2-Chlor-6-trifluormethyl-benzyl, 2-Chlor-6-fluor-benzyl, -CH₂-2-(4,6-dimethoxy)-pyrimidyl, 2,6-Dimethyl-benzyl, -CH₂-1-(3-nitro-5-methyl)-pyrazolyl, 2-(1-Methyl)-benzimidazolyl, 2-(5-Methyl)-oxadiazolyl, 2-[3-Methyl-6-(trifluormethyl)-imidazo[4.5]-pyridinyl, 3-[4-Ethyl-5-(trifluorinethyl)]-1,2,4-triazolyl, 3-[4-Methyl-5-(trifluormethyl)]-1,2,4-triazolyl, 3-[4-Methyl-5-(difluormethyl)]-1,2,4-triazolyl, 2-(5-Phenyl)-1,3,4-thiadiazolyl, 2-(1-Methyl-5-phenyl)-imidazolyl, 2-(4,5-Dimethyl)-oxazolyl, 2-(1-Methyl-5-methoxycarbonyl)-imidazolyl, 2-(1-Methyl)-imidazolyl, 1,2-Ethandiyl- steht,
- Y: für Methyl, Ethyl und Benzyl steht,
- n: für eine Zahl 0 oder 2 und bevorzugt für 0 steht,
- A: für einen Rest aus der Reihe Wasserstoff und Methyl und bevorzugt für Methyl steht,
- T: für ein Elektronenpaar steht
und
Verbindungen der Formel (I-B) 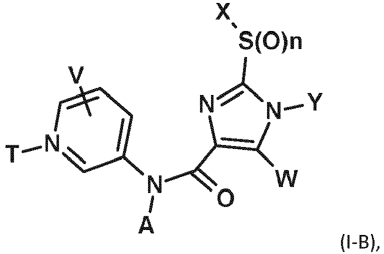 wobei
- V: für Wasserstoff steht,
- W: für einen Rest aus der Reihe Wasserstoff, Chlor und Brom und bevorzugt für Wasserstoff steht,
- X: für einen Rest aus der Reihe 4,6-Dimethylpyrimidyl, n-Butyl, n-Pentyl, Benzyl, Methyl, 3-Methylthiophenyl, 2,2,2-Trifluorethyl, Phenyl, 4-Methylphenyl, 2-Pyrimidyl, Ethylthioethyl, 2-Nitrophenyl, Cyclopropylmethyl und bevorzugt für einen 2-Pyrimidyl-Rest steht,
- Y: für Methyl steht,
- n: für eine Zahl 0 oder 2 und bevorzugt für 0 steht,
- A: für einen Rest aus der Reihe Wasserstoff und Methyl und bevorzugt für Methyl steht,
- T: für ein Elektronenpaar steht.
und deren Salze.

Es ist ebenfalls möglich, dass bei einem der Vorzugsbereiche (1-5) X und Y mit den Atomen, an die sie gebunden sind, für einen gesättigten oder ungesättigten Cyclus stehen. Bevorzugt stehen X und Y mit den Atomen, an die sie gebunden sind, für einen gesättigten oder ungesättigten 5-7-gliedrigen Ring. Ganz besonders bevorzugt stehen X und Y mit den Atomen, an die sie gebunden sind, für einen gesättigten 5-Ring.

Im Vorzugsbereich (1) ist, sofern nichts anderes angegeben ist,
Halogen ausgewählt aus der Reihe Fluor, Chlor, Brom und Iod, bevorzugt wiederum aus der Reihe Fluor, Chlor und Brom,
Hetaryl (gleichbedeutend mit Heteroaryl, auch als Teil einer größeren Einheit, wie beispielsweise Hetarylalkyl) ausgewählt aus der Reihe Furyl, Thienyl, Pyrrolyl, Pyrazolyl, Imidazolyl, 1,2,3-Triazolyl, 1,2,4-Triazolyl, Oxazolyl, Isoxazolyl, Thiazolyl, Isothiazolyl, 1,2,3-Oxadiazolyl, 1,2,4-Oxadiazolyl, 1,3,4-Oxadiazolyl, 1,2,5-Oxadiazolyl, 1,2,3-Thiadiazolyl, 1,2,4-Thiadiazolyl, 1,3,4-Thiadiazolyl, 1,2,5-Thiadiazolyl, Pyridyl, Pyrimidinyl, Pyridazinyl, Pyrazinyl, 1,2,3-Triazinyl, 1,2,4-Triazinyl, 1,3,5-Triazinyl, Benzofuryl, Benzisofuryl, Benzothienyl, Benzisothienyl, Indolyl, Isoindolyl, Indazolyl, Benzothiazolyl, Benzisothiazolyl, Benzoxazolyl, Benzisoxazolyl, Benzimidazolyl, 2,1,3-Benzoxadiazole, Chinolinyl, Isochinolinyl, Cinnolinyl, Phthalazinyl, Chinazolinyl, Chinoxalinyl, Naphthyridinyl, Benzotriazinyl, Purinyl, Pteridinyl und Indolizinyl,
Heterocyclyl für einen gesättigten 3-, 4-, 5- oder 6-Ring, der 1 oder 2 Stickstoffatome und/oder ein Sauerstoffatom und/oder ein Schwefelatom enthält, wobei jedoch nicht 2 Stickstoffatome direkt benachbart sein sollen, beispielsweise für Aziridinyl, Azetidinyl, Azolidinyl, Azinanyl, Oxiranyl, Oxetanyl, Oxolanyl, Oxanyl, Dioxanyl, Thiiranyl, Thietanyl, Thiolanyl, Thianyl, Tetrahydrofuryl.

Im Vorzugsbereich (2) steht, sofern nichts anderes angegeben ist,
Halogen für Fluor, Chlor, Brom und Iod, bevorzugt für Fluor, Chlor und Brom,
Hetaryl (auch als Teil einer größeren Einheit, wie Hetarylalkyl) für Pyridyl, Pyrimidyl, Thiazolyl, Oxazolyl, Pyrazolyl, Thienyl, Furanyl, Benzyl, Pyridinylmethyl und Thiazolylmethyl sowie
Heterocyclyl (auch als Teil einer größeren Einheit, wie Heterocyclylalkyl) für einen gesättigten oder ungesättigten 3-, 4- oder 5-Ring, der 1 oder 2 Stickstoffatome und/oder ein Sauerstoffatom und/oder ein Schwefelatom enthält, wobei jedoch nicht 2 Stickstoffatome direkt benachbart sein sollen, beispielsweise für 1- oder 2-Aziridinyl, 2-Oxiranyl, 2-Thiiranyl, 1- oder 2-Azetidinyl, 2- oder 3-Oxetanyl, 2- oder 3-Thietanyl, 1,3-Dioxetan-2-yl, 1-,2- oder 3-Pyrrolidinyl.

Im Vorzugsbereich (3) steht, sofern nichts anderes angegeben ist,
Halogen für Fluor, Chlor, Brom und Iod, bevorzugt für Fluor, Chlor und Brom und
Heterocyclyl (auch als Teil einer größeren Einheit, wie Heterocyclylalkyl) für einen gesättigten oder ungesättigten 3- oder 4-Ring, der 1 oder 2 Stickstoffatome und/oder ein Sauerstoffatom und/oder ein Schwefelatom enthält, wobei jedoch nicht 2 Stickstoffatome direkt benachbart sein sollen, beispielsweise für 1- oder 2-Aziridinyl, 2-Oxiranyl, 2-Thiiranyl, 1- oder 2-Azetidinyl, 2- oder 3-Oxetanyl, 2- oder 3-Thietanyl oder 1,3-Dioxetan-2-yl.Durch Halogen substituierte Reste, z.B. Halogenalkyl (= Haloalkyl), sind, sofern nichts anderes gesagt ist, einfach oder mehrfach bis zur maximal möglichen Substituentenzahl halogeniert. Bei mehrfacher Halogenierung können die Halogenatome gleich oder verschieden sein. Halogen steht dabei für Fluor, Chlor, Brom oder Iod, insbesondere für Fluor, Chlor oder Brom.

Gesättigte oder ungesättigte Kohlenwasserstoffreste wie Alkyl oder Alkenyl können, auch in Verbindung mit Heteroatomen, wie z.B. in Alkoxy, soweit möglich, jeweils geradkettig oder verzweigt sein.

Gegebenenfalls substituierte Reste können, wenn nichts anderes erwähnt ist, einfach oder mehrfach substituiert sein, wobei bei Mehrfachsubstitutionen die Substituenten gleich oder verschieden sein können.

Wenn T in den Verbindungen der Formel (I) für Sauerstoff steht, liegen diese Verbindungen als N-Oxide vor.

Wenn T in den Verbindungen der Formel (I) für ein Elektronenpaar steht, liegen diese Verbindungen als Pyridine vor.

Die oben aufgeführten allgemeinen oder in Vorzugsbereichen aufgeführten Restedefinitionen bzw. Erläuterungen gelten für die Endprodukte und für die Ausgangsprodukte und Zwischenprodukte entsprechend. Diese Restedefinitionen können untereinander, also auch zwischen den jeweiligen Vorzugsbereichen, beliebig kombiniert werden.

Erfindungsgemäß bevorzugt verwendet werden Verbindungen der Formel (I), in welchen eine Kombination der vorstehend als bevorzugt aufgeführten Bedeutungen vorliegt (Vorzugsbereich (1)).

Erfindungsgemäß besonders bevorzugt verwendet werden Verbindungen der Formel (I), in welchen eine Kombination der vorstehend als besonders bevorzugt aufgeführten Bedeutungen vorliegt (Vorzugsbereich (2)).

Erfindungsgemäß ganz besonders bevorzugt verwendet werden Verbindungen der Formel (I), in welchen eine Kombination der vorstehend als ganz besonders bevorzugt aufgeführten Bedeutungen vorliegt (Vorzugsbereich (3A und / oder 3B)).

Erfindungsgemäß insbesondere bevorzugt verwendet werden Verbindungen der Formel (I), in welchen eine Kombination der vorstehend als ganz besonders bevorzugt aufgeführten Bedeutungen vorliegt (Vorzugsbereich (4A und / oder 4B)).

Erfindungsgemäß noch weiter bevorzugt verwendet werden Verbindungen der Formel (I), in welchen eine Kombination der vorstehend als ganz besonders bevorzugt aufgeführten Bedeutungen vorliegt (Vorzugsbereich (5).

Die Verbindungen der Formel (I) können in Abhängigkeit von der Art der Substituenten als geometrische und/oder als optisch aktive Isomere oder entsprechende Isomerengemische in unterschiedlicher Zusammensetzung vorliegen. Diese Stereoisomere sind beispielsweise Enantiomere, Diastereomere, Atropisomere oder geometrische Isomere. Die Erfindung umfasst somit reine Stereoisomere als auch beliebige Gemische dieser Isomere. Weiter wurde gefunden, dass sich die neuen Verbindungen der Formel (I) nach den im Folgenden beschriebenen Verfahren herstellen lassen.

Die Verbindungen der Formel (I-A) können beispielsweise nach Verfahren A bis D wie in den folgenden Schemata dargestellt synthetisiert werden.

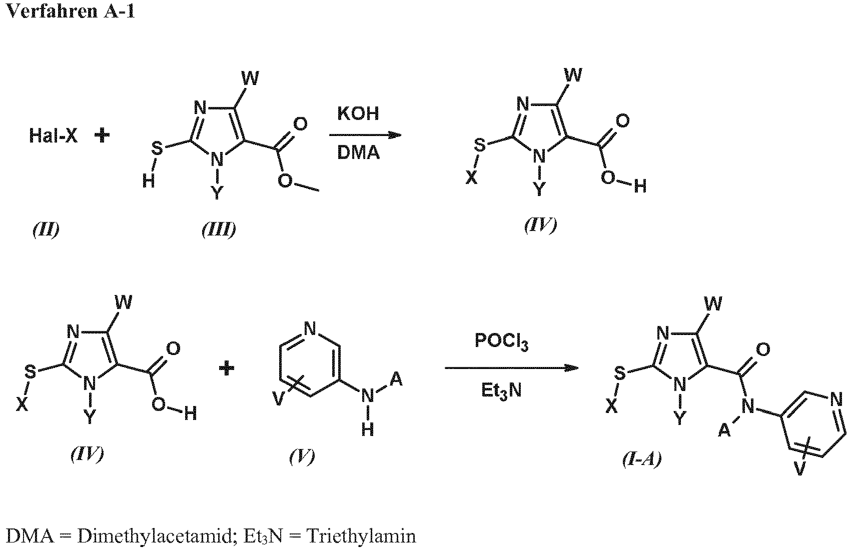

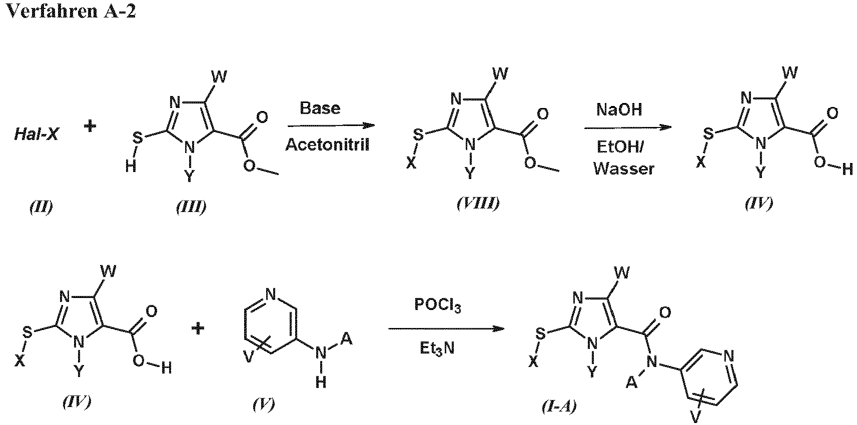

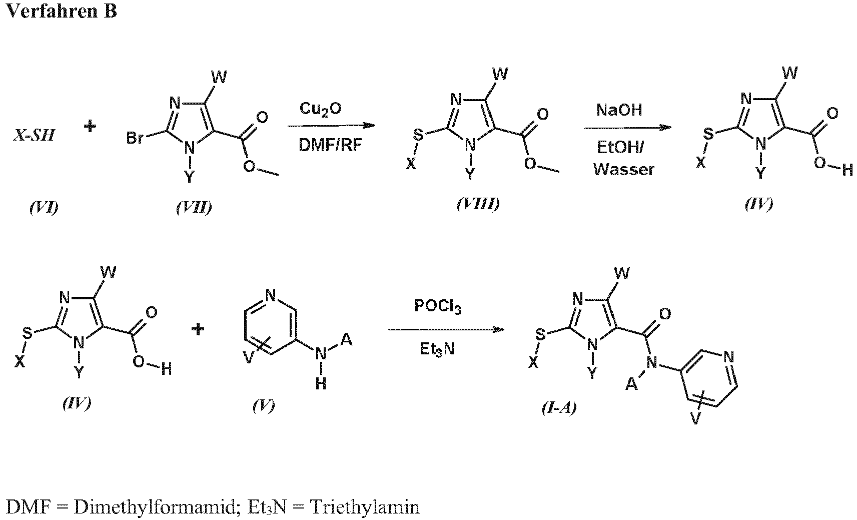

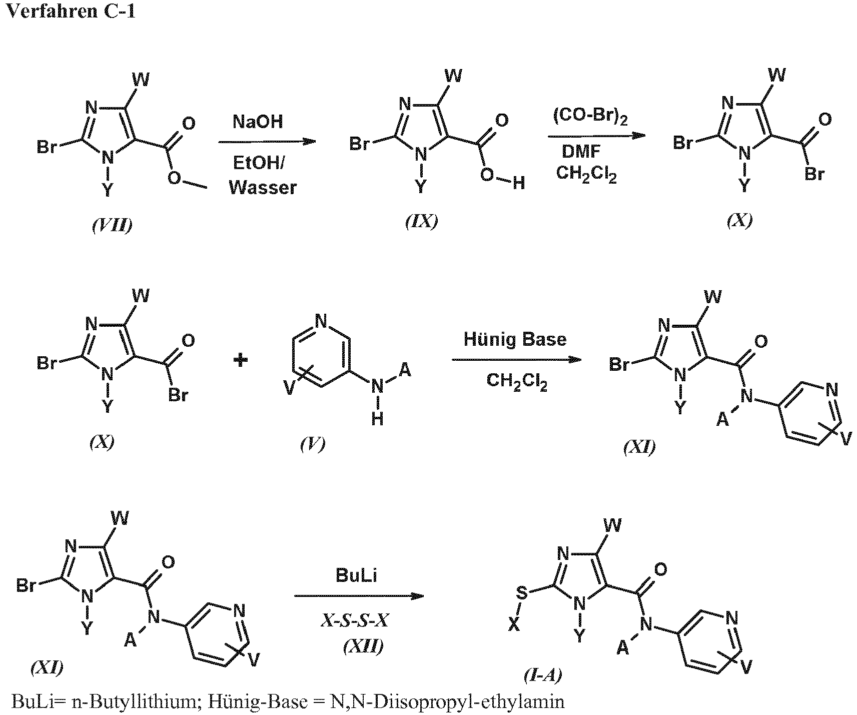

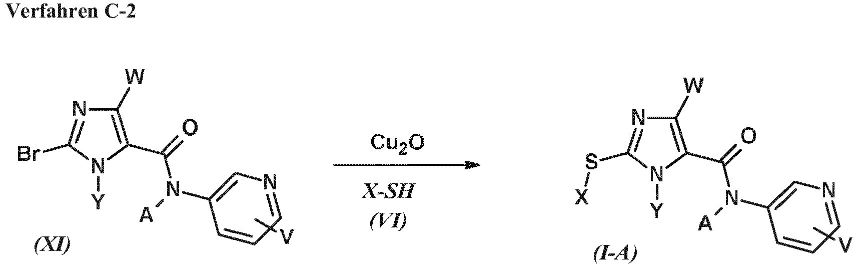

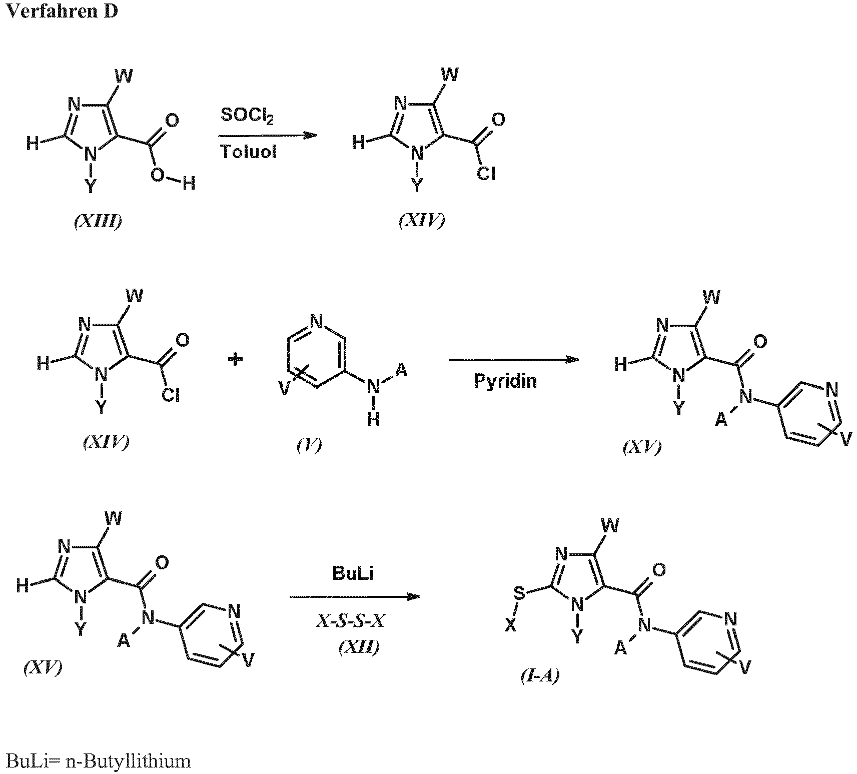

Die Verbindungen der Formel (I-B) können beispielsweise nach Verfahren E wie in dem folgenden Schema dargestellt synthetisiert werden.

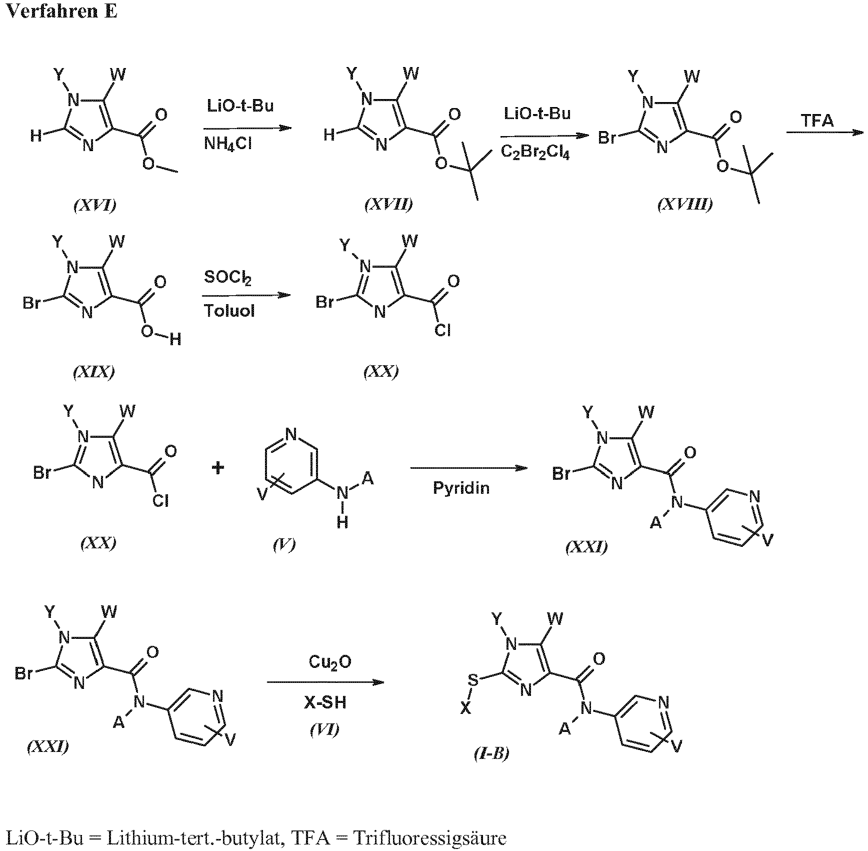

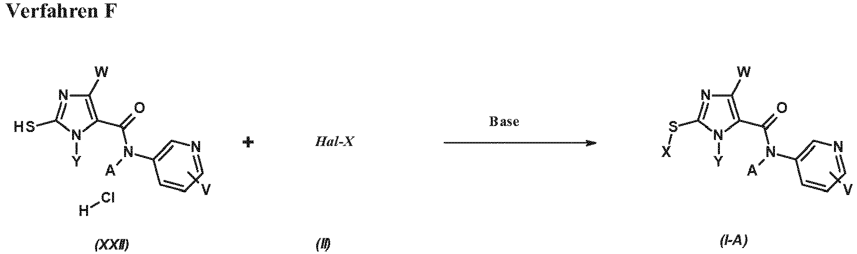

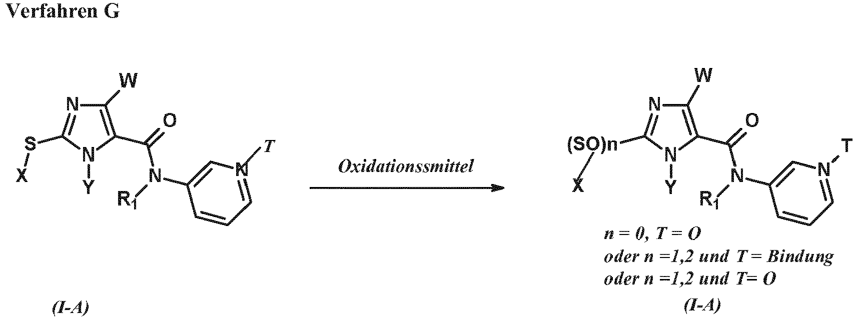

Die für das Verfahren A benötigten Halogenide (z. B. Alkylhalogenide, Hetarylhalogenide, etc.) der Formel (II) sind größtenteils Kaufprodukte oder lassen sich nach allgemein in der organischen Chemie bekannten Verfahren herstellen.

Die für das Verfahren A benötigten 2-Mercapto-imidazolyl-carbonsäureester der Formel (III) sind käuflich oder können beispielweise nach literaturbekannten Verfahren hergestellt werden, z. B., H. Rapoport et.al.; Synthesis 1988, 10, 767-771.

Die für das Verfahren B benötigten Thiole der Formel (VI) (z. B. Alkylmercaptane, Thiophenole, Mercaptopyrimidine, Mercaptopyridine etc.) sind größtenteils kommerziell erhältlich oder lassen sich nach allgemein in der organischen Chemie bekannten Verfahren herstellen.

Die für die Verfahren B und C benötigten 2-Brom-imidazolyl-carbonsäureester der Formel (VII) sind kommerziell erhältlich oder können beispielweise nach literaturbekannten Verfahren hergestellt werden, z. B., H. Rapoport et.al.; Synthesis 1988, 10, 767-771.

Die für das Verfahren C-1 und D benötigten Dithioether der Formel (XII) (z. B. Pyrimidyldithioether, Pyridyldithioehter, etc.) sind größtenteils kommerziell erhältlich oder lassen sich nach allgemein in der organischen Chemie bekannten Verfahren herstellen, z.B. Zeynizadeh, Belizad, Journal of Chemical Research - Part S, 2002, 564 - 566, Rütgerswerke Aktiengesellschaft Patent: US4256892 A1, 1981, Kesavan, Venkitasamy; Bonnet-Delpon, Daniele; Begue, Jean-Pierre Synthesis 2000, 2, 223 - 225.

Die für das Verfahren D benötigten Imidazolyl-carbonsäuren der Formel (XIII) sind kommerziell erhältlich oder können beispielweise nach literaturbekannten Verfahren hergestellt werden, z. B., H. Rapoport et.al.; Synthesis 1988, 10, 767-771, BASF Aktiengesellschaft Patent: US4864030 A1, 1989, Takeda Pharmaceutical Company Limited Patent: EP2530078 A1, 2012, TAISHO PHARMACEUTICAL CO., LTD. Patent: US2012/10414 A1, 2012, Subrayan, Ramachandran P.; Thurber, Ernest L.; Rasmussen, Paul G. Tetrahedron, 1994, 50, 2641 - 2656.

Die für das Verfahren E benötigten Imidazolyl-carbonsäureester der Formel (XVI) sind kommerziell erhältlich oder können beispielweise nach literaturbekannten Verfahren hergestellt werden, z. B., Nunami; Yamada; Fukui; Matsumoto, Journal of Organic Chemistry, 1994, vol. 59, 7635 - 7642, H. Rapoport et.al.; Synthesis 1988, 10, 767-771

Die für das Verfahren A bis E benötigten 3-Amino-pyridine der Formel (V) sind kommerziell erhältlich oder können beispielweise nach literaturbekannten Verfahren hergestellt werden, z. B., Liu, Zhen-Jiang; Vors, Jean-Pierre; Gesing, Ernst R. F.; Bolm, Carsten, Advanced Synthesis and Catalysis, 2010 , 352, 3158 - 3162, BAYER CROPSCIENCE AG Patent: US2010/305124 A1, 2010, Shafir, Alexandr; Buchwald, Stephen L., Journal of the American Chemical Society, 2006, 128, 8742 - 8743.

Wie aus den Verfahrensschemata A bis E ersichtlich sind die Verbindungen der Formel (I-A) und (I-B) prinzipiell durch ein Amidierungsverfahren oder einem Kupplungsverfahren auf der letzten Stufe zugänglich.

### Amidierungsverfahren

Die Verbindungen der Formel (I-A) und die Intermediate der Formel (XI), (XV) und (XXI) in den erfindungsgemäßen Verfahren A bis E lassen sich mit Hilfe von literaturbekannten Amidierungsreaktion oder analog den explizit genannten Beispielen synthetisieren.

Für den Amidierungsschritt sind zahlreiche Reaktionsbedingungen beschrieben worden, z.B. G. Benz in Comprehensive Organic Synthesis, 1st Ed., Pergamon Press, Oxford, 1991, Vol. 6, S. 381-417; P.D. Bailey et al. in Comprehensive Organic Functional Group Transformation, 1st Ed., Elsevier Science Ltd., Oxford, 1995, Vol. 5, S. 257-308 und R.C. Larock in Comprehensive Organic Transformations, 2nd Ed., Wiley-VCH, New York, Weinheim, 1999, S. 1929-1994. Einige dieser Reaktionen verlaufen über intermediäre Carbonsäurechloride, die isoliert oder in situ erzeugt eingesetzt werden können.

Die Amidierungsreaktionen erfolgen gegebenenfalls in Gegenwart eines Kondensationsmittels, gegebenenfalls in Gegenwart eines Säureakzeptors und gegebenenfalls in Gegenwart eines Lösungsmittels.

Als Kondensationsmittel kommen alle üblicherweise für derartige Amidierungsreaktionen verwendbaren Kondensationsmittel infrage. Beispielhaft genannt seien Aktivierungsreagenzien wie Phosgen, Phosphortrichlorid, Phosphoroxychlorid, Oxalylchlorid, Oxalylbromid oder Thionylchlorid; Carbodiimide, wie *N,N'*-Dicyclohexylcarbodiimid (DCC) und 1-(3-Dimethylaminopropyl)-3-ethyl-carbodiimid (EDCI), oder andere übliche Kondensationsmittel, wie Phosphorpentoxid, Polyphosphorsäure, *N,N'*-Carbonyldiimidazol, 2-Chlorpyridin 1-Methoiodid (Mukaiyamas Reagenz), 2-Ethoxy-N-ethoxycarbonyl-1,2-dihydrochinolin (EEDQ), Triphenylphosphin/Tetrachlorkohlenstoff, Brom-tripyrrolidino-phosphonium-hexafluorphosphat (BROP), O-(*1H*-Benzotriazol-1 -yloxy)tris(dimethylamino)phosphonium-hexafluorphosphat (BOP), *N,N,N',N'*-Bis(tetramethylen)cloruronium-tetrafluorborat, O-(*1H*-Benzotriazol-1-yl)-*N,N,N',N'-*tetramethyluronium-hexafluorphosphat (HBTU), O-(*1H*-Benzotriazol-1-yl)-*N,N,N',N'-*bis(tetramethylen)uronium-hexafluorphosphat, O-(*1H*-Benzotriazol-1-yl)-*N,N,N',N'-*tetramethyluronium-tetrafluorborat (TBTU), O-(*1H*-Benzotriazol-1-yl)-*N,N,N',N'-*bis(tetramethylen)uronium-tetrafluorborat, O-(7-Azabenzotriazol-1-yl)-*N,N,N',N'-*tetramethyluronium-hexafluorphosphat (HATU), 1-Hydroxybenzotriazol (HOBt) und 4-(4,6-Dimethoxy-1,3,5-triazin-2-yl)-4-methylmorpholiniumsalz (DMT.MM), meistens als Chlorid verfügbar. Diese Reagenzien können separat oder in Kombination eingesetzt werden.

Als Säureakzeptor kommen alle üblichen anorganischen oder organischen Basen infrage, beispielsweise organische Amine wie Triethylamin, Diisopropylethylamin, *N*-Methylmorpholin, Pyridin oder *N,N-*Dimethylaminopyridin, Alkali- und Erdalkalicarbonate wie Lithiumcarbonat Natriumcarbonat, Kaliumcarbonat oder Caesiumcarbonat; Alkalyhydrogencarbonate wie Natrumhydrogencarbonat oder Kaliumhydrogencarbonat. Die Amidierungsreaktion in den erfindungsgemäßen Verfahren wird gegebenenfalls in Gegenwart eines geeigneten Reaktionshilfstoffes wie beispielsweise *N,N*-Dimethylfonnamid oder *N,N*-Dimethylaminopyridin durchgeführt. Als Lösungs- oder Verdünnungsmittel kommen alle interten organischen Lösungsmittel in Betracht, beispielsweise aliphatische oder aromatische Kohlenwasserstoffe (wie Petrolether, Toluol, Cyclohexan), halogenierte Kohlenwasserstoffe (wie Chlortoluol, Dichlorbenzol, Dichlormethan, Chloroform, 1,2-Dichlorethan), Ether (wie Diethylether, Dioxan, Tetrahydrofuran, 1,2-Dimethoxyethan), Ester (wie Essigsäureethylester- oder -methylester), Nitrokohlenwasserstoffe (wie Nitromethan, Nitroethan, Nitrobenzol), Nitrile (wie Acetonitril, Propionitril, Butyronitril. Benzonitril), Amide (wie *N,N*-Dimethylformamid, *N,N*-Dimethylacetamide, *N*-Methylformanilid, *N-*Methylpyrrolidon, Hexamethylphosphorsäuretriamid) sowie Dimethylsulfoxid, Sulfolan oder Wasser oder Gemische der genannten Lösungsmittel.

Es können auch gemischte Anhydride zur Darstellung von Verbindungen der Formel (III) verwendet werden (vgl. J. Am. Chem. Soc. 1967, 5012). Bei diesem Verfahren können Chlorameisensäureester zum Einsatz kommen, wie z.B. Chlorameisensäuremethylester, Chlorameisensäureethylester, Chlorameisensäureisobutylester und Chlorameisensäureisopropylester. Ebenfalls können dafür Diethylacetylchlorid, Trimethylacetylchlorid und ähnliche Verbindungen verwendet werden.

### Kupplungsverfahren Variante A

Die Verbindungen der Formel (I-A) und (I-B) und die Intermediate der Formel (VIII), in den erfindungsgemäßen Verfahren **B, C-2 und E** lassen sich mit Hilfe von literaturbekannten Kupplungssreaktionen oder analog den explizit genannten Beispielen synthetisieren.

Für das übergangsmetallkatalysierte Kupplungsverfahren Variante A sind zahlreiche Reaktionsbedingungen beschrieben worden, z. B., J. P. Dickens et al.; Journal of Organic Chemistry, 1981, 46, 1781 ff. Harr, Molly S.; Presley, Alice L.; Thorarensen, Atli; Synlett; nb. 10; (1999); p. 1579 - 158. Babu, S. Ganesh; Karvembu, Tetrahedron Letters, 2013 , vol. 54, # 13 p. 1677 - 1680.

Die Kupplungsreaktionen erfolgen gegebenenfalls in Gegenwart eines Übergangsmetalls, gegebenenfalls in Gegenwart eines Säureakzeptors und gegebenenfalls in Gegenwart eines Lösungsmittels.

Als Lösungsmittel seien beispielhaft genannt: N,N-dimethylformamid, N,N-dimethylacetamid, Tetrahydrofuran, Toluol.

Als Metallverbindungen seien beispielhaft genannt: Cu(I)oxid, Cu(II)oxid, Tetrakis(triphenylphosphin) palladium(0), Tris-(dibenzylideneaceton)dipalladium(0).

Als Basen seien beispielhaft genannt: Kaliumhydroxid, Kalium-tert.-butylat, Triethylamin, ebenso alle üblichen anorganischen oder organischen Basen, beispielsweise organische Amine wie, Diisopropylethylamin, *N*-Methylmorpholin, Pyridin oder *N,N*-Dimethylaminopyridin, Alkali- und Erdalkalicarbonate wie Lithiumcarbonat, Natriumcarbonat, Kaliumcarbonat oder Caesiumcarbonat; Alkaihydrogencarbonate wie Natrumhydrogencarbonat oder Kaliumhydrogencarbonat

### Kupplungsverfahren Variante B

Die Verbindungen der Formel (I-A) in den erfindungsgemäßen Verfahren **C-1 und D** lassen sich mit Hilfe von literaturbekannten Kupplungssreaktionen oder analog den explizit genannten Beispielen synthetisieren.

Für das Lithiierungs-Kupplungsverfahren Variante B sind zahlreiche Reaktionsbedingungen beschrieben worden, z. B., Hoechst Aktiengesellschaft, Patent: US4764624 A1, 1988. Ohta; Yamamoto; Kawasaki; Yamashita; Katsuma; Nasako; Kobayashi; Ogawa, Chemical and Pharmaceutical Bulletin, 1992 , vol. 40, # 10 p. 2681 - 2685. Hara, Kenji; Iwahashi, Keiji; Kanamori, Yoshikazu; Naito, Satoshi; Takakusagi, Satoru; Uosaki, Kohei; Sawamura, Masaya Chemistry Letters, 2006 , vol. 35, # 8 p. 870 - 871.

Als Lösungsmittel seien beispielhaft genannt: Dieethylether, Tetrahydrofuran.

Als Lithiierungsmittel sein beispielhaft genannt: n-Butyllithium, Lithium-di-isoproylamin, Lithium-tert.-butylat.

Die Reaktionstemperatur liegt gegebenenfalls zwischen -100 und -75°C für den initialen Lithiierungsschritt.

### Verfahren und Verwendungen

Die Erfindung betrifft auch Verfahren zur Bekämpfung von tierischen Schädlingen, bei dem man Verbindungen der Formel (I) auf tierische Schädlinge und/oder ihren Lebensraum einwirken lässt. Bevorzugt wird die Bekämpfung der tierischen Schädlinge in der Land- und Forstwirtschaft und im Materialschutz durchgeführt. Hierunter vorzugsweise ausgeschlossen sind Verfahren zur chirurgischen oder therapeutischen Behandlung des menschlichen oder tierischen Körpers und Diagnostizierverfahren, die am menschlichen oder tierischen Körper vorgenommen werden.

Die Erfindung betrifft ferner die Verwendung der Verbindungen der Formel (I) als Schädlingsbekämpfungsmittel, insbesondere Pflanzenschutzmittel.

Im Rahmen der vorliegenden Anmeldung umfasst der Begriff Schädlingsbekämpfungsmittel immer auch den Begriff Pflanzenschutzmittel.

Die Verbindungen der Formel (I) eignen sich bei guter Pflanzenverträglichkeit, günstiger Warmblütertoxizität und guter Umweltverträglichkeit zum Schutz von Pflanzen und Pflanzenorganen vor biotischen und abiotischen Stressfaktoren, zur Steigerung der Ernteerträge, Verbesserung der Qualität des Erntegutes und zur Bekämpfung von tierischen Schädlingen, insbesondere Insekten, Spinnentieren, Helminthen, Nematoden und Mollusken, die in der Landwirtschaft, im Gartenbau, bei der Tierzucht, in Aquakulturen, in Forsten, in Gärten und Freizeiteinrichtungen, im Vorrats- und Materialschutz sowie auf dem Hygienesektor vorkommen. Sie können vorzugsweise als Schädlingsbekämpfungsmittel eingesetzt werden. Sie sind gegen normal sensible und resistente Arten sowie gegen alle oder einzelne Entwicklungsstadien wirksam. Zu den oben erwähnten Schädlingen gehören:
Schädlinge aus dem Stamm der Arthropoda, insbesondere aus der Klasse der Arachnida z.B. Acarus spp., z.B. Acarus siro, Aceria kuko, Aceria sheldoni, Aculops spp., Aculus spp., z.B. Aculus fockeui, Aculus schlechtendali, Amblyomma spp., Amphitetranychus viennensis, Argas spp., Boophilus spp., Brevipalpus spp., z.B. Brevipalpus phoenicis, Bryobia graminum, Bryobia praetiosa, Centruroides spp., Chorioptes spp., Dermanyssus gallinae, Dermatophagoides pteronyssinus, Dermatophagoides farinae, Dermacentor spp., Eotetranychus spp., z.B. Eotetranychus hicoriae, Epitrimerus pyri, Eutetranychus spp., z.B. Eutetranychus banksi, Eriophyes spp., z.B. Eriophyes pyri, Glycyphagus domesticus, Halotydeus destructor, Hemitarsonemus spp., z.B. Hemitarsonemus latus (=Polyphagotarsonemus latus), Hyalomma spp., Ixodes spp., Latrodectus spp., Loxosceles spp., Neutrombicula autumnalis, Nuphersa spp., Oligonychus spp., z.B. Oligonychus coniferarum, Oligonychus ilicis, Oligonychus indicus, Oligonychus mangiferus, Oligonychus pratensis, Oligonychus punicae, Oligonychus yothersi, Ornithodorus spp., Ornithonyssus spp., Panonychus spp., z.B. Panonychus citri (=Metatetranychus citri), Panonychus ulmi (=Metatetranychus ulmi), Phyllocoptruta oleivora, Platytetranychus multidigituli, Polyphagotarsonemus latus, Psoroptes spp., Rhipicephalus spp., Rhizoglyphus spp., Sarcoptes spp., Scorpio maurus, Steneotarsonemus spp., Steneotarsonemus spinki, Tarsonemus spp., z.B. Tarsonemus confusus, Tarsonemus pallidus, Tetranychus spp., z.B. Tetranychus canadensis, Tetranychus cinnabarinus, Tetranychus turkestani, Tetranychus urticae, Trombicula alfreddugesi, Vaejovis spp., Vasates lycopersici;
aus der Klasse der Chilopoda z.B. Geophilus spp., Scutigera spp.;
aus der Ordnung oder der Klasse der Collembola z.B. Onychiurus armatus; Sminthurus viridis;
aus der Klasse der Diplopoda z.B. Blaniulus guttulatus;
aus der Klasse der Insecta, z.B. aus der Ordnung der Blattodea z.B. Blatta orientalis, Blattella asahinai, Blattella germanica, Leucophaea maderae, Loboptera decipiens, Neostylopyga rhombifolia, Panchlora spp., Parcoblatta spp., Periplaneta spp., z.B. Periplaneta americana, Periplaneta australasiae, Pycnoscelus surinamensis, Supella longipalpa;
aus der Ordnung der Coleoptera z.B. Acalymma vittatum, Acanthoscelides obtectus, Adoretus spp., Aethina tumida, Agelastica alni, Agriotes spp., z.B. Agriotes linneatus, Agriotes mancus, Alphitobius diaperinus, Amphimallon solstitialis, Anobium punctatum, Anoplophora spp., Anthonomus spp., z.B. Anthonomus grandis, Anthrenus spp., Apion spp., Apogonia spp., Atomaria spp., z.B. Atomaria linearis, Attagenus spp., Baris caerulescens, Bruchidius obtectus, Bruchus spp., z.B. Bruchus pisorum, Bruchus rufimanus, Cassida spp., Cerotoma trifurcata, Ceutorrhynchus spp., z.B. Ceutorrhynchus assimilis, Ceutorrhynchus quadridens, Ceutorrhynchus rapae, Chaetocnema spp., z.B. Chaetocnema confinis, Chaetocnema denticulata, Chaetocnema ectypa, Cleonus mendicus, Conoderus spp., Cosmopolites spp., z.B. Cosmopolites sordidus, Costelytra zealandica, Ctenicera spp., Curculio spp., z.B. Curculio caryae, Curculio caryatrypes,Curculio obtusus, Curculio sayi, Cryptolestes ferrugineus, Cryptolestes pusillus, Cryptorhynchus lapathi, Cryptorhynchus mangiferae, Cylindrocopturus spp., Cylindrocopturus adspersus, Cylindrocopturus furnissi, Dermestes spp., Diabrotica spp., z.B. Diabrotica balteata, Diabrotica barberi, Diabrotica undecimpunctata howardi, Diabrotica undecimpunctata undecimpunctata, Diabrotica virgifera virgifera, Diabrotica virgifera zeae, Dichocrocis spp., Dicladispa armigera, Diloboderus spp., Epicaerus spp., Epilachna spp., z.B. Epilachna borealis, Epilachna varivestis, Epitrix spp., z.B. Epitrix cucumeris, Epitrix fuscula, Epitrix hirtipennis, Epitrix subcrinita, Epitrix tuberis, Faustinus spp., Gibbium psylloides, Gnathocerus cornutus, Hellula undalis, Heteronychus arator, Heteronyx spp., Hylamorpha elegans, Hylotrupes bajulus, Hypera postica, Hypomeces squamosus, Hypothenemus spp., z.B. Hypothenemus hampei, Hypothenemus obscurus, Hypothenemus pubescens, Lachnosterna consanguinea, Lasioderma serricorne, Latheticus oryzae, Lathridius spp., Lema spp., Leptinotarsa decemlineata, Leucoptera spp., z.B. Leucoptera coffeella, Lissorhoptrus oryzophilus, Listronotus (= Hyperodes) spp., Lixus spp., Luperomorpha xanthodera, Luperodes spp., Lyctus spp., Megascelis spp., Melanotus spp., z.B. Melanotus longulus oregonensis, Meligethes aeneus, Melolontha spp., z.B. Melolontha melolontha, Migdolus spp., Monochamus spp., Naupactus xanthographus, Necrobia spp., Neogalerucella spp., Niptus hololeucus, Oryctes rhinoceros, Oryzaephilus surnamensis, Oryzaphagus oryzae, Otiorhynchus spp., z.B. Otiorhynchus cribricollis, Otiorhynchus ligustici, Otiorhynchus ovatus, Otiorhynchus rugosostriarus, Otiorhynchus sulcatus, Oulema spp., Oulema oryzae, Oxycetonia jucunda, Phaedon cochleariae, Phyllophaga spp., Phyllophaga helleri, Phyllotreta spp., z.B. Phyllotreta armoraciae, Phyllotreta pusilla, Phyllotreta ramosa, Phyllotreta striolata, Popillia japonica, Premnotrypes spp., Prostephanus truncatus, Psylliodes spp., z.B. Psylliodes affinis, Psylliodes chrysocephala, Psylliodes punctulata, Ptinus spp., Rhizobius ventralis, Rhizopertha dominica, Rhynchophorus spp., Rhynchophorus ferrugineus, Rhynchophorus palmarum, Sinoxylon perforans, Sitophilus spp., z.B. Sitophilus granarius, Sitophilus linearis, Sitophilus oryzae, Sitophilus zeamais, Sphenophorus spp., Stegobium paniceum, Sternechus spp., z.B. Sternechus paludatus, Symphyletes spp., Tanymecus spp., z.B. Tanymecus dilaticollis, Tanymecus indicus, Tanymecus palliatus, Tenebrio molitor, Tenebrioides mauretanicus, Tribolium spp., z.B. Tribolium audax, Tribolium castaneum, Tribolium confusum, Trogoderma spp., Tychius spp., Xylotrechus spp., Zabrus spp., z.B. Zabrus tenebrioides;
aus der Ordnung der Dermaptera z.B. Anisolabis maritime, Forficula auricularia, Labidura riparia;
aus der Ordnung der Diptera z.B. Aedes spp., z.B. Aedes aegypti, Aedes albopictus, Aedes sticticus, Aedes vexans, Agromyza spp., z.B. Agromyza frontella, Agromyza parvicornis, Anastrepha spp., Anopheles spp., z.B. Anopheles quadrimaculatus, Anopheles gambiae, Asphondylia spp., Bactrocera spp., z.B. Bactrocera cucurbitae, Bactrocera dorsalis, Bactrocera oleae, Bibio hortulanus, Calliphora erythrocephala, Calliphora vicina, Ceratitis capitata, Chironomus spp., Chrysomya spp., Chrysops spp., Chrysozona pluvialis, Cochliomya spp., Contarinia spp., z.B. Contarinia johnsoni, Contarinia nasturtii, Contarinia pyrivora, Contarinia schulzi, Contarinia sorghicola, Contarinia tritici,Cordylobia anthropophaga, Cricotopus sylvestris, Culex spp., z.B. Culex pipiens, Culex quinquefasciatus, Culicoides spp., Culiseta spp., Cuterebra spp., Dacus oleae, Dasineura spp., z.B. Dasineura brassicae, Delia spp., z.B. Delia antiqua, Delia coarctata, Delia florilega, Delia platura, Delia radicum, Dermatobia hominis, Drosophila spp., z.B. Drosphila melanogaster, Drosophila suzukii, Echinocnemus spp., Euleia heraclei, Fannia spp., Gasterophilus spp., Glossina spp., Haematopota spp., Hydrellia spp., Hydrellia griseola, Hylemya spp., Hippobosca spp., Hypoderma spp., Liriomyza spp., z.B. Liriomyza brassicae, Liriomyza huidobrensis, Liriomyza sativae, Lucilia spp., z.B. Lucilia cuprina, Lutzomyia spp., Mansonia spp., Musca spp., z.B. Musca domestica, Musca domestica vicina, Oestrus spp., Oscinella frit, Paratanytarsus spp., Paralauterborniella subcincta, Pegomya spp., z.B. Pegomya betae, Pegomya hyoscyami, Pegomya rubivora, Phlebotomus spp., Phorbia spp., Phormia spp., Piophila casei, Platyparea poeciloptera, Prodiplosis spp., Psila rosae, Rhagoletis spp., z.B. Rhagoletis cingulata, Rhagoletis completa, Rhagoletis fausta, Rhagoletis indifferens, Rhagoletis mendax, Rhagoletis pomonella, Sarcophaga spp., Simulium spp., z.B. Simulium meridionale, Stomoxys spp., Tabanus spp., Tetanops spp., Tipula spp., z.B. Tipula paludosa, Tipula simplex, Toxotrypana curvicauda;
aus der Ordnung der Hemiptera z.B. Acizzia acaciaebaileyanae, Acizzia dodonaeae, Acizzia uncatoides, Acrida turrita, Acyrthosipon spp., z.B. Acyrthosiphon pisum, Acrogonia spp., Aeneolamia spp., Agonoscena spp., Aleurocanthus spp., Aleyrodes proletella, Aleurolobus barodensis, Aleurothrixus floccosus, Allocaridara malayensis, Amrasca spp., z.B. Amrasca bigutulla, Amrasca devastans, Anuraphis cardui, Aonidiella spp., z.B. Aonidiella aurantii, Aonidiella citrina, Aonidiella inornata, Aphanostigma piri, Aphis spp., z.B. Aphis citricola, Aphis craccivora, Aphis fabae, Aphis forbesi, Aphis glycines, Aphis gossypii, Aphis hederae, Aphis illinoisensis, Aphis middletoni, Aphis nasturtii, Aphis nerii, Aphis pomi, Aphis spiraecola, Aphis viburniphila, Arboridia apicalis, Arytainilla spp., Aspidiella spp., Aspidiotus spp., z.B. Aspidiotus nerii, Atanus spp., Aulacorthum solani, Bemisia tabaci, Blastopsylla occidentalis, Boreioglycaspis melaleucae, Brachycaudus helichrysi, Brachycolus spp., Brevicoryne brassicae, Cacopsylla spp., z.B. Cacopsylla pyricola, Calligypona marginata, Capulinia spp., Carneocephala fulgida, Ceratovacuna lanigera, Cercopidae, Ceroplastes spp., Chaetosiphon fragaefolii, Chionaspis tegalensis, Chlorita onukii, Chondracris rosea, Chromaphis juglandicola, Chrysomphalus aonidum, Chrysomphalus ficus, Cicadulina mbila, Coccomytilus halli, Coccus spp., z.B. Coccus hesperidum, Coccus longulus, Coccus pseudomagnoliarum, Coccus viridis, Cryptomyzus ribis, Cryptoneossa spp., Ctenarytaina spp., Dalbulus spp., Dialeurodes chittendeni, Dialeurodes citri, Diaphorina citri, Diaspis spp., Diuraphis spp., Drosicha spp., Dysaphis spp., z.B. Dysaphis apiifolia, Dysaphis plantaginea, Dysaphis tulipae, Dysmicoccus spp., Empoasca spp., z.B. Empoasca abrupta, Empoasca fabae, Empoasca maligna, Empoasca solana, Empoasca stevensi, Eriosoma spp., z.B. Eriosoma americanum, Eriosoma lanigerum, Eriosoma pyricola, Erythroneura spp., Eucalyptolyma spp., Euphyllura spp., Euscelis bilobatus, Ferrisia spp., Fiorinia spp., Furcaspis oceanica, Geococcus coffeae, Glycaspis spp., Heteropsylla cubana, Heteropsylla spinulosa, Homalodisca coagulata, Hyalopterus arundinis, Hyalopterus pruni, Icerya spp., z.B. Icerya purchasi, Idiocerus spp., Idioscopus spp., Laodelphax striatellus, Lecanium spp., z.B. Lecanium corni (=Parthenolecanium corni), Lepidosaphes spp., z.B. Lepidosaphes ulmi, Lipaphis erysimi, Lopholeucaspis japonica, Lycorma delicatula, Macrosiphum spp., z.B. Macrosiphum euphorbiae, Macrosiphum lilii, Macrosiphum rosae, Macrosteles facifrons, Mahanarva spp., Melanaphis sacchari, Metcalfiella spp., Metcalfa pruinosa, Metopolophium dirhodum, Monellia costalis, Monelliopsis pecanis, Myzus spp., z.B. Myzus ascalonicus, Myzus cerasi, Myzus ligustri, Myzus ornatus, Myzus persicae,. Myzus nicotianae, Nasonovia ribisnigri, Neomaskellia spp., Nephotettix spp., z.B. Nephotettix cincticeps,, Nephotettix nigropictus, Nettigoniclla spectra, Nilaparvata lugens, Oncometopia spp., Orthezia praelonga, Oxya chinensis, Pachypsylla spp., Parabemisia myricae, Paratrioza spp., z.B. Paratrioza cockerelli, Parlatoria spp., Pemphigus spp., z.B. Pemphigus bursarius, Pemphigus populivenae, Peregrinus maidis, Perkinsiella spp., Phenacoccus spp., z.B. Phenacoccus madeirensis, Phloeomyzus passerinii, Phorodon humuli, Phylloxera spp., z.B. Phylloxera devastatrix, Phylloxera notabilis, Pinnaspis aspidistrae, Planococcus spp., z.B. Planococcus citri, Prosopidopsylla flava, Protopulvinaria pyriformis, Pseudaulacaspis pentagona, Pseudococcus spp., z.B. Pseudococcus calceolariae, Pseudococcus comstocki, Pseudococcus longispinus, Pseudococcus maritimus, Pseudococcus viburni, Psyllopsis spp., Psylla spp., z.B. Psylla buxi, Psylla mali, Psylla pyri, Pteromalus spp., Pulvinaria spp., Pyrilla spp., Quadraspidiotus spp., z.B. Quadraspidiotus juglansregiae, Quadraspidiotus ostreaeformis, Quadraspidiotus perniciosus, Quesada gigas, Rastrococcus spp., Rhopalosiphum spp., z.B. Rhopalosiphum maidis, Rhopalosiphum oxyacanthae, Rhopalosiphum padi, Rhopalosiphum rufiabdominale, Saissetia spp., z.B. Saissetia coffeae, Saissetia miranda, Saissetia neglecta, Saissetia oleae, Scaphoideus titanus, Schizaphis graminum, Selenaspidus articulatus, Sitobion avenae, Sogata spp., Sogatella furcifera, Sogatodes spp., Stictocephala festina, Siphoninus phillyreae, Tenalaphara malayensis,Tetragonocephela spp., Tinocallis caryaefoliae, Tomaspis spp., Toxoptera spp., z.B. Toxoptera aurantii, Toxoptera citricidus, Trialeurodes vaporariorum, Trioza spp., z.B. Trioza diospyri, Typhlocyba spp., Unaspis spp., Viteus vitifolii, Zygina spp.;
aus der Unterordnung der Heteroptera z.B. Aelia spp., Anasa tristis, Antestiopsis spp., Boisea spp., Blissus spp., Calocoris spp., Campylomma livida, Cavelerius spp., Cimex spp., z.B. Cimex adjunctus, Cimex hemipterus, Cimex lectularius, Cimex pilosellus, Collaria spp., Creontiades dilutus, Dasynus piperis, Dichelops furcatus, Diconocoris hewetti, Dysdercus spp., Euschistus spp., z.B. Euschistus heros, Euschistus servus, Euschistus tristigmus, Euschistus variolarius, Eurydema spp., Eurygaster spp., Halyomorpha halys, Heliopeltis spp., Horcias nobilellus, Leptocorisa spp., Leptocorisa varicornis, Leptoglossus occidentalis, Leptoglossus phyllopus, Lygocoris spp., z.B. Lygocoris pabulinus, Lygus spp., z.B. Lygus elisus, Lygus hesperus, Lygus lineolaris, Macropes excavatus, Megacopta cribraria, Miridae, Monalonion atratum, Nezara spp., z.B. Nezara viridula, Nysius spp., Oebalus spp., Pentomidae, Piesma quadrata, Piezodorus spp., z.B. Piezodorus guildinii, Psallus spp., Pseudacysta persea, Rhodnius spp., Sahlbergella singularis, Scaptocoris castanea, Scotinophora spp., Stephanitis nashi, Tibraca spp., Triatoma spp.;
aus der Ordnung der Hymenoptera z.B. Acromyrmex spp., Athalia spp., z.B. Athalia rosae, Atta spp., Camponotus spp., Dolichovespula spp., Diprion spp., z.B. Diprion similis, Hoplocampa spp., z.B. Hoplocampa cookei, Hoplocampa testudinea, Lasius spp., Linepithema humile, Monomorium pharaonis, Paratrechina spp., Paravespula spp., Plagiolepis spp., Sirex spp., Solenopsis invicta, Tapinoma spp., Technomyrmex albipes, Urocerus spp., Vespa spp., z.B. Vespa crabro, Wasmannia auropunctata, Xeris spp.;
aus der Ordnung der Isopoda z.B. Armadillidium vulgare, Oniscus asellus, Porcellio scaber;
aus der Ordnung der Isoptera z.B. Coptotermes spp., z.B. Coptotermes formosanus, Cornitermes cumulans, Cryptotermes spp., Incisitermes spp., Kalotermes spp., Microtermes obesi, Nasutitermes spp., Odontotermes spp., Porotermes spp., Reticulitermes spp., z.B. Reticulitermes flavipes, Reticulitermes hesperus;
aus der Ordnung der Lepidoptera z.B. Achroia grisella, Acronicta major, Adoxophyes spp., z.B. Adoxophyes orana, Aedia leucomelas, Agrotis spp., z.B. Agrotis segetum, Agrotis ipsilon, Alabama spp., z.B. Alabama argillacea, Amyelois transitella, Anarsia spp., Anticarsia spp., z.B. Anticarsia gemmatalis, Argyroploce spp., Autographa spp., Barathra brassicae, Blastodacna atra, Borbo cinnara, Bucculatrix thurberiella, Bupalus piniarius, Busseola spp., Cacoecia spp., Caloptilia theivora, Capua reticulana, Carpocapsa pomonella, Carposina niponensis, Cheimatobia brumata, Chilo spp., z.B. Chilo plejadellus, Chilo suppressalis, Choreutis pariana, Choristoneura spp., Chrysodeixis chalcites, Clysia ambiguella, Cnaphalocerus spp., Cnaphalocrocis medinalis, Cnephasia spp., Conopomorpha spp., Conotrachelus spp., Copitarsia spp., Cydia spp., z.B. Cydia nigricana, Cydia pomonella, Dalaca noctuides, Diaphania spp., Diparopsis spp., Diatraea saccharalis, Earias spp., Ecdytolopha aurantium, Elasmopalpus lignosellus, Eldana saccharina, Ephestia spp., z.B. Ephestia elutella, Ephestia kuehniella, Epinotia spp., Epiphyas postvittana, Erannis spp., Erschoviella musculana, Etiella spp., Eudocima spp., Eulia spp., Eupoecilia ambiguella, Euproctis spp., z.B. Euproctis chrysorrhoea, Euxoa spp., Feltia spp., Galleria mellonella, Gracillaria spp., Grapholitha spp., z.B. Grapholita molesta, Grapholita prunivora, Hedylepta spp., Helicoverpa spp., z.B. Helicoverpa armigera, Helicoverpa zea, Heliothis spp., z.B. Heliothis virescens, Hofmannophila pseudospretella, Homoeosoma spp., Homona spp., Hyponomeuta padella, Kakivoria flavofasciata, Lampides spp., Laphygma spp., Laspeyresia molesta, Leucinodes orbonalis, Leucoptera spp., z.B. Leucoptera coffeella, Lithocolletis spp., z.B. Lithocolletis blancardella, Lithophane antennata, Lobesia spp., z.B. Lobesia botrana, Loxagrotis albicosta, Lymantria spp., z.b. Lymantria dispar, Lyonetia spp., z.B. Lyonetia clerkella, Malacosoma neustria, Maruca testulalis, Mamestra brassicae, Melanitis leda, Mocis spp., Monopis obviella, Mythimna separata, Nemapogon cloacellus, Nymphula spp., Oiketicus spp., Omphisa spp., Operophtera spp., Oria spp., Orthaga spp., Ostrinia spp., z.B. Ostrinia nubilalis, Oulema melanopus, Oulema oryzae, Panolis flammea, Parnara spp., Pectinophora spp., z.B. Pectinophora gossypiella, Perileucoptera spp., Phthorimaea spp., z.B. Phthorimaea operculella, Phyllocnistis citrella, Phyllonorycter spp., z.B. Phyllonorycter blancardella, Phyllonorycter crataegella, Pieris spp., z.B. Pieris rapae, Platynota stultana, Plodia interpunctella, Plusia spp., Plutella xylostella (=Plutella maculipennis), Prays spp., Prodenia spp., Protoparce spp., Pseudaletia spp., z.B. Pseudaletia unipuncta, Pseudoplusia includens, Pyrausta nubilalis, Rachiplusia nu, Schoenobius spp., z.B. Schoenobius bipunctifer, Scirpophaga spp., z.B. Scirpophaga innotata, Scotia segetum, Sesamia spp., z.B. Sesamia inferens, Sparganothis spp., Spodoptera spp., z.b. Spodoptera eradiana, Spodoptera exigua, Spodoptera frugiperda, Spodoptera praefica, Stathmopoda spp., Stenoma spp., Stomopteryx subsecivella, Synanthedon spp., Tecia solanivora, Thaumetopoea spp., Thermesia gemmatalis, Tinea cloacella, Tinea pellionella, Tineola bisselliella, Tortrix spp., Trichophaga tapetzella, Trichoplusia spp., z.B. Trichoplusia ni, Tryporyza incertulas, Tuta absoluta, Virachola spp.;
aus der Ordnung der Orthoptera oder Saltatoria z.B. Acheta domesticus, Dichroplus spp., Gryllotalpa spp., z.B. Gryllotalpa gryllotalpa, Hieroglyphus spp., Locusta spp., z.B. Locusta migratoria, Melanoplus spp., z.B. Melanoplus devastator, Paratlanticus ussuriensis, Schistocerca gregaria;
aus der Ordnung der Phthiraptera z.B. Damalinia spp., Haematopinus spp., Linognathus spp., Pediculus spp., Phylloxera vastatrix, Phthirus pubis, Trichodectes spp.;
aus der Ordnung der Psocoptera z.B. Lepinotus spp., Liposcelis spp.;
aus der Ordnung der Siphonaptera z.B. Ceratophyllus spp., Ctenocephalides spp., z.B. Ctenocephalides canis, Ctenocephalides felis, Pulex irritans, Tunga penetrans, Xenopsylla cheopis;
aus der Ordnung der Thysanoptera z.B. Anaphothrips obscurus, Baliothrips biformis, Chaetanaphothrips leeuweni, Drepanothrips reuteri, Enneothrips flavens, Frankliniella spp., z.B. Frankliniella fusca, Frankliniella occidentalis, Frankliniella schultzei, Frankliniella tritici, Frankliniella vaccinii, Frankliniella williamsi, Haplothrips spp., Heliothrips spp., Hercinothrips femoralis, Rhipiphorothrips cruentatus, Scirtothrips spp., Taeniothrips cardamomi, Thrips spp., z.B. Thrips palmi, Thrips tabaci;
aus der Ordnung der Zygentoma (= Thysanura), z. B. Ctenolepisma spp., Lepisma saccharina, Lepismodes inquilinus, Thermobia domestica;
aus der Klasse der Symphyla z.B. Scutigerella spp., z.B. Scutigerella immaculata;
Schädlinge aus dem Stamm der Mollusca, insbesondere aus der Klasse der Bivalvia, z.B. Dreissena spp.;
sowie aus der Klasse der Gastropoda z.B. Arion spp., z.B. Arion ater rufus, Biomphalaria spp., Bulinus spp., Deroceras spp., z.B. Deroceras laeve, Galba spp., Lymnaea spp., Oncomelania spp., Pomacea spp., Succinea spp.;
Tier- und Humanparasiten aus den Stämmen der Platyhelminthes und Nematoda, z.B. Aelurostrongylus spp., Amidostomum spp., Ancylostoma spp, Angiostrongylus spp., Anisakis spp., Anoplocephala spp., Ascaris spp., Ascaridia spp., Baylisascaris spp., Brugia spp., Bunostomum spp., Capillaria spp., Chabertia spp., Clonorchis spp., Cooperia spp., Crenosoma spp., Cyathostoma spp., Dicrocoelium spp., Dictyocaulus spp., Diphyllobothrium spp., Dipylidium spp., Dirofilaria spp., Dracunculus spp., Echinococcus spp., Echinostoma spp., Enterobius spp., Eucoleus spp., Fasciola spp., Fascioloides spp., Fasciolopsis spp., Filaroides spp., Gongylonema spp., Gyrodactylus spp., Habronema spp., Haemonchus spp., Heligmosomoides spp., Heterakis spp., Hymenolepis spp., Hyostrongylus spp., Litomosoides spp., Loa spp., Metastrongylus spp., Metorchis spp., Mesocestoides spp., Moniezia spp., Muellerius spp., Necator spp., Nematodirus spp., Nippostrongylus spp., Oesophagostomum spp., Ollulanus spp., Onchocerca spp, Opisthorchis spp., Oslerus spp., Ostertagia spp., Oxyuris spp., Paracapillaria spp., Parafilaria spp., Paragonimus spp., Paramphistomum spp., Paranoplocephala spp., Parascaris spp., Passalurus spp., Protostrongylus spp., Schistosoma spp., Setaria spp., Spirocerca spp., Stephanofilaria spp., Stephanurus spp., Strongyloides spp., Strongylus spp., Syngamus spp., Taenia spp., Teladorsagia spp., Thelazia spp., Toxascaris spp., Toxocara spp., Trichinella spp., Trichobilharzia spp., Trichostrongylus spp., Trichuris spp., Uncinaria spp., Wuchereria spp.;
Pflanzenschädlinge aus dem Stamm der Nematoda, d.h. pflanzenparasitäre Nematoden, insbesondere Aglenchus spp., z.B. Aglenchus agricola, Anguina spp., z.B. Anguina tritici, Aphelenchoides spp., z.B. Aphelenchoides arachidis, Aphelenchoides fragariae, Belonolaimus spp., z.B. Belonolaimus gracilis, Belonolaimus longicaudatus, Belonolaimus nortoni, Bursaphelenchus spp., z.B. Bursaphelenchus cocophilus, Bursaphelenchus eremus, Bursaphelenchus xylophilus, Cacopaurus spp., z.B. Cacopaurus pestis, Criconemella spp., z.B. Criconemella curvata, Criconemella onoensis, Criconemella ornata, Criconemella rusium, Criconemella xenoplax (= Mesocriconema xenoplax), Criconemoides spp., z.B. Criconemoides ferniae, Criconemoides onoense, Criconemoides ornatum, Ditylenchus spp., z.B. Ditylenchus dipsaci, Dolichodorus spp., Globodera spp., z.B. Globodera pallida, Globodera rostochiensis, Helicotylenchus spp., z.B. Helicotylenchus dihystera, Hemicriconemoides spp., Hemicycliophora spp., Heterodera spp., z.B. Heterodera avenae, Heterodera glycines, Heterodera schachtii, Hoplolaimus spp., Longidorus spp., z.B. Longidorus africanus, Meloidogyne spp., z.B. Meloidogyne chitwoodi, Meloidogyne fallax, Meloidogyne hapla, Meloidogyne incognita, Meloinema spp., Nacobbus spp., Neotylenchus spp., Paralongidorus spp., Paraphelenchus spp., Paratrichodorus spp., z.B. Paratrichodorus minor, Pratylenchus spp., z.B. Pratylenchus penetrans, Pseudohalenchus spp., Psilenchus spp., Punctodera spp., Quinisulcius spp., Radopholus spp., z.B. Radopholus citrophilus, Radopholus similis, Rotylenchulus spp., Rotylenchus spp., Scutellonema spp., Subanguina spp., Trichodorus spp., z.B. Trichodorus obtusus, Trichodorus primitivus, Tylenchulus spp., Tylenchorhynchus spp., z.B. Tylenchorhynchus annulatus, Tylenchulus spp., z.B. Tylenchulus semipenetrans, Xiphinema spp., z.B. Xiphinema index.

Weiterhin lässt sich aus dem Unterreich der Protozoa die Ordnung der Coccidia, z.B. Eimeria spp., bekämpfen.

### Nematoden

Der Begriff "Nematoden" umfasst im vorliegenden Zusammenhang alle Arten des Stammes Nematoda und hierbei insbesondere Arten, die Pflanzen oder Pilze (zum Beispiel Arten der Ordnung Aphelenchida, Meloidogyne, Tylenchida und andere) oder auch Menschen und Tiere (zum Beispiel Arten der Ordnungen Trichinellida, Tylenchida, Rhabditida und Spirurida) parasitieren oder in bzw. an diesen Lebewesen Schädigungen verursachen, sowie andere parasitäre Helminthen.

Ein Nematizid im Pflanzenschutz, wie hier beschrieben, besitzt die Fähigkeit, Nematoden zu kontro llieren.

Der Begriff "Nematoden kontrollieren" bedeutet das Abtöten der Nematoden oder das Verhindern oder Erschweren ihrer Entwicklung bzw. ihres Wachstums oder das Verhindern oder Erschweren ihres Eindringens in oder ihres Saugens am pflanzlichen Gewebe.

Dabei wird die Wirksamkeit der Verbindungen durch einen Vergleich von Mortalitäten, Gallenbildung, Zystenbildung, Nematodendichte pro Bodenvolumen, Nematodendichte pro Wurzel, Anzahl von Nematodeneier pro Bodenvolumen, Mobilität (Beweglichkeit) der Nematoden zwischen einer mit der Verbindung der Formel (I) behandelten Pflanze, Pflanzenteil oder dem behandelten Boden und einer unbehandelten Pflanze, Pflanzenteil oder unbehandelten Boden (100 %) ermittelt. Vorzugsweise wird eine Verringerung um 25-50 % im Vergleich mit einer unbehandelten Pflanze, Pflanzenteil oder unbehandelten Boden, besonders bevorzugt eine Verringerung um 51 - 79 % und ganz besonders bevorzugt das vollständige Abtöten oder die vollständige Verhinderung von Entwicklung und Wachstum der Nematoden durch eine Verringerung um 80 bis 100 % erreicht. Die Kontrolle von Nematoden, wie hier beschreiben, beinhaltet ebenso die Kontrolle der Nematoden-Vermehrung (Entwicklung von Zysten und/oder Eier). Verbindungen der Formel (I) können ebenso verwendet werden, um die Pflanzen oder Tiere gesund zu erhalten und können kurativ, präventiv oder systemisch zur Nematoden-Kontrolle eingesetzt werden.

Dem Fachmann sind Methoden bekannt, wie Mortalitäten, Gallenbildung, Zystenbildung, Nematodendichte pro Bodenvolumen, Nematodendichte pro Wurzel, Anzahl von Nematodeneier pro Bodenvolumen, Mobilität (Beweglichkeit) der Nematoden bestimmt werden.

Die Verwendung einer Verbindung der Formel (I) kann die Pflanze gesund erhalten und beinhaltet ebenso eine Reduktion der von Nematoden hervorgerufenen Schäden sowie eine Erhöhung der Erntemenge.

Der Begriff "Nematoden" bezieht sich im vorliegenden Zusammenhang auf Pflanzennematoden, unter die man alle Nematoden zusammenfasst, die Pflanzen schädigen. Pflanzennematoden umfassen pflanzenparasitäre Nematoden und im Boden lebende Nematoden. Zu den pflanzenparasitären Nematoden zählen Ektoparasiten wie Xiphinema spp., Longidorus spp. und Trichodorus spp.; Halbparasiten wie Tylenchulus spp.; migratorische Endoparasiten wie Pratylenchus spp., Radopholus spp. und Scutellonema spp.; ortsgebundene Parasiten wie Heterodera spp., Globodera spp. und Meloidogyne spp., sowie Stängel- und Blattendoparasiten wie Ditylenchus spp., Aphelenchoides spp. und Hirschmaniella spp.. Besonders schädliche wurzelparasitäre Bodennematoden sind zum Beispiel zystenbildende Nematoden der Gattungen Heterodera oder Globodera, und/oder Wurzelgallennematoden der Gattung Meloidogyne. Schädliche Arten dieser Gattungen sind zum Beispiel Meloidogyne incognita, Heterodera glycines (Sojabohnenzystennematode), Globodera pallida und Globodera rostochiensis (Kartoffelzystennematode), wobei diese Arten wirksam mit dem im vorliegenden Text beschriebenen Verbindungen bekämpft werden. Die Verwendung der im vorliegenden Text beschriebenen Verbindungen ist jedoch keineswegs auf diese Gattungen oder Arten beschränkt, sondern erstreckt sich in gleicher Weise auch auf andere Nematoden.

Zu den Pflanzennematoden zählen z.B. Aglenchus agricola, Anguina tritici, Aphelenchoides arachidis, Aphelenchoides fragaria und die Stängel- und Blattendoparasiten Aphelenchoides spp., Belonolaimus gracilis, Belonolaimus longicaudatus, Belonolaimus nortoni, Bursaphelenchus cocophilus, Bursaphelenchus eremus, Bursaphelenchus xylophilus und Bursaphelenchus spp., Cacopaurus pestis, Criconemella curvata, Criconemella onoensis, Criconemella ornata, Criconemella rusium, Criconemella xenoplax (= Mesocriconema xenoplax) und Criconemella spp.,

Criconemoides ferniae, Criconemoides onoense, Criconemoides ornatum und Criconemoides spp., Ditylenchus destructor, Ditylenchus dipsaci, Ditylenchus myceliophagus sowie die Stängel- und Blattendoparasiten Ditylenchus spp., Dolichodorus heterocephalus, Globodera pallida (=Heterodera pallida), Globodera rostochiensis (Kartoffelzystennematode), Globodera solanacearum, Globodera tabacum, Globodera virginia und die ortsgebundenen zystenbildenden Parasiten Globodera spp., Helicotylenchus digonicus, Helicotylenchus dihystera, Helicotylenchus erythrine, Helicotylenchus multicinctus, Helicotylenchus nannus, Helicotylenchus pseudorobustus und Helicotylenchus spp., Hemicriconemoides, Hemicycliophora arenaria, Hemicycliophora nudata, Hemicycliophora parvana, Heterodera avenae, Heterodera cruciferae, Heterodera glycines (Sojabohnenzystennematode), Heterodera oryzae, Heterodera schachtii, Heterodera zeae und die ortsgebundenen zystenbildenden Parasiten Heterodera spp., Hirschmaniella gracilis, Hirschmaniella oryzae, Hirschmaniella spinicaudata und die Stängel- und Blattendoparasiten Hirschmaniella spp., Hoplolaimus aegyptii, Hoplolaimus californicus, Hoplolaimus columbus, Hoplolaimus galeatus, Hoplolaimus indicus, Hoplolaimus magnistylus, Hoplolaimus pararobustus, Longidorus africanus, Longidorus breviannulatus, Longidorus elongatus, Longidorus laevicapitatus, Longidorus vineacola und die Ektoparasiten Longidorus spp., Meloidogyne acronea, Meloidogyne africana, Meloidogyne arenaria, Meloidogyne arenaria thamesi, Meloidogyne artiella, Meloidogyne chitwoodi, Meloidogyne coffeicola, Meloidogyne ethiopica, Meloidogyne exigua, Meloidogyne fallax, Meloidogyne graminicola, Meloidogyne graminis, Meloidogyne hapla, Meloidogyne incognita, Meloidogyne incognita acrita, Meloidogyne javanica, Meloidogyne kikuyensis, Meloidogyne minor, Meloidogyne naasi, Meloidogyne paranaensis, Meloidogyne thamesi und die ortsgebundenen Parasiten Meloidogyne spp., Meloinema spp., Nacobbus aberrans, Neotylenchus vigissi, Paraphelenchus pseudoparietinus, Paratrichodorus allius, Paratrichodorus lobatus, Paratrichodorus minor, Paratrichodorus nanus, Paratrichodorus porosus, Paratrichodorus teres und Paratrichodorus spp., Paratylenchus hamatus, Paratylenchus minutus, Paratylenchus projectus und Paratylenchus spp., Pratylenchus agilis, Pratylenchus alleni, Pratylenchus andinus, Pratylenchus brachyurus, Pratylenchus cerealis, Pratylenchus coffeae, Pratylenchus crenatus, Pratylenchus delattrei, Pratylenchus giibbicaudatus, Pratylenchus goodeyi, Pratylenchus hamatus, Pratylenchus hexincisus, Pratylenchus loosi, Pratylenchus neglectus, Pratylenchus penetrans,Pratylenchus pratensis, Pratylenchus scribneri, Pratylenchus teres, Pratylenchus thornei, Pratylenchus vulnus, Pratylenchus zeae und die migratorischen Endoparasiten Pratylenchus spp., Pseudohalenchus minutus, Psilenchus magnidens, Psilenchus tumidus, Punctodera chalcoensis, Quinisulcius acutus, Radopholus citrophilus, Radopholus similis, die migratorischen Endoparasiten Radopholus spp., Rotylenchulus borealis, Rotylenchulus parvus, Rotylenchulus reniformis und Rotylenchulus spp., Rotylenchus laurentinus, Rotylenchus macrodoratus, Rotylenchus robustus, Rotylenchus uniformis und Rotylenchus spp., Scutellonema brachyurum, Scutellonema bradys, Scutellonema clathricaudatum und die migratorischen Endoparasiten Scutellonema spp., Subanguina radiciola, Tetylenchus nicotianae, Trichodorus cylindricus, Trichodorus minor, Trichodorus primitivus, Trichodorus proximus, Trichodorus similis, Trichodorus sparsus und die Ektoparasiten Trichodorus spp., Tylenchorhynchus agri, Tylenchorhynchus brassicae, Tylenchorhynchus clarus, Tylenchorhynchus claytoni, Tylenchorhynchus digitatus, Tylenchorhynchus ebriensis, Tylenchorhynchus maximus, Tylenchorhynchus nudus, Tylenchorhynchus vulgaris und Tylenchorhynchus spp., Tylenchulus semipenetrans und die Semiparasiten Tylenchulus spp., Xiphinema americanum, Xiphinema brevicolle, Xiphinema dimorphicaudatum, Xiphinema index und die Ektoparasiten Xiphinema spp.

Zu den Nematoden, zu deren Bekämpfung eine Verbindung der Formel (I) eingesetzt werden kann, zählen Nematoden der Gattung Meloidogyne wie der Southern Root-Knot Nematode (Meloidogyne incognita), der Javanese Root-Knot Nematode (Meloidogyne javanica, der Northern Root-Knot Nematode (Meloidogyne hapla) und der Peanut Root-Knot Nematode (Meloidogyne arenaria); Nematoden der Gattung Ditylenchus wie das Kartoffelkrätzeälchen (Ditylenchus destructor) und das Stock- und Stängelälchen (Ditylenchus dipsaci); Nematoden der Gattung Pratylenchus wie der Cob Root-Lesion Nematode (Pratylenchus penetrans), der Chrysanthemum Root-Lesion Nematode (Pratylenchus fallax), der Kaffeewurzelnematode (Pratylenchus coffeae), der Teewurzelnematode (Pratylenchus loosi) und der Walnut Root-Lesion Nematode (Pratylenchus vulnus); Nematoden der Gattung Globodera wie das Goldfarbene Kartoffelzystenälchen (Globodera rostochiensis) und das Weiße Kartoffelzystenälchen (Globodera pallida); Nematoden der Gattung Heterodera wie der Sojabohnenzystennematode (Heterodera glycines) und das Rübenzystenälchen (Heterodera schachtii); Nematoden der Gattung Aphelenchoides wie der Rice White-tip Nematode (Aphelenchoides besseyi), das Chrysanthemenälchen (Aphelenchoides ritzemabosi) und das Erdbeerälchen (Aphelenchoides fragariae); Nematoden der Gattung Aphelenchus wie der fungivore Nematode (Aphelenchus avenae); Nematoden der Gattung Radopholus, wie der Burrowing-Nematode (Radopholus similis); Nematoden der Gattung Tylenchulus wie der Orangenwurzelnematode (Tylenchulus semipenetrans); Nematoden der Gattung Rotylenchulus wie der reniforme Nematode (Rotylenchulus reniformis); in Bäumen lebende Nematoden, wie der Kiefernholznematode (Bursaphelenchus xylophilus) und der Red Ring Nematode (Bursaphelenchus cocophilus) und dergleichen.

Zu den Pflanzen, zu deren Schutz eine Verbindung der Formel (I) verwendet werden kann, zählen Pflanzen wie Getreide (zum Beispiel Reis, Gerste, Weizen, Roggen, Hafer, Mais, und dergleichen), Bohnen (Sojabohne, Azukibohne, Bohne, Dicke Bohne, Erbsen, Erdnüsse und dergleichen), Obstbäume/Früchte (Äpfel, Zitrusarten, Birnen, Trauben, Pfirsiche, japanische Aprikosen, Kirschen, Walnüsse, Mandeln, Bananen, Erdbeeren und dergleichen), Gemüsearten (Kohl, Tomate, Spinat, Brokkoli, Salat, Zwiebel, Röhrenlauch, Pfeffer und dergleichen), Hackfrüchte (Karotte, Kartoffel, Süßkartoffel, Rettich, Lotuswurzel, Steckrübe und dergleichen), Pflanzen für industrielle Rohstoffe (Baumwolle, Hanf, Papiermaulbeere, Mitsumata, Raps, Rübe, Hopfen, Zuckerrohr, Zuckerrübe, Olive, Gummi, Palmen, Kaffee, Tabak, Tee und dergleichen), Kürbisgewächse (Kürbis, Gurke, Wassermelone, Melone und dergleichen), Weidepflanzen (Knaulgras, Sorgum, Wiesenlieschgras, Klee, Luzerne und dergleichen), Rasengräser (Maskarenengras, Straußgras und dergleichen), Gewürzpflanzen usw. (Lavendel, Rosmarin, Thymian, Petersilie, Pfeffer, Ingwer und dergleichen) und Blumenpflanzen (Chrysantheme, Rose, Orchidee und dergleichen) erwähnt werden.

Die Verbindungen der Formel (I) eignen sich besonders für die Bekämpfung von Nematoden des Kaffees, insbesondere von Pratylenchus brachyurus, Pratylenchus coffeae, Meloidogyne exigua, Meloidogyne incognita, Meloidogyne coffeicola, Helicotylenchus spp. sowie aus Meloidogyne paranaensis, Rotylenchus spp., Xiphinema spp., Tylenchorhynchus spp. und Scutellonema spp..

Die Verbindungen der Formel (I) eignen sich besonders für die Bekämpfung von Nematoden der Kartoffel, insbesondere von Pratylenchus brachyurus, Pratylenchus pratensis, Pratylenchus scribneri, Pratylenchus penetrans, Pratylenchus coffeae, Ditylenchus dipsaci sowie aus Pratylenchus alleni, Pratylenchus andinus, Pratylenchus cerealis, Pratylenchus crenatus, Pratylenchus hexincisus, Pratylenchus loosi, Pratylenchus neglectus, Pratylenchus teres, Pratylenchus thornei, Pratylenchus vulnus, Belonolaimus longicaudatus, Trichodorus cylindricus, Trichodorus primitivus, Trichodorus proximus, Trichodorus similis, Trichodorus sparsus, Paratrichodorus minor, Paratrichodorus allius, Paratrichodorus nanus, Paratrichodorus teres, Meloidogyne arenaria, Meloidogyne fallax, Meloidogyne hapla, Meloidogyne thamesi, Meloidogyne incognita, Meloidogyne chitwoodi, Meloidogyne javanica, Nacobbus aberrans, Globodera rostochiensis, Globodera pallida, Ditylenchus destructor, Radopholus similis, Rotylenchulus reniformis, Neotylenchus vigissi, Paraphelenchus pseudoparietinus, Aphelenchoides fragariae und Meloinema spp.

Die Verbindungen der Formel (I) eignen sich besonders für die Bekämpfung von Nematoden der Tomate, insbesondere von Meloidogyne arenaria, Meloidogyne hapla, Meloidogyne javanica, Meloidogyne incognita, Pratylenchus penetrans und aus Pratylenchus brachyurus, Pratylenchus coffeae, Pratylenchus scribneri, Pratylenchus vulnus, Paratrichodorus minor, Meloidogyne exigua, Nacobbus aberrans, Globodera solanacearum, Dolichodorus heterocephalus und Rotylenchulus reniformis.

Die Verbindungen der Formel (I) eignen sich besonders für die Bekämpfung von Nematoden von Gurkengewächsen, insbesondere von Meloidogyne arenaria, Meloidogyne hapla, Meloidogyne javanica, Meloidogyne incognita, Rotylenchulus reniformis und Pratylenchus thornei.

Die Verbindungen der Formel (I) eignen sich besonders für die Bekämpfung von Nematoden der Baumwolle, insbesondere von Belonolaimus longicaudatus, Meloidogyne incognita, Hoplolaimus columbus, Hoplolaimus galeatus und Rotylenchulus reniformis.

Die Verbindungen der Formel (I) eignen sich besonders für die Bekämpfung von Nematoden des Maises, insbesondere von Belonolaimus longicaudatus, Paratrichodorus minor und aus Pratylenchus brachyurus, Pratylenchus delattrei, Pratylenchus hexincisus, Pratylenchus penetrans, Pratylenchus zeae, (Belonolaimus gracilis), Belonolaimus nortoni, Longidorus breviannulatus, Meloidogyne arenaria, Meloidogyne arenaria thamesi, Meloidogyne graminis, Meloidogyne incognita, Meloidogyne incognita acrita, Meloidogyne javanica, Meloidogyne naasi, Heterodera avenae, Heterodera oryzae, Heterodera zeae, Punctodera chalcoensis, Ditylenchus dipsaci, Hoplolaimus aegyptii, Hoplolaimus magnistylus, Hoplolaimus galeatus, Hoplolaimus indicus, Helicotylenchus digonicus, Helicotylenchus dihystera, Helicotylenchus pseudorobustus, Xiphinema americanum, Dolichodorus heterocephalus, Criconemella ornata, Criconemella onoensis, Radopholus similis, Rotylenchulus borealis, Rotylenchulus parvus, Tylenchorhynchus agri, Tylenchorhynchus clarus, Tylenchorhynchus claytoni, Tylenchorhynchus maximus, Tylenchorhynchus nudus, Tylenchorhynchus vulgaris, Quinisulcius acutus, Paratylenchus minutus, Hemicycliophora parvana, Aglenchus agricola, Anguina tritici, Aphelenchoides arachidis, Scutellonema brachyurum und Subanguina radiciola.

Die Verbindungen der Formel (I) eignen sich besonders für die Bekämpfung von Nematoden der Sojabohne, insbesondere vonPratylenchus brachyurus, Pratylenchus pratensis, Pratylenchus penetrans, Pratylenchus scribneri, Belonolaimus longicaudatus, Heterodera glycines, Hoplolaimus columbus und aus Pratylenchus coffeae, Pratylenchus hexincisus, Pratylenchus neglectus, Pratylenchus crenatus, Pratylenchus alleni, Pratylenchus agilis, Pratylenchus zeae, Pratylenchus vulnus, (Belonolaimus gracilis), Meloidogyne arenaria, Meloidogyne incognita, Meloidogyne javanica, Meloidogyne hapla, Hoplolaimus columbus, Hoplolaimus galeatus und Rotylenchulus reniformis.

Die Verbindungen der Formel (I) eignen sich besonders für die Bekämpfung von Nematoden des Tabaks, insbesondere von Meloidogyne incognita, Meloidogyne javanica und aus Pratylenchus brachyurus, Pratylenchus pratensis, Pratylenchus hexincisus, Pratylenchus penetrans, Pratylenchus neglectus, Pratylenchus crenatus, Pratylenchus thornei, Pratylenchus vulnus, Pratylenchus zeae, Longidorus elongatu, Paratrichodorus lobatus, Trichodorus spp., Meloidogyne arenaria, Meloidogyne hapla, Globodera tabacum, Globodera solanacearum, Globodera virginiae, Ditylenchus dipsaci, Rotylenchus spp., Helicotylenchus spp., Xiphinema americanum, Criconemella spp., Rotylenchulus reniformis, Tylenchorhynchus claytoni, Paratylenchus spp. und Tetylenchus nicotianae.

Die Verbindungen der Formel (I) eignen sich besonders für die Bekämpfung von Nematoden von Zitrusgewächsen, insbesondere von Pratylenchus coffeae und aus Pratylenchus brachyurus, Pratylenchus vulnus, Belonolaimus longicaudatus, Paratrichodorus minor, Paratrichodorus porosus, Trichodorus , Meloidogyne incognita, Meloidogyne incognita acrita, Meloidogyne javanica, Rotylenchus macrodoratus, Xiphinema americanum, Xiphinema brevicolle, Xiphinema index, Criconemella spp., Hemicriconemoides, Radopholus similis bzw. Radopholus citrophilus, Hemicycliophora arenaria, Hemicycliophora nudata und Tylenchulus semipenetrans .

Die Verbindungen der Formel (I) eignen sich besonders für die Bekämpfung von Nematoden der Banane, insbesondere von Pratylenchus coffeae, Radopholus similis und aus Pratylenchus giibbicaudatus, Pratylenchus loosi, Meloidogyne spp., Helicotylenchus multicinctus, Helicotylenchus dihystera und Rotylenchulus spp..

Die Verbindungen der Formel (I) eignen sich besonders für die Bekämpfung von Nematoden der Ananas, insbesondere von Pratylenchus zeae, Pratylenchus pratensis, Pratylenchus brachyurus, Pratylenchus goodeyi., Meloidogyne spp., Rotylenchulus reniformis und aus Longidorus elongatus, Longidorus laevicapitatus, Trichodorus primitivus, Trichodorus minor, Heterodera spp., Ditylenchus myceliophagus, Hoplolaimus californicus, Hoplolaimus pararobustus, Hoplolaimus indicus, Helicotylenchus dihystera, Helicotylenchus nannus, Helicotylenchus multicinctus, Helicotylenchus erythrine, Xiphinema dimorphicaudatum, Radopholus similis, Tylenchorhynchus digitatus, Tylenchorhynchus ebriensis, Paratylenchus minutus, Scutellonema clathricaudatum, Scutellonema bradys, Psilenchus tumidus, Psilenchus magnidens, Pseudohalenchus minutus, Criconemoides ferniae, Criconemoides onoense und Criconemoides ornatum .

Die Verbindungen der Formel (I) eignen sich besonders für die Bekämpfung von Nematoden von Trauben, insbesondere von Pratylenchus vulnus, Meloidogyne arenaria, Meloidogyne incognita, Meloidogyne javanica, Xiphinema americanum, Xiphinema index und aus Pratylenchus pratensis, Pratylenchus scribneri, Pratylenchus neglectus, Pratylenchus brachyurus, Pratylenchus thornei und Tylenchulus semipenetrans.

Die Verbindungen der Formel (I) eignen sich besonders für die Bekämpfung von Nematoden von Baumkulturen - Kernobst, insbesondere von Pratylenchus penetrans und aus Pratylenchus vulnus, Longidorus elongatus, Meloidogyne incognita und Meloidogyne hapla.

Die Verbindungen der Formel (I) eignen sich besonders für die Bekämpfung von Nematoden von Baumkulturen - Steinfrüchten, insbesondere von Pratylenchus penetrans, Pratylenchus vulnus, Meloidogyne arenaria, Meloidogyne hapla, Meloidogyne javanica, Meloidogyne incognita, Criconemella xenoplax und aus Pratylenchus brachyurus, Pratylenchus coffeae, Pratylenchus scribneri, Pratylenchus zeae, Belonolaimus longicaudatus, Helicotylenchus dihystera, Xiphinema americanum, Criconemella curvata, Tylenchorhynchus claytoni, Paratylenchus hamatus, Paratylenchus projectus, Scutellonema brachyurum und Hoplolaimus galeatus.

Die Verbindungen der Formel (I) eignen sich besonders für die Bekämpfung von Nematoden in Baumkulturen, Zuckerrohr und Reis , insbesondere von Trichodorus spp., Criconemella spp. und aus Pratylenchus spp. , Paratrichodorus spp., Meloidogyne spp. , Helicotylenchus spp., Tylenchorhynchus spp., Aphelenchoides spp. Heterodera spp, Xiphinema spp. und Cacopaurus pestis.

Ebenso bezieht sich der Begriff "Nematoden" im vorliegenden Zusammenhang auf Nematoden, die Menschen oder Tiere schädigen.

Spezifische Nematodenarten, die für den Menschen oder für Tiere schädlich sind, sind:
Trichinellida, zum Beispiel: Trichuris spp., Capillaria spp., Paracapillaria spp., Eucoleus spp., Trichomosoides spp., Trichinella spp.

Aus der Ordnung der Tylenchida zum Beispiel: Micronema spp., Strongyloides spp.

Aus der Ordnung der Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp., Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.

Aus der Odnung der Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Viele bekannte Nematizide wirken gleichsam gegen andere parasitäre Helminthen und werden daher für die Bekämpfung von human- und tierparasitären Würmern, die nicht unbedingt zu der Gruppe Nematoda gehören, verwendet. Die vorliegende Erfindung bezieht sich auch auf die Verwendung der Verbindungen der Formel (I) als anthelmintische Arzneimittel. Zu den pathogenen endoparasitären Helminthen zählen Platyhelmintha (z.B. Monogenea, Cestodes und Trematodes), Acanthocephala und Pentastoma. Die folgenden Helminthen sind als bevorzugt zu erwähnen:
Monogenea: z.B.: Gyrodactylus spp., Dactylogyrus spp., Polystoma spp.
Cestodes: aus der Ordnung der Pseudophyllidea zum Beispiel: Diphyllobothrium spp., Spirometra spp., Schistocephalus spp., Ligula spp., Bothridium spp., Diplogonoporus spp.
Aus der Ordnung der Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.
Trematodes: aus der Klasse der Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp., Metorchis spp., Heterophyes spp., Metagonimus spp.
Acanthocephala: aus der Ordnung der Oligacanthorhynchida z.B.: Macracanthorhynchus spp., Prosthenorchis spp.; aus der Ordnung der Polymorphida zum Beispiel: Filicollis spp.; aus der Ordnung der Moniliformida zum Beispiel: Moniliformis spp.,
Aus der Ordnung der Echinorhynchida zum Beispiel Acanthocephalus spp., Echinorhynchus spp., Leptorhynchoides spp.
Pentastoma: aus der Ordnung der Porocephalida zum Beispiel Linguatula spp.

Auf dem Gebiet der Tiermedizin und in der Tierhaltung erfolgt die Verabreichung der Verbindungen der Formel (I) auf bekannte Weise direkt oder enteral, parenteral, dermal oder nasal in Form von geeigneten Anwendungsformen. Die Verabreichung kann prophylaktisch oder therapeutisch erfolgen.

Die Verbindungen der Formel (I) können gegebenenfalls in bestimmten Konzentrationen bzw. Aufwandmengen auch als Herbizide, Safener, Wachstumsregulatoren oder Mittel zur Verbesserung der Pflanzeneigenschaften, als Mikrobizide oder Gametozide, beispielsweise als Fungizide, Antimykotika, Bakterizide, Virizide (einschließlich Mittel gegen Viroide) oder als Mittel gegen MLO (Mycoplasma-like-organism) und RLO (Rickettsia-like-organism) verwendet werden. Sie lassen sich gegebenenfalls auch als Zwischen- oder Vorprodukte für die Synthese weiterer Wirkstoffe einsetzen.

### Formulierungen

Die vorliegende Erfindung betrifft weiterhin Formulierungen und daraus bereitete Anwendungsformen als Schädlingsbekämpfungsmittel wie z. B. Drench-, Drip- und Spritzbrühen, umfassend mindestens eine Verbindung der Formel (I). Gegebenenfalls enthalten die Anwendungsformen weitere Schädlingsbekämpfungsmittel und/oder die Wirkung verbessernde Adjuvantien wie Penetrationsförderer, z. B. vegetative Öle wie beispielsweise Rapsöl, Sonnenblumenöl, Mineralöle wie beispielsweise Paraffinöle, Alkylester vegetativer Fettsäuren wie beispielsweise Rapsöl- oder Sojaölmethylester oder Alkanol-alkoxylate und/oder Spreitmittel wie beispielsweise Alkylsiloxane und/oder Salze z.B. organische oder anorganische Ammonium- oder Phosphoniumsalze wie beispielsweise Ammoniumsulfat oder Diammonium-hydrogenphosphat und/oder die Retention fördernde Mittel wie z. B. Dioctylsulfosuccinat oder Hydroxypropyl-guar Polymere und/oder Humectants wie z.B. Glycerin und/oder Dünger wie beispielsweise Ammonium-, Kalium- oder Phosphor-enthaltende Dünger.

Übliche Formulierungen sind beispielsweise wasserlösliche Flüssigkeiten (SL), Emulsionskonzentrate (EC), Emulsionen in Wasser (EW), Suspensionskonzentrate (SC, SE, FS, OD), in Wasser dispergierbare Granulate (WG), Granulate (GR) und Kapselkonzentrate (CS); diese und weitere mögliche Formuliertypen sind beispielsweise durch Crop Life International und in Pesticide Specifications, Manual on development and use of FAO and WHO specifications for pesticides, FAO Plant Production and Protection Papers - 173, prepared by the FAO/WHO Joint Meeting on Pesticide Specifications, 2004, ISBN: 9251048576 beschrieben. Gegebenenfalls enthalten die Formulierungen neben einem oder mehreren Verbindungen der Formel (I) weitere agrochemische Wirkstoffe.

Vorzugsweise handelt es sich um Formulierungen oder Anwendungsformen, welche Hilfsstoffe wie beispielsweise Streckmittel, Lösemittel, Spontanitätsförderer, Trägerstoffe, Emulgiermittel, Dispergiermittel, Frostschutzmittel, Biozide, Verdicker und/oder weitere Hilfsstoffe wie beispielsweise Adjuvantien enthalten. Ein Adjuvant in diesem Kontext ist eine Komponente, die die biologische Wirkung der Formulierung verbessert, ohne dass die Komponente selbst eine biologische Wirkung hat. Beispiele für Adjuvantien sind Mittel, die die Retention, das Spreitverhalten, das Anhaften an der Blattoberfläche oder die Penetration fördern.

Diese Formulierungen werden in bekannter Weise hergestellt, z.B. durch Vermischen der Verbindungen der Formel (I) mit Hilfsstoffen wie beispielsweise Streckmitteln, Lösemitteln und/oder festen Trägerstoffen und/oder weiteren Hilfsstoffen wie beispielsweise oberflächenaktive Stoffe. Die Herstellung der Formulierungen erfolgt entweder in geeigneten Anlagen oder auch vor oder während der Anwendung.

Als Hilfsstoffe können solche Stoffe Verwendung finden, die geeignet sind, der Formulierung der Verbindungen der Formel (I) oder den aus diesen Formulierungen bereiteten Anwendungsformen (wie z.B. gebrauchsfähigen Schädlingsbekämpfungsmitteln wie Spritzbrühen oder Saatgutbeizen) besondere Eigenschaften, wie bestimmte physikalische, technische und/oder biologische Eigenschaften zu verleihen.

Als Streckmittel eignen sich z.B. Wasser, polare und unpolare organische chemische Flüssigkeiten z.B. aus den Klassen der aromatischen und nicht-aromatischen Kohlenwasserstoffe (wie Paraffine, Alkylbenzole, Alkylnaphthaline, Chlorbenzole), der Alkohole und Polyole (die ggf. auch substituiert, verethert und/oder verestert sein können), der Ketone (wie Aceton, Cyclohexanon), Ester (auch Fette und Öle) und (Poly-)Ether, der einfachen und substituierten Amine, Amide, Lactame (wie N-Alkylpyrrolidone) und Lactone, der Sulfone und Sulfoxide (wie Dimethylsulfoxid).

Im Falle der Benutzung von Wasser als Streckmittel können z.B. auch organische Lösemittel als Hilfslösemittel verwendet werden. Als flüssige Lösemittel kommen im Wesentlichen infrage: Aromaten wie Xylol, Toluol oder Alkylnaphthaline, chlorierte Aromaten oder chlorierte aliphatische Kohlenwasser-stoffe wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole wie Butanol oder Glykol sowie deren Ether und Ester, Ketone wie Aceton, Methylethylketon, Methylisobutylketon oder Cyclohexanon, stark polare Lösemittel wie Dimethylformamid und Dimethylsulfoxid sowie Wasser.

Grundsätzlich können alle geeigneten Lösemittel verwendet werden. Geeignete Lösemittel sind beispielsweise aromatische Kohlenwasserstoffe wie z.B. Xylol, Toluol oder Alkylnaphthaline, chlorierte aromatische oder aliphatische Kohlenwasserstoffe wie z.B. Chlorbenzol, Chlorethylen, oder Methylen-chlorid, aliphatische Kohlenwasserstoffe wie z.B. Cyclohexan, Paraffine, Erdölfraktionen, mineralische und pflanzliche Öle, Alkohole wie z.B. Methanol, Ethanol, iso-Propanol, Butanol oder Glykol sowie deren Ether und Ester, Ketone wie z.B. Aceton, Methylethylketon, Methylisobutylketon oder Cyclohexanon, stark polare Lösemittel wie Dimethylsulfoxid sowie Wasser.

Grundsätzlich können alle geeigneten Trägerstoffe eingesetzt werden. Als Trägerstoffe kommen insbesondere infrage: z.B. Ammoniumsalze und natürliche Gesteinsmehle wie Kaoline, Tonerden, Talkum, Kreide, Quarz, Attapulgit, Montmorillonit oder Diatomeenerde und synthetische Gesteinsmehl, wie hochdisperse Kieselsäure, Aluminiumoxid und natürliche oder synthetische Silikate, Harze, Wachse und /oder feste Düngemittel. Mischungen solcher Trägerstoffe können ebenfalls verwendet werden. Als Trägerstoffe für Granulate kommen infrage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Papier, Kokosnussschalen, Maiskolben und Tabakstängel.

Auch verflüssigte gasförmige Streckmittel oder Lösemittel können eingesetzt werden. Insbesondere eignen sich solche Streckmittel oder Trägerstoffe, welche bei normaler Temperatur und unter Normaldruck gasförmig sind, z.B. Aerosol-Treibgase wie Halogenkohlenwasserstoffe sowie Butan, Propan, Stickstoff und Kohlendioxid.

Beispiele für Emulgier- und/oder Schaum erzeugende Mittel, Dispergiermittel oder Benetzungsmittel mit ionischen oder nicht-ionischen Eigenschaften oder Mischungen dieser oberflächenaktiven Stoffe sind Salze von Polyacrylsäure, Salze von Lignosulphonsäure, Salze von Phenolsulphonsäure oder Naphthalinsulphonsäure, Polykondensate von Ethylenoxid mit Fettalkoholen oder mit Fettsäuren oder mit Fettaminen, mit substituierten Phenolen (vorzugsweise Alkylphenole oder Arylphenole), Salze von Sulphobernsteinsäureestern, Taurinderivate (vorzugsweise Alkyltaurate), Phosphorsäureester von polyethoxylierten Alkoholen oder Phenole, Fettsäureester von Polyolen und Derivate der Verbindungen enthaltend Sulphate, Sulphonate und Phosphate, z.B. Alkylarylpolyglycolether, Alkylsulfonate, Alkylsulfate, Arylsulfonate, Eiweißhydrolysate, Lignin-Sulfitablaugen und Methylcellulose. Die Anwesenheit einer oberflächenaktiven Substanz ist vorteilhaft, wenn eine der Verbindungen der Formel (I) und/oder einer der inerten Trägerstoffe nicht in Wasser löslich ist und wenn die Anwendung in Wasser erfolgt.

Als weitere Hilfsstoffe können in den Formulierungen und den daraus abgeleiteten Anwendungsformen Farbstoffe wie anorganische Pigmente, z.B. Eisenoxid, Titanoxid, Ferrocyanblau und organische Farbstoffe wie Alizarin-, Azo- und Metallphthalocyaninfarbstoffe und Nähr- und Spurennährstoffe wie Salze von Eisen, Mangan, Bor, Kupfer, Kobalt, Molybdän und Zink vorhanden sein.

Weiterhin enthalten sein können Stabilisatoren wie Kältestabilisatoren, Konservierungsmittel, Oxidationsschutzmittel, Lichtschutzmittel oder andere die chemische und / oder physikalische Stabilität verbessernde Mittel. Weiterhin enthalten sein können schaumerzeugende Mittel oder Entschäumer.

Ferner können die Formulierungen und daraus abgeleiteten Anwendungsformen als zusätzliche Hilfsstoffe auch Haftmittel wie Carboxymethylcellulose, natürliche und synthetische pulverige, körnige oder latexförmige Polymere enthalten wie Gummiarabikum, Polyvinylalkohol, Polyvinylacetat sowie natürliche Phospholipide wie Kephaline und Lecithine und synthetische Phospholipide. Weitere Hilfsstoffe können mineralische und vegetabile Öle sein.

Gegebenenfalls können noch weitere Hilfsstoffe in den Formulierungen und den daraus abgeleiteten Anwendungsformen enthalten sein. Solche Zusatzstoffe sind beispielsweise Duftstoffe, schützende Kolloide, Bindemittel, Klebstoffe, Verdicker, thixotrope Stoffe, Penetrationsförderer, Retentionsförderer, Stabilisatoren, Sequestiermittel, Komplexbildner, Humectans, Spreitmittel. Im Allgemeinen können die Verbindungen der Formel (I) mit jedem festen oder flüssigen Zusatzstoff, welches für Formulierungszwecke gewöhnlich verwendet wird, kombiniert werden.

Als Retentionsförderer kommen alle diejenigen Substanzen in Betracht, die die dynamische Oberflächenspannung verringern wie beispielsweise Dioctylsulfosuccinat oder die die Visko-Elastizität erhöhen wie beispielsweise Hydroxypropyl-guar Polymere.

Als Penetrationsförderer kommen im vorliegenden Zusammenhang alle diejenigen Substanzen in Betracht, die üblicherweise eingesetzt werden, um das Eindringen von agrochemischen Wirkstoffen in Pflanzen zu verbessern. Penetrationsförderer werden in diesem Zusammenhang dadurch definiert, dass sie aus der (in der Regel wässerigen) Applikationsbrühe und/oder aus dem Spritzbelag in die Kutikula der Pflanze eindringen und dadurch die Stoffbeweglichkeit (Mobilität) der Wirkstoffe in der Kutikula erhöhen können. Die in der Literatur (Baur et al., 1997, Pesticide Science 51, 131-152) beschriebene Methode kann zur Bestimmung dieser Eigenschaft eingesetzt werden. Beispielhaft werden genannt Alkoholalkoxylate wie beispielsweise Kokosfettethoxylat (10) oder Isotridecylethoxylat (12), Fettsäureester wie beispielsweise Rapsöl- oder Sojaölmethylester, Fettamine Alkoxylate wie beispielsweise Tallowamine-ethoxylat (15) oder Ammonium- und/oder Phosphonium-Salze wie beispielsweise Ammoniumsulfat oder Diammonium-hydrogenphosphat.

Die Formulierungen enthalten bevorzugt zwischen 0,00000001 und 98 Gew.-% der Verbindung der Formel (I), besonders bevorzugt zwischen 0,01 und 95 Gew.-% der Verbindung der Formel (I), ganz besonders bevorzugt zwischen 0,5 und 90 Gew.-% der Verbindung der Formel (I), bezogen auf das Gewicht der Formulierung.

Der Gehalt an der Verbindung der Formel (I) in den aus den Formulierungen bereiteten Anwendungsformen (insbesondere Schädlingsbekämpfungsmittel) kann in weiten Bereichen variieren. Die Konzentration der Verbindung der Formel (I) in den Anwendungsformen kann üblicherweise zwischen 0,00000001 und 95 Gew.-% der Verbindung der Formel (I), vorzugsweise zwischen 0,00001 und 1 Gew.-%, bezogen auf das Gewicht der Anwendungsform, liegen. Die Anwendung geschieht in einer den Anwendungsformen angepaßten üblichen Weise.

### Mischungen

Die Verbindungen der Formel (I) können auch in Mischung mit einem oder mehreren geeigneten Fungiziden, Bakteriziden, Akariziden, Molluskiziden, Nematiziden, Insektiziden, Mikrobiologika, Nützlingen, Herbizide, Düngemitteln, Vogelrepellentien, Phytotonics, Sterilantien, Safenern, Semiochemicals und/oder Pflanzenwachstumsregulatoren verwendet werden, um so z.B. das Wirkungsspektrum zu verbreitern, die Wirkdauer zu verlängern, die Wirkgeschwindigkeit zu steigern, Repellenz zu verhindern oder Resistenzentwicklungen vorzubeugen. Desweiteren können solche Wirkstoffkombinationen das Pflanzenwachstum und/oder die Toleranz gegenüber abiotischen Faktoren wie z. B. hohen oder niedrigen Temperaturen, gegen Trockenheit oder gegen erhöhten Wasser- bzw. Bodensalzgehalt verbessern. Auch lässt sich das Blüh- und Fruchtverhalten verbessern, die Keimfähigkeit und Bewurzelung optimieren, die Ernte erleichtern und Ernteerträge steigern, die Reife beeinflussen, die Qualität und/oder den Ernährungswert der Ernteprodukte steigern, die Lagerfähigkeit verlängern und/oder die Bearbeitbarkeit der Ernteprodukte verbessern.

Weiterhin können die Verbindungen der Formel (I) in Mischung mit weiteren Wirkstoffen oder Semiochemicals, wie Lockstoffen und/oder Vogelrepellentien und/oder Pflanzenaktivatoren und/oder Wachstumsregulatoren und/oder Düngemitteln vorliegen. Gleichfalls können die Verbindungen der Formel (I) in Mischungen mit Mitteln zur Verbesserung der Pflanzeneigenschaften wie zum Beispiel Wuchs, Ertrag und Qualität des Erntegutes eingesetzt werden.

In einer besonderen erfindungsgemäßen Ausführungsform liegen die Verbindungen der Formel (I) in Formulierungen bzw. in den aus diesen Formulierungen bereiteten Anwendungsformen in Mischung mit weiteren Verbindungen vor, vorzugsweise solchen wie nachstehend beschrieben.

Wenn eine der im Folgenden genannten Verbindungen in verschiedenen tautomeren Formen vorkommen kann sind auch diese Formen mit umfasst, auch wenn sie sie nicht in jedem Fall explizit genannt wurden.

### Insektizide / Akarizide / Nematizide

Die hier mit ihrem "common name" genannten Wirkstoffe sind bekannt und beispielsweise im Pestizidhandbuch ("The Pesticide Manual" 16th Ed., British Crop Protection Council 2012) beschrieben oder im Internet recherchierbar (z.B. http://www.alanwood.net/pesticides).
(1) Acetylcholinesterase (AChE) Inhibitoren, wie beispielsweise Carbamate, z.B. Alanycarb, Aldicarb, Bendiocarb, Benfuracarb, Butocarboxim, Butoxycarboxim, Carbaryl, Carbofuran, Carbosulfan, Ethiofencarb, Fenobucarb, Formetanate, Furathiocarb, Isoprocarb, Methiocarb, Methomyl, Metolcarb, Oxamyl, Pirimicarb, Propoxur, Thiodicarb, Thiofanox, Triazamate, Trimethacarb, XMC und Xylylcarb oder organophosphate, z.B. Acephate, Azamethiphos, Azinphosethyl, Azinphos-methyl, Cadusafos, Chlorethoxyfos, Chlorfenvinphos, Chlormephos, Chlorpyrifos, Chlorpyrifos-methyl, Coumaphos, Cyanophos, Demeton-S-methyl, Diazinon, Dichlorvos/DDVP, Dicrotophos, Dimethoate, Dimethylvinphos, Disulfoton, EPN, Ethion, Ethoprophos, Famphur, Fenamiphos, Fenitrothion, Fenthion, Fosthiazate, Heptenophos, Imicyafos, Isofenphos, Isopropyl O-(methoxyaminothio-phosphoryl) salicylat, Isoxathion, Malathion, Mecarbam, Methamidophos, Methidathion, Mevinphos, Monocrotophos, Naled, Omethoate, Oxydemeton-methyl, Parathion, Parathion-methyl, Phenthoate, Phorate, Phosalone, Phosmet, Phosphamidon, Phoxim, Pirimiphosmethyl, Profenofos, Propetamphos, Prothiofos, Pyraclofos, Pyridaphenthion, Quinalphos, Sulfotep, Tebupirimfos, Temephos, Terbufos, Tetrachlorvinphos, Thiometon, Triazophos, Triclorfon und Vamidothion.
(2) GABA-gesteuerte Chlorid-Kanal-Antagonisten, wie beispielsweise Cyclodien-organochlorine, z.B. Chlordane und Endosulfan oder Phenylpyrazole (Fiprole), z.B. Ethiprole und Fipronil.
(3) Natrium-Kanal-Modulatoren / Spannungsabhängige Natrium-Kanal-Blocker, wie beispielsweise Pyrethroide, z.B. Acrinathrin, Allethrin, d-cis-trans Allethrin, d-trans Allethrin, Bifenthrin, Bioallethrin, Bioallethrin S-cyclopentenyl Isomer, Bioresmethrin, Cycloprothrin, Cyfluthrin, beta-Cyfluthrin, Cyhalothrin, lambda-Cyhalothrin, gamma-Cyhalothrin, Cypemethrin, alpha-Cypermethrin, beta-Cypermethrin, theta-Cypermethrin, zeta-Cypermethrin, Cyphenothrin [(1R)-trans-Isomere], Deltamethrin, Empenthrin [(EZ)-(1R)-Isomere), Esfenvalerate, Etofenprox, Fenpropathrin, Fenvalerate, Flucythrinate, Flumethrin, tau-Fluvalinate, Halfenprox, Imiprothrin, Kadethrin, Momfluorothrin, Permethrin, Phenothrin [(1R)-trans-Isomer), Prallethrin, Pyrethrine (pyrethrum), Resmethrin, Silafluofen, Tefluthrin, Tetramethrin, Tetramethrin [(1R)- Isomere)], Tralomethrin und Transfluthrin oder DDT oder Methoxychlor.
(4) Nikotinerge Acetylcholin-Rezeptor (nAChR) Agonisten, wie beispielsweise Neonikotinoide, z.B. Acetamiprid, Clothianidin, Dinotefuran, Imidacloprid, Nitenpyram, Thiacloprid und Thiamethoxam oder Nikotin oder Sulfoxaflor oder Flupyradifurone.
(5) Nikotinerge Acetylcholin-Rezeptor (nAChR) allosterische Aktivatoren, wie beispielsweise Spinosine, z.B. Spinetoram und Spinosad.
(6) Chlorid-Kanal-Aktivatoren, wie beispielsweise Avermectine/Milbemycine, z.B. Abamectin, Emamectin-benzoat, Lepimectin und Milbemectin.
(7) Juvenilhormon-Imitatoren, wie beispielsweise Juvenilhormon-Analoge, z.B. Hydroprene, Kinoprene und Methoprene oder Fenoxycarb oder Pyriproxyfen.
(8) Wirkstoffe mit unbekannten oder nicht spezifischen Wirkmechanismen, wie beispielsweise
   Alkylhalide, z.B. Methylbromid und andere Alkylhalide; oderChloropicrin oder Sulfurylfluorid oder Borax oder Brechweinstein.
(9) Selektive Fraßhemmer, z.B. Pymetrozine oder Flonicamid.
(10) Milbenwachstumsinhibitoren, z.B. Clofentezine, Hexythiazox und Diflovidazin oder Etoxazole.
(11) Mikrobielle Disruptoren der Insektendarmmembran, z.B. Bacillus thuringiensis Subspezies israelensis, Bacillus sphaericus, Bacillus thuringiensis Subspezies aizawai, Bacillus thuringiensis Subspezies kurstaki, Bacillus thuringiensis Subspezies tenebrionis und BT Pflanzenproteine: Cry1Ab, Cry1Ac, Cry1Fa, Cry2Ab, mCry3A, Cry3Ab, Cry3Bb, Cry34/35Ab1.
(12) Inhibitoren der oxidativen Phosphorylierung, ATP-Disruptoren, wie beispielsweise Diafenthiuron oder Organozinnverbindungen, z.B. Azocyclotin, Cyhexatin und Fenbutatin-oxid oder Propargite oder Tetradifon.
(13) Entkoppler der oxidativen Phoshorylierung durch Unterbrechung des H-Protongradienten, wie beispielsweise Chlorfenapyr, DNOC und Sulfluramid.
(14) Nikotinerge Acetylcholin-Rezeptor-Antagonisten, wie beispielsweise Bensultap, Cartaphydrochlorid, Thiocyclam und Thiosultap-Natrium.
(15) Inhibitoren der Chitinbiosynthese, Typ 0, wie beispielsweise Bistrifluron, Chlorfluazuron, Diflubenzuron, Flucycloxuron, Flufenoxuron, Hexaflumuron, Lufenuron, Novaluron, Noviflumuron, Teflubenzuron und Triflumuron.
(16) Inhibitoren der Chitinbiosynthese, Typ 1, wie beispielsweise Buprofezin.
(17) Häutungshemmer (insbesondere bei Dipteren, d.h.Zweiflüglern), wie beispielsweise Cyromazine.
(18) Ecdyson-Rezeptor Agonisten, wie beispielsweise Chromafenozide, Halofenozide, Methoxyfenozide und Tebufenozide.
(19) Oktopaminerge Agonisten, wie beispielsweise Amitraz.
(20) Komplex-III-Elektronentransportinhibitoren, wie beispielsweise Hydramethylnon oder Acequinocyl oder Fluacrypyrim.
(21) Komplex-I-Elektronentransportinhibitoren, beispielsweise METI-Akarizide, z.B. Fenazaquin, Fenpyroximate, Pyrimidifen, Pyridaben, Tebufenpyrad und Tolfenpyrad oder Rotenone (Derris).
(22) Spannungsabhängige Natriumkanal-Blocker, z.B. Indoxacarb oder Metaflumizone.
(23) Inhibitoren der Acetyl-CoA-Carboxylase, wie beispielsweise Tetron- und Tetramsäurederivate, z.B. Spirodiclofen, Spiromesifen und Spirotetramat.
(24) Komplex-IV-Elektronentransportinhibitoren, wie beispielsweise Phosphine, z.B. Aluminiumphosphid, Calciumphosphid, Phosphin und Zinkphosphid oder Cyanid.
(25) Komplex-II-Elektronentransportinhibitoren, wie beispielsweise Cyenopyrafen und Cyflumetofen.
(28) Ryanodinrezeptor-Effektoren, wie beispielsweise Diamide, z.B. Chlorantraniliprole, Cyantraniliprole und Flubendiamide.

Weitere Wirkstoffe mit unbekanntem oder nicht eindeutigem Wirkmechanismus, wie beispielsweise Afidopyropen, Afoxolaner, Azadirachtin, Benclothiaz, Benzoximate, Bifenazate, Broflanilide, Bromopropylate, Chinomethionat, Cryolite, Cyclaniliprole, Cycloxaprid, Cyhalodiamide Dicloromezotiaz, Dicofol, Diflovidazin, Flometoquin, Fluazaindolizine, Fluensulfone, Flufenerim, Flufenoxystrobin, Flufiprole, Fluhexafon, Fluopyram, Fluralaner, Fluxametamide, Fufenozide, Guadipyr, Heptafluthrin, Imidaclothiz, Iprodione, Lotilaner, Meperfluthrin, Paichongding, Pyflubumide, Pyridalyl, Pyrifluquinazon, Pyriminostrobin, Sarolaner, Tetramethylfluthrin, Tetraniliprole, Tetrachlorantraniliprole, Tioxazafen, Triflumezopyrim und Iodmethan; desweiteren Präparate auf Basis von Bacillus firmus (I-1582, BioNeem, Votivo), sowie folgende bekannte wirksame Verbindungen: 1-{2-Fluor-4-methyl-5-[(2,2,2-trifluorethyl)sulfinyl]phenyl}-3-(trifluormethyl)-1H-1,2,4-triazol-5-amin (bekannt aus WO2006/043635), {1'-[(2E)-3-(4-Chlorphenyl)prop-2-en-1-yl]-5-fluorspiro[indol-3,4'-piperidin]-1(2H)-yl} (2-chlorpyridin-4-yl)methanon (bekannt aus WO2003/106457), 2-Chlor-N-[2-{1-[(2E)-3-(4-chlorphenyl)prop-2-en-1-yl]piperidin-4-yl}-4-(trifluormethyl)phenyl]isonicotinamid (bekannt aus WO2006/003494), 3-(2,5-Dimethylphenyl)-4-hydroxy-8-methoxy-1,8-diazaspiro[4.5]dec-3-en-2-on (bekannt aus WO2009/049851), 3-(2,5-Dimethylphenyl)-8-methoxy-2-oxo-1,8-diazaspiro[4.5]dec-3-en-4-ylethylcarbonat (bekannt aus WO2009/049851), 4-(But-2-in-1-yloxy)-6-(3,5-dimethylpiperidin-1-yl)-5-fluorpyrimidin (bekannt aus WO2004/099160), 4-(But-2-in-1-yloxy)-6-(3-chlorphenyl)pyrimidin (bekannt aus WO2003/076415), PF1364 (CAS-Reg.No. 1204776-60-2), Methyl-2-[2-({[3-brom-1-(3-chlorpyridin-2-yl)-1H-pyrazol-5-yl]carbonyl}amino)-5-chlor-3-methylbenzoyl]-2-methylhydrazincarboxylat (bekannt aus WO2005/085216), Methyl-2-[2-({[3-brom-1-(3-chlorpyridin-2-yl)-1H-pyrazol-5-yl]carbonyl}amino)-5-cyan-3-methylbenzoyl]-2-ethylhydrazincarboxylat (bekannt aus WO2005/085216), Metlyl-2-[2-({[3-brom-1-(3-chlorpyridin-2-yl)-1H-pyrazol-5-yl]carbonyl}amino)-5-cyan-3-methylbenzoyl]-2-methylhydrazincarboxylat (bekannt aus WO2005/085216), Methyl-2-[3,5-dibrom-2-({[3-brom-1-(3-chlorpyridin-2-yl)-1H-pyrazol-5-yl]carbonyl}amino)benzoyl]-2-ethylhydrazincarboxylat (bekannt aus WO2005/085216), N-[2-(5-Amino-1,3,4-thiadiazol-2-yl)-4-chlor-6-methylphenyl]-3-brom-1-(3-chlorpyridin-2-yl)-1H-pyrazol-5-carboxamid (bekannt aus CN102057925), 4-[5-(3,5-Dichlorphenyl)-5-(trifluormethyl)-4,5-dihydro-1,2-oxazol-3-yl]-2-methyl-N-(1-oxidothietan-3-yl)benzamid (bekannt aus WO2009/080250), N-[(2E)-1-[(6-Chlorpyridin-3-yl)methyl]pyridin-2(1H)-yliden] -2,2,2-trifluoracetamid (bekannt aus WO2012/029672), 1-[(2-Chlor-1,3-thiazol-5-yl)methyl]-4-oxo-3-phenyl-4H-pyrido[1,2-a]pyrimidin-1-ium-2-olat (bekannt aus WO2009/099929), 1-[(6-Chlorpyridin-3-yl)methyl]-4-oxo-3-phenyl-4H-pyrido[1,2-a]pyrimidin-1-ium-2-olat (bekannt aus WO2009/099929), 4-(3-{2,6-Dichlor-4-[(3,3-dichlorprop-2-en-1-yl)oxy]phenoxy}propoxy)-2-methoxy-6-(trifluormetliyl)pyrimidin (bekannt aus CN101337940), N-[2-(tert-Butylcarbamoyl)-4-chlor-6-methylphenyl]-1-(3-chlorpyridin-2-yl)-3-(fluormethoxy)-1H-pyrazol-5-carboxamid (bekannt aus WO2008/134969, Butyl-[2-(2,4-dichlorphenyl)-3-oxo-4-oxaspiro[4.5]dec-1-en-1-yl]-carbonat (bekannt aus CN 102060818), 3E)-3-[1-[(6-Chlor-3-pyridyl)methyl]-2-pyridyliden]-1,1,1-trifluorpropan-2-on (bekannt aus WO2013/144213, N-(Methylsulfonyl)-6-[2-(pyridin-3-yl)-1,3-thiazol-5-yl]pyridin-2-carboxamid (bekannt aus WO2012/000896), N-[3-(Benzylcarbamoyl)-4-chlorphenyl]-1-methyl-3-(pentafluorethyl)-4-(trifluormethyl)-1H-pyrazol-5-carboxamidbekanntaus WO2010/051926).

### Fungizide

Die hier mit ihrem "common name" spezifizierten Wirkstoffe sind bekannt, beispielsweise beschrieben im "Pesticide Manual" oder im Internet (beispielsweise: http://www.alanwood.net/pesticides).

Alle aufgeführten fungiziden Mischpartner der Klassen (1) bis (15) können optional Salze mit entsprechenden Basen oder Säuren bilden, sofern geeignete funktionelle Gruppen vorliegen. Außerdem sind für die aufgeführten fungiziden Mischpartner der Klassen (1) bis (15) auch tautomere Formen eingeschlossen, sofern Tautomerie möglich ist.
1) Inhibitoren der Ergosterolbiosynthese, zum Beispiel (1.01) Aldimorph, (1.02) Azaconazol, (1.03) Bitertanol, (1.04) Bromuconazol, (1.05) Cyproconazol, (1.06) Diclobutrazol, (1.07) Difenoconazol, (1.08) Diniconazol, (1.09) Diniconazol-M, (1.10) Dodemorph, (1.11) Dodemorphacetat, (1.12) Epoxiconazol, (1.13) Etaconazol, (1.14) Fenarimol, (1.15) Fenbuconazol, (1.16) Fenhexamid, (1.17) Fenpropidin, (1.18) Fenpropimorph, (1.19) Fluquinconazol, (1.20) Flurprimidol, (1.21) Flusilazol, (1.22) Flutriafol, (1.23) Furconazol, (1.24) Furconazol-cis, (1.25) Hexaconazol, (1.26) Imazalil, (1.27) Imazalilsulfat, (1.28) Imibenconazol, (1.29) Ipconazol, (1.30) Metconazol, (1.31) Myclobutanil, (1.32) Naftifin, (1.33) Nuarimol, (1.34) Oxpoconazol, (1.35) Paclobutrazol, (1.36) Pefurazoat, (1.37) Penconazol, (1.38) Piperalin, (1.39) Prochloraz, (1.40) Propiconazol, (1.41) Prothioconazol, (1.42) Pyributicarb, (1.43) Pyrifenox, (1.44) Quinconazol, (1.45) Simeconazol, (1.46) Spiroxamin, (1.47) Tebuconazol, (1.48) Terbinafin, (1.49) Tetraconazol, (1.50) Triadimefon, (1.51) Triadimenol, (1.52) Tridemorph, (1.53) Triflumizol, (1.54) Triforin, (1.55) Triticonazol, (1.56) Uniconazol, (1.57) Uniconazol-p, (1.58) Viniconazol, (1.59) Voriconazol, (1.60) 1-(4-Chlorphenyl)-2-(1H-1,2,4-triazol-1-yl)cycloheptanol, (1.61) 1-(2,2-Dimethyl-2,3-dihydro-1H-inden-1-yl)-1H-imidazol-5-carbonsäuremethylester, (1.62) N'-{5-(Difluormethyl)-2-methyl-4-[3-(trimethylsilyl)propoxy]phenyl}-N-ethyl-N-methylimidoformamid, (1.63) N-Ethyl-N-methyl-N'-{2-methyl-5-(trifluormethyl)-4-[3-(trimethylsilyl)propoxy]phenyl}imidoformamid, (1.64) O-[1-(4-Methoxyphenoxy)-3,3-dimethylbutan-2-yl]-1H-imidazol-1-carbothioat, (1.65) Pyrisoxazol, (1.66) 2-{[3-(2-Chlorphenyl)-2-(2,4-difluorphenyl)oxiran-2-yl]methyl}-2,4-dihydro-3H-1,2,4-triazol-3-thion, (1.67) 1-{[3-(2-Chlorphenyl)-2-(2,4-difluorphenyl)oxiran-2-yl]methyl}-1H-1,2,4-triazol-5-ylthiocyanat, (1.68) 5-(Allylsulfanyl)-1-{[3-(2-chlorphenyl)-2-(2,4-difluorphenyl)oxiran-2-yl]methyl}-1H-1,2,4-triazol, (1.69) 2-[1-(2,4-Dichlorphenyl)-5-hydroxy-2,6,6-trimethylheptan-4-yl]-2,4-dihydro-3H-1,2,4-triazol-3-thion, (1.70) 2-{[rel(2R,3S)-3-(2-Chlorphenyl)-2-(2,4-difluorphenyl)oxiran-2-yl]methyl}-2,4-dihydro-3H-1,2,4-triazol-3-thion, (1.71) 2-{[rel(2R,3R)-3-(2-Chlorphenyl)-2-(2,4-difluorphenyl)oxiran-2-yl]methyl}-2,4-dihydro-3H-1,2,4-triazol-3-thion, (1.72) 1-{[rel(2R,3S)-3-(2-Chlorphenyl)-2-(2,4-difluorphenyl)oxiran-2-yl]methyl}-1H-1,2,4-triazol-5-ylthiocyanat, (1.73) 1-{[rel(2R,3R)-3-(2-Chlorphenyl)-2-(2,4-difluorphenyl)oxiran-2-yl]methyl}-1H-1,2,4-triazol-5-ylthiocyanat, (1.74) 5-(Allylsulfanyl)-1-{[rel(2R,3S)-3-(2-chlorphenyl)-2-(2,4-diauorphenyl)oxiran-2-yl]methyl}-1H-1,2,4-triazol, (1.75) 5-(Allylsulfanyl)-1-{[rel(2R,3R)-3-(2-chlorphenyl)-2-(2,4-difluorphenyl)oxiran-2-yl]methyl}-1H-1,2,4-triazol, (1.76) 2-[(2S,4S,5S)-1-(2,4-Dichlorphenyl)-5-hydroxy-2,6,6-trimethylheptan-4-yl]-2,4-dihydro-3H-1,2,4-triazol-3-thion, (1.77) 2-[(2R,4S,5S)-1-(2,4-Dichlorphenyl)-5-hydroxy-2,6,6-trimethylheptan-4-yl]-2,4-dihydro-3H-1,2,4-triazol-3-thion, (1.78) 2-[(2R,4R,5R)-1-(2,4-Dichlorphenyl)-5-hydroxy-2,6,6-trimethylheptan-4-yl]-2,4-dihydro-3H-1,2,4-triazol-3-thion, (1.79) 2-[(2S,4R,5R)-1-(2,4-Dichlorphenyl)-5-hydroxy-2,6,6-trimethylheptan-4-yl]-2,4-dihydro-3H-1,2,4-triazol-3-thion, (1.80) 2-[(2S,4S,5R)-1-(2,4-Dichlorphenyl)-5-hydroxy-2,6,6-trimethylheptan-4-yl]-2,4-dihydro-3H-1,2,4-triazol-3-thion, (1.81) 2-[(2R,4S,5R)-1-(2,4-Dichlorphenyl)-5-hydroxy-2,6,6-trimethylheptan-4-yl]-2,4-dihydro-3H-1,2,4-triazol-3-thion, (1.82) 2-[(2R,4R,5S)-1-(2,4-Dichlorphenyl)-5-hydroxy-2,6,6-trimethylheptan-4-yl]-2,4-dihydro-3H-1,2,4-triazol-3-thion, (1.83) 2-[(2S,4R,5S)-1-(2,4-Dichlorphenyl)-5-hydroxy-2,6,6-trimethylheptan-4-yl]-2,4-dihydro-3H-1,2,4-triazol-3-thion, (1.84) 2-[4-(4-Chlorphenoxy)-2-(trifluormethyl)phenyl]-1-(1H-1,2,4-triazol-1-yl)propan-2-ol, (1.85) 2-[4-(4-Chlorphenoxy)-2-(trifluormethyl)phenyl]-1-(1H-1,2,4-triazol-1-yl)butan-2-ol, (1.86) 2-[4-(4-Chlorphenoxy)-2-(trifluormethyl)phenyl]-1-(1H-1,2,4-triazol-1-yl)pentan-2-ol, (1.87) 2-[2-Chlor-4-(4-chlorphenoxy)phenyl]-1-(1H-1,2,4-triazol-1-yl)butan-2-ol, (1.88) 2-[2-Chlor-4-(2,4-dichlorphenoxy)phenyl]-1-(1H-1,2,4-triazol-1-yl)propan-2-ol, (1.89) (2R)-2-(1-Chlorcyclopropyl)-4-[(1R)-2,2-dichlorcyclopropyl]-1-(1H-1,2,4-triazol-1-yl)butan-2-ol, (1.90) (2R)-2-(1-Chlorcyclopropyl)-4-[(1S)-2,2-dichlorcyclopropyl]-1-(1H-1,2,4-triazol-1-yl)butan-2-ol, (1.91) (2S)-2-(1-Chlorcyclopropyl)-4-[(1S)-2,2-dichlorcyclopropyl]-1-(1H-1,2,4-triazol-1-yl)butan-2-ol, (1.92) (2S)-2-(1-Chlorcyclopropyl)-4-[(1R)-2,2-dichlorcyclopropyl]-1-(1H-1,2,4-triazol-1-yl)butan-2-ol, (1.93) (1S,2R,5R)-5-(4-Chlorbenzyl)-2-(chlormethyl)-2-methyl-1-(1H-1,2,4-triazol-1-ylmethyl)cyclopentanol, (1.94) (1R,2S,5S)-5-(4-Chlorbenzyl)-2-(chlormethyl)-2-methyl-1-(1H-1,2,4-triazol-1-ylmethyl)cyclopentanol, (1.95) 5-(4-Chlorbenzyl)-2-(chlormethyl)-2-methyl-1-(1H-1,2,4-triazol-1-ylmethyl)cyclopentanol.
2) Inhibitoren der Atmungskette am Komplex I oder II, zum Beispiel (2.01) Bixafen, (2.02) Boscalid, (2.03) Carboxin, (2.04) Diflumetorim, (2.05) Fenfuram, (2.06) Fluopyram, (2.07) Flutolanil, (2.08) Fluxapyroxad, (2.09) Furametpyr, (2.10) Furmecyclox, (2.11) Isopyrazam (Mischung von synepimerem Racemat 1RS,4SR,9RS und anti-epimerem Racemat 1RS,4SR,9SR), (2.12) Isopyrazam (anti-epimeres Racemat 1RS,4SR,9SR), (2.13) Isopyrazam (anti-epimeres Enantiomer 1R,4S,9S), (2.14) Isopyrazam (anti-epimeres Enantiomer 1S,4R,9R), (2.15) Isopyrazam (syn-epimeres Racemat 1RS,4SR,9RS), (2.16) Isopyrazam (syn-epimeres Enantiomer 1R,4S,9R), (2.17) Isopyrazam (syn-epimeres Enantiomer 1S,4R,9S), (2.18) Mepronil, (2.19) Oxycarboxin, (2.20) Penflufen, (2.21) Penthiopyrad, (2.22) Sedaxan, (2.23) Thifluzamid, (2.24) 1-Methyl-N-[2-(1,1,2,2-tetrafluorethoxy)phenyl]-3-(trifluormethyl)-1H-pyrazol-4-carboxamid, (2.25) 3-(Difluormethyl)-1-methyl-N-[2-(1,1,2,2-tetrafluorethoxy)phenyl]-1H-pyrazol-4-carboxamid, (2.26) 3-(Difluormethyl)-N-[4-fluor-2-(1,1,2,3,3,3-hexafluorpropoxy)phenyl]-1-methyl-1H-pyrazol-4-carboxamid, (2.27) N-[1-(2,4-Dichlorphenyl)-1-methoxypropan-2-yl]-3-(difluormethyl)-1-methyl-1H-pyrazol-4-carboxamid, (2.28) 5,8-Difluor-N-[2-(2-fluor-4-{[4-(trifluormethyl)pyridin-2-yl]oxy}phenyl)ethyl]chinazolin-4-amin, (2.29) Benzovindiflupyr, (2.30) N-[(1S,4R)-9-(Dichlormethylen)-1,2,3,4-tetrahydro-1,4-methanonaphthalin-5-yl]-3-(difluormethyl)-1-methyl-1H-pyrazol-4-carboxamid, (2.31) N-[(1R,4S)-9-(Dichlormethylen)-1,2,3,4-tetrahydro-1,4-methanonaphthalin-5-yl]-3-(difluormethyl)-1-methyl-1H-pyrazol-4-carboxamid, (2.32) 3-(Difluormethyl)-1-methyl-N-(1,1,3-trimethyl-2,3-dihydro-1H-inden-4-yl)-1H-pyrazol-4-carboxamid, (2.33) 1,3,5-Trimethyl-N-(1,1,3-trimethyl-2,3-dihydro-1H-inden-4-yl)-1H-pyrazol-4-carboxamid, (2.34) 1-Methyl-3-(trifluormethyl)-N-(1,1,3-trimethyl-2,3-dihydro-1H-inden-4-yl)-1H-pyrazol-4-carboxamid, (2.35) 1-Methyl-3-(trifluormethyl)-N-[(3R)-1,1,3-trimethyl-2,3-dihydro-1H-inden-4-yl]-1H-pyrazol-4-carboxamid, (2.36) 1-Methyl-3-(trifluormethyl)-N-[(3S)-1,1,3-trimethyl-2,3-dihydro-1H-inden-4-yl]-1H-pyrazol-4-carboxamid, (2.37) 3-(Difluormethyl)-1-methyl-N-[(3S)-1,1,3-trimethyl-2,3-dihydro-1H-inden-4-yl]-1H-pyrazol-4-carboxamid, (2.38) 3-(Difluormethyl)-1-methyl-N-[(3R)-1,1,3-trimethyl-2,3-dihydro-1H-inden-4-yl]-1H-pyrazol-4-carboxamid, (2.39) 1,3,5-Trimethyl-N-[(3R)-1,1,3-trimethyl-2,3-dihydro-1H-inden-4-yl]-1H-pyrazol-4-carboxamid, (2.40) 1,3,5-Trimethyl-N-[(3S)-1,1,3-trimethyl-2,3-dihydro-1H-inden-4-yl]-1H-pyrazol-4-carboxamid, (2.41) Benodanil, (2.42) 2-Chlor-N-(1,1,3-trimethyl-2,3-dihydro-1H-inden-4-yl)pyridin-3-carboxamid, (2.43) Isofetamid, (2.44) 1-Methyl-3-(trifluormethyl)-N-[2'-(trifluormethyl)biphenyl-2-yl]-1H-pyrazol-4-carboxamid, (2.45) N-(4'-Chlorbiphenyl-2-yl)-3-(difluormethyl)-1-methyl-1H-pyrazol-4-carboxamid, (2.46) N-(2',4'-Dichlorbiphenyl-2-yl)-3-(difluormethyl)-1-methyl-1H-pyrazol-4-carboxamid, (2.47) 3-(Difluormethyl)-1-methyl-N-[4'-(trifluormethyl)biphenyl-2-yl]-1H-pyrazol-4-carboxamid, (2.48) N-(2',5'-Difluorbiphenyl-2-yl)-1-methyl-3-(trifluormethyl)-1H-pyrazol-4-carboxamid, (2.49) 3-(Difluormethyl)-1-methyl-N-[4'-(prop-1-in-1-yl)biphenyl-2-yl]-1H-pyrazol-4-carboxamid, (2.50) 5-Fluor-1,3-dimethyl-N-[4'-(prop-1-in-1-yl)biphenyl-2-yl]-1H-pyrazol-4-carboxamid, (2.51) 2-Chlor-N-[4'-(prop-1-in-1-yl)biphenyl-2-yl]nicotinamid, (2.52) 3-(Difluormethyl)-N-[4'-(3,3-dimethylbut-1-in-1-yl)biphenyl-2-yl]-1-methyl-1H-pyrazol-4-carboxamid, (2.53) N-[4'-(3,3-Dimethylbut-1-in-1-yl)biphenyl-2-yl]-5-fluor-1,3-dimethyl-1H-pyrazol-4-carboxamid, (2.54) 3-(Difluormethyl)-N-(4'-ethinylbiphenyl-2-yl)-1-methyl-1H-pyrazol-4-carboxamid, (2.55) N-(4'-Ethinylbiphenyl-2-yl)-5-fluor-1,3-dimethyl-1H-pyrazol-4-carboxamid, (2.56) 2-Chlor-N-(4'-ethinylbiphenyl-2-yl)nicotinamid, (2.57) 2-Chlor-N-[4'-(3,3-dimethylbut-1-in-1-yl)biphenyl-2-yl]nicotinamid, (2.58) 4-(Difluormethyl)-2-methyl-N-[4'-(trifluormethyl)biphenyl-2-yl]-1,3-thiazol-5-carboxamid, (2.59) 5-Fluor-N-[4'-(3-hydroxy-3-methylbut-1-in-1-yl)biphenyl-2-yl]-1,3-dimethyl-1H-pyrazol-4-carboxamid, (2.60) 2-Chlor-N-[4'-(3-hydroxy-3-methylbut-1-in-1-yl)biphenyl-2-yl]nicotinamid, (2.61) 3-(Difluormethyl)-N-[4'-(3-methoxy-3-methylbut-1-in-1-yl)biphenyl-2-yl]-1-methyl-1H-pyrazol-4-carboxamid, (2.62) 5-Fluor-N-[4'-(3-methoxy-3-methylbut-1-in-1-yl)biphenyl-2-yl]-1,3-dimethyl-1H-pyrazol-4-carboxamid, (2.63) 2-Chlor-N-[4'-(3-methoxy-3-methylbut-1-in-1-yl)biphenyl-2-yl]nicotinamid, (2.64) 1,3-Dimethyl-N-(1,1,3-trimethyl-2,3-dihydro-1H-inden-4-yl)-1H-pyrazol-4-carboxamid, (2.65) 1,3-Dimethyl-N-[(3R)-1,1,3-trimethyl-2,3-dihydro-1H-inden-4-yl]-1H-pyrazol-4-carboxamid, (2.66) 1,3-Dimethyl-N-[(3S)-1,1,3-trimethyl-2,3-dihydro-1H-inden-4-yl]-1H-pyrazol-4-carboxamid, (2.67) 3-(Difluormethyl)-N-methoxy-1-methyl-N-[1-(2,4,6-trichlorphenyl)propan-2-yl]-1H-pyrazol-4-carboxamid, (2.68) 3-(Difluormethyl)-N-(7-fluor-1,1,3-trimethyl-2,3-dihydro-1H-inden-4-yl)-1-methyl-1H-pyrazol-4-carboxamid, (2.69) 3-(Difluormethyl)-N-[(3R)-7-fluor-1,1,3-trimethyl-2,3-dihydro-1H-inden-4-yl]-1-methyl-1H-pyrazol-4-carboxamid, (2.70) 3-(Difluormethyl)-N-[(3S)-7-fluor-1,1,3-trimethyl-2,3-dihydro-1H-inden-4-yl]-1-methyl-1H-pyrazol-4-carboxamid.
3) Inhibitoren der Atmungskette am Komplex III, zum Beispiel (3.01) Ametoctradin, (3.02) Amisulbrom, (3.03) Azoxystrobin, (3.04) Cyazofamid, (3.05) Coumethoxystrobin, (3.06) Coumoxystrobin, (3.07) Dimoxystrobin, (3.08) Enoxastrobin, (3.09) Famoxadon, (3.10) Fenamidon, (3.11) Flufenoxystrobin, (3.12) Fluoxastrobin, (3.13) Kresoxim-methyl, (3.14) Metominostrobin, (3.15) Orysastrobin, (3.16) Picoxystrobin, (3.17) Pyraclostrobin, (3.18) Pyrametostrobin, (3.19) Pyraoxystrobin, (3.20) Pyribencarb, (3.21) Triclopyricarb, (3.22) Trifloxystrobin, (3.23) (2E)-2-(2-{[6-(3-Chlor-2-methylphenoxy)-5-fluorpyrimidin-4-yl]oxy}phenyl)-2-(methoxyimino)-N-methyl-acetamid, (3.24) (2E)-2-(Methoxyimino)-N-methyl-2-(2-{[({(1E)-1-[3-(trifluormethyl)phenyl]-ethyliden}amino)oxy]methyl}phenyl)acetamid, (3.25) (2E)-2-(Methoxyimino)-N-methyl-2-{2-[(E)-({1-[3-(trifluormethyl)phenyl]ethoxy}imino)methyl]phenyl}acetamid, (3.26) (2E)-2-{2-[({[(1E)-1-(3-{[(E)-1-Fluor-2-phenylvinyl]oxy}phenyl)ethyliden]amino}oxy)methyl]phenyl}-2-(methoxyimino)-N-methylacetamid, (3.27) Fenaminostrobin, (3.28) 5-Methoxy-2-methyl-4-(2-{[({(1E)-1-[3-(trifluormethyl)phenyl]ethyliden}amino)oxy]methyl}phenyl)-2,4-dihydro-3H-1,2,4-triazol-3-on, (3.29) (2E)-2-{2-[({Cyclopropyl[(4-methoxyphenyl)imino]methyl}sulfanyl)methyl]phenyl}-3-methoxyacrylsäuremethylester, (3.30) N-(3-Ethyl-3,5,5-trimethylcyclohexyl)-3-formamido-2-hydroxybenzamid, (3.31) 2-{2-[(2,5-Dimethylphenoxy)methyl]phenyl}-2-methoxy-N-methylacetamid, (3.32) 2-{2-[(2,5-Dimethylphenoxy)methyl]phenyl}-2-methoxy-N-methylacetamid, (3.33) (2E,3Z)-5-{[1-(4-Chlorphenyl)-1H-pyrazol-3-yl]oxy}-2-(methoxyimino)-N,3-dimethylpent-3-enamid.
4) Inhibitoren der Mitose und Zellteilung, zum Beispiel (4.01) Benomyl, (4.02) Carbendazim, (4.03) Chlorfenazol, (4.04) Diethofencarb, (4.05) Ethaboxam, (4.06) Fluopicolid, (4.07) Fuberidazol, (4.08) Pencycuron, (4.09) Thiabendazol, (4.10) Thiophanat-methyl, (4.11) Thiophanat, (4.12) Zoxamid, (4.13) 5-Chlor-7-(4-methylpiperidin-1-yl)-6-(2,4,6-trifluorphenyl)[1,2,4]triazolo[1,5-a]pyrimidin, (4.14) 3-Chlor-5-(6-chlorpyridin-3-yl)-6-methyl-4-(2,4,6-trifluorphenyl)pyridazin.
5) Verbindungen, die dazu fähig sind, an mehreren Stellen anzugreifen ("Multisite Action"), zum Beispiel (5.01) Bordeaux-Mischung, (5.02) Captafol, (5.03) Captan, (5.04) Chlorothalonil, (5.05) Kupferhydroxid, (5.06) Kupfernaphthenat, (5.07) Kupferoxid, (5.08) Kupferoxychlorid, (5.09) Kupfer(2+)-sulfat, (5.10) Dichlofluanid, (5.11) Dithianon, (5.12) Dodin, (5.13) Dodin freie Base, (5.14) Ferbam, (5.15) Fluorofolpet, (5.16) Folpet, (5.17) Guazatin, (5.18) Guazatinacetat, (5.19) Iminoctadin, (5.20) Iminoctadinalbesilat, (5.21) Iminoctadintriacetat, (5.22) Mancopper, (5.23) Mancozeb, (5.24) Maneb, (5.25) Metiram, (5.26) Metiram-Zink, (5.27) Oxin-Kupfer, (5.28) Propamidin, (5.29) Propineb, (5.30) Schwefel und Schwefelzubereitungen einschließlich Calciumpolysulfid, (5.31) Thiram, (5.32) Tolylfluanid, (5.33) Zineb, (5.34) Ziram, (5.35) Anilazin.
6) Verbindungen, die dazu fähig sind, eine Abwehrreaktion des Wirtes zu induzieren, zum Beispiel (6.01) Acibenzolar-S-methyl, (6.02) Isotianil, (6.03) Probenazol, (6.04) Tiadinil, (6.05) Laminarin.
7) Inhibitoren der Aminosäure- und/oder Proteinbiosynthese, zum Beispiel (7.01) Andoprim, (7.02) Blasticidin-S, (7.03) Cyprodinil, (7.04) Kasugamycin, (7.05) Kasugamycinhydrochlorid-hydrat, (7.06) Mepanipyrim, (7.07) Pyrimethanil, (7.08) 3-(5-Fluor-3,3,4,4-tetramethyl-3,4-dihydroisochinolin-1-yl)chinolin, (7.09) Oxytetracyclin, (7.10) Streptomycin.
8) Inhibitoren der ATP-Produktion, zum Beispiel (8.01) Fentinacetat, (8.02) Fentinchlorid, (8.03) Fentinhydroxid, (8.04) Silthiofam.
9) Inhibitoren der Zellwandsynthese, zum Beispiel (9.01) Benthiavalicarb, (9.02) Dimethomorph, (9.03) Flumorph, (9.04) Iprovalicarb, (9.05) Mandipropamid, (9.06) Polyoxine, (9.07) Polyoxorim, (9.08) Validamycin A, (9.09) Valifenalat, (9.10) Polyoxin B, (9.11) (2E)-3-(4-tert.-Butylphenyl)-3-(2-chlorpyridin-4-yl)-1-(morpholin-4-yl)prop-2-en-1-on, (9.12) (2Z)-3-(4-tert.-Butylphenyl)-3-(2-chlorpyridin-4-yl)-1-(morpholin-4-yl)prop-2-en-1-on.
10) Inhibitoren der Lipid- und Membransynthese, zum Beispiel (10.01) Biphenyl, (10.02) Chloroneb, (10.03) Dicloran, (10.04) Edifenphos, (10.05) Etridiazol, (10.06) Iodocarb, (10.07) Iprobenfos, (10.08) Isoprothiolan, (10.09) Propamocarb, (10.10) Propamocarbhydrochlorid, (10.11) Prothiocarb, (10.12) Pyrazophos, (10.13) Quintozen, (10.14) Tecnazen, (10.15) Tolclofos-methyl.
11) Inhibitoren der Melaninbiosynthese, zum Beispiel (11.01) Carpropamid, (11.02) Diclocymet, (11.03) Fenoxanil, (11.04) Phthalid, (11.05) Pyroquilon, (11.06) Tricyclazol, (11.07) 2,2,2-Trifluorethyl {3-methyl-1-[(4-methylbenzoyl)amino]butan-2-yl}carbamat.
12) Inhibitoren der Nukleinsäuresynthese, zum Beispiel (12.01) Benalaxyl, (12.02) Benalaxyl-M (Kiralaxyl), (12.03) Bupirimat, (12.04) Clozylacon, (12.05) Dimethirimol, (12.06) Ethirimol, (12.07) Furalaxyl, (12.08) Hymexazol, (12.09) Metalaxyl, (12.10) Metalaxyl-M (Mefenoxam), (12.11) Ofurace, (12.12) Oxadixyl, (12.13) Oxolinsäure, (12.14) Octhilinon.
13) Inhibitoren der Signalvermittlung, zum Beispiel (13.01) Chlozolinat, (13.02) Fenpiclonil, (13.03) Fludioxonil, (13.04) Iprodion, (13.05) Procymidon, (13.06) Quinoxyfen, (13.07) Vinclozolin, (13.08) Proquinazid.
14) Verbindungen, die als Entkoppler wirken können, zum Beispiel (14.01) Binapacryl, (14.02) Dinocap, (14.03) Ferimzon, (14.04) Fluazinam, (14.05) Meptyldinocap.
15) Weitere Verbindungen, zum Beispiel (15.001) Benthiazol, (15.002) Bethoxazin, (15.003) Capsimycin, (15.004) Carvon, (15.005) Chinomethionat, (15.006) Pyriofenon (Chlazafenon), (15.007) Cufraneb, (15.008) Cyflufenamid, (15.009) Cymoxanil, (15.010) Cyprosulfamid, (15.011) Dazomet, (15.012) Debacarb, (15.013) Dichlorophen, (15.014) Diclomezin, (15.015) Difenzoquat, (15.016) Difenzoquatmetilsulfat, (15.017) Diphenylamin, (15.018) Ecomat, (15.019) Fenpyrazamin, (15.020) Flumetover, (15.021) Fluoroimid, (15.022) Flusulfamid, (15.023) Flutianil, (15.024) Fosetyl-Aluminium, (15.025) Fosetyl-Calcium, (15.026) Fosetyl-Natrium, (15.027) Hexachlorbenzol, (15.028) Irumamycin, (15.029) Methasulfocarb, (15.030) Methylisothiocyanat, (15.031) Metrafenon, (15.032) Mildiomycin, (15.033) Natamycin, (15.034) Nickeldimethyldithiocarbamat, (15.035) Nitrothal-isopropyl, (15.036) Oxamocarb, (15.037) Oxyfenthiin, (15.038) Pentachlorphenol und Salze, (15.039) Phenothrin, (15.040) phosphorige Säure und deren Salze, (15.041) Propamocarb-fosetylat, (15.042) Propanosin-Natrium, (15.043) Pyrimorph, (15.044) Pyrrolnitrin, (15.045) Tebufloquin, (15.046) Tecloftalam, (15.047) Tolnifanid, (15.048) Triazoxid, (15.049) Trichlamid, (15.050) Zarilamid, (15.051) 2-Methylpropansäure-(3S,6S,7R,8R)-8-benzyl-3-[({3-[(isobutyryloxy)methoxy]-4-methoxypyridin-2-yl}carbonyl)amino]-6-methyl-4,9-dioxo-1,5-dioxonan-7-ylester, (15.052) 1-(4-{4-[(5R)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.053) 1-(4-{4-[(5S)-5-(2,6-Difluorphenyl)-4,5-dihydro-1,2-oxazol-3-yl]-1,3-thiazol-2-yl}piperidin-1-yl)-2-[5-methyl-3-(trifluormethyl)-1H-pyrazol-1-yl]ethanon, (15.054) Oxathiapiprolin, (15.055) 1H-Imidazol-1-carbonsäure-1-(4-methoxyphenoxy)-3,3-dimethylbutan-2-ylester, (15.056) 2,3,5,6-Tetrachlor-4-(methylsulfonyl)pyridin, (15.057) 2,3-Dibutyl-6-chlorthieno[2,3-d]pyrimidin-4(3H)-on, (15.058) 2,6-Dimethyl-1H,5H-[1,4]dithiino[2,3-c:5,6-c']dipyrrol-1,3,5,7(2H,6H)-tetron, (15.059) 2-[5-Methyl-3-(trifluormethyl)-1H-pyrazol-1-yl]-1-(4-{4-[(5R)-5-phenyl-4,5-dihydro-1,2-oxazol-3-yl]-1,3-thiazol-2-yl}piperidin-1-yl)ethanon, (15.060) 2-[5-Methyl-3-(trifluormethyl)-1H-pyrazol-1-yl]-1-(4-{4-[(5S)-5-phenyl-4,5-dihydro-1,2-oxazol-3-yl]-1,3-thiazol-2-yl}piperidin-1-yl)ethanon, (15.061) 2-[5-Methyl-3-(trifluormethyl)-1H-pyrazol-1-yl]-1-{4-[4-(5-phenyl-4,5-dihydro-1,2-oxazol-3-yl)-1,3-thiazol-2-yl]piperidin-1-yl}ethanon, (15.062) 2-Butoxy-6-iod-3-propyl-4H-chromen-4-on, (15.063) 2-Chlor-5-[2-chlor-1-(2,6-difluor-4-methoxyphenyl)-4-methyl-1H-imidazol-5-yl]pyridin, (15.064) 2-Phenylphenol und Salze, (15.065) 3-(4,4,5-Trifluor-3,3-dimethyl-3,4-dihydroisochinolin-1-yl)chinolin, (15.066) 3,4,5-Trichlorpyridin-2,6-dicarbonsäurenitril, (15.067) 3-Chlor-5-(4-chlorphenyl)-4-(2,6-difluorphenyl)-6-methylpyridazin, (15.068) 4-(4-Chlorphenyl)-5-(2,6-difluorphenyl)-3,6-dimethylpyridazin, (15.069) 5-Amino-1,3,4-thiadiazol-2-thiol, (15.070) 5-Chlor-N'-phenyl-N'-(prop-2-in-1-yl)thiophen-2-sulfonohydrazid, (15.071) 5-Fluor-2-[(4-fluorbenzyl)oxy]pyrimidin-4-amin, (15.072) 5-Fluor-2-[(4-methylbenzyl)oxy]pyrimidin-4-amin, (15.073) 5-Methyl-6-octyl[1,2,4]triazolo[1,5-a]pyrimidin-7-amin, (15.074) (2Z)-3-Amino-2-cyano-3-phenylacrylsäureethylester, (15.075) N'-(4-{[3-(4-Chlorbenzyl)-1,2,4-thiadiazol-5-yl]oxy}-2,5-dimethylphenyl)-N-ethyl-N-methylimidoformamid, (15.076) N-(4-Chlorbenzyl)-3-[3-methoxy-4-(prop-2-in-1-yloxy)phenyl]propanamid, (15.077) N-[(4-Chlorphenyl)(cyano)methyl]-3-[3-methoxy-4-(prop-2-in-1-yloxy)phenyl]propanamid, (15.078) N-[(5-Brom-3-chlorpyridin-2-yl)methyl]-2,4-dichlornicotinamid, (15.079) N-[1-(5-Brom-3-chlorpyridin-2-yl)ethyl]-2,4-dichlornicotinamid, (15.080) N-[1-(5-Brom-3-chlorpyridin-2-yl)ethyl]-2-fluor-4-iodnicotinamid, (15.081) N-{(E)-[(Cyclopropylmethoxy)imino][6-(difluormethoxy)-2,3-difluorphenyl]methyl}-2-phenylacetamid, (15.082) N-{(Z)-[(Cyclopropylmethoxy)imino][6-(difluormethoxy)-2,3-difluorphenyl]methyl}-2-phenylacetamid, (15.083) N'-{4-[(3-tert.-Butyl-4-cyano-1,2-thiazol-5-yl)oxy]-2-chlor-5-methylphenyl}-N-ethyl-N-methylimidoformamid, (15.084) N-Methyl-2-(1-{[5-methyl-3-(trifluormethyl)-1H-pyrazol-1-yl]acetyl}piperidin-4-yl)-N-(1,2,3,4-tetrahydronaphthalin-1-yl)-1,3-thiazol-4-carboxamid, (15.085) N-Methyl-2-(1-{[5-methyl-3-(trifluormethyl)-1H-pyrazol-1-yl]acetyl}piperidin-4-yl)-N-[(1R)-1,2,3,4-tetrahydronaphthalin-1-yl]-1,3-thiazol-4-carboxamid, (15.086) N-Methyl-2-(1-{[5-methyl-3-(trifluormethyl)-1H-pyrazol-1-yl]acetyl}piperidin-4-yl)-N-[(1S)-1,2,3,4-tetrahydronaphthalin-1-yl]-1,3-thiazol-4-carboxamid, (15.087) {6-[({[(1-Methyl-1H-tetrazol-5-yl)(phenyl)methylen]amino}oxy)methyl]pyridin-2-yl}-carbamidsäurepentylester, (15.088) Phenazin-1-carbonsäure, (15.089) Chinolin-8-ol, (15.090) Chinolin-8-olsulfat (2:1), (15.091) {6-[({[(1-Methyl-1H-tetrazol-5-yl)(phenyl)methylen]amino}-oxy)methyl]pyridin-2-yl}carbamidsäure-tert.-butylester, (15.092) (5-Brom-2-methoxy-4-methyl-pyridin-3-yl)(2,3,4-trimethoxy-6-methylphenyl)methanon, (15.093) N-[2-(4-{[3-(4-Chlorphenyl)prop-2-in-1-yl]oxy}-3-methoxyphenyl)ethyl]-N2-(methylsulfonyl)valinamid, (15.094) 4-Oxo-4-[(2-phenylethyl)amino]butansäure, (15.095) {6-[({[(Z)-(1-Methyl-1H-tetrazol-5-yl)(phenyl)methylen]amino}oxy)methyl]pyridin-2-yl}carbamidsäurebut-3-in-1-ylester, (15.096) 4-Amino-5-fluorpyrimidin-2-ol (tautomere Form: 4-Amino-5-fluorpyrimidin-2(1H)-on), (15.097) 3,4,5-Trihydroxybenzoesäurepropylester, (15.098) [3-(4-Chlor-2-fluorphenyl)-5-(2,4-difluorphenyl)-1,2-oxazol-4-yl](pyridin-3-yl)methanol, (15.099) (S)-[3-(4-Chlor-2-fluorphenyl)-5-(2,4-difluorphenyl)-1,2-oxazol-4-yl](pyridin-3-yl)methanol, (15.100) (R)-[3-(4-Chlor-2-fluorphenyl)-5-(2,4-difluorphenyl)-1,2-oxazol-4-yl](pyridin-3-yl)methanol, (15.101) 2-Fluor-6-(trifluormethyl)-N-(1,1,3-trimethyl-2,3-dihydro-1H-inden-4-yl)benzamid, (15.102) 2-(6-Benzylpyridin-2-yl)chinazolin, (15.103) 2-[6-(3-Fluor-4-methoxyphenyl)-5-methylpyridin-2-yl]chinazolin, (15.104) 3-(4,4-Difluor-3,3-dimethyl-3,4-dihydroisochinolin-1-yl)chinolin, (15.105) Abscisinsäure, (15.106) N'-[5-Brom-6-(2,3-dihydro-1H-inden-2-yloxy)-2-methylpyridin-3-yl]-N-ethyl-N-methylimidoformamid, (15.107) N'-{5-Brom-6-[1-(3,5-difluorphenyl)ethoxy]-2-methylpyridin-3-yl}-N-ethyl-N-methylimidoformamid, (15.108) N'-{5-Brom-6-[(1R)-1-(3,5-difluorphenyl)ethoxy]-2-methylpyridin-3-yl}-N-ethyl-N-methylimidoformamid, (15.109) N'-{5-Brom-6-[(1S)-1-(3,5-difluorphenyl)ethoxy]-2-methylpyridin-3-yl}-N-ethyl-N-methylimidoformamid, (15.110) N'-{5-Brom-6-[(cis-4-isopropylcyclohexyl)oxy]-2-methylpyridin-3-yl}-N-ethyl-N-methylimidoformamid, (15.111) N'-{5-Brom-6-[(trans-4-isopropylcyclohexyl)oxy]-2-methylpyridin-3-yl}-N-ethyl-N-methylimidoformamid, (15.112) N-Cyclopropyl-3-(difluormethyl)-5-fluor-N-(2-isopropylbenzyl)-1-methyl-1H-pyrazol-4-carboxamid, (15.113) N-Cyclopropyl-N-(2-cyclopropylbenzyl)-3-(difluormethyl)-5-fluor-1-methyl-1H-pyrazol-4-carboxamid, (15.114) N-(2-tert.-Butylbenzyl)-N-cyclopropyl-3-(difluormethyl)-5-fluor-1-methyl-1H-pyrazol-4-carboxamid, (15.115) N-(5-Chlor-2-ethylbenzyl)-N-cyclopropyl-3-(difluormethyl)-5-fluor-1-methyl-1H-pyrazol-4-carboxamid, (15.116) N-(5-Chlor-2-isopropylbenzyl)-N-cyclopropyl-3-(difluormethyl)-5-fluor-1-methyl-1H-pyrazol-4-carboxamid, (15.117) N-Cyclopropyl-3-(difluormethyl)-N-(2-ethyl-5-fluorbenzyl)-5-fluor-1-methyl-1H-pyrazol-4-carboxamid, (15.118) N-Cyclopropyl-3-(difluormethyl)-5-fluor-N-(5-fluor-2-isopropylbenzyl)-1-methyl-1H-pyrazol-4-carboxamid, (15.119) N-Cyclopropyl-N-(2-cyclopropyl-5-fluorbenzyl)-3-(difluormethyl)-5-fluor-1-methyl-1H-pyrazol-4-carboxamid, (15.120) N-(2-Cyclopentyl-5-fluorbenzyl)-N-cyclopropyl-3-(difluormethyl)-5-fluor-1-methyl-1H-pyrazol-4-carboxamid, (15.121) N-Cyclopropyl-3-(difluormethyl)-5-fluor-N-(2-fluor-6-isopropylbenzyl)-1-methyl-1H-pyrazol-4-carboxamid, (15.122) N-Cyclopropyl-3-(difluormethyl)-N-(2-ethyl-5-methylbenzyl)-5-fluor-1-methyl-1H-pyrazol-4-carboxamid, (15.123) N-Cyclopropyl-3-(difluormethyl)-5-fluor-N-(2-isopropyl-5-methylbenzyl)-1-methyl-1H-pyrazol-4-carboxamid, (15.124) N-Cyclopropyl-N-(2-cyclopropyl-5-methylbenzyl)-3-(difluormethyl)-5-fluor-1-methyl-1H-pyrazol-4-carboxamid, (15.125) N-(2-tert.-Butyl-5-methylbenzyl)-N-cyclopropyl-3-(difluormethyl)-5-fluor-1-methyl-1H-pyrazol-4-carboxamid, (15.126) N-[5-Chlor-2-(trifluormethyl)benzyl]-N-cyclopropyl-3-(difluormethyl)-5-fluor-1-methyl-1H-pyrazol-4-carboxamid, (15.127) N-Cyclopropyl-3-(difluormethyl)-5-fluor-1-methyl-N-[5-methyl-2-(trifluormethyl)benzyl]-1H-pyrazol-4-carboxamid, (15.128) N-[2-Chlor-6-(trifluormethyl)benzyl]-N-cyclopropyl-3-(difluormethyl)-5-fluor-1-methyl-1H-pyrazol-4-carboxamid, (15.129) N-[3-Chlor-2-fluor-6-(trifluormethyl)benzyl]-N-cyclopropyl-3-(difluormethyl)-5-fluor-1-methyl-1H-pyrazol-4-carboxamid, (15.130) N-Cyclopropyl-3-(difluormethyl)-N-(2-ethyl-4,5-dimethylbenzyl)-5-fluor-1-methyl-1H-pyrazol-4-carboxamid, (15.131) N-Cyclopropyl-3-(difluormethyl)-5-fluor-N-(2-isopropylbenzyl)-1-methyl-1H-pyrazol-4-carbothioamid, (15.132) N'-(2,5-Dimethyl-4-phenoxyphenyl)-N-ethyl-N-methylimidoformamid, (15.133) N'-{4-[(4,5-Dichlor-1,3-thiazol-2-yl)oxy]-2,5-dimethylphenyl}-N-ethyl-N-methylimidoformamid, (15.134) N-(4-Chlor-2,6-difluorphenyl)-4-(2-chlor-4-fluorphenyl)-1,3-dimethyl-1H-pyrazol-5-amin, (15.135) 9-Fluor-2,2-dimethyl-5-(chinolin-3-yl)-2,3-dihydro-1,4-benzoxazepin, (15.136) 2-{2-Fluor-6-[(8-fluor-2-methylchinolin-3-yl)oxy]phenyl}propan-2-ol, (15.137) 2-{2-[(7,8-Difluor-2-methylchinolin-3-yl)oxy]-6-fluorphenyl}propan-2-ol, (15.138) 4-(2-Chlor-4-fluorphenyl)-N-(2-fluorphenyl)-1,3-dimethyl-1H-pyrazol-5-amin, (15.139) 4-(2-Chlor-4-fluorphenyl)-N-(2,6-difluorphenyl)-1,3-dimethyl-1H-pyrazol-5-amin, (15.140) 4-(2-Chlor-4-fluorphenyl)-N-(2-chlor-6-fluorphenyl)-1,3-dimethyl-1H-pyrazol-5-amin, (15.141) 4-(2-Brom-4-fluorphenyl)-N-(2-chlor-6-fluorphenyl)-1,3-dimethyl-1H-pyrazol-5-amin, (15.142) N-(2-Brom-6-fluorphenyl)-4-(2-chlor-4-fluorphenyl)-1,3-dimethyl-1H-pyrazol-5-amin, (15.143) 4-(2-Brom-4-fluorphenyl)-N-(2-bromphenyl)-1,3-dimethyl-1H-pyrazol-5-amin, (15.144) 4-(2-Brom-4-fluorphenyl)-N-(2-brom-6-fluorphenyl)-1,3-dimethyl-1H-pyrazol-5-amin, (15.145) 4-(2-Brom-4-fluorphenyl)-N-(2-chlorphenyl)-1,3-dimethyl-1H-pyrazol-5-amin, (15.146) N-(2-Bromphenyl)-4-(2-chlor-4-fluorphenyl)-1,3-dimethyl-1H-pyrazol-5-amin, (15.147) 4-(2-Chlor-4-fluorphenyl)-N-(2-chlorphenyl)-1,3-dimethyl-1H-pyrazol-5-amin, (15.148) 4-(2-Brom-4-fluorphenyl)-N-(2,6-difluorphenyl)-1,3-dimethyl-1H-pyrazol-5-amin, (15.149) 4-(2-Brom-4-fluorphenyl)-N-(2-fluorphenyl)-1,3-dimethyl-1H-pyrazol-5-amin, (15.150) N'-(4-{3-[(Difluormethyl)sulfanyl]phenoxy}-2,5-dimethylphenyl)-N-ethyl-N-methylimidoformamid, (15.151) N'-(2,5-Dimethyl-4-{3-[(1,1,2,2-tetrafluorethyl)sulfanyl]phenoxy}phenyl)-N-ethyl-N-methylimidoformamid, (15.152) N'-(2,5-Dimethyl-4-{3-[(2,2,2-trifluorethyl)sulfanyl]phenoxy}phenyl)-N-ethyl-N-methylimidoformamid, (15.153) N'-(2,5-Dimethyl-4-{3-[(2,2,3,3-tetrafluorpropyl)sulfanyl]phenoxy}phenyl)-N-ethyl-N-methylimidoformamid, (15.154) N'-(2,5-Dimethyl-4-{3-[(pentafluorethyl)sulfanyl]phenoxy}phenyl)-N-ethyl-N-methylimidoformamid, (15.155) N'-(4-{[3-(Difluormethoxy)phenyl]sulfanyl}-2,5-dimethylphenyl)-N-ethyl-N-methylimidoformamid, (15.156) N'-(2,5-Dimethyl-4-{[3-(1,1,2,2-tetrafluorethoxy)phenyl]sulfanyl}phenyl)-N-ethyl-N-methylimidoformamid, (15.157) N'-(2,5-Dimethyl-4-{[3-(2,2,2-trifluorethoxy)phenyl]sulfanyl}phenyl)-N-ethyl-N-methylimidoformamid, (15.158) N'-(2,5-Dimethyl-4-{[3-(2,2,3,3-tetrafluorpropoxy)phenyl]sulfanyl}phenyl)-N-ethyl-N-methylimidoformamid, (15.159) N'-(2,5-Dimethyl-4-{[3-(pentafluorethoxy)phenyl]sulfanyl}phenyl)-N-ethyl-N-methylimidoformamid, (15.160) 2-[3,5-Bis(difluormethyl)-1H-pyrazol-1-yl]-1-[4-(4-{5-[2-(prop-2-in-1-yloxy)phenyl]-4,5-dihydro-1,2-oxazol-3-yl}-1,3-thiazol-2-yl)piperidin-1-yl]ethanon, (15.161) 2-[3,5-Bis(difluormethyl)-1H-pyrazol-1-yl]-1-[4-(4-{5-[2-fluor-6-(prop-2-in-1-yloxy)phenyl]-4,5-dihydro-1,2-oxazol-3-yl}-1,3-thiazol-2-yl)piperidin-1-yl]ethanon, (15.162) 2-[3,5-Bis(difluormethyl)-1H-pyrazol-1-yl]-1-[4-(4-{5-[2-chlor-6-(prop-2-in-1-yloxy)phenyl]-4,5-dihydro-1,2-oxazol-3-yl}-1,3-thiazol-2-yl)piperidin-1-yl]ethanon, (15.163) 2-{3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.164) 2-{3-[2-(1-{[3,5-Bis(difluormethyl)-1H-pyrazol-1-yl]-acetyl}piperidin-4-yl)-1,3-thiazol-4-yl]-4,5-dihydro-1,2-oxazol-5-yl}-3-chlorphenylmethansulfonat, (15.165) 2-[3,5-Bis(difluormethyl)-1H-pyrazol-1-yl]-1-[4-(4-{(5S)-5-[2-(prop-2-in-1-yloxy)phenyl]-4,5-dihydro-1,2-oxazol-3-yl}-1,3-thiazol-2-yl)piperidin-1-yl]ethanon, (15.166) 2-[3,5-Bis(difluormethyl)-1H-pyrazol-1-yl]-1-[4-(4-{(5R)-5-[2-(prop-2-in-1-yloxy)phenyl]-4,5-dihydro-1,2-oxazol-3-yl}-1,3-thiazol-2-yl)piperidin-1-yl]ethanon, (15.167) 2-[3,5-Bis(difluormethyl)-1H-pyrazol-1-yl]-1-[4-(4-{(5S)-5-[2-fluor-6-(prop-2-in-1-yloxy)phenyl]-4,5-dihydro-1,2-oxazol-3-yl}-1,3-thiazol-2-yl)piperidin-1-yl]ethanon, (15.168) 2-[3,5-Bis(difluormethyl)-1H-pyrazol-1-yl]-1-[4-(4-{(5R)-5-[2-fluor-6-(prop-2-in-1-yloxy)phenyl]-4,5-dihydro-1,2-oxazol-3-yl}-1,3-thiazol-2-yl)piperidin-1-yl]ethanon, (15.169) 2-[3,5-Bis(difluormethyl)-1H-pyrazol-1-yl]-1-[4-(4-{(5S)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.170) 2-[3,5-Bis(difluormethyl)-1H-pyrazol-1-yl]-1-[4-(4-{(5R)-5-[2-chlor-6-(prop-2-in-1-yloxy)phenyl]-4,5-dihydro-1,2-oxazol-3-yl}-1,3-thiazol-2-yl)piperidin-1-yl]ethanon, (15.171) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.172) 2-{(5R)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}phenylmethansulfonat, (15.173) 2-{(5S)-3-[2-(1-{[3,5-Bis(difluormethyl)-1H-pyrazol-1-yl]acetyl}piperidin-4-yl)-1,3-thiazol-4-yl]-4,5-dihydro-1,2-oxazol-5-yl}-3-chlorphenylmethansulfonat, (15.174) 2-{(5R)-3-[2-(1-{[3,5-Bis(difluormethyl)-1H-pyrazol-1-yl]acelyl}piperidin-4-yl)-1,3-thiazol-4-yl]-4,5-dihydro-1,2-oxazol-5-yl}-3-chlorphenylmethansulfonat.

### Biologische Schädlingsbekämpfungsmittel als Mischungspartner

Die Verbindungen der Formel (I) können mit biologischen Schädlingsbekämpfungsmitteln kombiniert werden.

Biologische Schädlingsbekämpfungsmittel umfassen insbesondere Bakterien, Pilze, Hefen, Pflanzenextrakte, und solche Produkte, die von Mikroorganismen gebildet wurden inklusive Proteine und sekundäre Stoffwechselprodukte.

Biologische Schädlingsbekämpfungsmittel umfassen Bakterien wie sporenbildende Bakterien, wurzelbesiedelnde Bakterien und Bakterien, die als biologische Insektizide, Fungizide oder Nematizide wirken.

Beispiele für solche Bakterien, die als biologische Schädlingsbekämpfungsmittel eingesetzt werden bzw. verwendet werden können, sind:
*Bacillus amyloliquefaciens*, Stamm FZB42 (DSM 231179), oder *Bacillus cereus*, insbesondere *B. cereus* Stamm CNCM I-1562 oder *Bacillus firmus,* Stamm I-1582 (Accession number CNCM I-1582) oder *Bacillus pumilus,* insbesondere Stamm GB34 (Accession No. ATCC 700814) und Stamm QST2808 (Accession No. NRRL B-30087), oder *Bacillus subtilis,* insbesondere Stamm GB03 (Accession No. ATCC SD-1397), oder *Bacillus subtilis* Stamm QST713 (Accession No. NRRL B-21661) oder *Bacillus subtilis* Stamm OST 30002 (Accession No. NRRL B-50421) *Bacillus thuringiensis,* insbesondere *B. thuringiensis* subspecies *israelensis* (serotype H-14), Stamm AM65-52 (Accession No. ATCC 1276), oder *B. thuringiensis subsp. aizawai,* insbesondere Stamm ABTS-1857 (SD-1372), oder *B. thuringiensis subsp. kurstaki* Stamm HD-1, oder *B. thuringiensis subsp. tenebrionis* Stamm NB 176 (SD-5428), *Pasteuria penetrans, Pasteuria spp.* (Rotylenchulus reniformis nematode)-PR3 (Accession Number ATCC SD-5834), *Streptomyces microflavus* Stamm AQ6121 (= QRD 31.013, NRRL B-50550), *Streptomyces galbus* Stamm AQ 6047 (Acession Number NRRL 30232).

Beispiele für Pilze und Hefen, die als biologische Schädlingsbekämpfungsmittel eingesetzt werden bzw. verwendet werden können, sind:
*Beauveria bassiana,* insbesondere Stamm ATCC 74040, *Coniothyrium minitans,* insbesondere Stamm CON/M/91-8 (Accession No. DSM-9660), *Lecanicillium spp.,* insbesondere Stamm HRO LEC 12, *Lecanicillium lecanii,* (ehemals bekannt als *Verticillium lecanii*), insbesondere Stamm KV01, *Metarhizium anisopliae,* insbesondere Stamm F52 (DSM3884/ ATCC 90448), *Metschnikowia fructicola,* insbesondere Stamm NRRL Y-30752, *Paecilomyces fumosoroseus* (neu: *Isaria fumosorosea),* insbesondere Stamm IFPC 200613, oder Stamm Apopka 97 (Accesion No. ATCC 20874), *Paecilomyces lilacinus,* insbesondere *P. lilacinus* Stamm 251 (AGAL 89/030550), *Talaromyces flavus,* insbesondere Stamm V117b, *Trichoderma atroviride,* insbesondere Stamm SC1 (Accession Number CBS 122089), *Trichoderma harzianum,* insbesondere *T. harzianum rifai T39.* (Accession Number CNCM I-952).

Beispiele für Viren, die als biologische Schädlingsbekämpfungsmittel eingesetzt werden bzw. verwendet werden können, sind:
*Adoxophyes orana* (Apfelschalenwickler) Granulosevirus (GV), *Cydia pomonella* (Apfelwickler) Granulosevirus (GV), *Helicoverpa armigera* (Baumwollkapselwurm) Nuklear Polyhedrosis Virus (NPV), *Spodoptera exigua* (Zuckerrübeneule) mNPV, *Spodoptera frugiperda* (Heerwurm) mNPV, *Spodoptera littoralis* (Afrikanischer Baumwollwurm) NPV.

Es sind auch Bakterien und Pilze umfasst, die als 'Inokulant' Pflanzen oder Pflanzenteilen oder Pflanzenorganen beigegeben werden und durch ihre besonderen Eigenschaften das Pflanzenwachstum und die Pflanzengesundheit fördern. Als Beispiele sind genannt:
*Agrobacterium spp., Azorhizobium caulinodans, Azospirillum spp., Azotobacter spp., Bradyrhizobium spp., Burkholderia spp.,* insbesondere *Burkholderia cepacia* (ehemals bekannt als *Pseudomonas cepacia), Gigaspora spp.,* oder *Gigaspora monosporum, Glomus spp., Laccaria spp., Lactobacillus buchneri, Paraglomus spp., Pisolithus tinctorus, Pseudomonas spp., Rhizobium spp.,* insbesondere *Rhizobium trifolii, Rhizopogon spp., Scleroderma spp., Suillus spp., Streptomyces spp..*

Beispiele für Pflanzenextrakte und solche Produkte, die von Mikroorganismen gebildet wurden inklusive Proteine und sekundäre Stoffwechselprodukte, die als biologische Schädlingsbekämpfungsmittel eingesetzt werden bzw. verwendet werden können, sind:
Allium sativum, Artemisia absinthium, Azadirachtin, Biokeeper WP, Cassia nigricans, Celastrus angulatus, Chenopodium anthelminticum, Chitin, Armour-Zen, Dryopteris filix-mas, Equisetum arvense,Fortune Aza, Fungastop, Heads Up (Chenopodium quinoa-Saponinextrakt), Pyrethrum/Pyrethrins, Quassia amara, Quercus, Quillaja, Regalia, "Requiem ™ Insecticide", Rotenon, Ryania/Ryanodine, Symphytum officinale, Tanacetum vulgare, Thymol, Triact 70, TriCon, Tropaeulum majus, Urtica dioica, Veratrin, Viscum album, Brassicacaeen-Extrakt, insbesondere Raps- oder Senfpulver.

### Safener als Mischpartner

Die Verbindungen der Formel (I) können mit Safenern kombiniert werden, wie zum Beispiel Benoxacor, Cloquintocet (-mexyl), Cyometrinil, Cyprosulfamide, Dichlormid, Fenchlorazole (-ethyl), Fenclorim, Flurazole, Fluxofenim, Furilazole, Isoxadifen (-ethyl), Mefenpyr (-diethyl), Naphthalic anhydride, Oxabetrinil, 2-Methoxy-N-({4-[(methylcarbamoyl)amino]phenyl}sulfonyl)benzamid (CAS 129531-12-0), 4-(Dichloroacetyl)-1-oxa-4-azaspiro[4.5]decane (CAS 71526-07-3), 2,2,5-Trimethyl-3-(dichloroacetyl)-1,3-oxazolidine (CAS 52836-31-4).

### Pflanzen und Pflanzenteile

Erfindungsgemäß können alle Pflanzen und Pflanzenteile behandelt werden. Unter Pflanzen werden hierbei alle Pflanzen und Pflanzenpopulationen verstanden wie erwünschte und unerwünschte Wildpflanzen oder Kulturpflanzen (einschließlich natürlich vorkommender Kulturpflanzen), beispielsweise Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben). Kulturpflanzen können Pflanzen sein, die durch konventionelle Züchtungs- und Optimierungsmethoden oder durch biotechnologische und gentechnologische Methoden oder Kombinationen dieser Methoden erhalten werden können, einschließlich der transgenen Pflanzen und einschließlich der durch Sortenschutzrechte schützbaren oder nicht schützbaren Pflanzensorten. Unter Pflanzenteilen sollen alle oberirdischen und unterirdischen Teile und Organe der Pflanzen wie Spross, Blatt, Blüte und Wurzel verstanden werden, wobei beispielhaft Blätter, Nadeln, Stängel, Stämme, Blüten, Fruchtkörper, Früchte und Samen sowie Wurzeln, Knollen und Rhizome aufgeführt werden. Zu den Pflanzenteilen gehört auch Erntegut sowie vegetatives und generatives Vermehrungsmaterial, beispielsweise Stecklinge, Knollen, Rhizome, Ableger und Samen.

Die erfindungsgemäße Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, Sprühen, Verdampfen, Vernebeln, Streuen, Aufstreichen, Injizieren und bei Vermehrungsmaterial, insbesondere bei Saatgut, weiterhin durch ein- oder mehrschichtiges Umhüllen.

Wie bereits oben erwähnt können erfindungsgemäß alle Pflanzen und deren Teile behandelt werden. In einer bevorzugten Ausführungsform werden wild vorkommende oder durch konventionelle biologische Zuchtmethoden wie Kreuzung oder Protoplastenfusion erhaltene Pflanzenarten und Pflanzensorten sowie deren Teile behandelt. In einer weiteren bevorzugten Ausführungsform werden transgene Pflanzen und Pflanzensorten, die durch gentechnologische Methoden gegebenenfalls in Kombination mit konventionellen Methoden erhalten wurden (Genetically Modified Organisms) und deren Teile behandelt. Der Begriff "Teile" bzw. "Teile von Pflanzen" oder "Pflanzenteile" wurde oben erläutert. Besonders bevorzugt werden erfindungsgemäß Pflanzen der jeweils handelsüblichen oder in Gebrauch befindlichen Pflanzensorten behandelt. Unter Pflanzensorten versteht man Pflanzen mit neuen Eigenschaften ("Traits"), die sowohl durch konventionelle Züchtung, durch Mutagenese oder durch rekombinante DNA-Techniken gezüchtet worden sind. Dies können Sorten, Rassen, Bio- und Genotypen sein.

### Transgene Pflanze, Saatgutbehandlung und Integrationsereignisse

Zu den bevorzugten erfindungsgemäß zu behandelnden transgenen (gentechnologisch erhaltenen) Pflanzen bzw. Pflanzensorten gehören alle Pflanzen, die durch die gentechnologische Modifikation genetisches Material erhielten, welches diesen Pflanzen besondere vorteilhafte wertvolle Eigenschaften ("Traits") verleiht. Beispiele für solche Eigenschaften sind besseres Pflanzenwachstum, erhöhte Toleranz gegenüber hohen oder niedrigen Temperaturen, erhöhte Toleranz gegen Trockenheit oder gegen Wasser- bzw. Bodensalzgehalt, erhöhte Blühleistung, erleichterte Ernte, Beschleunigung der Reife, höhere Ernteerträge, höhere Qualität und/oder höherer Ernährungswert der Ernteprodukte, höhere Lagerfähigkeit und/oder Bearbeitbarkeit der Ernteprodukte. Weitere und besonders hervorgehobene Beispiele für solche Eigenschaften sind eine erhöhte Abwehrfähigkeit der Pflanzen gegen tierische und mikrobielle Schädlinge, wie-Insekten, Spinnentiere, Nematoden, Milben, Schnecken, bewirkt z.B. durch in den Pflanzen entstehende Toxine, insbesondere solche, die durch das genetische Material aus Bacillus Thuringiensis (z.B. durch die Gene CryIA(a), CryIA(b), CryIA(c), CryIIA, CryIIIA, CryIIIB2, Cry9c Cry2Ab, Cry3Bb und CryIF sowie deren Kombinationen) in den Pflanzen erzeugt werden, ferner eine erhöhte Abwehrfähigkeit der Pflanzen gegen pflanzenpathogene Pilze, Bakterien und/oder Viren, bewirkt z.B. durch Systemisch Akquirierte Resistenz (SAR), Systemin, Phytoalexine, Elicitoren sowie Resistenzgene und entsprechend exprimierte Proteine und Toxine, sowie eine erhöhte Toleranz der Pflanzen gegen bestimmte herbizide Wirkstoffe, beispielsweise Imidazolinonen, Sulfonylharnstoffen, Glyphosate oder Phosphinotricin (z.B. "PAT"-Gen). Die jeweils die gewünschten Eigenschaften ("Traits") verleihenden Gene können auch in Kombinationen miteinander in den transgenen Pflanzen vorkommen. Als Beispiele transgener Pflanzen werden die wichtigen Kulturpflanzen, wie Getreide (Weizen, Reis, Triticale, Gerste, Roggen, Hafer), Mais, Soja, Kartoffel, Zuckerrüben, Zuckerrohr, Tomaten, Erbsen und andere Gemüsesorten, Baumwolle, Tabak, Raps, sowie Obstpflanzen (mit den Früchten Äpfel, Birnen, Zitrusfrüchten und Weintrauben) erwähnt, wobei Mais, Soja, Weizen, Reis, Kartoffel, Baumwolle, Zuckerrohr, Tabak und Raps besonders hervorgehoben werden. Als Eigenschaften ("Traits") werden besonders hervorgehoben die erhöhte Abwehrfähigkeit der Pflanzen gegen Insekten, Spinnentiere, Nematoden und Schnecken.

### Pflanzenschutz - Behandlungsarten

Die Behandlung der Pflanzen und Pflanzenteile mit den Verbindungen der Formel (I) erfolgt direkt oder durch Einwirkung auf deren Umgebung, Lebensraum oder Lagerraum nach den üblichen Behandlungsmethoden, z.B. durch Tauchen, (Ver-) Spritzen, (Ver-)Sprühen, Berieseln, Verdampfen, Zerstäuben, Vernebeln, (Ver-)Streuen, Verschäumen, Bestreichen, Verstreichen, Injizieren, Gießen (drenchen), Tröpfchenbewässerung und bei Vermehrungsmaterial, insbesondere bei Saatgut, weiterhin durch Trockenbeizen, Nassbeizen, Schlämmbeizen, Inkrustieren, ein- oder mehrschichtiges Umhüllen, usw. Es ist ferner möglich, die Verbindungen der Formel (I) nach dem Ultra-Low-Volume-Verfahren auszubringen oder die Anwendungsform oder die Verbindung der Formel (I) selbst in den Boden zu injizieren.

Eine bevorzugte direkte Behandlung der Pflanzen ist die Blattapplikation, d.h. Verbindungen der Formel (I) werden auf das Blattwerk aufgebracht, wobei die Behandlungsfrequenz und die Aufwandmenge auf den Befallsdruck des jeweiligen Schädlings abgestimmt sein sollte.

Bei systemisch wirksamen Verbindungen gelangen die Verbindungen der Formel (I) auch über das Wurzelwerk in die Pflanzen. Die Behandlung der Pflanzen erfolgt dann durch Einwirkung der Verbindungen der Formel (I) auf den Lebensraum der Pflanze. Das kann beispielsweise durch Drenchen, Einmischen in den Boden oder die Nährlösung sein, d.h. der Standort der Pflanze (z.B. Boden oder hydroponische Systeme) wird mit einer flüssigen Form der Verbindungen der Formel (I) getränkt, oder durch die Bodenapplikation, d.h. die Verbindungen der Formel (I) werden in fester Form, (z.B. in Form eines Granulats) in den Standort der Pflanzen eingebracht. Bei Wasserreiskulturen kann das auch durch Zudosieren der Verbindung der Formel (I) in einer festen Anwendungsform (z.B. als Granulat) in ein überflutetes Reisfeld sein.

### Saatgutbehandlung

Die Bekämpfung von tierischen Schädlingen durch die Behandlung des Saatguts von Pflanzen ist seit langem bekannt und ist Gegenstand ständiger Verbesserungen. Dennoch ergeben sich bei der Behandlung von Saatgut eine Reihe von Problemen, die nicht immer zufriedenstellend gelöst werden können. So ist es erstrebenswert, Verfahren zum Schutz des Saatguts und der keimenden Pflanze zu entwickeln, die das zusätzliche Ausbringen von Schädlingsbekämpfungsmitteln bei der Lagerung, nach der Saat oder nach dem Auflaufen der Pflanzen überflüssig machen oder zumindest deutlich verringern. Es ist weiterhin erstrebenswert, die Menge des eingesetzten Wirkstoffs dahingehend zu optimieren, dass das Saatgut und die keimende Pflanze vor dem Befall durch tierische Schädlinge bestmöglich geschützt werden, ohne jedoch die Pflanze selbst durch den eingesetzten Wirkstoff zu schädigen. Insbesondere sollten Verfahren zur Behandlung von Saatgut auch die intrinsischen insektiziden bzw. nematiziden Eigenschaften schädlingsresistenter bzw. - toleranter transgener Pflanzen einbeziehen, um einen optimalen Schutz des Saatguts und der keimenden Pflanze bei einem minimalen Aufwand an Schädlingsbekämpfungsmitteln zu erreichen.

Die vorliegende Erfindung bezieht sich daher insbesondere auch auf ein Verfahren zum Schutz von Saatgut und keimenden Pflanzen vor dem Befall von Schädlingen, indem das Saatgut mit einer der Verbindungen der Formel (I) behandelt wird. Das erfindungsgemäße Verfahren zum Schutz von Saatgut und keimenden Pflanzen vor dem Befall von Schädlingen umfasst ferner ein Verfahren, in dem das Saatgut gleichzeitig in einem Vorgang oder sequentiell mit einer Verbindung der Formel (I) und Mischungspartner behandelt wird. Es umfasst ferner auch ein Verfahren, in dem das Saatgut zu unterschiedlichen Zeiten mit einer Verbindung der Formel (I) und Mischungspartner behandelt wird.

Die Erfindung bezieht sich ebenfalls auf die Verwendung der Verbindungen der Formel (I) zur Behandlung von Saatgut zum Schutz des Saatguts und der daraus entstehenden Pflanze vor tierischen Schädlingen.

Weiterhin bezieht sich die Erfindung auf Saatgut, welches zum Schutz vor tierischen Schädlingen mit einer Verbindung der Formel (I) behandelt wurde. Die Erfindung bezieht sich auch auf Saatgut, welches zur gleichen Zeit mit einer Verbindung der Formel (I) und Mischungspartner behandelt wurde. Die Erfindung bezieht sich weiterhin auf Saatgut, welches zu unterschiedlichen Zeiten mit einer Verbindung der Formel (I) und Mischungspartner behandelt wurde. Bei Saatgut, welches zu unterschiedlichen Zeiten mit einer Verbindung der Formel (I) und Mischungspartner behandelt wurde, können die einzelnen Substanzen in unterschiedlichen Schichten auf dem Saatgut enthalten sein. Dabei können die Schichten, die eine Verbindung der Formel (I) und Mischungspartner enthalten, gegebenenfalls durch eine Zwischenschicht getrennt sein. Die Erfindung bezieht sich auch auf Saatgut, bei dem eine Verbindung der Formel (I) und Mischungspartner als Bestandteil einer Umhüllung oder als weitere Schicht oder weitere Schichten zusätzlich zu einer Umhüllung aufgebracht sind.

Des Weiteren bezieht sich die Erfindung auf Saatgut, welches nach der Behandlung mit einer Verbindung der Formel (I) einem Filmcoating - Verfahren unterzogen wird, um Staubabrieb am Saatgut zu vermeiden.

Einer der auftretenden Vorteile, wenn eine der Verbindungen der Formel (I) systemisch wirkt, ist es, dass die Behandlung des Saatguts nicht nur das Saatgut selbst, sondern auch die daraus hervorgehenden Pflanzen nach dem Auflaufen vor tierischen Schädlingen schützt. Auf diese Weise kann die unmittelbare Behandlung der Kultur zum Zeitpunkt der Aussaat oder kurz danach entfallen.

Ein weiterer Vorteil ist darin zu sehen, dass durch die Behandlung des Saatguts mit einer Verbindung der Formel (I) Keimung und Auflauf des behandelten Saatguts gefördert werden können.

Ebenso ist es als vorteilhaft anzusehen, dass Verbindungen der Formel (I) insbesondere auch bei transgenem Saatgut eingesetzt werden können.

Verbindungen der Formel (I) können ferner in Kombination mit Mitteln der Signaltechnologie eingesetzt werden, wodurch eine bessere Besiedlung mit Symbionten, wie zum Beispiel Rhizobien, Mycorrhiza und/oder endophytischen Bakterien oder Pilzen, stattfindet und/oder es zu einer optimierten Stickstofffixierung kommt.

Die Verbindungen der Formel (I) eignen sich zum Schutz von Saatgut jeglicher Pflanzensorte, die in der Landwirtschaft, im Gewächshaus, in Forsten oder im Gartenbau eingesetzt wird. Insbesondere handelt es sich dabei um Saatgut von Getreide (z. B. Weizen, Gerste, Roggen, Hirse und Hafer), Mais, Baum-wolle, Soja, Reis, Kartoffeln, Sonnenblume, Kaffee, Tabak, Canola, Raps, Rübe (z.B. Zuckerrübe und Futterrübe), Erdnuss, Gemüse (z. B. Tomate, Gurke, Bohne, Kohlgewächse, Zwiebeln und Salat), Obstpflanzen, Rasen und Zierpflanzen. Besondere Bedeutung kommt der Behandlung des Saatguts von Getreide (wie Weizen, Gerste, Roggen und Hafer), Mais, Soja, Baumwolle, Canola, Raps und Reis zu.

Wie vorstehend bereits erwähnt, kommt auch der Behandlung von transgenem Saatgut mit einer Verbindung der Formel (I) eine besondere Bedeutung zu. Dabei handelt es sich um das Saatgut von Pflanzen, die in der Regel zumindest ein heterologes Gen enthalten, das die Expression eines Polypeptids mit insbesondere insektiziden bzw. nematiziden Eigenschaften steuert. Die heterologen Gene in transgenem Saatgut können dabei aus Mikro-organismen wie Bacillus, Rhizobium, Pseudomonas, Serratia, Trichoderma, Clavibacter, Glomus oder Gliocladium stammen. Die vorliegende Erfindung eignet sich besonders für die Behandlung von trans-genem Saatgut, das zumindest ein heterologes Gen enthält, das aus Bacillus sp. stammt. Besonders bevorzugt handelt es sich dabei um ein heterologes Gen, das aus Bacillus thuringiensis stammt.

Im Rahmen der vorliegenden Erfindung wird die Verbindung der Formel (I) auf das Saatgut aufgebracht. Vorzugsweise wird das Saatgut in einem Zustand behandelt, in dem es so stabil ist, dass keine Schäden bei der Behandlung auftreten. Im Allgemeinen kann die Behandlung des Saatguts zu jedem Zeitpunkt zwischen der Ernte und der Aussaat erfolgen. Üblicherweise wird Saatgut verwendet, das von der Pflanze getrennt und von Kolben, Schalen, Stängeln, Hülle, Wolle oder Fruchtfleisch befreit wurde. So kann zum Beispiel Saatgut verwendet werden, das geerntet, gereinigt und bis zu einem lagerfähigen Feuchtigkeitsgehalt getrocknet wurde. Alternativ kann auch Saatgut verwendet werden, das nach dem Trocknen z.B. mit Wasser behandelt und dann erneut getrocknet wurde, zum Beispiel Priming. Im Falle von Reissaatgut ist es auch möglich Saatgut zu verwenden, das zum Beispiel in Wasser bis zu einem bestimmten Stadium vorgequollen wurde (pigeon breast Stadium), was zu einer verbesserten Keimung und zu einem gleichmäßigeren Auflaufen führt.

Im Allgemeinen muss bei der Behandlung des Saatguts darauf geachtet werden, dass die Menge der auf das Saatgut aufgebrachten Verbindung der Formel (I) und/oder weiterer Zusatzstoffe so gewählt wird, dass die Keimung des Saatguts nicht beeinträchtigt bzw. die daraus hervorgehende Pflanze nicht geschädigt wird. Dies ist vor allem bei Wirkstoffen zu beachten, die in bestimmten Aufwandmengen phytotoxische Effekte zeigen können.

Die Verbindungen der Formel (I) werden in der Regel in Form einer geeigneten Formulierung auf das Saatgut aufgebracht. Geeignete Formulierungen und Verfahren für die Saatgutbehandlung sind dem Fachmann bekannt.

Die Verbindungen der Formel (I) können in die üblichen Beizmittel-Formulierungen überführt werden, wie Lösungen, Emulsionen, Suspensionen, Pulver, Schäume, Slurries oder andere Hüllmassen für Saatgut, sowie ULV-Formulierungen.

Diese Formulierungen werden in bekannter Weise hergestellt, indem man Verbindungen der Formel (I) mit üblichen Zusatzstoffen vermischt, wie zum Beispiel übliche Streckmittel sowie Lösungs- oder Verdünnungsmittel, Farbstoffe, Netzmittel, Dispergiermittel, Emulgatoren, Entschäumer, Konservierungsmittel, sekundäre Verdickungsmittel, Kleber, Gibberelline und auch Wasser.

Als Farbstoffe, die in den erfindungsgemäß verwendbaren Beizmittel-Formulierungen enthalten sein können, kommen alle für derartige Zwecke üblichen Farbstoffe in Betracht. Dabei sind sowohl in Wasser wenig lösliche Pigmente als auch in Wasser lösliche Farbstoffe verwendbar. Als Beispiele genannt seien die unter den Bezeichnungen Rhodamin B, C.I. Pigment Red 112 und C.I. Solvent Red 1 bekannten Farbstoffe.

Als Netzmittel, die in den erfindungsgemäß verwendbaren Beizmittel-Formulierungen enthalten sein können, kommen alle zur Formulierung von agrochemischen Wirkstoffen üblichen, die Benetzung fördernden Stoffe in Frage. Vorzugsweise verwendbar sind Alkylnaphthalin-Sulfonate, wie Diisopropyl- oder Diisobutyl-naphthalin-Sulfonate.

Als Dispergiermittel und/oder Emulgatoren, die in den erfindungsgemäß verwendbaren Beizmittel-Formulierungen enthalten sein können, kommen alle zur Formulierung von agrochemischen Wirkstoffen üblichen nichtionischen, anionischen und kationischen Dispergiermittel in Betracht. Vor-zugsweise verwendbar sind nichtionische oder anionische Dispergiermittel oder Gemische von nichtionischen oder anionischen Dispergiermitteln. Als geeignete nichtionische Dispergiermittel sind insbesondere Ethylenoxid-Propylenoxid Blockpolymere, Alkylphenolpolyglykolether sowie Tristryrylphenolpolyglykolether und deren phosphatierte oder sulfatierte Derivate zu nennen. Geeignete anionische Dispergiermittel sind insbesondere Ligninsulfonate, Polyacrylsäuresalze und Arylsulfonat-Formaldehydkondensate.

Als Entschäumer können in den erfindungsgemäß verwendbaren Beizmittel-Formulierungen alle zur Formulierung von agrochemischen Wirkstoffen üblichen schaumhemmenden Stoffe enthalten sein. Vorzugsweise verwendbar sind Silikonentschäumer und Magnesiumstearat.

Als Konservierungsmittel können in den erfindungsgemäß verwendbaren Beizmittel-Formulierungen alle für derartige Zwecke in agrochemischen Mitteln einsetzbaren Stoffe vorhanden sein. Beispielhaft genannt seien Dichlorophen und Benzylalkoholhemiformal.

Als sekundäre Verdickungsmittel, die in den erfindungsgemäß verwendbaren Beizmittel-Formulierungen enthalten sein können, kommen alle für derartige Zwecke in agrochemischen Mitteln einsetzbaren Stoffe in Frage. Vorzugsweise in Betracht kommen Cellulosederivate, Acrylsäurederivate, Xanthan, modifizierte Tone und hochdisperse Kieselsäure.

Als Kleber, die in den erfindungsgemäß verwendbaren Beizmittel-Formulierungen enthalten sein können, kommen alle üblichen in Beizmitteln einsetzbaren Bindemittel in Frage. Vorzugsweise genannt seien Polyvinylpyrrolidon, Polyvinylacetat, Polyvinylalkohol und Tylose.

Als Gibberelline, die in den erfindungsgemäß verwendbaren Beizmittel-Formulierungen enthalten sein können, kommen vorzugsweise die Gibberelline A1, A3 (= Gibberellinsäure), A4 und A7 infrage, be-sonders bevorzugt verwendet man die Gibberellinsäure. Die Gibberelline sind bekannt (vgl. R. Wegler "Chemie der Pflanzenschutz- und Schädlingsbekämpfungsmittel", Bd. 2, Springer Verlag, 1970, S. 401-412).

Die erfindungsgemäß verwendbaren Beizmittel-Formulierungen können entweder direkt oder nach vorherigem Verdünnen mit Wasser zur Behandlung von Saatgut der verschiedensten Art eingesetzt werden. So lassen sich die Konzentrate oder die daraus durch Verdünnen mit Wasser erhältlichen Zubereitungen einsetzen zur Beizung des Saatgutes von Getreide, wie Weizen, Gerste, Roggen, Hafer und Triticale, sowie des Saatgutes von Mais, Reis, Raps, Erbsen, Bohnen, Baumwolle, Sonnenblumen, Soja und Rüben oder auch von Gemüsesaatgut der verschiedensten Natur. Die erfindungsgemäß verwendbaren Beizmittel-Formulierungen oder deren verdünnte Anwendungsformen können auch zum Beizen von Saatgut transgener Pflanzen eingesetzt werden.

Zur Behandlung von Saatgut mit den erfindungsgemäß verwendbaren Beizmittel-Formulierungen oder daraus hergestellten Anwendungsformen kommen alle üblicherweise für die Beizung einsetzbaren Mischgeräte in Betracht. Im einzelnen geht man bei der Beizung so vor, dass man das Saatgut in einen Mischer im diskontinuierlichem oder kontinuierlichem Betrieb gibt, die jeweils gewünschte Menge an Beizmittel-Formulierungen entweder als solche oder nach vorherigem Verdünnen mit Wasser hinzufügt und bis zur gleichmäßigen Verteilung der Formulierung auf dem Saatgut mischt. Gegebenenfalls schließt sich ein Trocknungsvorgang an.

Die Aufwandmenge an den erfindungsgemäß verwendbaren Beizmittel-Formulierungen kann innerhalb eines größeren Bereiches variiert werden. Sie richtet sich nach dem jeweiligen Gehalt der Verbindungen der Formel (I) in den Formulierungen und nach dem Saatgut. Die Aufwandmengen bei der Verbindung der Formel (I) liegen im Allgemeinen zwischen 0,001 und 50 g pro Kilogramm Saatgut, vorzugsweise zwischen 0,01 und 15 g pro Kilogramm Saatgut.

### Tiergesundheit

Auf dem Gebiet der Tiergesundheit, d.h. dem Gebiet der Tiermedizin, sind die Verbindungen der Formel (I) gegen Tierparasiten, insbesondere Ektoparasiten oder Endoparasiten, wirksam. Der Begriff Endoparasiten umfasst insbesondere Helminthen und Protozoa wie Kokzidien. Ektoparasiten sind typischerweise und bevorzugt Arthropoden, insbesondere Insekten und Akariden.

Auf dem Gebiet der Tiermedizin eignen sich die Verbindungen der Formel (I), die eine günstige Toxizität gegenüber Warmblütern aufweisen, für die Bekämpfung von Parasiten, die in der Tierzucht und Tierhaltung bei Nutztieren, Zuchttieren, Zootieren, Laboratoriumstieren, Versuchstieren und Haustieren auftreten. Sie sind gegen alle oder einzelne Entwicklungsstadien der Parasiten wirksam.

Zu den landwirtschaftlichen Nutztieren zählen zum Beispiel Säugetiere wie Schafe, Ziegen, Pferde, Esel, Kamele, Büffel, Kaninchen, Rentiere, Damhirsche und insbesondere Rinder und Schweine; Geflügel wie Truthähne, Enten, Gänse und insbesondere Hühner; Fische und Krustentiere, z.B. in der Aquakultur und auch Insekten wie Bienen.

Zu den Haustieren zählen zum Beispiel Säugetiere wie Hamster, Meerschweinchen, Ratten, Mäuse, Chinchillas, Frettchen und insbesondere Hunde, Katzen, Stubenvögel, Reptilien, Amphibien und Aquariumfische.

Gemäß einer bevorzugten Ausführungsform werden die Verbindungen der Formel (I) an Säugetiere verabreicht.

Gemäß einer weiteren bevorzugten Ausführungsform werden die Verbindungen der Formel (I) an Vögel, nämlich Stubenvögel und insbesondere Geflügel, verabreicht.

Durch Verwendung der Verbindungen der Formel (I) für die Bekämpfung von Tierparasiten sollen Krankheit, Todesfälle und Leistungsminderungen (bei Fleisch, Milch, Wolle, Häuten, Eiern, Honig und dergleichen) verringert bzw. vorgebeugt werden, so dass eine wirtschaftlichere und einfachere Tierhaltung ermöglicht wird und ein besseres Wohlbefinden der Tiere erzielbar ist.

In Bezug auf das Gebiet der Tiergesundheit bedeutet der Begriff "Bekämpfung" oder "bekämpfen", dass durch die Verbindungen der Formel (I) wirksam das Auftreten des jeweiligen Parasiten in einem Tier, das mit solchen Parasiten in einem harmlosen Ausmaß infiziert ist, reduziert werden kann. Genauer gesagt bedeutet "bekämpfen" im vorliegenden Zusammenhang, dass die Verbindung der Formel (I) den jeweiligen Parasiten abtöten, sein Wachstum verhindern oder seine Vermehrung verhindern kann.

### Zu den Arthropoden zählen:

aus der Ordnung Anoplurida, zum Beispiel Haematopinus spp., Linognathus spp., Pediculus spp., Phtirus spp., Solenopotes spp.; aus der Ordnung Mallophagida und den Unterordnungen Amblycerina and Ischnocerina, zum Beispiel Trimenopon spp., Menopon spp., Trinoton spp., Bovicola spp., Werneckiella spp., Lepikentron spp., Damalina spp., Trichodectes spp., Felicola spp.; aus der Ordnung Diptera und den Unterordnungen Nematocerina und Brachycerina, zum Beispiel Aedes spp., Anopheles spp., Culex spp., Simulium spp., Eusimulium spp., Phlebotomus spp., Lutzomyia spp., Culicoides spp., Chrysops spp., Odagmia spp., Wilhelmia spp., Hybomitra spp., Atylotus spp., Tabanus spp., Haematopota spp., Philipomyia spp., Braula spp., Musca spp., Hydrotaea spp., Stomoxys spp., Haematobia spp., Morellia spp., Fannia spp., Glossina spp., Calliphora spp., Lucilia spp., Chrysomyia spp., Wohlfahrtia spp., Sarcophaga spp., Oestrus spp., Hypoderma spp., Gasterophilus spp., Hippobosca spp., Lipoptena spp., Melophagus spp., Rhinoestrus spp., Tipula spp.; aus der Ordnung Siphonapterida, zum Beispiel Pulex spp., Ctenocephalides spp., Tunga spp., Xenopsylla spp., Ceratophyllus spp.;
aus der Ordnung Heteropterida, zum Beispiel Cimex spp., Triatoma spp., Rhodnius spp., Panstrongylus spp.; sowie Lästlinge und Hygieneschädlinge aus der Ordnung Blattarida.

### Weiterhin zählen zu den Arthropoden:

Aus der Unterklasse Akari (Acarina) und der Ordnung Metastigmata, zum Beispiel aus der Familie Argasidae, wie Argas spp., Ornithodorus spp., Otobius spp., aus der Familie Ixodidae, wie Ixodes spp., Amblyomma spp., Rhipicephalus (Boophilus) spp. Dermacentor spp., Haemophysalis spp., Hyalomma spp., Rhipicephalus spp. (die ursprüngliche Gattung der mehrwirtigen Zecken); aus der Ordnung Mesostigmata, wie Dermanyssus spp., Ornithonyssus spp., Pneumonyssus spp., Raillietia spp., Pneumonyssus spp., Sternostoma spp., Varroa spp., Acarapis spp.; aus der Ordnung Actinedida (Prostigmata), zum Beispiel Acarapis spp., Cheyletiella spp., Ornithocheyletia spp., Myobia spp., Psorergates spp., Demodex spp., Trombicula spp., Neotrombiculla spp., Listrophorus spp.; und aus der Ordnung Acaridida (Astigmata), zum Beispiel Acarus spp., Tyrophagus spp., Caloglyphus spp., Hypodectes spp., Pterolichus spp., Psoroptes spp., Chorioptes spp., Otodectes spp., Sarcoptes spp., Notoedres spp., Knemidocoptes spp., Cytodites spp., Laminosioptes spp..

### Zu parasitären Protozoen zählen:

Mastigophora (Flagellata), wie zum Beispiel Trypanosomatidae, zum Beispiel Trypanosoma b. brucei, T.b. gambiense, T.b. rhodesiense, T. congolense, T. cruzi, T. evansi, T. equinum, T. lewisi, T. percae, T. simiae, T. vivax, Leishmania brasiliensis, L. donovani, L. tropica, wie zum Beispiel Trichomonadidae, zum Beispiel Giardia lamblia, G. canis;
Sarcomastigophora (Rhizopoda), wie Entamoebidae, zum Beispiel Entamoeba histolytica, Hartmanellidae, zum Beispiel Acanthamoeba sp., Harmanella sp.;
Apicomplexa (Sporozoa), wie Eimeridae, zum Beispiel Eimeria acervulina, E. adenoides, E. alabamensis, E. anatis, E. anserina, E. arloingi, E. ashata, E. auburnensis, E. bovis, E. brunetti, E. canis, E. chinchillae, E. clupearum, E. columbae, E. contorta, E. crandalis, E. debliecki, E. dispersa, E. ellipsoidales, E. falciformis, E. faurei, E. flavescens, E. gallopavonis, E. hagani, E. intestinalis, E. iroquoina, E. irresidua, E. labbeana, E. leucarti, E. magna, E. maxima, E. media, E. meleagridis, E. meleagrimitis, E. mitis, E. necatrix, E. ninakohlyakimovae, E. ovis, E. parva, E. pavonis, E. perforans, E. phasani, E. piriformis, E. praecox, E. residua, E. scabra, E. spec., E. stiedai, E. suis, E. tenella, E. truncata, E. truttae, E. zuernii, Globidium spec., Isospora belli, I. canis, I. felis, I. ohioensis, I. rivolta, I. spec., I. suis, Cystisospora spec., Cryptosporidium spec., insbesondere C. parvum; wie Toxoplasmadidae, zum Beispiel Toxoplasma gondii, Hammondia heydornii, Neospora caninum, Besnoitia besnoitii; wie Sarcocystidae, zum Beispiel Sarcocystis bovicanis, S. bovihominis, S. ovicanis, S. ovifelis, S. neurona, S. spec., S. suihominis, wie Leucozoidae, zum Beispiel Leucozytozoon simondi, wie Plasmodiidae, zum Beispiel Plasmodium berghei, P. falciparum, P. malariae, P. ovale, P. vivax, P. spec., wie Piroplasmea, zum Beispiel Babesia argentina, B. bovis, B. canis, B. spec., Theileria parva, Theileria spec., wie Adeleina, zum Beispiel Hepatozoon canis, H. spec..

Zu pathogenen Endoparasiten, bei denen es sich um Helminthen handelt, zählen Plattwürmer (z.B. Monogenea, Cestodes und Trematodes), Rundwürmer, Acanthocephala und Pentastoma. Dazu zählen:
Monogenea: z.B.: Gyrodactylus spp., Dactylogyrus spp., Polystoma spp.;
Cestodes: aus der Ordnung Pseudophyllidea zum Beispiel: Diphyllobothrium spp., Spirometra spp., Schistocephalus spp., Ligula spp., Bothridium spp., Diplogonoporus spp.;
aus der Ordnung Cyclophyllida zum Beispiel: Mesocestoides spp., Anoplocephala spp., Paranoplocephala spp., Moniezia spp., Thysanosoma spp., Thysaniezia spp., Avitellina spp., Stilesia spp., Cittotaenia spp., Andyra spp., Bertiella spp., Taenia spp., Echinococcus spp., Hydatigera spp., Davainea spp., Raillietina spp., Hymenolepis spp., Echinolepis spp., Echinocotyle spp., Diorchis spp., Dipylidium spp., Joyeuxiella spp., Diplopylidium spp.;
Trematodes: aus der Klasse Digenea zum Beispiel: Diplostomum spp., Posthodiplostomum spp., Schistosoma spp., Trichobilharzia spp., Ornithobilharzia spp., Austrobilharzia spp., Gigantobilharzia spp., Leucochloridium spp., Brachylaima spp., Echinostoma spp., Echinoparyphium spp., Echinochasmus spp., Hypoderaeum spp., Fasciola spp., Fascioloides spp., Fasciolopsis spp., Cyclocoelum spp., Typhlocoelum spp., Paramphistomum spp., Calicophoron spp., Cotylophoron spp., Gigantocotyle spp., Fischoederius spp., Gastrothylacus spp., Notocotylus spp., Catatropis spp., Plagiorchis spp., Prosthogonimus spp., Dicrocoelium spp., Eurytrema spp., Troglotrema spp., Paragonimus spp., Collyriclum spp., Nanophyetus spp., Opisthorchis spp., Clonorchis spp. Metorchis spp., Heterophyes spp., Metagonimus spp.;
Rundwürmer: Trichinellida zum Beispiel: Trichuris spp., Capillaria spp., Paracapillaria spp., Eucoleus spp., Trichomosoides spp., Trichinella spp.;
aus der Ordnung Tylenchida zum Beispiel: Micronema spp., Strongyloides spp.;
aus der Ordnung Rhabditida zum Beispiel: Strongylus spp., Triodontophorus spp., Oesophagodontus spp., Trichonema spp., Gyalocephalus spp., Cylindropharynx spp., Poteriostomum spp., Cyclococercus spp., Cylicostephanus spp., Oesophagostomum spp., Chabertia spp., Stephanurus spp., Ancylostoma spp., Uncinaria spp., Necator spp., Bunostomum spp., Globocephalus spp., Syngamus spp., Cyathostoma spp., Metastrongylus spp., Dictyocaulus spp., Muellerius spp., Protostrongylus spp., Neostrongylus spp., Cystocaulus spp., Pneumostrongylus spp., Spicocaulus spp., Elaphostrongylus spp. Parelaphostrongylus spp., Crenosoma spp., Paracrenosoma spp., Oslerus spp., Angiostrongylus spp., Aelurostrongylus spp., Filaroides spp., Parafilaroides spp., Trichostrongylus spp., Haemonchus spp., Ostertagia spp., Teladorsagia spp., Marshallagia spp., Cooperia spp., Nippostrongylus spp., Heligmosomoides spp., Nematodirus spp., Hyostrongylus spp., Obeliscoides spp., Amidostomum spp., Ollulanus spp.;
aus der Ordnung Spirurida zum Beispiel: Oxyuris spp., Enterobius spp., Passalurus spp., Syphacia spp., Aspiculuris spp., Heterakis spp.; Ascaris spp., Toxascaris spp., Toxocara spp., Baylisascaris spp., Parascaris spp., Anisakis spp., Ascaridia spp.; Gnathostoma spp., Physaloptera spp., Thelazia spp., Gongylonema spp., Habronema spp., Parabronema spp., Draschia spp., Dracunculus spp.; Stephanofilaria spp., Parafilaria spp., Setaria spp., Loa spp., Dirofilaria spp., Litomosoides spp., Brugia spp., Wuchereria spp., Onchocerca spp., Spirocerca spp.;
Acanthocephala: aus der Ordnung Oligacanthorhynchida z.B: Macracanthorhynchus spp., Prosthenorchis spp.; aus der Ordnung Polymorphida zum Beispiel: Filicollis spp.; aus der Ordnung Moniliformida zum Beispiel: Moniliformis spp.;
aus der Ordnung Echinorhynchida zum Beispiel Acanthocephalus spp., Echinorhynchus spp., Leptorhynchoides spp.;
Pentastoma: aus der Ordnung Porocephalida zum Beispiel Linguatula spp..

Auf dem Gebiet der Tiermedizin und der Tierhaltung erfolgt die Verabreichung der Verbindungen der Formel (I) nach allgemein fachbekannten Verfahren, wie enteral, parenteral, dermal oder nasal in Form von geeigneten Präparaten. Die Verabreichung kann prophylaktisch oder therapeutisch erfolgen.

So bezieht sich eine Ausführungsform der vorliegenden Erfindung auf die Verwendung einer Verbindung der Formel (I) als Arzneimittel.

Ein weiterer Aspekt bezieht sich auf die Verwendung einer Verbindung der Formel (I) als Antiendoparasitikum, insbesondere als ein Helminthizid oder ein Mittel gegen Protozoen. Verbindungen der Formel (I) eignen sich für die Verwendung als Antiendoparasitikum, insbesondere als ein Helminthizid oder Mittel gegen Protozoen, beispielsweise in der Tierzucht, in der Tierhaltung, in Ställen und auf dem Hygienesektor.

Ein weiterer Aspekt wiederum betrifft die Verwendung einer Verbindung der Formel (I) als Antiektoparasitikum, insbesondere ein Arthropodizid wie ein Insektizid oder ein Akarizid Ein weiterer Aspekt betrifft die Verwendung einer Verbindung der Formel (I) als Antiektoparasitikum, insbesondere ein Arthropodizid wie ein Insektizid oder Akarizid, zum Beispiel in der Tierhaltung, in der Tierzucht, in Ställen oder auf dem Hygienesektor.

### Anthelminthische Mischungspartner

Beispielhaft seien folgende anthelmintische Mischungspartner genannt:
Anthelminthische Wirkstoffe, einschließlich trematicide und cestocide Wirkstoffe:
aus der Klasse der **macrocyclischen Lactone,** z. B.: Abamectin, Doramectin, Emamectin, Eprinomectin, Ivermectin, Milbemycin, Moxidectin, Nemadectin, Selamectin;
aus der Klasse der **Benzimidazole** und **Probenzimidazole,** z. B.: Albendazol, Albendazol- Sulfoxid, Cambendazol, Cyclobendazol, Febantel, Fenbendazol, Flubendazol, Mebendazol, Netobimin, Oxfendazol, Oxibendazol, Parbendazol, Thiabendazol, Thiophanat, Triclabendazol;
aus der Klasse der **Cyclooctadepsipeptide,** z. B.: Emodepsid, PF1022;
aus der Klasse der **Aminoacetonitril-Derivate,** z. B.: Monepantel;
aus der Klasse der **Tetrahydropyrimidine,** z. B.: Morantel, Pyrantel, Oxantel;
aus der Klasse der **Imidazothiazole,** z. B.: Butamisol, Levamisol, Tetramisol;
aus der Klasse der **Salicylanilide,** z. B.: Bromoxanid, Brotianid, Clioxanid, Closantel, Niclosamid, Oxyclozanid, Rafoxanid, Tribromsalan;
aus der Klasse der **Paraherquamide,** z. B.: Derquantel, Paraherquamid;
aus der Klasse der **Aminophenylamidine,** z. B.: Amidantel, deacyliertes Amidantel (dAMD), Tribendimidin;
aus der Klasse der **Organophosphate,** z. B.: Coumaphos, Crufomat, Dichlorvos, Haloxon, Naphthalofos, Trichlorfon;
aus der Klasse der **substituierten Phenole,** z. B.: Bithionol, Disophenol, Hexachlorophen, Niclofolan, Meniclopholan, Nitroxynil;
aus der Klasse der **Piperazinone,** z. B.: Praziquantel, Epsiprantel;
aus anderen **diversen Klassen,** z. B.: Amoscanat, Bephenium, Bunamidin, Clonazepam, Clorsulon, Diamfenetid, Dichlorophen, Diethylcarbamazin, Emetin, Hetolin, Hycanthon, Lucanthon, Miracil, Mirasan, Niclosamid, Niridazol, Nitroxynil, Nitroscanat, Oltipraz, Omphalotin, Oxamniquin, Paromomycin, Piperazin, Resorantel.

### Vektorkontrolle

Die Verbindungen der Formel (I) können auch in der Vektorkontrolle eingesetzt werden. Ein Vektor im Sinne der vorliegenden Erfindung ist ein Arthropode, insbesondere ein Insekt oder Arachnide, der in der Lage ist, Krankheitserreger wie z. B. Viren, Würmer, Einzeller und Bakterien aus einem Reservoir (Pflanze, Tier, Mensch, etc.) auf einen Wirt zu übertragen. Die Krankheitserreger können entweder mechanisch (z.B. Trachoma durch nicht-stechende Fliegen) auf einem Wirt, oder nach Injektion (z.B. Malaria-Parasiten durch Mücken) in einen Wirt übertragen werden.

Beispiele für Vektoren und die von ihnen übertragenen Krankheiten bzw. Krankheitserreger sind:
1) Mücken
   - Anopheles: Malaria, Filariose;
   - Culex: Japanische Encephalitis, Filariasis, weitere virale Erkrankungen, Übertragung von Würmern;
   - Aedes: Gelbfieber, Dengue-Fieber, Filariasis, weitere virale Erkrankungen;
   - Simulien: Übertragung von Würmern insbesondere Onchocerca volvulus;
2) Läuse: Hautinfektionen, Fleckfieber (epidemic typhus);
3) Flöhe: Pest, endemisches Fleckfieber;
4) Fliegen: Schlafkrankheit (Trypanosomiasis); Cholera, weitere bakterielle Erkrankungen;
5) Milben: Acariose, Fleckfieber, Rickettsipocken, Tularämie, Saint-Louis-Enzephalitis, virale Hirnhautentzündung (FSME), Krim-Kongo-Fieber, Borreliose;
6) Zecken: Borelliosen wie Borrelia duttoni, Frühsommer-Meningoenzephalitis, Q-Fieber (Coxiella burnetii), Babesien (Babesia canis canis).

Beispiele für Vektoren im Sinne der vorliegenden Erfindung sind Insekten wie Aphiden, Fliegen, Zikaden oder Thripse, die Pflanzenviren auf Pflanzen übertragen können. Weitere Vektoren, die Pflanzenviren übertragen können, sind Spinnmilben, Läuse, Käfer und Nematoden.

Weitere Beispiele für Vektoren im Sinne der vorliegenden Erfindung sind Insekten und Arachniden wie Mücken, insbesondere der Gattungen Aedes, Anopheles, z.B. A. gambiae, A. arabiensis, A. funestus, A. dirus (Malaria) und Culex, Läuse, Flöhe, Fliegen, Milben und Zecken, die Krankheitserreger auf Tiere und/oder Menschen übertragen können.

Eine Vektorkontrolle ist auch möglich, wenn die Verbindungen der Formel (I) Resistenz-brechend sind.

Verbindungen der Formel (I) sind zur Verwendung in der Prävention von Krankheiten bzw. vor Krankheitserregern, die durch Vektoren übertragen werden, geeignet. Somit ist ein weiterer Aspekt der vorliegenden Erfindung die Verwendung von Verbindungen der Formel (I) zur Vektorkontrolle, z.B. in der Landwirtschaft, im Gartenbau, in Forsten, in Gärten und Freizeiteinrichtungen sowie im Vorrats- und Materialschutz.

### Schutz von technischen Materialen

Die Verbindungen der Formel (I) eignen sich zum Schutz von technischen Materialien gegen Befall oder Zerstörung durch Insekten, z.B. aus der Ordnung Coleoptera, Hymenoptera, Isoptera, Lepidoptera, Psocoptera und Zygentoma.

Unter technischen Materialien sind im vorliegenden Zusammenhang nicht lebende Materialien zu verstehen, wie vorzugsweise Kunststoffe, Klebstoffe, Leime, Papiere und Kartone, Leder, Holz, Holzverarbeitungsprodukte und Anstrichmittel. Die Anwendung der Erfindung zum Schutz von Holz ist besonders bevorzugt.

In einer weiteren Ausfuhrungsform werden die Verbindungen der Formel (I) zusammen mit mindestens einem weiteren Insektizid und/oder mindestens einem Fungizid eingesetzt.

In einer weiteren Ausfuhrungsform liegen die Verbindungen der Formel (I) als ein anwendungsfertiges (ready-to-use) Schädlingsbekämpfungsmittel vor, d.h., es kann ohne weitere Änderungen auf das entsprechende Material aufgebracht werden. Als weitere Insektizide oder als Fungizide kommen insbesondere die oben genannten in Frage.

Überraschenderweise wurde auch gefunden, dass die Verbindungen der Formel (I) zum Schutz vor Bewuchs von Gegenständen, insbesondere von Schiffskörpern, Sieben, Netzen, Bauwerken, Kaianlagen und Signalanlagen, welche mit See- oder Brackwasser in Verbindung kommen, verwendet werden können. Gleichfalls können die Verbindungen der Formel (I) allein oder in Kombinationen mit anderen Wirkstoffen als Antifouling-Mittel eingesetzt werden.

### Bekämpfung von tierischen Schädlingen auf dem Hygienesektor

Die Verbindungen der Formel (I) eignen sich zur Bekämpfung von tierischen Schädlingen auf dem Hygienesektor. Insbesondere kann die Erfindung im Haushalts-, Hygiene- und Vorratsschutz verwendet werden, vor allem zur Bekämpfung von Insekten, Spinnentieren und Milben, die in geschlossenen Räumen, wie beispielsweise Wohnungen, Fabrikhallen, Büros, Fahrzeugkabinen vorkommen. Zur Bekämpfung der tierischen Schädlinge werden die Verbindungen der Formel (I) allein oder in Kombination mit anderen Wirk- und/oder Hilfsstoffen verwendet. Bevorzugt werden sie in Haushaltsinsektizid-Produkten verwendet. Die Verbindungen der Formel (I) sind gegen sensible und resistente Arten sowie gegen alle Entwicklungsstadien wirksam.

Zu diesen Schädlingen gehören beispielsweise Schädlinge aus der Klasse Arachnida, aus den Ordnungen Scorpiones, Araneae und Opiliones, aus den Klassen Chilopoda und Diplopoda, aus der Klasse Insecta die Ordnung Blattodea, aus den Ordnungen Coleoptera, Dermaptera, Diptera, Heteroptera, Hymenoptera, Isoptera, Lepidoptera, Phthiraptera, Psocoptera, Saltatoria oder Orthoptera, Siphonaptera und Zygentoma und aus der Klasse Malacostraca die Ordnung Isopoda.

Die Anwendung erfolgt beispielsweise in Aerosolen, drucklosen Sprühmitteln, z.B. Pump- und Zerstäubersprays, Nebelautomaten, Foggern, Schäumen, Gelen, Verdampferprodukten mit Verdampferplättchen aus Cellulose oder Kunststoff, Flüssigverdampfern, Gel- und Membranverdampfern, propellergetriebenen Verdampfern, energielosen bzw. passiven Verdampfungssystemen, Mottenpapieren, Mottensäckchen und Mottengelen, als Granulate oder Stäube, in Streuködern oder Köderstationen.

### Erläuterung der Verfahren und Zwischenprodukte

Die folgenden Herstellungs- und Verwendungsbeispiele illustrieren die Erfindung, ohne sie zu beschränken. Die Produkte wurden mittels 1H-NMR Spektroskopie und/oder LC/MS (Liquid Chromatography Mass Spectrometry) charakterisiert.

Die Bestimmung der logP Werte erfolgte gemäß OECD Guideline 117 (EC Directive 92/69/EEC) durch HPLC (High Performance Liquid Chromatography) an reversed-phase (RP) Säulen (C18), mit nachfolgenden Methoden:
[a] Die Bestimmung mit der LC-MS im sauren Bereich erfolgte bei pH 2,7 mit 0,1 % wässriger Ameisensäure und Acetonitril (enthält 0,1% Ameisensäure) als Eluenten; linearer Gradient von 10% Acetonitril bis 95% Acetonitril.
[b] Die Bestimmung mit der LC-MS im neutralen Bereich erfolgte bei pH 7.8 mit 0,001 molarer wässriger Ammoniumhydrogencarbonat-Lösung und Acetonitril als Eluenten; linearer Gradient von 10 % Acetonitril bis 95 % Acetonitril.

Die Eichung erfolgte mit unverzweigten Alkan-2-onen (mit 3 bis 16 Kohlenstoffatomen), deren logP-Werte bekannt sind (Bestimmung der logP-Werte anhand der Retentionszeiten durch lineare Interpolation zwischen zwei aufeinander folgenden Alkanonen).

Die NMR-Spektren wurden mit einem Bruker Avance 400, ausgestattet mit einem Durchflussprobenkopf (60 µl Volumen), bestimmt. In Einzelfällen wurden die NMR Spektren mit einem Bruker Avance II 600 gemessen.

Die NMR-Daten ausgewählter Beispiele werden in klassischer Form (δ-Werte, Multiplettaufspaltung, Anzahl der H-Atome) aufgeführt. Die Aufspaltung der Signale wurde wie folgt beschrieben: s (Singulett), d (Duplett), t (Triplett), q (Quartett), quint (Quintuplett), m (Multiplett), br (für breite Signale). Als Lösungsmittel wurden CD₃CN, CDCl₃ oder D6-DMSO verwendet, wobei als Referenz Tetramethylsilan (0.00 ppm) eingesetzt wurde.

### Herstellbeispiele

### Verfahren A und B

### Beispiel (I-A-1)

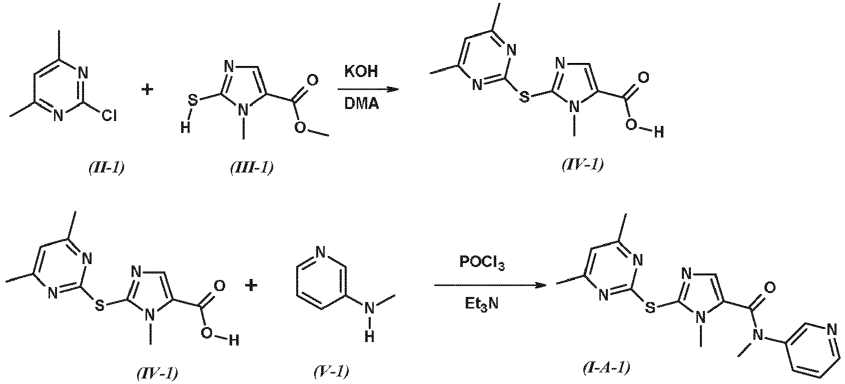

### Herstellung der Verbindung (IV-1)

1.46g (10mmol) 2-Chlor-4,6-dimethyl-pyrimidin, 0.62g (11mmol) gepulvertes Kaliumhydroxid und 1.72g (10mmol) 1-Methyl-2-sulfanyl-imidazol-5-carbonsäuremethylester wurden in 30ml Dimethylacetamid (DMA) zusammengegeben und bei einer Temperatur von 120° C über Nacht gerührt. Das Lösungsmittel wurde im Vakkum abrotiert und der Rückstand in Wasser und Methylenchlorid aufgenommen und extrahiert. Die wässrige Phase wurde mit 2N Salzsäure angesäuert, der Niederschlag abgesaugt und getrocknet. Ausbeute: 443mg (15.8% d. Theorie)
logP[a]: 0.81
¹H-NMR (d₆-DMSO, 400MHz); δ = 2.30 (s, 6H), 3.34 (s, 3H), 7.07 (s, 1H), 7.75 (s, 1H), 13.19 (s,br, 1H) ppm.

### Herstellung Beispiel (I-A-1)

0,339g (1.18 mmol) der Verbindung (IV-1) wurden in 15ml absolutem Tetrahydrofuran (THF) bei Raumtemperatur vorgelegt und 0.17ml (1.18 mmol) Triethylamin zugegeben. Es wurde 5 Min. gerührt, dann wurden 0.14g (1.3 mmol) 3-Methylamino-pyridin zugegeben und weitere 15 Min. gerührt. Anschließend wurden 0.46 ml (3.31 mmol) Triethylamin zugegeben, direkt dannach 0.11g (0.71 mmol) Phosphoroxychlorid zugetropft und dann 30 Min. unter Rückfluss gekocht. Der Ansatz wurde im Vakuum eingedampft und der Rückstand durch RP-Mitteldrucksäulenchromatographie mit einem Wassser/ Acetonitril Laufmittelgradienten gereinigt. Ausbeute: 77mg (17.7% d. Theorie)
logP[a]: 1.31; logP [n]: 1.41
¹H-NMR (CD₃CN, 400MHz); δ = 2.32 (s, 6H), 3.43 (s, 3H), 3.79 (s, 3H), 6.43 (s, 1H), 6.90 (s, 1H), 7.36-7.39 (m, 1H), 7.70-7.73 (m, 1H), 8.41-8.42 (d, 1H), 8.45-8.47 (m, 1H) ppm.

### Beispiel (I-A-2)

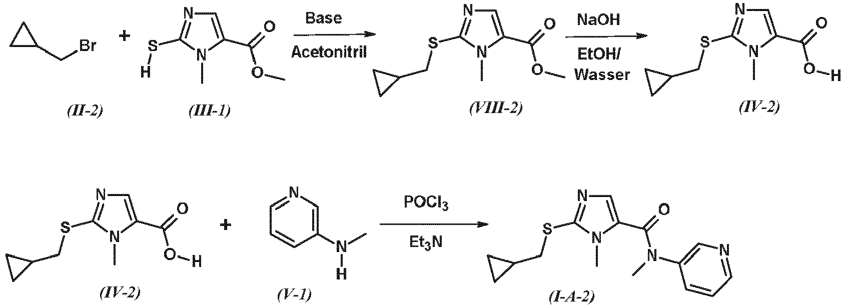

### Herstellung der Verbindung (VIII-2)

0.81g (6 mmol) Cyclopropyl-methylbromid, 0.83g (6 mmol) gepulvertes Kaliumcarbonat und 0.861g (5mmol) 1-Methyl-2-sulfanyl-imidazol-5-carbonsäuremethylester wurden in 20ml Acetonitril zusammengegeben und unter Rühren 5h unter Rückfluss erhitzt. Beim Lösen in saurem MeOH fiel ein weißes Pulver aus, das abgesaugt und dann das durch MPLC an Kieselgel mit Cyclohexan/Essigester 1:1 als Laufmittel aufgereinigt wurde. Ausbeute: 210mg (18.6% d. Theorie)
logP[a]: 2.06; logP[n]: 2.26
¹H-NMR (CD₃CN, 400MHz); δ = 0.26-0.30 (m, 2H), 0.56-0.61 (m, 2H), 1.10 (cm, 1H), 3.37-3.47 (m, 2H), 3.88 (s, 3H), 3.96 (s, 3H), 7.97 (s, 1H) ppm.

### Herstellung der Verbindung (IV-2)

0.210g (0.928 mmol) der Verbindung (VIII-2) und 0.186g (0.928 mmol) 20%-ige Natronlauge in 5ml Ethanol wurden 1h bei 40°C gerührt. Das Lösungsmittel wurde im Vakkum abgedampft, der Rückstand mit 1ml Wasser in Lösung und mit 1N HCl auf pH2 eingestellt. Die Lösung wurde im Vakuum eingedampft und der Rückstand an RP durch MPLC mit einem Wasser/Acetonitril-Gradienten gereinigt. Ausbeute: 155mg (78.7 % d. Theorie)
logP[a]: 0.62; logP[n]: -0.18
¹H-NMR (CD₃CN, 400MHz); δ = 0.23-0.26 (m, 2H), 0.52-0.56 (m, 2H), 1.12 (cm, 1H), 3.08-3.10 (d, 2H), 3.77 (s, 3H), 7.66 (s, 1H) ppm.

### Herstellung der Beispiel (I-A-2)

0,14g (0.66 mmol) der Verbindung (IV-2) wurden in 10ml absolutem Tetrahydrofuran (THF) bei Raumtemperatur vorgelegt und 0.1ml (0.66 mmol) Triethylamin zugegeben. Es wurde 5 Min. gerührt, dann wurden 0.078g (0.73 mmol) 3-Methylamino-pyridin zugegeben und weitere 15 Min. gerührt. Anschließend wurden 0.26 ml (1.85 mmol) Triethylamin zugegeben, dannach direkt 0.061g (0.4 mmol) Phosphoroxychlorid zugetropft und dann 30 Min. unter Rückfluss gekocht. Der Ansatz wurde im Vakuum eingedampft und der Rückstand an Kieselgel durch MPLC mit Cyclohexan/Essigester 1:1 als Laufmittel aufgereinigt. Ausbeute: 35mg (14% d. Theorie)
logP[a]: 1.28; logP[n]: 1.81
¹H-NMR (CD₃CN, 400MHz); δ = 0.12-0.16 (m, 2H), 0.47-0.51 (m, 2H), 1.03 (cm, 1H), 2.94-2.96 (d, 2H), 3.38 (s, 3H), 3.74 (s, 3H), 6,25 (s, 1H), 7.34-7.38 (m, 1H), 7.66-7.69 (m, 1H), 8.410-8.415 (d, 1H), 8.45-8.46 (m, 1H) ppm.

### Herstellung der Zwischenprodukte für Verfahren B

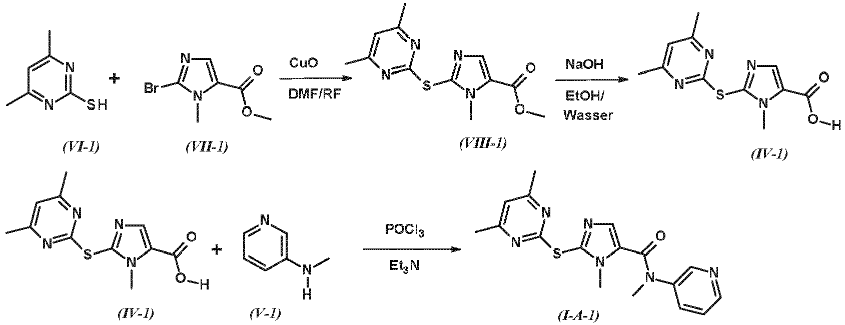

### Herstellung der Verbindung (VIII-1)

0.7g (5 mmol) 2-Mercapto-4,6-dimethyl-pyrimidin, 0.75g (5.25 mmol) Kupfer-(I)-oxid und 1.1g (5 mmol) 1-Methyl-2-brom-imidazol-5-carbonsäuremethylester wurden in 60 ml trockenem Dimethylformamid (DMF) unter Argon zusammengegeben und unter Rückfluss über Nacht erhitzt. Nach Abkühlen auf Raumtemperatur wurde das Reaktionsgemisch über Celite abgesaugt, mit Essigsäureethylester und heißem Methanol nachgewaschen, mit EDTA-Lösung extrahiert, die organische Phase abgetrennt, das Lösungsmittel im Vakuum abgedampft und der Rückstand an Kieselgel durch MPLC mit Cyclohexan/Essigsäureethylester 1:1 als Laufmittel gereinigt. Ausbeute: 247mg (17.3% d. Theorie)
logP[a]: 1.78; logP[n]: 1.80
¹H-NMR (CD₃CN, 400MHz); δ = 2.23 (s, 6H), 3.85 (s, 3H), 3.87 (s, 3H), 6.92 (s, 1H), 7.77 (s, 1H) ppm.

### Herstellung der Verbindung (IV-1)

0.396g (1.42 mmol) der Verbindung (VIII-1) und 0.28g (1.42 mmol) 20%-ige Natronlauge in 10ml Ethanol wurden 1h bei 40°C gerührt. Das Lösungsmittel wurde im Vakkum abgedampft, der Rückstand mit 2ml Wasser in Lösung und mit 1N HCl auf pH2 eingestellt. Der Niederschlag wurde abgesaugt und getrocknet. Ausbeute: 339mg (83% d. Theorie)
logP[a]: 0.81
¹H-NMR (CD₃CN, 400MHz); δ = 2.23 (s, 6H), 3.86 (s, 3H), 6.92 (s, 1H), 7.77 (s, 1H) ppm.

### Verfahren C-1

### Beispiel (I-A-3)

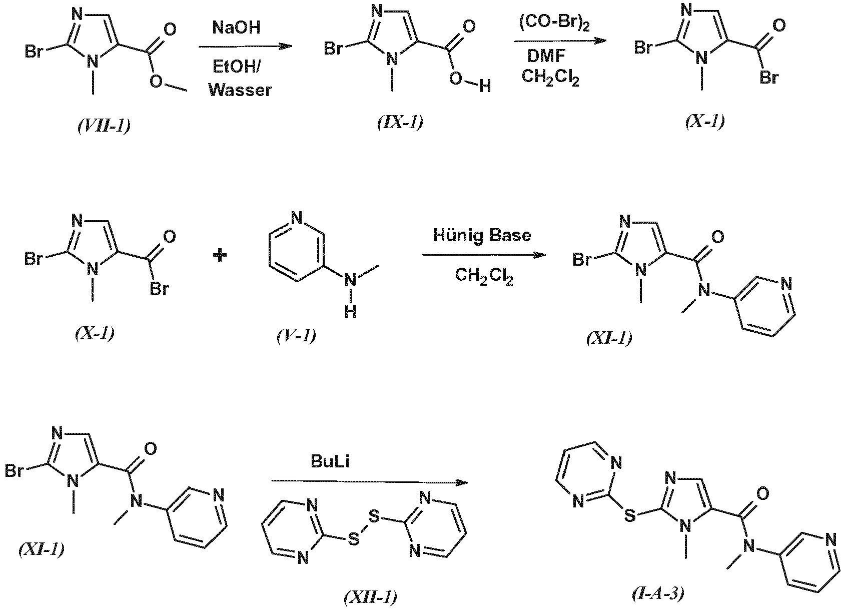

### Herstellung der Verbindung (IX-1)

1g (4.565 mmol) 1-Methyl-2-brom-imidazol-5-carbonsäuremethylester (VII-1) wurden in 10ml Ethanol gelöst, mit 5.48ml 1 N NaOH(aq.) versetzt und 60 Min bei Raumtemperatur gerührt. Nach Zugabe von 5.5mL 1N HCl(aq.) (auf pH ∼ 3 eingestellt), entsteht ein weißer Niederschlag. Der Ansatz wurde bis zur Trockene eingeengt und im Ultraschallbad in 6mL Wasser suspendiert. Die weißen Kristalle wurden abfiltriert und mit 2mL Wasser gewaschen. Die Mutterlauge wurde fast bis zur Trockenen eingeengt. Die Kristalle wurden abgesaugt und mit wenig Wasser gewaschen. Die vereinigten Kristalle wurden im Ölpumpenvakuum getrocknet. Ausbeute: 890mg (95% d. Theorie).
logP[a]:0.31;
¹H-NMR (d₆-DMSO, 400MHz); δ = 3.82 (s, 3H), 7.60 (s, 1H), 13.15 (s, 1H) ppm.

### Herstellung der Verbindung (X-1)

Eine Suspension von 890mg (4.341 mmol) 1-Methyl-2-brom--imidazol-5-carbonsäure (IX-1) in 15 ml Dichlormethan, wurde mit ca. 60 mg DMF aus einer Spritzenkanüle versetzt. Bei Raumtemperatur wurden 940mg (4.341 mmol) Oxalylbromid hinzugefügt, wobei eine intensive Gasentwicklung einsetzte. Am nächsten Tag wurden weitere 10 ml Dichlormethan und 140 mg Oxalylbromid hinzugegeben. Nach 1h Rühren bei Raumtemperatur erfolgte eine weitere Zugabe von 110mg Oxalylbromid und 30 mg DMF und es wurde weitere Stunde bei Raumtemperatur nachgerührt, LC/MS-Kontrolle zeigte 96% Umsatz. Die Suspension wurde ohne weitere Aufarbeitung zur Synthese der Verbindung (XI-1) eingesetzt.

### Herstellung der Verbindung (XI-1)

14.02g (40.19 mmol) 1-Methyl-2-brom-imidazol-5-carbonsäurebromid (X-1) wurden in 80ml Dichlormethan suspendiert und auf 0°C abgekühlt. Eine Lösung aus 4.35g (40.19 mmol) 3-Methylamino-pyridin (V-1) und 41 ml (241.2 mmol) Hünig-Base gelöst in 40ml Dichlormethan wurden bei 0°C zugeben. Es wurde 1h bei Raumttemperatur nachgerührt, anschließend 4h unter Rückfluss gekocht und über Nacht bei RT stehen gelassen. Der Ansatz wurde im Vakkum eingeengt Der Rückstand wurde in 500mL Dichlormethan aufgenommen und 3x mit insgesamt 400 ml Wasser gewaschen (2 x ca. 200ml und 1x ca. 100ml). Die vereinigten wässrigen Phasen wurden mit ca. 50ml Dichlormethan extrahiert und die organischen Phasen vereinigt. Die vereinigten organischen Phasen wurden 2x mit einer wässrigen NaHCO₃-Lsg. gewaschen (3,5g NaHCO3 in 150ml Wasser). Die vereinigten wässrigen Phasen wurden mit ca. 50ml Dichlormethan extrahiert, die organischen Phasen vereinigt, getrocknet und im Vakkum eingeengt. Ausbeute: 10.45g (81.9% d. Theorie) braunes, zähes Öl in einer Reinheit LC/MS 93%.
logP[a]: 0.65; logP[n]: 0.93
¹H-NMR (d₆-DMSO, 400MHz); δ = 3.37 (s, 3H), 3.75 (s, 3H), 6.25 (s, 1H), 7.43-7.47 (m, 1H), 7.84-7.87 (m, 1H), 8.48-8.49 (m, 1H), 8.52 (m, 1H) ppm.

### Verbindung (I-A-3)

0.54g (1.83 mmol) der Verbindung (XI-1) wurden in 5mL THF gelöst und auf <-70°C abgekühlt. 0,75ml (1.83 mmol) 2.5 molare n-BuLi-Lösung in Hexan wurden innerhalb von 10 Minuten bei dieser Temperatur zugetropft und 15 Minuten nachgerührt. Anschließend wurden 0.407g der Verbindung (XII-1) gelöst in 5ml THF zugetropft. Es wurden weitere 45 Minuten bei <-70°C nachgerührt. Der Ansatz wurde bei -70°C mit Ammoniumchloridlösung gequencht, das Produkt mit Dichlormethan extrahiert, die Lösung getrocknet und Vakuum eingeengt. Der Rückstand wurde an Kieselgel durch MPLC mit einem Methylenchlorid/Ethanol-Gradienten als Laufmittel isoliert. Ausbeute: 113mg (17% d. Theorie)
logP[a]: 0.69; logP[n]: 0.98
¹H-NMR (d₆-DMSO, 400MHz); δ = 3.41 (s, 3H), 3.75 (s, 3H), 6.49 (s, 1H), 6.90 (s, 1H), 7.32-7.34 (t, 1H), 7.45-7.48 (m, 1H), 7.84-7.86 (m, 1H), 8.48-8.50 (m, 1H), 8.51-8.53 (m, 1H), 8.63-8.64 (d, 2H) ppm.

### Verfahren C-2

### Verbindung (I-A-3)

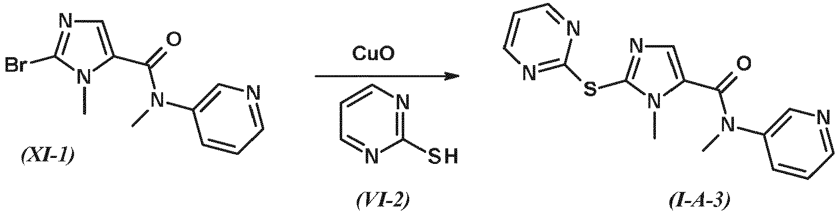

0.456g (4.066 mmol) 2-Mercapto -pyrimidin, 0.64g (4.473 mmol) Kupfer-(I)-oxid und 1.2g (4.066 mmol) der Verbindung (XI-1) wurden in 12 ml trockenem Dimethylformamid (DMF) zusammengegeben und 3h unter Rückfluss erhitzt. Nach Abkühlen auf Raumtemperatur wurde das Reaktionsgemisch über Celite filtriert, das Lösungsmittel im Vakuum abgedampft und der Rückstand an Kieselgel durch MPLC mit einem Dichlormethan/Methanol-Gradienten als Laufmittel gereinigt. Ausbeute: 408mg (30% d. Theorie)
logP[n]: 0.98
¹H-NMR (d₆-DMSO, 400MHz); δ = 3.41 (s, 3H), 3.75 (s, 3H), 6.49 (s, 1H), 6.90 (s, 1H), 7.32-7.34 (t, 1H), 7.45-7.48 (m, 1H), 7.84-7.86 (m, 1H), 8.48-8.50 (m, 1H), 8.51-8.53 (m, 1H), 8.63-8.64 (d, 2H) ppm.

### Verfahren D

### Verbindung (I-A-1)

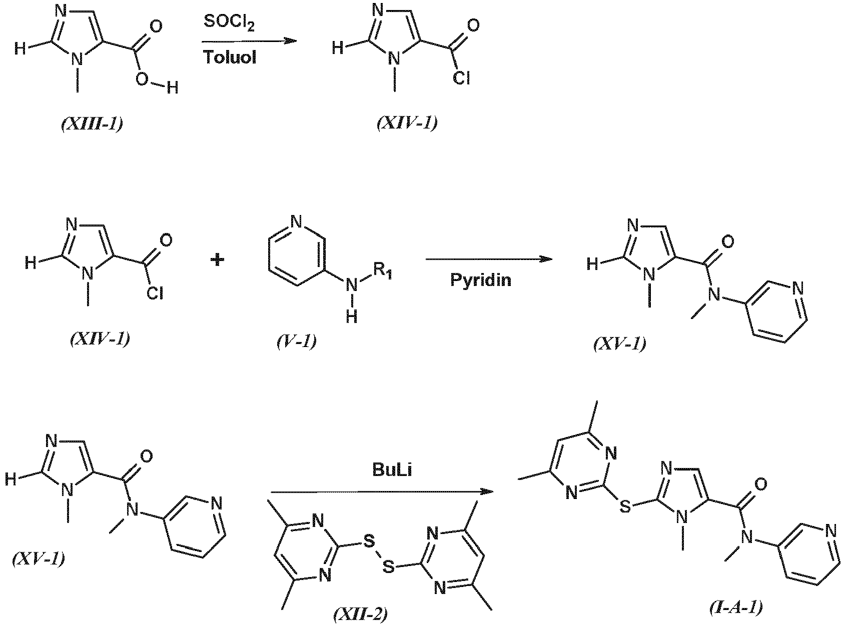

### Herstellung der Verbindung (XV-1)

Eine Suspension von 12g (95.2 mmol) 1-Methyl-imidazol-5-carbonsäure (XIII-1) in 72 ml Toluol wurde mit 12.71g (104.7 mmol) Thionylchlorid versetzt und über Nacht bei 130°C gerührt. Das Reaktionsgemisch wurde im Vakuum eingeengt. Der Rückstand wurde mit einer Lösung von 10.3g (95.2 mmol) 3-Methylamino-pyridin (V-1) in 72ml Pyridin versetzt und die resultierende Reaktionsmischung 4h auf 115°C erwärmt. Anschließend wurde erneut im Vakuum eingeengt und der Rückstand an Kieselgel säulenchromatographisch mit Acetonitril/Methanol 3:1 als Laufmittel gereinigt. Es wurden 8.1g (39.3 % d. Theorie) der Titelverbindung (XV-1) erhalten sowie 9.5g (37.1% d. Theorie) des HCl-Salzes der Titelverbindung.
logP[n]: 0.42
¹H-NMR (CD₃CN, 400MHz); δ = 3.39 (s, 3H), 3.81 (s, 3H), 6.17 (s, 1H), 7.36-7.40 (m, 2H), 7.67-7.70 (m, 1H), 8.41 (m, 1 H) 8.47 (m, 1H) ppm.

### Beispiel (I-A-1)

0.500g (2.31 mmol) der Verbindung (XV-1) wurden in 10ml THF gelöst und auf -85°C abgekühlt. Die Temperatur wurde während der Reaktion zwischen -82 und -90°C gehalten. 0,91ml (2.4 mmol) 2.5 molare n-BuLi-Lösung in n-Hexan wurden innerhalb von 5 Minuten bei dieser Temperatur zugetropft und 10 Minuten nachgerührt. Anschließend wurden 0.407g der Verbindung (XII-2) suspendiert in 5ml THF innerhalb von 3 Min. zugetropft. Es wurden weitere 30 Minuten bei <-85°C nachgerührt. Der Ansatz wurde innerhalb von 1.5h auf 0°C erwärmt und anschließend über Nacht bei Raumtemperatur stehen gelassen. Der Feststoff wurde abgesaugt und mit Dichlormethan nachgewaschen. Das Filtrat wurde im Vakuum eingedampft und der Rückstand an Kieselgel durch MPLC mit Essigsäureethylester/Methanol 95:5 als Laufmittel gereinigt. Es wurden 200mg 70%-iges Produkt erhalten, die mittels RP-HPLC (Acetonitril/Wasser + 0.1% Ameisensäure) weiter aufgereinigt wurden. Nach Aufreinigung wurden 105 mg (12.8% d. Theorie) erhalten.
logP[a]: 1.26; logP[n]: 1.46
¹H-NMR (d₆-DMSO, 400MHz); δ = 2.31 (s, 6H), 3.41 (s, 3H), 3.74 (s, 3H), 6.46 (s, 1H), 7.06 (s, 1H), 7.43-7.46 (m, 1H), 7.85-7.87 (m, 1H), 8.46-8.49 (m, 2H) ppm.

### Herstellung von 1-Benzyl-N-methyl-N-(pyridin-3-yl)-1H-imidazol-5-carboxamid

### (Verbindung XV-2)

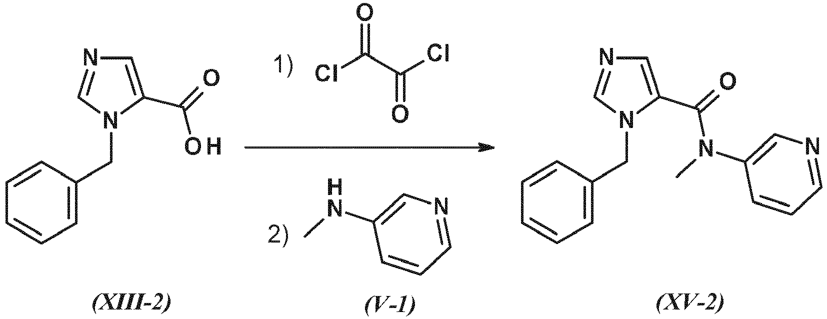

[1-Benzyl-1H-imidazol-5-carbonsäurehydrochlorid (XIII-2) wurde laut dem Protokoll von Tetrahedron 2004, 60, 6079-6083 hergestellt.] 1.00 g (4.94 mmol) 1-Benzyl-1H-imidazol-5-carbonsäurehydrochlorid (XIII-2) wurden in 10 mL Dichlormethan mit einem Tropfen Dimethylformamid gelöst. 0.475 mL (5.44 mmol) Oxalsäuredichlorid wurden zugetropf. Es wurde 3 h bei Raumtemperatur gerührt. Das Lösungsmittel wurde im Vakuum abgedampft. Zum Rückstand wurde eine Lösung von 0.535 g (4.94 mmol) N-Methylpyridin-3-amin (V-1) in 7.2 mL Pyridin zugetropft und über Nacht bei 100 °C gerührt. Das Lösungsmittel wurde im Vakuum abgedampft. Der Rückstand wurde in 8.3 mL Dichlormethan gelöst und 1.57 g (14.8 mmol) Natriumcarbonat wurden zugegeben. Der Ansatz wurde 3 h bei Raumtemperatur gerührt, dann filtriert und eingeengt. Der Rückstand wurde an Kieselgel durch MPLC mit Essigsäureethylester/Methanol als Laufmittel gereinigt. Nach Aufreinigung wurden 0.326 g (21.9% d. Theorie) erhalten.
logP[a]: 0.85; logP[n]: 1.40;
¹H-NMR (d₆-DMSO, 400MHz); δ = 3.26 (s, 3H), 5.45 (s, 2H), 6.27 (s, 1H), 7.19-7.21 (m, 2H), 7.30-7.42 (m, 5H), 7.858-7.863 (m, 1H), 7.90 (s, 1H), 8.40-8.41 (m, 1H) ppm.

### Beispiel (I-A-111): 1-Benzyl-N-methyl-N-(pyridin-3-yl)-2-(pyrimidin-2-ylsulfanyl)-1H-imidazol-5-carboxamid

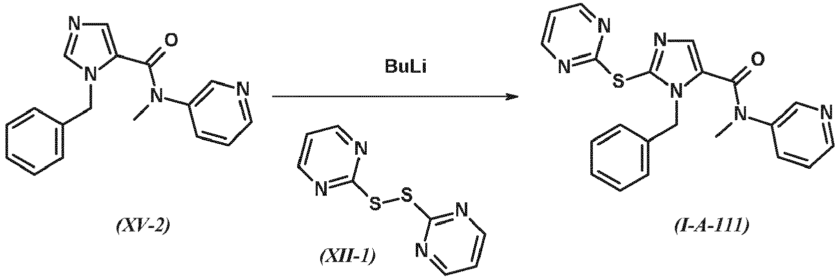

0.272 g (0.930 mmol) 1-Benzyl-N-methyl-N-(pyridin-3-yl)-1H-imidazol-5-carboxamid (XV-2) wurden in 11 mL THF gelöst und auf -90 °C abgekühlt. Die Temperatur wurde während der Reaktion zwischen -85 °C und -90 °C gehalten. 0.39 mL (0.977 mmol) 2.5 molare *n*-BuLi-Lösung in *n*-Hexan wurden innerhalb von 5 Minuten bei -90 °C zugetropft und 5 Minuten nachgerührt. Anschließend wurden innerhalb von 5 Minuten 0.620 g 2,2'-Disulfandiyldipyrimidin (2.79 mmol) (XII-1), in 1 mL THF gelöst, zugetropft. Es wurden weitere 30 Minuten bei -80 °C nachgerührt und dann innerhalb von 30 Minuten auf Raumtemperatur erwärmt. Der Ansatz wurde mit halbkonzentrierter Natriumhydrogencarbonatlösung/Dichlormethan extrahiert. Die wässrige Phase wurde nochmals dreimal mit Dichlormethan extrahiert. Die organischen Phasen wurden vereinigt, mit wenig Wasser gewaschen, mit Natriumsulfat getrocknet, filtriert und eingeengt. Der Rückstand wurde an Kieselgel durch MPLC mit Essigsäureethylester/Methanol als Laufmittel gereinigt. Die gewünschte Fraktion wurde über RP18 durch HPLC mit Acetonitril/Wasser als Laufmittel gereinigt. Nach Aufreinigung wurden 140 mg (35.1% d. Theorie) erhalten.
Analytik siehe Tabelle 2

### Verfahren E

### Beispiel (I-B-1)

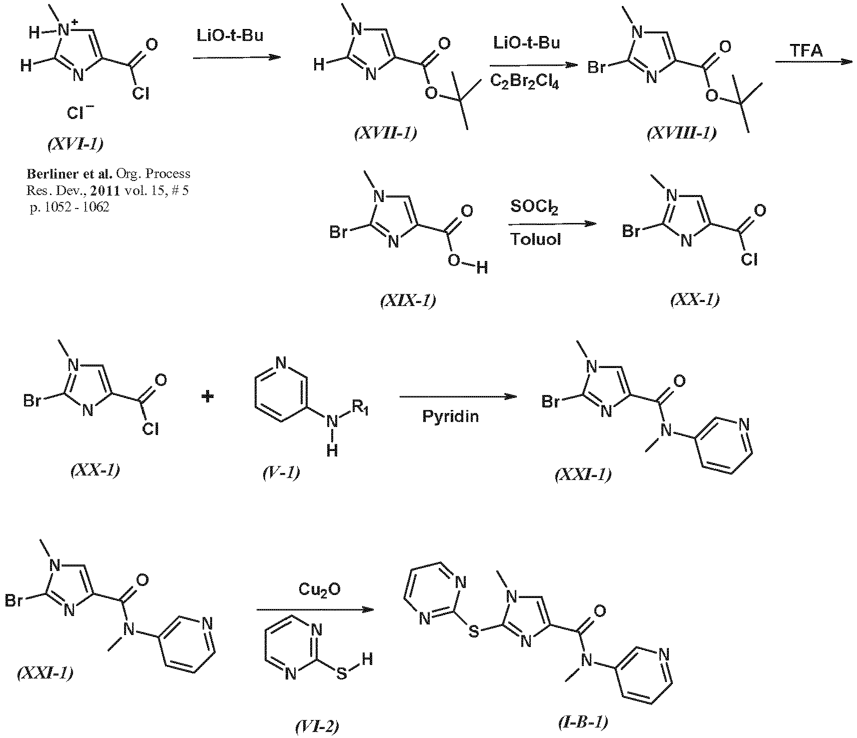

### Herstellung der Verbindung (XVII-1)

Zu einer Suspension von 36.5g (0.201 mol) 1-Methyl-1H-imidazol-4-carbonylchlorid Hydrochlorid in 400ml Tetrahydrofuran wurden unter Argon bei -40°C 33.9 g (0.493 mol) Lithium-tert.- butanolat unter Rühren zugegeben. Es wurde dann ohne weitere Kühlung für 12 Stunden gerührt. Nach Zugabe von 170g Natriumhydrogencarbonat wurde das Gemisch über etwas Kieselgel filtriert und mit Essigsäureethylester nachgewaschen. Die vereinigten organischen Phasen wurden eingedampft und der erhaltene Rückstand mit Dichlormethan extrahiert. Der Eindampfrückstand der Dichlormethanphase wurde zwischen gesättigter Natriumhydrogencarbontlösung und Essigsäureethylester verteilt, die organische Phase mit Magnesiumsulfat getrocknet, filtriert, eingedampft und es wurden erhält 22.7g tert-Butyl-1-methyl-1H-imidazol-4-carboxylat (73% Ausbeute d. Theorie) erhalten.
logP[a]:0.74;
¹H-NMR (CD₃CN, 400MHz); δ = 1.52 (s, 9H), 3.66 (s, 3H), 7.43 (s, 1H), 7.54 (s, 1H) ppm.

### Herstellung der Verbindung (XVIII-1)

0.5g (2.744 mmol) 1-Methyl-imidazol-4-carbonsäure-tert.-butylester (XVII-1) wurden in 10ml Tetrahydrofuran (THF) vorgelegt, 0.894g (2.744 mmol) 1,2-Dibrom-1,1,2,2-tetrachlorethan zugeben und bei Raumtemperatur 0.88g (11 mmol) Lithium-tert.-butylat hinzugefügt. Es wurde über Nacht bei Raumtemperatur gerührt, das Reaktionsgemisch im Vakkum eingeengt und der Rückstand über RP18 durch MPLC mit einem Acetonitril/Wasser + 0.1% Ameisensäure Laufmittel gereinigt. Ausbeute: 250mg (34.8% d. Theorie).
logP[a]: 1.27
¹H-NMR (CD₃CN, 400MHz); δ = 1.51 (s, 9H), 3.60 (s, 3H), 7.65 (s, 1H) ppm.

### Herstellung der Verbindung (XIX-1)

645 mg (2.47 mmol) tert-Butyl-2-brom-1-methyl-1H-imidazol-4-carboxylat wurden in einer Mischung aus 1.7 g Trifluoressigsäure und 7 ml Dichlormethan bei Raumtemperatur für 12 Stunden gerührt. Das Lösungsmittel wurde entfernt. Der erhaltene Eindampfrückstand bestand zu 95% aus dem Zielprodukt 2-Brom-1-methyl-1H-imidazol-4-carbonsäure, was praktisch einer quantitativen Ausbeute entspricht.
logP[a]: 0.0;
¹H-NMR (CD₃CN, 400MHz); δ = 3.65 (s, 3H), 7.78 (s, 1H) ppm.

### Herstellung der Verbindung (XXI-1)

Zu einer Lösung von 681 mg (3.32 mmol) 2-Brom-1-methyl-1H-imidazol-4-carbonsäure in 7ml Dichlormethan und 3 Tropfen Dimethylformamid wurden 0.32 ml Oxalsäuredichlorid (1.2 Eq.) getropft. Nach 4 Stunden wurde vollständig eingedampft. Zum Rückstand wurden 10ml Dichlormethan, 359 mg (1 Eq.) N-Methylpyridin-3-amin und 6 Eq. N,N-Di-isopropylethylamin gegeben und dann für eine Stunde gerührt. Anschließend wurde vollständig eingedampft und durch MPLC mit Essigsäureethylester/Methanol als Laufmittel an Kieselgel chromatographiert. Ausbeute 411mg (43 % d. Th.).
logP[n]: 0.76;
¹H-NMR (CD₃CN, 400MHz); δ = 3.41 (s, 3H); 3.51 (s, 3H), 7.28-7.35 (m, 2H), 7.59-7.61 (m, 1H), 8.37-8.43 (m, 2H) ppm.

### Herstellung der Verbindung (I-B-1)

250mg (0.618 mmol) der Verbindung (XXI-1) und 69mg (0.618 mmol) 2-Mercapto-pyrimidin wurden in einem Rollrandgefäß zusammengegeben und 2 Stunden bei 150°C gerührt. Der Ansatz wurde mit gesättigter EDTA Lösung gewaschen und dreimal mit Essigester extrahiert. Die organische Lösung wurde mit Magnesiumsulfat getrocknet, filtriert und im Vakkum eingedampft. Der Rückstand wurde durch MPLC mit einem Essigester/Methanol-Gradienten als Laufmittel an Kieselgel aufgereinigt. Ausbeute: 65mg (30.6% d. Theorie).
logP[a]: 0.42; logP[n]: 0.83;
¹H-NMR (CD3CN, 400MHz); δ = 3.47 (s, 3H), 3.58 (s, 3H), 7.15 (m, 1H), 7.30 (br. s, 1H), 7.54 (br. s, 1H), 7.60 (br. d, 1H), 8.39 (br. s, 2H), 8.48-8.49 (m, 2H) ppm.

### Verfahren F

### Beispiel (I-A-45)

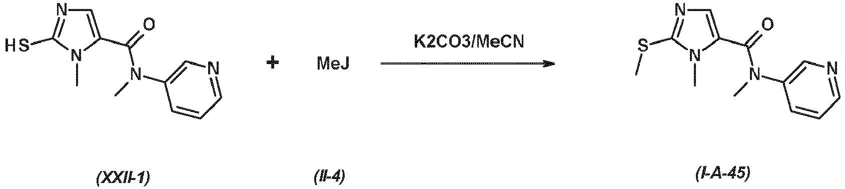

0.496g (2 mmol) der Verbindung (XXII-1), 0.332g (2.4 mmol) Kaliumcarbonat und 0.125ml (2 mmol) Methyljodid wurden in 10ml Acetonitril unter Argon 1h unter Rückfluss gekocht. Die Reaktionsmischung wurde im Vakkum eingedampft, mit Natriumhydrogencarbonatlösung versetzt, erneut eingedampft, der Rückstand mit Ethanol verrührt, die Salze abfiltriert und das Lösungsmittel abgedampft. Der Rückstand lieferte nach Reversed Phase Chromatographie 237 mg der Zielverbindung (I-A-45).
Analytik siehe Tabelle 2

### Beispiel (I-A-37)

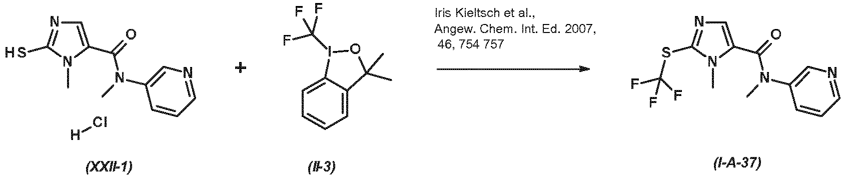

0.420g (1.47 mmol) der Verbindung (XXII-l)-Hydrochlorid wurden in 5ml Methanol unter Argon gelöst und auf -78°C abgekühlt. Nach Zugabe des Alkylierungsmittesl (II-3) (2 Äq.) ließ man eine Stunde nachrühren, erwärmte auf Raumtemp., versetzte mit 1ml gesättigter AmmoniumchloridLösung und engte vollständig ein. Der Rückstand lieferte nach Chromatographie an Kieselgel 220 mg der Zielverbindung (I-A-37).
Analytik siehe Tabelle 2

### Beispiel (I-A-38)

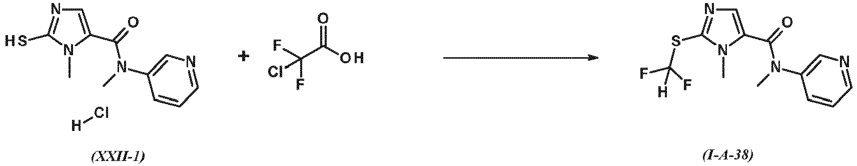

0.200g (0.70 mmol) der Verbindung (XXII-1) Hydrochlorid wurden in 10ml Dimethylformamid gelöst und nacheinander unter Rühren mit 3 Äq. Kaliumcarbonat und 2 Äq. Natrium-chlor-difluoracetat versetzt. Nach 3 stdg. Erhitzen auf 95°C ließ man auf Raumtemp abkühlen, dampfte vollständig ein, nahm in 10 ml Methanol auf und filtrierte über Celite. Das Filtrat wurde vollständig eingedampft und an Kieselgel chromatographiert und man erhielt 56 mg der Zielverbindung (I-A-38).
Analytik siehe Tabelle 2

### Herstellungsweg zu Verbindung (XXII-1)

### Verbindung (XXIV)

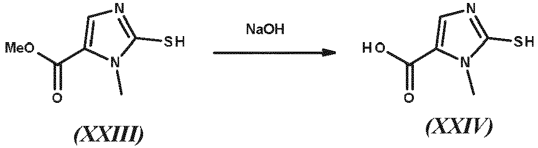

428g (2.49 Mol) der kommerziell verfügbaren Verbindung (XXIII) wurden 30 Min. in 2.81 3M Natronlauge bei 15°C gerührt. Anschließend wurde mit 6M Sakzsäure auf pH1-2 eingestellt, der Niederschlag abgesaugt. Nach Trocknen erhielt man 363g der Verbindung (XXIV).
1H-NMR (400MHz, CD₃OD): δ = 3.82 (s, 3H), 7.58 (s,1H)

### Verbindung (XXV)

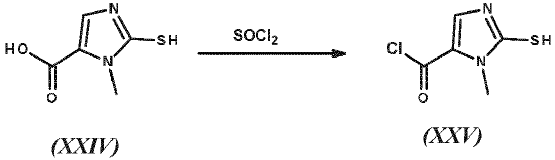

400g (2.53 Mol) der Verbindung (XXIV) wurden in 3.21 Dichlormethan und 30ml Dimethylformamid vorgelegt. Es wurden 1.21 Thionylchlorid hinzugetropft und anschließend 2 Stunden bei 50°C gerührt. Die Reaktionsmischung wurde im Vakkum eingedampft. Man erhielt 580g Rohprodukt als Feststoff, der direkt weiter für die nächste Reaktion eingesetzt wurde.

### Verbindung (XXII-1)

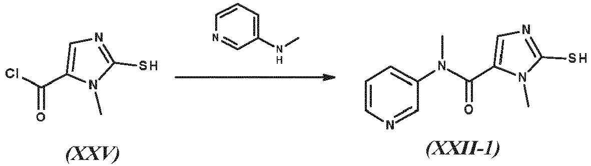

106g (601 mmol) der Verbindung (XXV) (1.3 eq) und 50g (462 mmol) 3-Methylamino-pyridin (1 eq) wurden in 750ml Pyridin bei 90°C für 1h gerührt. Danach wurde die Reaktionsmischung im Vakkum eingedampft und der Rückstand an Kieselgel mit Dichlormethan/Methanol 100:1/5:1-Gradienten gereinigt. Man erhielt 67.3g der Verbindung (XXII-1).
¹H-NMR (**DMSO-d6,** 400MHz); δ = 3.43 (s, 3H), 3.61 (s, 3H), 6.76 (s, 1H), 7.81-7.84 (m, 1H), 8.33 (d, 1H), 8.67 (d, 1H), 8.91 (d, 1H).

### Verfahren G

### Beispiel (I-A-43)

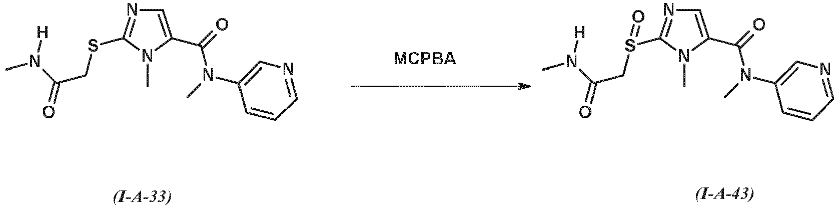

0.18g (0.564 mmol) der Verbindung (I-A-33) wurden in 30ml Dichlormethan gelöst und nacheinander unter Rühren mit 321mg (2.82 mmol) Trifluoressigsäure und 243mg (1.41 mmol) 3-Chlorperoxybenzoesäure bei 0°C versetzt. Die Reaktionsmischuung wurde eingedampft. Nach Chromatographie an Kieselgel in Essigester/Methanol-Gradient erhielt man 62mg der Zielverbindung (I-A-43).
Analytik siehe Tabelle 2

### Beispiel (I-A-83)

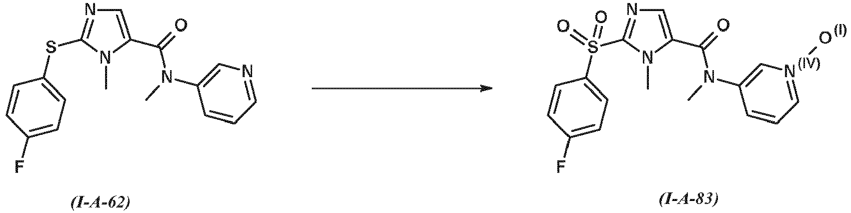

0.100g (0.29 mmol) der Verbindung (I-A-62) wurden in 15ml Dichlormethan gelöst und nacheinander unter Rühren mit 5 Äq. Trifluoressigsäure und 2 Äq. 3-Chlorperoxybenzoesäure versetzt. Nach 24 stdg. Rühren bei Raumtemp. fügte man ein weiteres Äq. Trifluoressigsäure zu, ließ noch eine weitere Stunde rühren und dampfte fast ganz vollständig ein und erhielt nach RP-Chromatographie 59mg der Zielverbindung (I-A-83).
Analytik siehe Tabelle 2

Weitere Verbindungen der Formel (I), die gemäß den Verfahren A bis D, F und G in analoger Weise hergestellt wurden sind in der folgenden Tabelle aufgeführt.

### Tabelle 1

### Verbindungen der Formel (I-A)

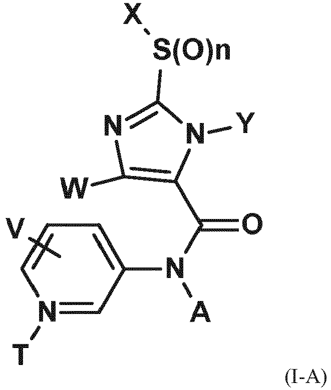

in welcher die Substituenten, die in der Tabelle angegebenen Bedeutungen haben:

**Tabelle 2**

| **Bsp.-Nr.** | **X** | **n** | **W** | **Y** | **A** | **V** | **T** |
|---|---|---|---|---|---|---|---|
| I-A-4 | 2-(4,6-Dimethyl)-pyrimidyl- | 0 | H | CH₃ | H | H | Elektronenpaar |
| I-A-5 | n-Butyl- | 0 | H | CH₃ | CH₃ | H | Elektronenpaar |
| I-A-6 | n-Pentyl- | 0 | H | CH₃ | CH₃ | H | Elektronenpaar |
| I-A-7 | Benzyl- | 0 | H | CH₃ | CH₃ | H | Elektronenpaar |
| I-A-8 | Methyl | 2 | H | CH₃ | CH₃ | H | Elektronenpaar |
| I-A-9 | 3-Methylthiophenyl- | 0 | H | CH₃ | CH₃ | H | Elektronenpaar |
| I-A-10 | 2,2,2-Trifluorethyl- | 0 | H | CH₃ | CH₃ | H | Elektronenpaar |
| I-A-11 | Phenyl- | 0 | H | CH₃ | CH₃ | H | Elektronenpaar |
| I-A-12 | 4-Methyl-phenyl- | 0 | H | CH₃ | CH₃ | H | Elektronenpaar |
| I-A-13 | 2-Pyrimidyl- | 0 | Br | CH₃ | CH₃ | H | Elektronenpaar |
| I-A-14 | Ethylthio-ethyl- | 0 | H | CH₃ | CH₃ | H | Elektronenpaar |
| I-A-15 | 2-Pyrimidyl- | 0 | Cl | CH₃ | CH₃ | H | Elektronenpaar |
| I-A-16 | 2-Nitro-phenyl- | 0 | H | CH₃ | CH₃ | H | Elektronenpaar |
| I-A-17 | Cyclohexyl | 0 | H | CH₃ | CH₃ | H | Elektronenpaar |
| I-A-18 | -(CH₂)₂-O-CH₃ | 0 | H | CH₃ | CH₃ | H | Elektronenpaar |
| I-A-19 | i-Propyl | 0 | H | CH₃ | CH₃ | H | Elektronenpaar |
| I-A-20 | -(CH₂)₃-O-CH₃ | 0 | H | CH₃ | CH₃ | H | Elektronenpaar |
| I-A-21 | n-Propyl | 0 | H | CH₃ | CH₃ | H | Elektronenpaar |
| I-A-22 | -(CH₂)₂-O-C₂H₅ | 0 | H | CH₃ | CH₃ | H | Elektronenpaar |
| I-A-23 | -CH₂-CO-CH₃ | 0 | H | CH₃ | CH₃ | H | Elektronenpaar |
| I-A-24 | -CH₂-CO₂-C₂H₅ | 0 | H | CH₃ | CH₃ | H | Elektronenpaar |
| I-A-25 | -CH₂-CO₂-CH₃ | 0 | H | CH₃ | CH₃ | H | Elektronenpaar |
| I-A-26 | -(CH₂)₂-S-CH₃ | 0 | H | CH₃ | CH₃ | H | Elektronenpaar |
| I-A-27 | Allyl | 0 | H | CH₃ | CH₃ | H | Elektronenpaar |
| I-A-28 | -CH₂-CO-C(CH₃)₃ | 0 | H | CH₃ | CH₃ | H | Elektronenpaar |
| I-A-29 | -CH₂-CO₂H | 0 | H | CH₃ | CH₃ | H | Elektronenpaar |
| I-A-30 | Propargyl | 0 | H | CH₃ | CH₃ | H | Elektronenpaar |
| I-A-31 | -CH₂-CO-c-Propyl | 0 | H | CH₃ | CH₃ | H | Elektronenpaar |
| I-A-32 | -CH₂-CO-NH₂ | 0 | H | CH₃ | CH₃ | H | Elektronenpaar |
| I-A-33 | -CH₂-CO-NH-CH₃ | 0 | H | CH₃ | CH₃ | H | Elektronenpaar |
| I-A-34 | -CH₂-CO-N(CH₃)₂ | 0 | H | CH₃ | CH₃ | H | Elektronenpaar |
| I-A-35 | 3,3-Dimethyl-allyl | 0 | H | CH₃ | CH₃ | H | Elektronenpaar |
| I-A-36 | Ethyl | 0 | H | CH₃ | CH₃ | H | Elektronenpaar |
| I-A-37 | CF₃ | 0 | H | CH₃ | CH₃ | H | Elektronenpaar |
| I-A-38 | CHF₂ | 0 | H | CH₃ | CH₃ | H | Elektronenpaar |
| I-A-39 | -CH₂-CNOCH₃-CH₃ | 0 | H | CH₃ | CH₃ | H | Elektronenpaar |
| I-A-40 | Ethyl | 2 | H | CH₃ | CH₃ | H | Elektronenpaar |
| I-A-41 | Ethyl | 0 | H | CH₃ | H | H | Elektronenpaar |
| I-A-42 | -CH₂-CO-NH-CH₃ | 2 | H | CH₃ | CH₃ | H | Elektronenpaar |
| I-A-43 | -CH₂-CO-NH-CH₃ | 1 | H | CH₃ | CH₃ | H | Elektronenpaar |
| I-A-44 | Methyl | 0 | H | CH₃ | C₂H₅ | H | Elektronenpaar |
| I-A-45 | Methyl | 0 | H | CH₃ | CH₃ | H | Elektronenpaar |
| I-A-46 | -CH₂-CF₂-CF₂Cl | 0 | H | CH₃ | CH₃ | H | Elektronenpaar |
| I-A-47 | -CH₂-CH₂-CF₃ | 0 | H | CH₃ | CH₃ | H | Elektronenpaar |
| I-A-48 | -CH₂-CH₂-CH₂F | 0 | H | CH₃ | CH₃ | H | Elektronenpaar |
| I-A-49 | -CH₂-CF₂-CHF₂ | 0 | H | CH₃ | CH₃ | H | Elektronenpaar |
| I-A-50 | -(CH₂)₂-S-CH₂-CF₃ | 0 | H | CH₃ | CH₃ | H | Elektronenpaar |
| I-A-51 | 4-tetrahydrothiopyranyl | 0 | H | CH₃ | CH₃ | H | Elektronenpaar |
| I-A-52 | 4-tetrahydropyranyl | 0 | H | CH₃ | CH₃ | H | Elektronenpaar |
| I-A-53 | -(CH₂)₂-iC₃F₇ | 0 | H | CH₃ | CH₃ | H | Elektronenpaar |
| I-A-54 | 3-oxetanyl | 0 | H | CH₃ | H₃ | H | Elektronenpaar |
| I-A-55 | 5-oxa-[3.3.0]-bicycloheptan | 0 | H | CH₃ | CH₃ | H | Elektronenpaar |
| I-A-56 | 2,4,5-Trichlorphenyl- | 0 | H | CH₃ | CH₃ | H | Elektronenpaar |
| I-A-57 | 4-Chlor-phenyl- | 0 | H | CH₃ | CH₃ | H | Elektronenpaar |
| I-A-58 | 4-Methoxyphenyl- | 0 | H | CH₃ | CH₃ | H | Elektronenpaar |
| I-A-59 | 4-Dimethlyaminophenyl | 0 | H | CH₃ | CH₃ | H | Elektronenpaar |
| I-A-60 | 2,5-Dichlorphenyl- | 0 | H | CH₃ | CH₃ | H | Elektronenpaar |
| I-A-61 | 3-Trifluormethylphenyl- | 0 | H | CH₃ | CH₃ | H | Elektronenpaar |
| I-A-62 | 4-Fluor-phenyl- | 0 | H | CH₃ | CH₃ | H | Elektronenpaar |
| I-A-63 | 4-tert.-Butylphenyl- | 0 | H | CH₃ | CH₃ | H | Elektronenpaar |
| I-A-64 | 4-Chlor-3-trifluormethylphenyl- | 0 | H | CH₃ | CH₃ | H | Elektronenpaar |
| I-A-65 | 2-Pyridyl | 0 | H | CH₃ | CH₃ | H | Elektronenpaar |
| I-A-66 | 3-Chlor-phenyl- | 0 | H | CH₃ | CH₃ | H | Elektronenpaar |
| I-A-67 | 2-Dimethylaminocarbamoyl-phenyl- | 0 | H | CH₃ | CH₃ | H | Elektronenpaar |
| I-A-68 | 3-Nitro-phenyl- | 0 | H | CH₃ | CH₃ | H | Elektronenpaar |
| I-A-69 | 2-Dimethylaminosulfonyl-phenyl- | 0 | H | CH₃ | CH₃ | H | Elektronenpaar |
| I-A-70 | 5-(2-Chlor)-pyridyl- | 0 | H | CH₃ | CH₃ | H | Elektronenpaar |
| I-A-71 | 2-Fluor-phenyl- | 0 | H | CH₃ | CH₃ | H | Elektronenpaar |
| I-A-72 | 2-Methoxyphenyl- | 0 | H | CH₃ | CH₃ | H | Elektronenpaar |
| I-A-73 | 3-Chlor-phenyl- | 2 | H | CH₃ | CH₃ | H | Elektronenpaar |
| I-A-74 | 3-Chlor-phenyl- | 1 | H | CH₃ | CH₃ | H | Elektronenpaar |
| I-A-75 | 4-tert.-Butylphenyl- | 1 | H | CH₃ | CH₃ | H | Elektronenpaar |
| I-A-76 | 4-tert.-Butylphenyl- | 2 | H | CH₃ | CH₃ | H | Elektronenpaar |
| I-A-77 | 3,5-Dichlor- | 1 | H | CH₃ | CH₃ | H | Elektronenpaar |
| I-A-78 | 3,5-Dichlor- | 2 | H | CH₃ | CH₃ | H | Elektronenpaar |
| I-A-79 | 4-Chlor-3-trifluormethylphenyl- | 1 | H | CH₃ | CH₃ | H | Elektronenpaar |
| I-A-80 | 4-Chlor-3-trifluormethylphenyl- | 2 | H | CH₃ | CH₃ | H | Elektronenpaar |
| I-A-81 | 4-Methoxyphenyl- | 2 | H | CH₃ | CH₃ | H | Elektronenpaar |
| I-A-82 | 4-Fluor-phenyl- | 2 | H | CH₃ | CH₃ | H | Elektronenpaar |
| I-A-83 | 4-Fluor-phenyl- | 2 | H | CH₃ | CH₃ | H | Sauerstoff |
| I-A-84 | 4-Fluor-phenyl- | 1 | H | CH₃ | CH₃ | H | Elektronenpaar |
| I-A-85 | 2-Pyridyl- | 2 | H | CH₃ | CH₃ | H | Elektronenpaar |
| I-A-86 | 2-Pyridyl- | 1 | H | CH₃ | CH₃ | H | Elektronenpaar |
| I-A-87 | Phenyl- | 1 | H | CH₃ | CH₃ | H | Elektronenpaar |
| I-A-88 | Phenyl- | 2 | H | CH₃ | CH₃ | H | Elektronenpaar |
| I-A-89 | 4-Nitro-phenyl- | 1 | H | CH₃ | CH₃ | H | Elektronenpaar |
| I-A-90 | 4-Nitro-phenyl- | 2 | H | CH₃ | CH₃ | H | Elektronenpaar |
| I-A-91 | 2-Dimethylaminocarbamoyl-phenyl- | 1 | H | CH₃ | CH₃ | H | Elektronenpaar |
| I-A-92 | 2-Dimethylaminocarbamoyl-phenyl- | 2 | H | CH₃ | CH₃ | H | Elektronenpaar |
| I-A-93 | 4-Methoxyphenyl- | 1 | H | CH₃ | CH₃ | H | Elektronenpaar |
| I-A-94 | 2-(5-fluor)-pyridyl | 0 | H | CH₃ | CH₃ | H | Elektronenpaar |
| I-A-95 | 3-Trifluormethylphenyl- | 1 | H | CH₃ | CH₃ | H | Sauerstoff |
| I-A-96 | 3-Trifluormethylphenyl- | 1 | H | CH₃ | CH₃ | H | Elektronenpaar |
| I-A-97 | 2-Pyrimidyl- | 1 | H | CH₃ | CH₃ | H | Elektronenpaar |
| I-A-98 | 2-Pyrimidyl- | 0 | H | CH₃ | C₂H₅ | H | Elektronenpaar |
| I-A-99 | 2-Pyrimidyl- | 2 | H | CH₃ | CH₃ | H | Elektronenpaar |
| I-A-100 | 2-(4-Trifluormethyl)-pyrimidyl- | 0 | H | CH₃ | CH₃ | H | Elektronenpaar |
| I-A-101 | 2-(4-Methyl)-pyrimidyl- | 0 | H | CH₃ | CH₃ | H | Elektronenpaar |
| I-A-102 | 2-(4,6-Dimethoxy)-pyrimidyl- | 0 | H | CH₃ | CH₃ | H | Elektronenpaar |
| I-A-103 | 2-(4,5-Dimethyl)-pyrimidyl- | 0 | H | CH₃ | CH₃ | H | Elektronenpaar |
| I-A-104 | 2-(5-Methyl)-pyrimidyl- | 0 | H | CH₃ | CH₃ | H | Elektronenpaar |
| I-A-105 | 2-(5-Trifluormethyl)-pyrimidyl- | 0 | H | CH₃ | CH₃ | H | Elektronenpaar |
| I-A-106 | 2-(4-Methoxy)-pyrimidyl- | 0 | H | CH₃ | CH₃ | H | Elektronenpaar |
| I-A-107 | 2-(5-Fluor)-pyrimidyl- | 0 | H | CH₃ | CH₃ | H | Elektronenpaar |
| I-A-108 | 2-(6-Methyl)-pyridyl- | 0 | H | CH₃ | CH₃ | H | Elektronenpaar |
| I-A-109 | 2-(5-Methyl)-pyridyl- | 0 | H | CH₃ | CH₃ | H | Elektronenpaar |
| I-A-110 | 2-(3-Trifluormethyl)-pyridyl- | 0 | H | CH₃ | CH₃ | H | Elektronenpaar |
| I-A-111 | 2-Pyrimidyl- | 0 | H | Benzyl | CH₃ | H | Elektronenpaar |
| I-A-112 | -CH₂-2-pyrimidyl- | 0 | H | CH₃ | CH₃ | H | Elektronenpaar |
| I-A-113 | -CH₂-2-pyrazinyl | 0 | H | CH₃ | CH₃ | H | Elektronenpaar |
| I-A-114 | -CH₂-5-(1-methyl)-imidazolyl | 0 | H | CH₃ | CH₃ | H | Elektronenpaar |
| I-A-115 | -CH₂-3-(1-methyl)-pyrazolyl | 0 | H | CH₃ | CH₃ | H | Elektronenpaar |
| I-A-116 | -CH₂-4-pyridyl- | 0 | H | CH₃ | CH₃ | H | Elektronenpaar |
| I-A-117 | -CH₂-2-pyridyl- | 0 | H | CH₃ | CH₃ | H | Elektronenpaar |
| I-A-118 | -CH₂-2-(1-methyl)-imidazolyl | 0 | H | CH₃ | CH₃ | H | Elektronenpaar |
| I-A-119 | -CH₂-3-pyridyl- | 0 | H | CH₃ | CH₃ | H | Elektronenpaar |
| I-A-120 | -CH₂-2-furanyl- | 0 | H | CH₃ | CH₃ | H | Elektronenpaar |
| I-A-121 | 3,4-Dichlor-benzyl | 0 | H | CH₃ | CH₃ | H | Elektronenpaar |
| I-A-122 | -CH₂-5-(2-chlor)-pyridyl- | 0 | H | CH₃ | CH₃ | H | Elektronenpaar |
| I-A-123 | 2,6-Difluorbenzyl | 0 | H | CH₃ | CH₃ | H | Elektronenpaar |
| I-A-124 | 2-Fluor-6-methoxy-benzyl | 0 | H | CH₃ | CH₃ | H | Elektronenpaar |
| I-A-125 | 2,6-Dichlor-benzyl | 0 | H | CH₃ | CH₃ | H | Elektronen paar |
| I-A-126 | 2-Chlor-6-trifluormethylbenzyl | 0 | H | CH₃ | CH₃ | H | Elektronenpaar |
| I-A-127 | 2-Chlor-6-fluorbenzyl | 0 | H | CH₃ | CH₃ | H | Elektronenpaar |
| I-A-128 | -CH₂-2-(4,6-dimethoxy)-pyrimidyl | 0 | H | CH₃ | CH₃ | H | Elektronenpaar |
| I-A-129 | 2,6-Dimethylbenzyl | 0 | H | CH₃ | CH₃ | H | Elektronenpaar |
| I-A-130 | Benzyl | 1 | H | CH₃ | CH₃ | H | Elektronenpaar |
| I-A-131 | -CH₂-1-(3-nitro-5-methyl)-pyrazolyl | 0 | H | CH₃ | CH₃ | H | Elektronenpaar |
| I-A-132 | -CH₂-CO-NH-c-propyl | 0 | H | CH₃ | CH₃ | H | Elektronenpaar |
| I-A-133 | -CH₂-CONH-C(CH₃)₂-CO₂CH₃ | 0 | H | CH₃ | CH₃ | H | Elektronenpaar |
| I-A-135 | 2-(5-Methyl)-oxdiazolyl- | 0 | H | CH₃ | CH₃ | H | Elektronenpaar |
| I-A-138 | 3-[4-Methyl-5-(trifluormethyl)]-1,2,4-triazolyl- | 0 | H | CH₃ | CH₃ | H | Elektronenpaar |
| I-A-139 | 3-[4-Methyl-5-(difluormethyl)]-1,2,4-triazolyl- | 0 | H | CH₃ | CH₃ | H | Elektronenpaar |
| I-A-140 | 2-(5-Phenyl)-1.3.4-thiadiazolyl- | 0 | H | CH₃ | CH₃ | H | Elektronenpaar |
| I-A-141 | 2-(1-Methyl-5-phenyl)-imidazolyl- | 0 | H | CH₃ | CH₃ | H | Elektronenpaar |
| I-A-142 | 2-(4,5-Dimethyl)-oxazolyl- | 0 | H | CH₃ | CH₃ | H | Elektronenpaar |
| I-A-143 | 2-(1-Methyl-5-methoxycarbonyl)-imidazolyl | 0 | H | CH₃ | CH₃ | H | Elektronenpaar |
| I-A-144 | 2-(1-Methyl)- imidazolyl- | 0 | H | CH₃ | CH₃ | H | Elektronenpaar |
| I-A-145 | 1,2-Ethandiyl- | 0 | H | CH₃ | CH₃ | H | Elektronenpaar |
| I-A-146 | 2-Pyrimidyl- | 0 | H | C₂H₅ | CH₃ | H | Elektronenpaar |

Analytische Daten zu den in der Tabelle 1 angegebenen Verbindungen
Ab Beispiel I-A-17 sind die NMR-Daten nach dem NMR-Peak-Listenverfahren erstellt worden.

Die 1H-NMR-Daten ausgewählter Beispiele werden in Form von 1H-NMR-Peaklisten notiert. Zu jedem Signalpeak wird erst der δ-Wert in ppm und dann die Signalintensität in runden Klammern aufgeführt. Die δ-Wert - Signalintensitäts- Zahlenpaare von verschiedenen Signalpeaks werden durch Semikolons voneinander getrennt aufgelistet.

Die Peakliste eines Beispieles hat daher die Form:
δ1 (Intensität1); δ2 (Intensität2);........; δi (Intensitäti);......; δn (Intensitätn)

Die Intensität scharfer Signale korreliert mit der Höhe der Signale in einem gedruckten Beispiel eines NMR-Spektrums in cm und zeigt die wirklichen Verhältnisse der Signalintensitäten. Bei breiten Signalen können mehrere Peaks oder die Mitte des Signals und ihre relative Intensität im Vergleich zum intensivsten Signal im Spektrum gezeigt werden.

Zur Kalibrierung der chemischen Verschiebung von 1H-NMR-Spektren benutzen wir Tetramethylsilan und/oder die chemische Verschiebung des Lösungsmittels, besondern im Falle von Spektren, die in DMSO gemessen werden. Daher kann in NMR-Peaklisten der Tetramethylsilan-Peak vorkommen, muss es aber nicht.

Die Listen der 1H-NMR-Peaks sind ähnlich den klassischen 1H-NMR-Ausdrucken und enthalten somit gewöhnlich alle Peaks, die bei einer klassischen NMR-Interpretation aufgeführt werden.

Darüber hinaus können sie wie klassische 1H-NMR-Ausdrucke Lösungsmittelsignale, Signale von Stereoisomeren der Zielverbindungen, die ebenfalls Gegenstand der Erfindung sind, und/oder Peaks von Verunreinigungen zeigen.

Bei der Angabe von Verbindungssignalen im Delta-Bereich von Lösungsmitteln und/oder Wasser sind in unseren Listen von 1H-NMR-Peaks die gewöhnlichen Lösungsmittelpeaks, zum Beispiel Peaks von DMSO in DMSO-D6 und der Peak von Wasser, gezeigt, die gewöhnlich im Durchschnitt eine hohe Intensität aufweisen.

Die Peaks von Stereoisomeren der Targetverbindungen und/oder Peaks von Verunreinigungen haben gewöhnlich im Durchschnitt eine geringere Intensität als die Peaks der Zielverbindungen (zum Beispiel mit einer Reinheit von >90%).

Solche Stereoisomere und/oder Verunreinigungen können typisch für das jeweilige Herstellungsverfahren sein. Ihre Peaks können somit dabei helfen, die Reproduktion unseres Herstellungsverfahrens anhand von "Nebenprodukt-Fingerabdrücken" zu erkennen.

Einem Experten, der die Peaks der Zielverbindungen mit bekannten Verfahren (MestreC, ACD-Simulation, aber auch mit empirisch ausgewerteten Erwartungswerten) berechnet, kann je nach Bedarf die Peaks der Zielverbindungen isolieren, wobei gegebenenfalls zusätzliche Intensitätsfilter eingesetzt werden. Diese Isolierung wäre ähnlich dem betreffenden Peak-Picking bei der klassischen 1H-NMR-Interpretation.

Weitere Details zu 1H-NMR-Peaklisten können der Research Disclosure Database Number 564025 entnommen werden.

| **Bsp.-Nr.** | **logP[a]** | **logP[b]** | **1H-NMR; δ (ppm)** |
|---|---|---|---|
| I-A-4 | 1,55 | 1,97 | ¹H-NMR (CD₃CN 400MHz); δ = 2.31 (s, 6H), 3.90 (s, 3H), 6.92 (s, 1H), 7.33-7.37 (m, 1H), 7.85 (s, 1H) 8.12-8.15 (m, 1H), 8.33-8.34 (d, 1H), 8.77 (br, 1H), 8.826-8.831 (d, 1H) ppm. |
| I-A-5 | 1,67 | 1,99 | ¹H-NMR (CD₃CN, 400MHz); δ = 0.87 (t, 3H), 1.34-1.42 (m, 2H), 1.53-1.61(m, 2H), 3.02-3.06 (m, 2H), 3.38 (s 3H), 3,70 (s, 3H), 6.24 (s, 1H), 7.35-7.38 (m, 1H), 7.66-7.69 (m, 1H) 8.407-8.413 (d, 1H), 8.45-8.47 (m, 1H) ppm. |
| I-A-6 | 2.03 | 2.33 | ¹H-NMR (CD₃CN, 400MHz); δ = 0.86 (t, 3H), 1.26-1.36 (m, 4H), 1.57-1.61(m, 2H), 3.02-3.06 (m, 2H), 3.38 (s 3H), 3,71 (s, 3H), 6.25 (s, 1H), 7.35-7.38 (m, 1H), 7.66-7.69 (m, 1H) 8.41-8.42 (d, 1H), 8.45-8.47 (m, 1H) ppm. |
| I-A-7 | 1,68 | 1,91 | ¹H-NMR (CD₃CN, 400MHz); δ = 3.37 (s 3H), 3,53 (s, 3H), 4.18 (s, 2H), 6.28 (s, 1H), 7.16-7.18 (m, 2H), 7.25-7.30 (m, 3H), 7.37-7.39 (m, 1H), 7.66-7.68 (m, 1H) 8.380-8.384 (m, 1H), 8.47-8.48 (m, 1H) ppm. |
| I-A-8 | 0.45 | 0.67 | ¹H-NMR (CD₃CN, 400MHz); δ = 3.25 (s 3H), 3,42 (s, 3H), 4.05 (s, 3H), 6.45 (s, 1H), 7.34-7.39 (m, 1H), 7.68-7.71 (m, 1H), 8.435-8.441 (d, 1H), 8.47-8.49 (m, 1H) ppm. |
| I-A-9 | 2.0 | 2.12 | ¹H-NMR (d6-DMSO,400MHz); δ = 2.41 (s, 3H), 3.39 (s, 3H), 3.75 (s, 3H), 6.48 (s, 1H), 6.76 (d, 1H), 6.92-6.93 (m, 1H), 7.12-7.14 (m, 1H), 7.25 (t, 1H), 7.43-7.46 (m, 1H), 7.84-7.87 (m, 1H), 8.48-8.53 (m, 2H) ppm. |
| I-A-10 | 1,41 | 1,54 | ¹H-NMR (CD₃CN 400MHz); δ = 3.39 (s 3H), 3,76 (s, 3H), 3.80-3.89 (q, 2H), 6.29 (s, 1H), 7.34-7.38 (m, 1H), 7.66-7.69 (m, 1H), 8.406-8.411 (d, 1H), 8.46-8.47 (m, 1H) ppm. |
| I-A-11 | 1,55 | 1,75 | ¹H-NMR (d₆-DMSO,400MHz); δ = 3.39 (s, 3H), 3.73 (s, 3H), 6.48 (s, 1H), 7.00-7.16 (m, 2H), 7.26-7.28 (m, 1H), 7.31-7.35 (m, 2H), 7.43-7.46 (m, 1H),7.83-7.86 (m, 1H), 8.48-8.52 (m, 2 H) ppm |
| I-A-12 | 1,89 | 2.03 | ¹H-NMR (d₆-DMSO,400MHz); δ = 2,27 (s, 3H),3.38 (s, 3H), 3.72 (s, 3H), 6.42 (s, 1H), 7.05-7.07 (m, 4H), 7.42-7.45 (m, 1H), 7.81-7.85 (m, 1H), 8.47-8.50 (m, 2 H) ppm |
| I-A-13 | 1,25 | 1,31 | ¹H-NMR (d₆-DMSO,40OMHz); δ = 3.46 (s, 3H), 3.68 (s, 3H), 7.34-7.43 (m, 2H), 7.75-7.77 (m, 1H), 8.44-8.47 (m, 2H), 8.66-8.68 (m, 2 H) ppm |
| I-A-14 | 1,61 | 1,87 | ¹H-NMR (d₆-DMSO, 400MHz); δ = 1.10-1.14 (t, 3H), 2.47-2.50 (m, 2H), 2.71-2.73 (m, 2H), 3.22-3.26 (m, 2H), 3.36 (s, 3H), 3.67 (s, 3H), 6.24 (s, 1H), 7.42-7.45 (m, 1H), 7.82-7.85 (m, 1H), 8.46-8.50 (m, 2H) ppm |
| I-A-15 | 1,24 | 1,34 | ¹H-NMR (d₆-DMSO,400MHz); δ = 3.45 (s, 3H), 3.70 (s, 3H), 7.35-7.43 (m, 2H), 7.76-7.78 (m, 1H), 8.44-8.47 (m, 2H), 8.67-8.68 (m, 2H) ppm |
| I-A-16 | 1,68 | 1,78 | ¹H-NMR (d₆-DMSO,400MHz); δ = 3.42 (s, 3H), 3.72 (s, 3H), 6.26 (d, 1H), 6.66 (s, 1H), 7.47-7.51 (m, 2H), 7.61-7.65 (m, 1H), 7.87-7.90 (m, 1H), 8.30-8.32 (m, 1H), 8.51-8.55 (m, 2 H) ppm |
| I-A-17 | 1,89 | 2,26 | ¹H-NMR(400,0 MHz, d₆-DMSO): δ= 8,491(2,5);8,485(2,6);8,477(1,9);8,474(1,9);8,465(1,8);8,462(1,8);7,844(0,9);7,841(1,2);7,838(1,2);7,834(1,0);7,824(1,1);7,820(1,3);7,818(1,3);7,814(1,1);7,450(1,3);7,438(1,3);7,430(1,3);7,418(1,2);6,283(4,0);5,754(1,4);3,689(16,0);3,431(0,5);3,406(0,9);3,397(0,7);3,364(15,4);3,321(4,2);2,506(23,8);2,502(30,5);2,498(23,9);1,885(1,2);1,858(1,6);1,669(1,3);1,662(1,2);1,650(1,3);1,642(1,2);1,539(0,6);1,527(0,6);1,512(0,7);1,394(0,4);1,388(0,4);1,362(1,2);1,335(1,9);1,301(1,7);1,270(1,3);1,241(1,2);1,211(0,6);1,186(0,5);0,000(27,0) |
| I-A-18 | 0,76 | 1,14 | ¹H-NMR(400,0 MHz, d₆-DMSO): δ= 8,502(1,8);8,495(1,8);8,484(1,2);8,481(1,3);8,473(1,2);8,469(1,3);7,857(0,6);7,854(0,8);7,851(0,8);7,848(0,7);7,837(0,7);7,833(0,8);7,831((0,9);7,827(0,7);7,460(1,0);7,448(1,0);7,440(0,9);7,428(0,9);6,230(3,2);3,677(12,9);3,504(1,7);3,488(4,1);3,473(2,2);3,361(12,2);3,325(12,0);3,240(2,1);3,224(3,9);3,209(16,0);3,166(0,8);2,525(0,3);2,508(16,1);2,503(21,6);2,499(16,3);0,008(0,4);0,000(11,1);-0,008(0,5) |

| **Bsp.-Nr.** | **logP[a]** | **logP[b]** | **1H-NMR ;δ (ppm)** |
|---|---|---|---|
| I-A-19 | 1,23 | 1,53 | ¹H-NMR(400,0 MHz, d₆-DMSO): δ= 8,491(2,1);8,485(2,2);8,478(1,6);8,474(1,7);8,466(1,6);8,462(1,6);7,847(0,8);7,843(1,0);7,841(0,9);7,837(0,8);7,827(1,0);7,823(1,1);7,820(1,1);7,817(0,9);7,452(1,1);7,440(1,2);7,431(1,1);7,419(1,0);6,295(3,7);3,702(15,9);3,597(0,4);3,580(1,1);3,563(1,5);3,547(1,1);3,530(0,5);3,368(14,9);3,318(18,5);2,510(12,4);2,506(23,6);2,502(30,4);2,497(22,5);1,244(16,0);1,227(15,8);0,000(5,4) |
| I-A-20 | 1,04 | 1,35 | ¹H-NMR(400,0 MHz, d₆-DMSO): δ= 8,502(1,9);8,496(1,9);8,483(1,4);8,479(1,4);8,471(1,4);8,467(1,4);7,854(0,7);7,850(0,8);7,848(0,8);7,844(0,7);7,834(0,8);7,830(0,9);7,827(0,9);7,824(0,8);7,458(1,0);7,446(1,0);7,437(1,0);7,426(0,9);6,234(3,4);3,679(14,0);3,361(14,6);3,344(4,7);3,325(7,3);3,218(0,4);3,187(16,0);3,080(1,9);3,063(3,1);3,044(2,0);2,512(7,4);2,507(14,8);2,503(19,6);2,499(14,4);2,494(7,3);1,817(0,5);1,801(1,5);1,783(2,0);1,766(1,4);1,750(0,5);0,000(8,5);-0,008(0,4) |
| I-A-21 | 1,31 | 1,60 | ¹H-NMR(400,0 MHz, d₆-DMSO): δ= 8,500(2,2);8,494(2,3);8,483(1,6);8,480(1,7);8,471(1,6);8,468(1,6);7,852(0,8);7,848(1,0);7,846(1,0);7,842(0,8);7,831(0,9);7,828(1,1);7,825(1,1);7,822(0,9);7,457(1,3);7,445(1,3);7,437(1,2);7,425(1,1);6,230(4,0);3,677(16,0);3,361(15,2);3,320(9,3);3,034(2,5);3,016(4,2);2,999(2,6);2,687(0,4);2,674(0,5);2,507(13,7);2,502(18,1);2,498(13,6);1,596(1,4);1,578(2,7);1,560(2,7);1,542(1,5);1,524(0,3);0,922(4,2);0,904(8,3);0,885(3,8);0,000(7,6);-0,008(0,3) |
| I-A-22 | 1,04 | 1,41 | ¹H-NMR(400,0 MHz, d₆-DMSO): δ= 8,499(2,2);8,493(2,2);8,484(1,6);8,480(1,6);8,472(1,6);8,468(1,6);7,853(0,8);7,849(1,0);7,846(1,0);7,843(0,8);7,832(0,9);7,829(1,1);7,826(1,1);7,822(0,9);7,458(1,2);7,446(1,2);7,438(1,1);7,426(1,0);6,232(4,0);3,680(16,0);3,537(2,1);3,521(4,8);3,506(2,5);3,417(1,4);3,399(4,4);3,382(4,5);3,361(15,2);3,311(8,2);3,229(2,4);3,213(4,6);3,197(2,0);2,511(7,8);2,506(15,6);2,502(20,5);2,498(14,9);2,493(7,4);1,080(4,5);1,063(8,9);1,045(4,3);0,008(0,4);0,000(10,1);-0,008(0,4) |
| I-A-23 | 0,67;0,71 | | ¹H-NMR(400,0 MHz, CD3CN): δ= 8,464(1,6);8,452(1,6);8,411(2,2);8,404(2,3);7,680(0,8);7,676(1,1);7,672(0,8);7,660(0,9);7,655(1.2);7.651(0.9);7,376(1,3);7,364(1,3);7,355(1,2);7,343(1,2);6,185(4,3);3,961(10,2);3,726(16,0);3,371(16,0);2,166(20,2);1,962(0,4);1,956(0,5);1,950(2,5);1,944(4,5);1,938(6,1);1,932(4,2);1,926(2,1);0,007(0,4);0,000(10,5);-0,001(10,5);-0,008(0,5) |
| I-A-24 | 1,20 | 1,34 | ¹H-NMR(400,0 MHz, CD3CN): δ= 8,468(1,4);8,464(1,5);8,456(1,5);8,452(1,5);8,412(2,0);8,406(2,1);7,687(0,8);7,683(1,0);7,680(0,9);7,677(0,8);7,666(1,0);7,662(1,1);7,660(1,1);7,656(0,9);7,377(1,2);7,365(1,2);7,357(1,1);7,345(1,0);6,224(3,9);4,102(1,4);4,084(4,0);4,067(4,1);4,049(1,4);3,820(10,7);3,738(16,0);3,375(16,0);2,502(1,1);2,182(0,3);1,957(0,3);1,951(1,8);1,945(3,2);1,939(4,3);1,932(3,0);1,926(1,6);1,174(4,5);1, 156(8,5);1,138(4,3);0,000(5,4) |
| I-A-25 | 0,88 | 1,09 | ¹H-NMR(400,0 MHz, CD3CN): δ= 8,471(1,3);8,468(1,3);8,459(1,3);8,456(1,3);8,411(1,8);8,405(1,8);7,688(0,8);7,684(0,9);7,682(1,0);7,678(0,8);7,668(0,9);7,664(1,0);7,661(1,0);7,658(0,8);7,380(1,0);7,368(1,0);7,360(0,9);7,348(0,8);6,220(3,4);3,836(9,5);3,734(16,0);3,692(0,3);3,665(0,5);3,660(0,5);3,623(14,8);3,432(0,5);3,389(1,0);3,377(15,4);2,500(2,2);2,135(0,4);2,126(0,4);2,118(0,5);2,112(0,5);2,105(0,5);2,099(0,4);1,962(0,8);1,950(10,0);1,944(18,1);1,938(24,1);1,932(17,0);1,926(8,9);0,008(1,3);0,000(31,4) |
| I-A-26 | 1,23 | 1,55 | ¹H-NMR(400,0 MHz, d₆-DMSO): δ= 8,500(1,9);8,494(1,9);8,483(1,4);8,480(1,4);8,471(1,4);8,468(1,4);7,852(0,7);7,848(0,9);7,845(0,8);7,842(0,7);7,831(0,8);7,828(0,9);7,825(0,9);7,821(0,8);7,456(1,0);7,445(1,0);7,436(1,0);7,424(0,9);6,251(3,6);3,677(14,4);3,363(13,4);3,311(7,4);3,284(1,9);3,270(1,5);3,266(2,2);3,260(1,5);3,246(2,1);3,178(0,4);3,164(0,4);2,711(2,2);2,697(1,5);2,691(2,5);2,689(2,4);2,673(2,1);2,511(4,3);2,506(8,7);2,502(11,5);2,497(8,4);2,493(4,2);2,041(16,0);0,000(2,2) |
| I-A-27 | 1,08; 1,11 | 1,40 | ¹H-NMR(400,0 MHz, CD3CN): δ= 8,463(1,4);8,460(1,5);8,451(1,5);8,448(1,5);8,400(2,0);8,394(2,1);7,673(0,8);7,669(1,0);7,666(1,0);7,663(0,8);7,652(0,9);7,648(1,1);7,646(1,1);7,642(0,9);7,374(1,2);7,362(1,2);7,353(1,1);7,342(1,0);6,264(3,9);5,911(0,6);5,904(0,3);5,893(0,3);5,886(0,9);5,869(1,0);5,861(0,4);5,851(0,4);5,844(0,7);5,826(0,3);5,096(1,3);5,093(1,3);5,054(1,2);5,051(1,2);5,026(1,4);5,024(1,4);5,001(1,3);4,999(1,4);3,725(16,0);3,658(3,4);3,641(3,3);3,379(16,0);2,166(6,6);1,957(0,7);1,951(3,2);1,945(5,8);1,939(7,6);1,933(5,3);1,926(2,8);0,000(7,0) |
| I-A-28 | 1,53 | 1,78 | ¹H-NMR(400,0 MHz, CD3CN): δ= 8,463(0,5);8,460(0,6);8,451(0,5);8,448(0,6);8,408(0,7);8,402(0,7);8,034(0,4);7,677(0,4);7,674(0,4);7,670(0,4);7,656(0,4);7,653(0,5);7,650(0,4);7,372(0,5);7,361(0,5);7,352(0,4);7,340(0,4);6,188(1,3);4,259(3,6);3,741(5,4);3,705(0,8);3,390(0,9);3,371(5,4);2,502(2,4);2,138(0,3);1,956(0,7);1,950(3,2);1,944(5,9);1,938(7,9);1,932(5,8);1,926(3,1);1,118(16,0);1,093(1,3);0,000(7,5) |
| I-A-29 | 0,28 | 1,05 | ¹H-NMR(601,6 MHz, d₆-DMSO): δ= 8,506(2,0);8,502(2,0);8,482(1,4);8,480(1,4);8,475(1,5);8,472(1,4);8,139(1,2);7,843(0,9);7,841(1,0);7,839(1,0);7,837(0,8);7,830(1,0);7,827(1,1);7,826(1,1);7,823(0,9);7,452(1,1);7,444(1,1);7,438(1,0);7,430(1,0);6,202(2,4);5,754(0,6);3,884(8,4);3,697(16,0);3,358(15,5);3,340(0,4);3,333(0,4);3,327(0,4);2,541(11,6);2,507(5,9);2,504(11,9);2,501(16,0);2,498(11,6);2,495(5,5) |
| I-A-30 | 0,87 | 1,22 | ¹H-NMR(400,0 MHz, CD3CN): δ= 8,469(1,5);8,466 (1,5);8,457(1,5);8,454(1,5);8,412(2,0);8,406(2,0);7,689(0,8);7,685(1,0);7,682(1,0);7,679(0,8);7,668(1,0);7,665(1,1);7,662(1,1);7,658(0,9);7,380(1,2);7,379(1,1);7,368(1,2);7,367(1,1);7,360(1,1);7,359(1,0);7,348(1,0);7,347(1,0);6,302(3,7);3,781(6,1);3,775(6,1);3,757(16,0);3,386(16,0);2,466(1,4);2,459(2,6);2,453(1,3);2,170(1,7);1,957(0,4);1,952(1,9);1,946(3,4);1,939(4,5);1,933(3,1);1,927(1,6);0,000(2,7) |
| I-A-31 | 0,97 | 1,28 | ¹H-NMR(400,0 MHz, CD3CN): δ= 8,466(1,3);8,463(1,4);8,455(1,3);8,451(1,4);8,404(1,8);8,398(1,9);7,682(0,7);7,678(0,9);7,676(0,9);7,672(0,8);7,661(0,9);7,658(1,0);7,655(1,0);7,651(0,8);7,378(1,1);7,366(1,1);7,357(1,0);7,345(0,9);6,202(3,5);4,107(10,4);3,737(15,9);3,384(0,4);3,373(16,0);2,354(0,4);2,129(45,1);2,107(1,3);2,096(1,5);2,085(0,6);2,078(0,7);2,066(0,4);1,963(3,4);1,957(8,8);1,951(47,5);1,945(87,0);1,939(117,5);1,933(82,7);1,927(43,6);1,774(0,5);1,767(0,7);1,761(0,5);0,901(1,0);0,893(3,1);0,875(5,6);0,864(3,5);0,856(0,9);0,008(2,1);0,000(65,6) |
| I-A-32 | 0,11 | 0,50 | ¹H-NMR(400,0 MHz, CD3CN): δ= 8,475(1,5);8,472(1,6);8,463(1,6);8,460(1,6);8,418(2,1);8,412(2,2);7,695(1,0);7,690(0,8);7,673(1,1);7,388(1,2);7,376(1,2);7,368(1,1);7,356(1,1);6,258(3,1);3,734(10,9);3,654(9,4);3,383(16,0);3,280(0,7);3,269(0,7);2,149(58,1);2,119(0,5);2,113(0,6);2,106(0,7);2,101(0,5);1,963(2,7);1,957(7,2);1,951(35,9);1,945(64,9);1,939(86,7);1,933(60,5);1,927(31,8);1,774(0,4);1,768(0,5);1,762(0,4);0,007(1,3);0,000(34,8) |
| I-A-33 | 0,33 | 0,62 | ¹H-NMR(600,1 MHz, CD3CN): δ= 8,472(1,1);8,470(1,1);8,464(1,2);8,462(1,1);8,4222(1,4);8,4216(1,4);8,418(1,5);7,691(0,7);7,689(0,8);7,687(0,8);7,685(0,7);7,678(0,7);7,675(0,8);7,673(0,8);7,671(0,7);7,382(0,9);7,381(0,9);7,374(0,9);7,373(0,9);7,368(0,9);7,367(0,8);7,360(0,8);7,359(0,8);6,245(2,1);3,719(16,0);3,652(7,8);3,380(15,5);2,613(6,4);2,605(6,4);2,195(14,1);1,949(1,7);1,945(3,1);1,941(4,5);1,937(3,0);1,932(1,5) |
| I-A-34 | 0,59 | 0,81 | ¹H-NMR(400,0 MHz, CD3CN): δ= 8,464(1,6);8,453(1,6);8,414(2,2);8,408(2,3);7,684(1,1);7,680(0,9);7,664(1,2);7,660(1,0);7,381(1,3);7,369(1,3);7,360(1,2);7,348(1,1);6,228(4,1);4,009(9,2);3,743(16,0);3,378(16,0);3,014(0,4);2,950(14,7);2,867(0,4);2,848(13,5);2,170(28,7);1,963(1,0);1,952(12,6);1,946(23,5);1,940(32,2);1,934(23,6);1,928(12,9);0,000(7,7) |
| I-A-35 | 1,61 | 1,96 | ¹H-NMR(400,0 MHz, CD3CN): δ= 8,469(1,3);8,466(1,4);8,457(1,4);8,454(1,4);8,410(1,9);8,404(1,9);7,693(0,8);7,689(0,9);7,687(0,9);7,683(0,7);7,672(0,9);7,669(1,1);7,666(1,1);7,663(0,8);7,382(1,1);7,370(1,1);7,362(1,1);7,350(1,0);6,259(3,6);5,277(0,5);5,273(0,4);5,260(0,8);5,256(0,9);5,253(0,8);5,240(0,4);5,237(0,5);3,718(14,7);3,694(0,5);3,625(2,9);3,605(2,8);3,379(16,0);2,180(14,8);1,964(0,4);1,958(1,2);1,952(5,8);1,946(10,5);1,940(13,9);1,934(9,7);1,928(5,0);1,670(8,1);1,486(7,9);0,000(4,5) |
| I-A-36 | 0,95 | 1,31 | ¹H-NMR(400,0 MHz, CD3CN): δ= 8,466(1,4);8,463(1,4);8,454(1,5);8,451(1,4);8,415(2,0);8,409(2,0);7,688(0,8);7,685(1,0);7,682(0,9);7,678(0,8);7,668(1,0);7,664(1,1);7,662(1,1);7,658(0,9);7,379(1,1);7,367(1,1);7,359(1,0);7,347(1,0);6,254(3,7);3,748(0,8);3,703(15,9);3,380(16,0);3,069(1,3);3,051(3,9);3,033(4,0);3,014(1,4);2,759(1,5);2,193(4,9);2,119(0,4);2,113(0,4);2,107(0,4);1,963(1,1);1,952(9,8);1,946(17,1);1,940(22,3);1,933(15,6);1,927(8,2);1,322(0,5);1,269(0,4);1,259(4,2);1,241(8,1);1,223(4,0);0,000(3,5) |
| I-A-37 | 1,29 | 1,45 | ¹H-NMR(400,0 MHz, d₆-DMSO): δ= 8,501(2,4);8,4 94(2,5);8,483(1,7);8,480(2,0);8,472(1,8);8,468(2,0);7, 864(1,0);7,861(1,3);7,858(1,4);7,854(1,2);7,844(1,1); 7,840(1,4);7,838(1,5);7,834(1,2);7,450(1,5);7,438(1,5 );7,429(1,4);7,418(1,3);6,592(1,4);3,867(16,0);3,402( 17,1);3,372(1,4);3,321(15,5);2,506(21,8);2,502(29,3); 2,498(23,9);1,621(1,7);1,236(0,8);0,000(6,0) |
| I-A-38 | 0,91 | 1,17 | ¹H-NMR(400,0MHz, d₆-DMSO): δ= 8,504(2,9);8,4 98(2,8);8,486(2,2);8,474(2,2);7,926(0,4);7,919(0,4);7, 856(1,4);7,835(1,5);7,582(1,3);7,456(1,6);7,444(4,1); 7,437(1,6);7,424(1,3);7,305(1,4);6,468(2,4);3,820(16, 0);3,600(0,4);3,390(16,2);3,360(0,6);3,319(42,4);2,68 7(1,3);2,674(1,6);2,505(57,0);2,501(63,0);2,328(0,4); 0,003(2,9);0,000(5,5) |
| I-A-39 | 1,13 | 1,37;1,45 | H-NMR(600,1 MHz, CD3CN): δ= 8,465(0,9);8,463 (0,9);8,457(0,9);8,455(0,9);8,412(0,4);8,407(1,2);8,40 3(1,0);7,679(0,6);7,676(0,6);7,675(0,6);7,672(0,5);7,6 65(0,6);7,663(0,7);7,661(0,7);7,659(0,5);7,373(0,7);7, 372(0,8);7,365(0,8);7,364(0,8);7,359(0,7);7,358(0,7); 7,3513(0,7);7,3505(0,7);6,278(1,5);6,261(0,5);3,762( 2,4);3,754(3,8);3,745(12,4);3,711(13,1);3,698(7,3);3, 680(3,9);3,382(16,0);2,144(3,4);1,955(0,6);1,951(0,6) ;1,947(3,7);1,943(6,4);1,939(9,4);1,935(6,4);1,931(3, 2);1,816(12,2);1,802(3,7);0,000(2,6) |
| I-A-40 | 0,68 | 0,88 | ¹H-NMR(400,0 MHz, CD3CN): δ= 8,479(1,5);8,476 (1,6);8,467(1,6);8,464(1,6);8,431(2,0);8,426(2,1);7,69 5(0,8);7,692(1,1);7,689(1,1);7,686(1,0);7,675(1,0);7,6 71(1,2);7,669(1,2);7,665(1,0);7,379(1,2);7,367(1,3);7, 358(1,2);7,346(1,0);6,495(1,2);4,062(0,5);4,047(16,0) ;3,423(16,0);3,391(1,4);3,372(4,0);3,354(4,1);3,335(1 ,4);2,755(0,8);2,176(7,0);1,952(2,6);1,946(4,5);1,940( 6,0);1,934(4,5);1,928(2,5);1,227(4,2);1,209(8,1);1,19 0(4,0);0,000(0,4) |
| I-A-41 | 0,71 | 1,54 | ¹H-NMR(400,0 MHz, CD3CN): δ= 8,815(2,1);8,810 (2,1);8,651(0,8);8,316(1,6);8,304(1,7);8,139(1,1);8,11 8(1,2);7,742(4,4);7,365(1,2);7,353(1,3);7,344(1,2);7,3 33(1,1);3,804(16,0);3,206(1,3);3,188(3,9);3,170(4,0); 3,151(1,3);3,033(0,4);2,154(170,7);2,113(2,4);2,107( 2,2);2,101(1,6);1,951(60,2);1,945(107,1);1,939(142,1 );1,933(100,9);1,927(53,4);1,779(0,4);1,774(0,7);1,76 8(0,9);1,761(0,6);1,755(0,4);1,357(4,1);1,339(8,0);1,3 20(3,9);0,146(3,0);0,000(555,3);-0,008(49,4);-0,150(3,0) |
| I-A-42 | 0,35 | 0,55 | ¹H-NMR(400,0 MHz, CD3CN): δ= 8,478(1,4);8,475(1,4);8,467(1,5);8,463(1,5);8,430(1,8);8,424(1,9);7,685(0,8);7,682(0,9);7,676(0,8);7,665(0,9);7,662(1,1);7,659(1,1);7,656(0,8);7,378(1,1);7,366(1,1);7,358(1,1);7,346(1,0);6,770(0,3);6,524(0,8);4,179(9,3);4,019(15,9) ;3,425(16,0);2,661(7,6);2,649(7,6);2,147(33,0);2,106(0,3);1,963(1,3);1,951(17,8);1,945(32,6);1,939(44,6);1,933(32,1);1,927(16,9);0,146(1,1);0,000(210,5);-0,008(16,6);-0,150(1,1) |
| I-A-43 | 0,27 | 0,22 | ¹H-NMR(400,0 MHz, CD3CN): δ= 8,480(1,8);8,470(1,8);8,427(2,3);8,421(2,4);7,703(1,2);7,682(1,3);7,389(1,3);7,377(1,4);7,369(1,3);7,357(1,1);6,727(0,5);6,439(1,9);5,446(0,4);4,167(1,9);4,132(2,9);4,010(3,4);3,998(16,0);3,975(2,0);3,816(0,5);3,413(15,8);3,389(0,6);2,652(7,8);2,640(7,7);2,178(4,7);1,951(4,6);1,945(8,2);1,939(11,1);1,933(8,2);1,927(4,4);0,000(52,2);-0,001(52,2) |
| I-A-44 | 0,80 | 1,30 | ¹H-NMR(400,0 MHz, CD3CN): δ= 8,488(1,1);8,485(1,2);8,476(1,2);8,473(1,1);8,386(1,6);8,380(1,6);7,679(0,7);7,675(0,9);7,672(0,9);7,669(0,7);7,658(0,9);7,655(1,0);7,652(1,0);7,648(0,8);7,395(1,0);7,383(1,0);7,375(0,9);7,363(0,9);7,345(0,5);6,170(3,7);4,608(0,5);4,521(0,5);3,898(1,1);3,880(3,5);3,862(4,5);3,844(1,2);3,801(1,1);3,687(15,5);2,499(16,0);2,209(18,0);1,958(0,6);1,953(3,2);1,946(5,8);1,940(7,8);1,934(5,4);1,928(2,8);1,162(3,7);1,144(7,4);1,126(3,6);0,008(0,6);0,000(13,5);-0,008(0,6) |
| I-A-45 | 0,54 | 1,04 | ¹H-NMR(400,0MHz, CD3CN): δ= 8,814(2,0);8,809(1,8);8,600(1,4);8,586(1,4);8,392(1,0);8,389(1,0);8,370(1,1);8,368(1,0);7,949(1,0);7,935(1,1);7,928(1,0);7,914(0,9);7,212(3,0);3,814(15,0);3,501(15,0);2,921(16,0);2,514(1,7);2,042(0,5);1,965(0,6);1,953(7,8);1,947(14,0);1,941(18,5);1,935(12,7);1,928(6,5);0,000(49,4);-0,008(2,5) |
| I-A-46 | 2,09 | 2,17 | ¹H-NMR(400,0 MHz, CD3CN): δ= 8,502(1,4);8,498(1,5);8,490(1,4);8,486(1,4);8,445(1,9);8,439(1,9);7,71 5(0,8);7,711(0,9);7,708(0,9);7,704(0,8);7,694(0,9);7,690(1,0);7,688(1,0);7,684(0,8);7,406(1,1);7,395(1,1);7,386(1,0);7,374(0,9);6,306(3,2);5,477(1,2);4,012(1,5);3,970(2,6);3,969(2,6);3,927(1,6);3,809(1,1);3,797(16,0);3,426(1,2);3,415(15,9);2,170(5,5);1,994(0,4);1,988(0,8);1,982(4,0);1,976(7,1);1,970(9,7);1,964(6,7);1,958(3,4) |
| I-A-47 | 1,65 | 1,84 | ¹H-NMR(400,0 MHz, CD3CN): δ= 8,506(1,3);8,502(1,4);8,494(1,4);8,490(1,4);8,453(1,9);8,447(1,9);7,724(0,8);7,720(0,8);7,718(0,8);7,714(0,7);7,704(0,9);7,700(1,0);7,697(0,9);7,694(0,8);7,414(1,0);7,402(1,1);7,394(1,0);7,382(0,9);6,293(3,6);5,477(2,0);3,730(16,0);3,413(15,8);3,272(2,0);3,253(2,2);3,248(1,0);3,233(2,3);2,646(0,3);2,638(1,0);2,626(0,5);2,623(0,5);2,619(0,9);2,611(1,1);2,599(1,0);2,591(0,9);2,584(0,5);2,572(0,9);2,564(0,3);2,173(3,3);1,994(0,4);1,988(0,8);1,982(4,1);1,976(7,5);1,970(10,3);1,964(7,1);1,958(3,6) |
| I-A-48 | 1,11 | 1,40 | ¹H-NMR(400,0 MHz, CD3CN): δ= 8,497(1,4);8,495(1,6);8,486(1,5);8,483(1,6);8,446(2,2);8,440(2,2);7,718(0,8);7,714(1,0);7,708(0,8);7,697(0,9);7,693(1,1);7,409(1,2);7,397(1,2);7,388(1,1);7,376(1,1);6,282(4,1);5, 478(2,5);4,594(1,3);4,579(2,5);4,565(1,3);4,475(1,3);4,461(2,5);4,446(1,3);3,741(16,0);3,410(16,0);3,181(2,4);3,163(4,4);3,145(2,6);2,204(17,0);2,078(0,3);2,063(0,8);2,045(1,2);2,028(0,8);2,013(0,6);1,995(1,1);1,982(5,2);1,976(9,0);1,970(11,4);1,964(8,6);1,958(4,2);1,948(0,4) |
| I-A-49 | 1,55 | 1,71 | ¹H-NMR(400,0 MHz, CD3CN): δ 8,501(1,9);8,498(1,7);8,489(1,6);8,486(1,3);8,444(2,3);8,438(2,0);7,714(1,1);7,710(1,2);7,708(1,1);7,704(1,0);7,694(1,2);7,690(1,2);7,684(0,8);7,407(1,4);7,396(1,4);7,387(1,2);7,375(1,0);6,305(3,4);6,278(0,4);6,268(0,6);6,146(0,8);6,136(1,3);6,125(0,6);6,014(0,4);6,004(0,6);5,478(3,9);3,826(1,6);3,796(16,0);3,784(3,0);3,742(1,7);3,414(15,2);2,198(8,5);2,194(8,2);1,982(6,0);1,976(8,6);1,970(10,0);1,964(6,5);1,958(3,1) |
| I-A-50 | 1,94 | 2,10 | ¹H-NMR(400,0 MHz, CD3CN): δ 8,498(1,4);8,495(1,4);8,486(1,4);8,483(1,4);8,446(2,0);8,440(2,1);7,720(0,7);7,716(0,9);7,714(0,9);7,710(0,8);7,699(0,9);7,695(1,1);7,693(1,0);7,689(0,9);7,409(1,2);7,397(1,1);7,388(1,0);7,376(1,0);6,276(3,9);5,478(0,8);3,730(16,0);3,410(15,9);3,344(1,2);3,318(5,8);3,300(2.6);3,292(4,3);3,280(2,6);3,266(1,3);2,936(1,9);2,917(2,2);2,899(1,6);2,195(17,4);1,995(0,4);1,988(0,7);1,983(4,5);1,976(8,3);1,970(11,5);1,964(8,0);1,95 8(4,1) |
| I-A-51 | 1,47 | 1,73 | ¹H-NMR(400,0 MHz, CD3CN): δ 8,493(1,3);8,489(1,3);8,481(1,4);8,477(1,4);8,437(1,8);8,431(1,9);7,706(0,8);7,702(0,9);7,700(0,8);7,696(0,7);7,686(0,9);7,682(1,0);7,679(0,9);7,676(0,8);7,403(1,1);7,391(1,1);7,382(1,0);7,370(0,9);6,328(3,7);5,478(3,9);3,762(16,0);3,505(0,5);3,487(0,5);3,479(1,0);3,470(0,5);3,452(0,5);3,429(0,4);3,414(15,8);2,686(2,4);2,677(4,9);2,663(2,7);2,655(1,9);2,628(0,3);2,261(0,4);2,251(0,8);2,241(0,9);2,229(0,6);2,218(1,2);2,208(1,2);2,181(4,9);1,994(0,4);1,988(0,7);1,983(4,1);1,976(7,6);1,970(10,5);1,964(7,2);1,958(3,7);1,806(0,5);1,793(0,5);1,781(0,6);1,772(0,9);1,760(0,7);1,747(0,9);1,738(0,6);1,724(0, 4);1,712(0,4) |
| I-A-52 | 0,90 | 1,25 | ¹H-NMR(400,0 MHz, CD3CN): δ 8,488(1,7);8,477(1,7);8,436(2,4);8,430(2,5);7,708(0,8);7,706(1,0);7,704(1,1);7,700(0,9);7,688(0,9);7,686(1,1);7,684(1,3);7,680(1,0);7,401(1,4);7,390(1,4);7,381(1,3);7,369(1,2);6,329(4,3);5,478(1,8);5,477(1,6);3,894(1,0);3,885(1,7);3,876(1,1);3,865(1,2);3,856(1,9);3,846(1,1);3,769(16,0);3,768(15,2);3,617(0,6);3,607(0,3);3,600(0,6);3,590(1,3);3,580(0,6);3,573(0,4);3,562(0,6);3,434(1,4);3,429(1,5);3,416(16,0);3,414(15,2);3,406(2,8);3,400(2,4);3,377(1,2);3,372(1,2);2,199(18,1);1,993(0,4);1,983(4,0);1,982(4,0);1,977(7,4);1,976(7,1);1,971(10,1);1,969(9,5);1,965(7,2);1,963(6,6);1,958(3,8);1,903(1,1);1,867(1,5);1,673(0,6);1,662(0,6);1,645(1,2);1,635(1,3);1,613(1,1);1,602(1,0);1,585(0,5);1,575(0,5) |
| I-A-53 | 2,68 | 2,76 | ¹H-NMR(400,0 MHz, CD3CN): δ 8,505(1,3);8,502(1,3);8,494(1,3);8,490(1,3);8,453(1,7);8,447(1,8);7,723(0,7);7,719(0,8);7,716(0,8);7,712(0,7);7,702(0,8);7,698(0,9);7,696(0,9);7,692(0,8);7,412(1,0);7,411(1,0);7,400(1,0);7,399(0,9);7,392(0,9);7,391(0,9);7,380(0,8);6,282(3,5);5,478(1,0);3,731(16,0);3,412(15,6);3,304(1,2);3,284(1,2);3,263(1,4);2,694(0,5);2,672(0,5);2,653(0,5);2,643(0,5);2,621(0,5);2,602(0,5);2,181(7,5);1,988(0,7);1,983(3,7);1,976(6,8);1,970(9,4);1,964(6,5);1,958(3,3) |
| I-A-54 | 0,63 | 0,98 | ¹H-NMR(400,0 MHz, CD3CN): δ 8,469(1,4);8,466(1,4);8,457(1,4);8,454(1,4);8,411(1,9);8,405(1,9);7,687(0,8);7,683(0,9);7,680(0,9);7,676(0,8);7,666(0,9);7,662(1,0);7,660(1,0);7,656(0,8);7,380(1,1);7,378(1,0);7,368(1,1);7,366(1,0);7,360(1,0);7,348(1,0);7,346(0,9);6,223(3,6);5,448(1,2);4,940(2,1);4,923(4,1);4,905(2,3);4,602(0,7);4,584(1,3);4,569(1,0);4,564(0,4);4,551(0,5);4,507(2,8);4,491(4,0);4,475(1,8);3,716(16,0);3,377(16,0);2,175(18,4);1,964(0,4);1,958(0,9);1,952(4,4);1,946(8,0);1,940(10,8);1,934(7,4);1,927(3,7);0,008(1,0);0,000(21,4);-0,008(0,7) |
| I-A-55 | 1,01 | 1,27 | 1H-NMR(601,6 MHz, d6-DMSO): δ= 8,632(0,5);8,629(0,5);8,546(0,4);8,539(0,4);8,486(2,2);8,481(2,2);8,478(1,7);8,475(1,5);8,470(1,6);8,467(1,4);7,842(0,8);7,840(1,0);7,838(0,9);7,836(0,8);7,829(0,9);7,826(1,0);7,825(1,0);7,822(0,8);7,448(1,1);7,440(1,1);7,434(1,1);7,426(1,0);6,256(2,7);4,536(8,2);4,427(8,1);4,244(0,7);3,900(3,3);3,790(1,2);3,778(1,8);3,771(3,8);3,765(1,2);3,675(16,0);3,449(2,9);3,361(14,7);3,338(0,9);3,330(1,3);3,306(255,1);3,280(0,3);3,173(0,7);3,164(0,7);2,660(1,3);2,655(0,7);2,646(1,4);2,642(1,3);2,637(1,5);2,628(0,7);2,624(1,4);2,615(1,0);2,612(1,3);2,609(0,9);2,521(2,4);2,518(3,0);2,515(3,1);2,506(79,7);2,503(156,7);2,500(210,1);2,497(156,0);2,494(76,4);2,387(0,9);2,384(1,3);2,381(0,9);2,169(1,5);2,165(0,7);2,157(1,5);2,152(1,3);2,147(1,4);2,139(0,7);2,134(1,3);2,033(0,4);2,010(0,4);0,000(1,3) |
| I-A-56 | 2,86 | 2,92 | ¹H-NMR(400,0 MHz, d₆-DMSO): δ= 8,559(2,4);8,552(2,5);8,499(1,7);8,496(1,8);8,487(1,8);8,484(1,7);7,974(5,6);7,862(0,9);7,858(1,1);7,855(1,0);7,852(0,9);7,841(1,1);7,837(1,2);7,835(1,2);7,831(1,0);7,452(1,4);7,440(1,4);7,431(1,3);7,420(1,2);6,721(5,0);6,558(2,3);5,754(2,1);3,800(16,0);3,758(0,5);3,409(15,5);3,318(23,4);2,671(0,4);2,507(46,4);2,502(60,3);2,498(45,2);2,329(0,4);1,989(0,5);0,008(2,5);0,000(62,0) |
| I-A-57 | 2,02 | 2,14 | ¹H-NMR(400,0 MHz, d₆-DMSO): δ= 8,515(2,4);8,509(2,6);8,495(1,7);8,493(1,8);8,484(1,7);8,481(1,8);8,135(1,5);7,855(0,9);7,851(1,1);7,850(1,1);7,846(1,0);7,834(1,0);7,831(1,3);7,829(1,3);7,825(1,0);7,466(1,4);7,454(1,4);7,446(1,3);7,434(1,3);7,423(0,5);7,416(4,3);7,399(1,6);7,394(4,9);7,115(0,6);7,108(4,8);7,087(4,1);6,475(2,9);5,754(2,7);3,741(16,0);3,392(15,6);3,321(2,5);2,507(15,4);2,503(20,1);2,499(15,7);2,086(8,1);0,008(1,2);0,000(26,8) |
| I-A-58 | 1,62 | 1,79 | ¹H-NMR(400,0 MHz, d₆-DMSO): δ= 8,496(1,9);8,489(2,0);8,478(1,4);8,475(1,5);8,467(1,4);8,463(1,4);7,839(0,7);7,835(0,9);7,832(0,8);7,829(0,7);7,818(0,8);7,814(1,0);7,812(0,9);7,808(0,8);7,448(1,1);7,436(1,1);7,428(1,0);7,416(1,0);7,218(3,3);7,213(1,1);7,201(1,1);7,196(3,9);7,188(0,4);6,944(0,4);6,936(3,9);6,931(1,3);6,919(1,1);6,914(3,3);6,906(0,3);6,344(2,6);4,108(0,4);4,095(1,3);4,082(1,3);4,069(0,5);3,741(16,0);3,736(14,8);3,369(13,3);3,320(9,1);3,177(5,7);3,163(5,5);2,688(0,9);2,675(1,0);2,506(15,5);2,502(20,5);2,497(15,3);0,008(1,1);0,000(27,6);-0,009(1,1) |
| I-A-59 | 1,55 | 2,11 | ¹H-NMR(400,0MHz, d₆-DMSO): δ 8,486(1,2);8,480(1,3);8,469(0,9);8,466(0,9);8,458(0,9);8,454(0,9);8,140(1,9);7,830(0,5);7,827(0,6);7,824(0,6);7,821(0,5);7,810(0,5);7,806(0,6);7,804(0,6);7,800(0,5);7,441(0,7);7,429(0,7);7,421(0,6);7,409(0,6);7,180(2,0);7,175(0,8);7,158(2,1);6,676(2,0);6,654(1,9);6,263(1,7);3,732(8,0);3,357(9,0);2,891(16,0);2,506(13,5);2,502(17,4);2,498(13,4);2,073(0,7);0,007(0,9);0,000(16,4) |
| I-A-60 | 2,55 | 2,58 | ¹H-NMR(400,0 MHz, d₆-DMSO): δ= 8,548(2,3);8,542(2,3);8,495(1,6);8,491(1,7);8,483(1,7);8,479(1,7);7,853(0,9);7,850(1,1);7,847(1,0);7,843(0,9);7,833(1,0);7,829(1,2);7,827(1,2);7,823(1,0);7,517(1,7);7,512(3,1);7,508(1,7);7,446(1,3);7,434(1,3);7,425(1,2);7,414(1,1);7,078(6,4);7,074(6,3);6,542(2,3);5,754(1,8);3,784(16,0);3,403(15,3);3,318(15,9);2,524(0,7);2,511(12,8);2,507(24,6);2,502(31,9);2,498(24,0);2,086(1,3);0,000(0,4) |
| I-A-61 | | | ¹H-NMR(400,0 MHz, d₆-DMSO): δ= 8,531(3,6);8,525(3,7);8,493(2,5);8,481(2,5);7,928(1,0);7,921(1,0);7,860(1,3);7,856(1,7);7,854(1,7);7,840(1,4);7,837(1,6);7,758(0,8);7,747(0,9);7,638(1,2);7,619(2,3);7,591(1,6);7,571(2,2);7,552(0,9);7,459(3,2);7,446(2,1);7,434(1,9);7,425(1,5);7,413(1,4);7,315(1,7);7,295(1,5);7,090(0,5);7,079(0,5);7,070(0,7);7,058(0,6);6,862(0,6);6,859(0,6);6,855(0,6);6,838(0,5);6,515(3,0);6,209(1,0);5,843(0,4);5,755(1,1);3,774(16,0);3,750(5,7);3,398(15,7);3,377(5,8);3,353(0,5);3,321(30,3);2,688(3,8);2,675(4,0);2,502(54,8);2,328(0,4);0,032(0,3);0,000(38,1) |
| I-A-62 | 1,66 | 1,87 | ¹H-NMR(400,0 MHz, d₆-DMSO): δ= 8,509(2,4);8,503(2,6);8,489(1,8);8,486(1,8);8,477(1,9);8,474(1,8);7,929(0,4);7,923(0,4);7,849(0,9);7,845(1,2);7,843(1,2);7,839(1.0);7,828(1,1);7,825(1,3);7,822(1,3);7,819(1,0);7,762(0,3);7,751(0,3);7,458(1,4);7,446(1,4);7,438(1,3);7,426(1,3);7,222(10,5);7,207(5,0);7,202(5,1);7,186(0,3);7,179(0,4);6,427(3,0);5,755(3,7);3,747(16,0);3,384(15,4);3,323(5,6);2,689(1,3);2,677(1,4);2,507(16,7);2,503(20,8);0,008(0,9);0,000(18,4) |
| I-A-63 | 2,75 | 2,92 | ¹H-NMR(400,0 MHz, d₆-DMSO): δ= 8,516(0,8);8,509(0,8);8,490(0,6);8,487(0,6);8,478(0,6);8,475(0,6);7,854(0,4);7,837(0,3);7,833(0,4);7,831(0,4);7,460(0,5);7,448(0,5);7,440(0,4);7,428(0,4);7,367(1,4);7,346(1,6);7,071(1,6);7,050(1,4);6,424(1,0);3,740(5,6);3,384(5,5);3,320(5,7);3,177(0,4);3,163(0,4);2,506(7,0);2,502(9,2);2,498(6,8);1,246(16,0);1,074(0,4);1,057(0,9);1,039(0,4);0,000(5,8) |
| I-A-64 | 2,63 | 2,71 | ¹H-NMR(400,0 MHz, d₆-DMSO): δ= 8,526(2,8);8,520(2,9);8,493(2,2);8,482(2,2);7,854(1,5);7,852(1,4);7,834(1,6);7,832(1,5);7,702(2,3);7,681(2,5);7,592(2,9);7,589(2,9);7,448(1,5);7,436(1,6);7,428(1,5);7,416(1,4);7,330(1,6);7,309(1,5);6,514(3,0);5,756(3,5);5,755(4,1);3,778(16,0);3,398(15,7);3,322(15,0);2,503(30,6);0,000(22,0) |
| I-A-65 | 1,01 | 1,18 | ¹H-NMR(400,0 MHz, d₆-DMSO): δ= 8,535(2,3);8,529(2,4);8,505(1,6);8,502(1,7);8,493(1,7);8,490(1,7);8,397(1,3);8,387(1,3);8,384(1,3);7,878(0,8);7,874(1,0);7,873(1,0);7,869(0,9);7,858(1,0);7,854(1,1);7,852(1,1);7,848(0,9);7,727(0,8);7,722(0,8);7,707(1,4);7,703(1,5);7,688(0,9);7,683(0,9);7,479(1,3);7,468(1,3);7,459(1,3);7,447(1,2);7,229(1,1);7,217(1,2);7,211(1,2);7,198(1,0);6,814(2,0);6,794(1,9);6,542(2,2);3,753(16,0);3,414(15,4);3,320(14,3);2,507(21,1);2,502(27,9);2,498(21,2);0,008(0,6);0,000(13,8) |
| I-A-66 | 1,97 | 2,11 | ¹H-NMR(400,0 MHz, d₆-DMSO): δ= 8,532(2,3);8,526(2,4);8,495(1,6);8,491(1,7);8,483(1,7);8,479(1,7);7,858(0,8);7,854(1,0);7,852(1,0);7,848(0,9);7,838(0,9);7,834(1,1);7,832(1,1);7,828(1,0);7,456(1,3);7,444(1,3);7,435(1,2);7,423(1,2);7,380(0,7);7,360(2,2);7,341(3,1);7,335(2,7);7,332(1,7);7,320(0,5);7,315(0,7);7,312(0,4);7,136(1,5);7,131(2,8);6,993(0,9);6,989(1,7);6,986(1,0);6,976(0,9);6,971(1,5);6,967(0,9);6,512(2,5);5,755(1,2);3,760(16,0);3,398(15,4);3,321(12,6);2,507(17,4);2,502(23,0);2,498(17,5);2,086(1,8);0,007(0,5);0,000(13,1) |
| I-A-67 | 1,17 | 1,25 | ¹H-NMR(400,0 MHz, d₆-DMSO): δ= 8,513(2,1);8,506(2,2);8,490(1,5);8,487(1,6);8,478(1,5);8,475(1,5);8,137(1,7);7,859(0,8);7,855(1,0);7,853(1,0);7,849(0,9);7,839(1,0);7,835(1,1);7,832(1,1);7,829(0,9);7,460(1,3);7,448(1,3);7,439(1,2);7,428(1,2);7,342(2,1);7,334(2,2);7,327(2,4);7,319(3,7);7,309(0,9);7,297(2,1);7,290(1,0);7,287(0,8);7,283(1,1);7,274(0,7);6,823(1,3);6,815(1,3);6,810(1,0);6,801(1,3);6,450(2,7);5,754(5,4);3,712(16,0);3,700(0,4);3,388(15,4);3,319(3,8);2,988(11,2);2,722(11,2);2,671(0,3);2,524(0,7);2,510(18,9);2,50 6(38,0);2,502(50,3);2,497(37,5);2,328(0,3);0,008(1,9);0,000(48,7);-0,008(2,3) |
| I-A-68 | 1,71 | 1,77 | ¹H-NMR(400,0 MHz, d₆-DMSO): δ= 8,539(2,1);8,534(2,2);8,493(1,6);8,490(1,7);8,482(1,7);8,478(1,6);8,136(0,5);8,108(1,0);8,106(1,1);8,102(1,1);8,100(1,0);8,087(1,1);8,085(1,2);8,082(1,3);8,080(1,1);7,881(1,5);7,876(2,6);7,871(1,5);7,857(0,9);7,853(1,1);7,850(1,0);7,847(0,9);7,836(1,0);7,833(1,1);7,830(1,1);7,826(1,0);7,653(1,3);7,633(2,6);7,613(1,5);7,513(1,3);7,511(1,5);7,509(1,5);7,507(1,3);7,494(1,0);7,491(1,1);7,489(1,1);7,487(0,9);7,454(1,2);7,453(1,2);7,443(1,2);7,441(1,2);7,434(1,1);7,433(1,1);7,422(1,1);7,421(1,0);6,544(2,3);3,783(16,0);3,404(15,3);3,311(4,4);2,524(0,4);2,511(8,9);2,507(17,7);2,502(23,0);2,498(17,0);2 ,073(10,4);0,000(1,9) |
| I-A-69 | 1,56 | 1,63 | ¹H-NMR(400,0 MHz, d₆-DMSO): δ= 8,529(1,0);8,522(1,1);8,513(0,7);8,510(0,8);8,502(0,8);8,498(0,8);8,151(0,3);7,886(0,4);7,882(0,5);7,880(0,5);7,876(0,4);7,866(0,5);7,862(0,5);7,859(0,5);7,856(0,5);7,851(0,7);7,847(0,7);7,831(0,8);7,828(0,8);7,528(0,3);7,513(0,6);7,510(0,6);7,494(0,5);7,489(0,8);7,476(0,6);7,467(0,5);7,455(0,6);7,451(0,6);7,448(0,6);7,429(0,8);6,598(1,0);6,354(0,8);6,334(0,7);5,754(1,0);3,708(7,2);3,414(6,8);3,318(4,7);2,815(16,0);2,524(0,6);2,510(11,6);2,506(23,5);2,502(31,1);2,497(23,2);2,493(11,8);0,008(1,1);0,000(29,5);-0,008(1,3) |
| I-A-70 | 1,45 | 1,52 | ¹H-NMR(400,0 MHz, d₆-DMSO): δ= 8,513(2,0);8,508(2,1);8,485(1,5);8,482(1,6);8,473(1,6);8,470(1,6);8,233(2,1);8,227(2,1);8,226(2,1);8,141(0,4);7,853(0,8);7,849(1,0);7,847(1,0);7,843(0,9);7,833(1,0);7,829(1,1);7,826(1,1);7,823(1,0);7,648(1,4);7,641(1,4);7,627(1,9);7,620(1,9);7,521(2,5);7,520(2,6);7,500(1,8);7,499(1,8);7,452(1,1);7,441(1,1);7,440(1,1);7,433(1,1);7,431(1,1);7,421(1,0);7,419(1,0);6,435(2,6);5,751(1,2);3,786(16,0);3,388(15,3);3,309(13,5);2,524(0,7);2,510(17,8);2,506(35,9);2,501(47,4);2,497(35,8);0,000(3,7) |
| I-A-71 | 1,68 | 1,78 | ¹H-NMR(400,0 MHz, d₆-DMSO): δ= 8,513(2,5);8,507(2,4);8,498(0,9);8,487(2,1);8,484(2,1);8,475(1,7);8,472(1,6);7,855(1,2);7,851(1,3);7,849(1,3);7,845(1,3);7,835(1,3);7,831(1,3);7,828(1,2);7,825(1,0);7,456(1,7);7,444(1,6);7,435(1,5);7,423(1,2);7,397(0,4);7,392(0,5);7,383(0,6);7,376(1,0);7,358(1,1);7,354(0,8);7,344(0,6);7,340(0,5);7,312(1,1);7,309(1,1);7,292(0,9);7,288(1,5);7,285(1,1);7,267(0,7);7,264(0,6);7,188(1,1);7,185(1,1);7,169(1,7);7,166(1,6);7,151(0,9);7,147(0,8);6,920(0,9);6,916(1,0);6,900(1,6);6,896(1,5);6,881(0,7);6,877(0,7);6,438(2,9);6,237(0,4);3,779(3,2);3,767(16,0);3,674(1,6);3,400(3,2);3,389(15,6);3,373(0,4);3,360(1,6);3,322(3,5);3,311(12,5);3,037(0,4);2,510(27,1);2,506(37,5);2,501(41,6);2,497(28,4);0,858(0,5);0,840(0,9);0,821(0,4);0,011(0,5);0,000(2,8) |
| I-A-72 | 1,59 | 1,74 | ¹H-NMR(400,0 MHz, d₆-DMSO): δ= 8,522(3,3);8,492(2,4);8,481(2,4);7,867(1,6);7,847(1,8);7,467(1,5);7,455(1,7);7,447(1,5);7,435(1,3);7,249(1,0);7,229(2,2);7,210(1,3);7,051(2,7);7,031(2,2);6,879(1,3);6,860(2,5);6,841(1,3);6,475(3,7);6,421(2,3);6,402(2,2);5,754(1,3);3,819(16,0);3,732(16,0);3,398(15,7);3,321(8,9);2,502(36,3);0,000(2,8) |
| I-A-73 | 1,81 | 1,87 | ¹H-NMR(400,0 MHz, d₆-DMSO): δ= 8,509(2,2);8,503(2,2);8,460(1,6);8,457(1,7);8,448(1,7);8,445(1,7);7,927(1,2);7,923(2,5);7,918(2,0);7,907(1,4);7,888(2,4);7,873(1,4);7,868(1,3);7,850(0,9);7,846(1,1);7,844(1,0);7,840(0,9);7,830(1,0);7,826(1,1);7,824(1,2);7,820(1,0);7,725(1,7);7,705(2,7);7,685(1,2);7,420(1,3);7,408(1,3);7,399(1,2);7,387(1,1);6,583(0,6);4,030(16,0);4,016(0,3);3,388(15,2);3,315(50,4);2,675(0,4);2,670(0,6);2,666(0,5);2,510(36,0);2,506(69,7);2,501(93,6);2,497(72,1);2,333(0,5);2,328(0,6);2,324(0,5);0,146(0,5);0,008(4,9);0,000(99,4);-0,008(4,9);-0,019(0,4);-0,150(0,5) |
| I-A-74 | 1,45 | 1,55 | ¹H-NMR(400,0 MHz, d₆-DMSO): δ= 8,505(2,3);8,499(2,4);8,476(1,7);8,472(1,8);8,464(1,8);8,461(1,9);8,137(2,6);7,842(0,9);7,839(1,1);7,836(1,1);7,832(1,0);7,822(1,1);7,818(1,3);7,816(1,3);7,812(1,3);7,661(0,9);7,657(1,2);7,654(1,7);7,650(3,3);7,645(4,2);7,642(2,9);7,629(2,0);7,610(2,5);7,601(0,5);7,598(0,4);7,589(1,2);7,551(1,3);7,547(2,1);7,543(1,3);7,532(0,9);7,528(1,3);7,525(0,8);7,431(1,3);7,419(1,3);7,411(1,3);7,399(1,2);6,471(1,1);3,907(16,0);3,376(15,3);3,316(5,7);2,670(0,4);2,666(0,3);2,506(46,5);2,501(62,9);2,497(50,5);2,328(0,4);2,324(0,3);0,008(2,8);0,000(63,9) |
| I-A-75 | 2,14 | 2,19 | ¹H-NMR(400,0 MHz, d₆-DMSO): δ= 8,504(0,8);8,498(0,8);8,481(0,6);8,478(0,6);8,470(0,6);8,466(0,6);8,139(0,6);7,839(0,4);7,837(0.4);7,833(0,3);7,823(0,3);7,819(0,4);7,816(0,4);7,813(0,3);7,626(1,1);7,604(1,7);7,519(1,7);7,498(1,1);7,437(0,4);7,425(0,5);7,417(0,4);7,405(0,4);6,459(0,4);3,845(5,4);3,369(5,1);3,316(8,7);2,510(11,9);2,506(23,5);2,501(31,8);2,497(24,8); 1,298(16,0);0,008(1,7);0,000(35,6);-0,008(1,8) |
| I-A-76 | 2,53 | 2,55 | ¹H-NMR(400,0 MHz, d₆-DMSO): δ= 8,515(0,9);8,509(0,9);8,461(0,7);8,449(0,7);7,852(1,6);7,831(2,1);7,697(1,7);7,675(1,3);7,424(0,5);7,412(0,5);7,404(0,6);7,392(0,5);5,752(1,9);4,017(5,4);3,383(5,2);3,371(0,3);3,316(5,0);2,502(9,5);1,300(16,0);0,000(6,2) |
| I-A-77 | | | ¹H-NMR(400,0 MHz, CD3CN): δ= 8,467(1,2);8,464(1,3);8,455(1,3);8,452(1,3);8,400(1,7);8,394(1,7);7,651(0,7);7,647(0,9);7,645(0,8);7,641(0,7);7,630(1,0);7,626(2,2);7,621(3,2);7,617(1,6);7,515(5,3);7,511(4,8);7,355(1,0);7,343(1,0);7,334(0,9);7,322(0,9);6,424(1,2);3,918(16,0);3,391(16,0);2,142(23,5);2,113(1,1);2,106(0,9);2,100(0,7);2,094(0,4);1,963(2,1);1,957(4,1);1,951(26,4);1,945(48,5);1,939(66,8);1,933(46,1);1,926(23,8);1,767(0,4);0,146(1,2);0,008(10,3);0,000(243,1);-0,009(11,1);-0,033(0,6);-0,150(1,2) |
| I-A-78 | 2,37 | 2,53 | ¹H-NMR(400,0 MHz, d₆-DMSO): δ= 8,512(2,5);8,506(2,5);8,462(1,8);8,460(1,9);8,451(1,9);8,448(1,9);8,118(1,8);8,114(2,9);8,110(1,8);7,933(6,3);7,928(6,0);7,848(1,3);7,827(1,4);7,422(1,4);7,410(1,4);7,401(1,3);7,390(1,2);6,625(0,8);5,753(1,7);4,042(16,0);3,394(15,4);3,315(45,2);2,671(0,6);2,501(97,2);2,328(0,7);0,000(22,3) |
| I-A-79 | 2,16 | 2,29 | ¹H-NMR(400,0 MHz, d₆-DMSO): δ= 8,503(2,9);8,498(2,9);8,467(2,3);8,455(2,3);8,072(3,7);7,973(0,8);7,952(5,1);7,922(0,6);7,845(1,5);7,824(1,7);7,425(1,5);7,413(1,6);7,405(1,5);7,393(1,4);6,450(1,5);5,752(3,7);3,985(16,0);3,380(15,6);3,313(43,1);2,891(0,7);2,731(0,7);2,670(0,9);2,501(137,8);2,327(0,9);0,000(30,9) |
| I-A-80 | 2,56 | 2,72 | ¹H-NMR(400,0 MHz, d₆-DMSO): δ= 8,503(2,8);8,498(2,8);8,455(2,2);8,444(2,2);8,256(1,5);8,235(1,8);8,192(3,3);8,063(2,7);8,042(2,2);7,848(1,5);7,829(1,6);7,419(1,5);7,407(1,6);7,399(1,4);7,387(1,3);6,604(0,9);4,043(16,0);3,390(15,6);3,314(43,1);2,671(0,8);2,501(125,8);2,328(0,8);0,000(25,2) |
| I-A-81 | 1,53 | 1,58 | ¹H-NMR(400,0 MHz, d₆-DMSO): δ= 8,506(2,4);8,500(2,5);8,463(1,7);8,460(2,0);8,451(1,8);8,448(1,9);7,861(4,1);7,852(1,6);7,839(5,0);7,832(1,8);7,828(1,6);7,825(1,5);7,822(1,2);7,428(1,4);7,417(1,4);7,408(1,3);7,396(1,2);7,185(0,7);7,178(4,4);7,155(4,2);6,486(0,9);5,754(4,5);4,002(15,6);3,862(16,0);3,820(0,4);3,379(14,9);3,330(1,5);3,319(33,0);2,687(0,7);2,674(1,0);2,506(54,0);2,502(71,2);2,497(57,9);2,328(0,5);2,086(3,8);0,008(2,6);0,000(38,0) |
| I-A-82 | 1,53 | 1,62 | ¹H-NMR(400,0 MHz, d₆-DMSO): δ= 8,506(1,8);8,501(1,9);8,460(1,5);8,448(1,4);8,314(0,5);8,027(2,0);8,014(2,1);8,009(1,5);8,004(2,2);7,996(1,1);7,992(2,0);7,901(1,2);7,898(1,1);7,882(0,6);7,851(0,9);7,847(1,1);7,845(1,1);7,841(0,9);7,831(1,0);7,827(1,2);7,824(1,2);7,821(1,0);7,530(2,3);7,508(4,0);7,486(2,0);7,426(1,2);7,414(1,2);7,406(1,1);7,394(1,0);6,536(0,7);4,021(16,0);3,384(15,2);3,317(96,9);2,675(1,0);2,670(1,4);2,666(1,1);2,506(159,2);2,501(207,5);2,497(158,2);2,333(1,0);2,328(1,3);2,324(1,0);0,000(5,0) |
| I-A-83 | 1,12 | 1,23 | ¹H-NMR(400,0 MHz, d₆-DMSO): δ= 8,464(2,9);8,136(0,9);8,132(1,6);8,126(1,1);8,123(1,1);8,119(1,6);8,114(1,0);8,058(2,1);8,053(1,0);8,045(2,3);8,040(1,5);8,035(2,4);8,027(1,1);8,023(2,3);7,543(2,3);7,521(4,3);7,503(0,8);7,499(2,1);7,421(0,4);7,417(0,4);7,401(4,9);7,389(2,2);7,368(0,4);6,788(3,0);4,099(0,9);4,085(0,9);4,072(0,3);4,032(16,0);3,348(15,6);3,323(27,4);3,176(3,4);3,163(3,3);2,507(23,5);2,503(30,9);2,498(23,6);0,000(0,5) |
| I-A-84 | 1,22 | 1,27 | ¹H-NMR(400,0 MHz, d₆-DMSO): δ= 8,497(2,2);8,491(2,3);8,478(1,6);8,475(1,7);8,466(1,6);8,463(1,6);7,842(0,9);7,838(1,1);7,832(0,9);7,821(1,0);7,818(1,2);7,815(1,1);7,812(0,9);7,684(1,9);7,679(1,0);7,671(2,1);7,667(1,5);7,662(2,4);7,654(1,1);7,649(2,2);7,472(0,3);7,465(2,3);7,460(0,9);7,442(4,4);7,426(2,0);7,420(2,6);7,406(1,2);6,452(1,1);5,754(2,0);3,880(16,0);3,372(15,2);3,318(34,3);2,671(0,4);2,506(49,2);2,502(63,2);2,497(47,1);2,328(0,4);2,086(0,5);0,008(0,5);0,000(7,4) |
| I-A-85 | 1,01 | 1,10 | ¹H-NMR(400,0 MHz, d₆-DMSO): δ= 8,744(1,4);8,742(1,2);8,736(1,0);8,733(1,6);8,730(1,1);8,514(1,9);8,508(2,0);8,461(1,4);8,458(1,6);8,449(1,5);8,446(1,6);8,208(0,4);8,204(0,5);8,189(1,4);8,185(1,4);8,172(3,3);8,168(3,7);8,153(0,5);8,151(0,5);7,854(0,8);7,851(1,0);7,848(1,0);7,844(0,9);7,834(1,0);7,830(1,1);7,828(1,1);7,824(0,9);7,790(0,9);7,785(0,9);7,778(0,9);7,773(1,6);7,768(0,9);7,762(0,9);7,757(0,8);7,430(1,2);7,418(1,2);7,410(1,1);7,398(1,0);6,628(0,5);5,754(2,5);4,095(16,0);3,402(15,1);3,317(19,4);2,675(0,3);2,670(0,5);2,666(0,4);2,510(26,1);2,506(51,0);2,501(68,0);2,497(51,7);2,492(26,7);2,328(0,4);0,000(0,4) |
| I-A-86 | 0,59 | 0,80 | ¹H-NMR(400,0 MHz, d₆-DMSO): δ= 8,591(1,8);8,581(1,8);8,501(2,6);8,495(2,8);8,474(2,1);8,462(2,1);8,171(0,8);8,155(1,7);8,152(1,9);8,136(4,8);8,035(2,5);8,016(1,8);7,839(1,3);7,835(1,2);7,819(1,5);7,815(1,2);7,584(1,3);7,573(1,4);7,566(1,3);7,554(1,2);7,439(1,5);7,427(1,5);7,419(1,5);7,407(1,3);6,433(1,3);3,857(16,0);3,370(16,0);3,320(3,9);2,671(0,4);2,501(55,8);2,329(0,4) |
| I-A-87 | 1,04 | 1,20 | ¹H-NMR(400,0 MHz, d₆-DMSO): δ= 8,494(2,3);8,487(2,3);8,480(1,7);8,476(1,7);8,468(1,7);8,464(1,8);8,138(1,2);7,836(0,9);7,832(1,0);7,831(1,0);7,827(0,9);7,816(1,1);7,812(1,1);7,810(1,1);7,806(0,9);7,605(0,7);7,601(0,8);7,597(1,1);7,584(8,1);7,578(7,3);7,568(1,6);7,557(0,8);7,435(1,3);7,423(1,3);7,415(1,2);7,403(1,2);7,395(0,3);6,466(1,0);5,754(3,4);4,021(0,9);4,012(0,8);3,832(16,0);3,382(1,0);3,367(15,2);3,343(1,4);3,320(10,5);2,510(18,0);2,506(33,9);2,502(43,7);2,497(32,5);2,493(16,2);0,000(2,1) |
| I-A-88 | 1,37 | 1,47 | ¹H-NMR(400,0 MHz, d₆-DMSO): δ= 8,507(2,2);8,500(2,2);8,460(1,6);8,457(1,6);8,448(1,7);8,445(1,6);7,927(2,9);7,908(3,5);7,847(0,9);7,843(1,1);7,838(0,9);7,827(1,0);7,822(1,2);7,817(1,0);7,809(0,7);7,790(1,9);7,772(1,2);7,691(2,6);7,671(3,6);7,652(1,5);7,420(1,3);7,408(1,3);7,400(1,3);7,388(1,2);6,544(0,7);5,754(4,3);4,012(16,0);3,382(15,3);3,320(33,6);2,506(34,7);2,501(44,4);2,497(33,4);0,000(1,9);-0,001(2,0) |
| I-A-89 | 1,20 | 1,38 | ¹H-NMR(400,0 MHz, d₆-DMSO): δ= 8,495(2,5);8,489(2,7);8,463(1,8);8,460(2,0);8,447(2,7);8,441(2,9);8,436(2,3);8,418(1,5);8,398(1,5);8,394(1,3);8,039(1,5);8,019(1,9);7,892(1,7);7,872(2,8);7,852(1,3);7,844(1,0);7,838(1,3);7,835(1,1);7,824(1,1);7,818(1,4);7,814(1,1);7,424(1,4);7,412(1,4);7,404(1,4);7,392(1,4);6,462(1,2);5,754(8,3);3,957(16,0);3,376(15,3);3,349(0,6);3,319(46,3);2,671(0,4);2,666(0,3);2,506(48,2);2,502(63,4);2,497(51,2);2,328(0,4);0,000(13,5) |
| I-A-90 | 1,58 | 1,68 | ¹H-NMR(400,0 MHz, d₆-DMSO): δ= 8,610(1,4);8,606(1,5);8,604(1,5);8,589(1,4);8,586(1,6);8,584(1,7);8,550(2,9);8,502(2,3);8,497(2,4);8,449(1,9);8,438(1,9);8,377(1,7);8,357(1,8);7,988(1,6);7,968(2,8);7,948(1,3);7,848(1,3);7,845(1,4);7,827(1,4);7,825(1,5);7,418(1,4);7,406(1,4);7,397(1,4);7,385(1,2);6,610(0,8);5,754(10,9);4,049(16,0);3,387(15,5);3,330(29,8);2,670(0,5);2,501(69,6);2,328(0,5);0,000(11,9) |
| I-A-91 | 0,76 | 0,96 | ¹H-NMR(400,0 MHz, d₆-DMSO): δ= 8,539(2,4);8,533(2,4);8,480(1,7);8,477(1,8);8,469(1,8);8,465(1,7);8,142(0.4);8,053(1,8);8,033(2,0);7,902(0.9);7,898(1,1);7,892(0,9);7,882(1,1);7,877(1,2);7,872(1,0);7,756(0,9);7,754(0,9);7,735(1,8);7,718(1,0);7,715(1,0);7,607(1,0);7,590(1,9);7,588(1,9);7,572(1,0);7,569(1,0);7,458(1,5);7,447(3,1);7,437(1,5);7,428(2,0);6,362(1,2);5,754(5,1);3,978(16,0);3,384(15,4);3,321(46,6);2,743(4,1);2,675(0,4);2,670(0,5);2,506(54,4);2,501(71,4);2,497(54,1);2,439(4,0);2,333(0,3);2,328(0,5);2,324(0,3);0,000(7,4) |
| I-A-92 | 1,12 | 1,29 | ¹H-NMR(400,0 MHz, d₆-DMSO): δ= 8,522(2,3);8,516(2,3);8,453(1,8);8,443(1,8);8,134(1,2);8,034(1,8);8,014(2.1);7,839(1,3);7,819(2,1);7.802(2,0);7,783(1,3);7,710(1,3);7,691(1,9);7,673(0,8);7,455(2,1);7,436(1,9);7,416(1,4);7,404(1,4);7,396(1,3);7,384(1,3);6,548(0,7);5,754(6,4);3,986(15,8);3,395(15,4);3,321(37,3);2,958(16,0);2,670(0,5);2,646(14,9);2,506(45,7);2,502(58,2);2,498(45,4);2,329(0,4);0,000(4,4) |
| I-A-93 | 1,12 | 1,28 | ¹H-NMR(400,0 MHz, d₆-DMSO): δ= 8,497(0,6);8,313(1,0);8,134(0,8);8,016(0,5);7,855(0,9);7,834(1,0);7,634(0,4);7,629(0,4);7,554(0,4);7,546(3,2);7,524(3,6);7,459(0,6);7,447(0,7);7,438(0,7);7,427(0,6);7,142(3,6);7,120(3,3);6,447(1,0);4,432(0,3);4,349(0,5);4,002(3,7);3,972(3,3);3,961(3,3);3,863(3,1);3,852(15,2);3,820(16,0);3,749(1,3);3,742(1,2);3,671(0,8);3,637(0,6);3,6 05(0,5);3,544(0,4);3,510(0,4);3,381(1,0);3,370(12,8);2,789(3,7);2,675(1,3);2,671(1,8);2,666(1,3);2,510(112,8);2,506(220,1);2,501(293,3);2,497(224,0);2,437(0,6);2,400(0,4);2,385(0,3);2,370(0,4);2,333(1,5);2,328(2,0);2,324(1,6);0,008(0,6);0,000(14,9);-0,008(0,6) |
| I-A-94 | 1,18 | 1,39 | ¹H-NMR(400,0 MHz, CD3CN): δ= 8,484(1,3);8,481(1,4);8,473(1,4);8,469(1,4);8,430(1,9);8,424(2,0);8,287(1,9);8,280(2,0);7,705(0,7);7,702(0,9);7,699(0,8);7,696(0,7);7,685(0,8);7,681(1,0);7,679(1,0);7,675(0,8);7,473(0,7);7,465(0,6);7,451(1,2);7,444(1,2);7,430(0,7);7,422(0,7);7,396(1,1);7,384(1,1);7,376(1,0);7,364(0,9);6,957(1,2);6,947(1,2);6,935(1,1);6,925(1,1);6,487(2,5);5,447(5,3);3,779(16,0);3,423(15,8);2,763(0,7);2,751(0,7);2,165(8,4);1,958(0,5);1,952(3,2);1,946(5,8);1,940(7,9);1,934(5,4);1,927(2,8);0,008(0,7);0,000(17,4);-0,009(0,7) |
| I-A-95 | 1,41 | 1,39 | ¹H-NMR(400,0 MHz, d₆-DMSO): δ= 8,449(2,9);8,138(0,9);8,134(1,7);8,129(1,1);8,124(1,1);8,120(1,7);8,116(1,0);8,020(2,5);7,984(1,2);7,965(1,6);7,941(1,2);7,921(1,8);7,854(1,4);7,834(1,9);7,815(0,8);7,419(0,5);7,415(0,4);7,398(4,7);7,383(2,0);7,362(0,5);6,740(3,7);5,755(8,3);3,962(16,0);3,375(0,4);3,340(15,9);3,320(34,9);2,671(0,4);2,506(49,1);2,502(64,8);2,497(49,9);2,328(0,4);0,008(0,8);0,000(16,2);-0,008(0,6) |
| I-A-96 | 1,74 | 1,80 | ¹H-NMR(400,0 MHz, d₆-DMSO): δ= 8,502(2,4);8,496(2,4);8,468(1,7);8,465(1,9);8,456(1,8);8,453(1,9);7,971(3,7);7,955(1,6);7,915(1,1);7,895(1,8);7,841(2,1);7,823(2,6);7,814(1,2);7,803(0,6);7,539(0,4);7,422(1,4);7,410(2,1);7,402(1,3);7,390(1,3);6,807(0,8);6,457(1,0);5,754(1,7);3,953(16,0);3,774(2,5);3,375(15,4);3,364(2,8);3,349(0,6);3,318(96,5);2,675(0,7);2,671(0,9);2,666(0,7);2,506(102,8);2,501(134,3);2,497(102,4);2,333(0,6);2,328(0,9);0,008(1,5);0,000(31,3);-0,008(1,3) |
| I-A-97 | 0,45 | 0,48 | ¹H-NMR(400,0 MHz, d₆-DMSO): δ= 8,661(6,4);8,649(6,5);8,423(2,7);8,163(0,9);8,159(1,1);8,156(1,1);8,153(1,6);8,146(1,3);8,141(1,0);7,436(2,6);7,427(4,1);7,424(3,8);7,340(1,7);7,328(3,2);7,316(1,7);6,802(4,7);3,740(16,0);3,380(15,7);3,320(12,1);2,506(21,1);2,502(28,0);2,498(21,3);1,236(0,5);0,008(0,9);0,000(26,2) |
| I-A-98 | 1,04 | 1,22 | ¹H-NMR(400,0 MHz, CD3CN): δ= 8,518(4,6);8,506(4,7);8,499(1,4);8,495(1,4);8,487(1,3);8,483(1,4);8,402(1,7);8,396(1,9);7,697(0,6);7,693(0,8);7,691(0,8);7,687(0,7);7,677(0,8);7,673(1,0);7,671(0,9);7,667(0,8);7,409(1,0);7,397(1,0);7,388(0,9);7,376(0,9);7,177(1,3);7,165(2,5);7,152(1,2);6,433(1,8);3,950(1,1);3,932(3,4);3,914(3,5);3,896(1,3);3,883(0,4);3,874(0,4);3,868(0,5);3,860(0,5);3,853(0,4);3,844(0,4);3,771(15,0);2,527(0,7);2,164(4,4);1,958(0,7);1,952(3,9);1,946(7,1);1,940(9,5);1,934(6,7);1,928(3,5);1,197(3,7);1,179(7,4);1,161(3,6);1,099(16,0);1,084(15,7);0,000(4,6) |
| I-A-99 | 0,66 | 0,87 | ¹H-NMR(400,0 MHz, CD3CN): δ= 8,553(1,9);8,543(6,2);8,531(5,7);7,924(0,9);7,904(1,1);7,575(0,9);7,563(1,0);7,555(1,0);7,542(0,9);7,209(1,3);7,197(2,5);7,185(1,2);6,700(2,3);4,099(0,5);4,073(1,2);4,040(0,8);3,986(0,8);3,951(0,7);3,907(0,6);3,878(0,6);3,853(0,5);3,810(15,8);3,760(0,4);3,654(1,0);3,467(16,0);3,453(0,8);3,409(1,0);3,337(0,5);3,277(6,0);1,964(0,6);1,958(1,5);1,952(8,2);1,946(15,0);1,940(20,2);1,934(14,1);1,928(7,4);0,000(7,2) |
| I-A-100 | 1,58 | | ¹H-NMR(400,0 MHz, CD3CN): δ= 9,012(1,2);8,999(1,3);8,913(1,2);8,908(1,2);8,677(0,9);8,663(1,0);8,540(0,6);8,537(0,6);8,516(0,7);8,078(0,7);8,063(0,7);8,057(0,7);8,043(0,8);7,953(2,1);7,724(1,5);7,711(1,5);7,549(0,9);3,995(9,5);3,554(9,5);2,970(0,3);2,919(116,0);2,834(0,6);2,799(15,7);2,769(0,8);2,745(0,9);2,623(15);2,607(3,8);2,593(6,2);2,578(3,9);2,545(1,7);2,500(1,6);2,485(1,5);2,276(0,5);2,201(0,4);2,120(0,3);2,114(0,4);2,108(0,5);2,101(0,4);1,965(1,2);1,958(2,8);1,953(16,1);1,947(29,7);1,940(40,5);1,934(28,2);1,928(14,8);1,769(0,3);1,285(0,4);1,272(1,0);0,008(2,3);0,000(63,5);-0,008(3,0) |
| I-A-101 | 1,00 | | ¹H-NMR(400,0 MHz, CD3CN): δ= 8,471(1,0);8,461(1,1);8,428(1,4);8,423(1,4);8,342(1,1);8,329(1,2);8,032(0,8);7,719(0,7);7,716(0,9);7,713(0,8);7,709(0,7);7,699(0,9);7,695(1,0);7,693(0,9);7,689(0,8);7,398(0,9);7,385(0,9);7,377(0,9);7,365(0,8);7,040(1,7);7,027(1,6);6,458(1,3);3,780(16,0);3,428(16,0);2,380(12,8);2,153(65,7);2,119(0,5);2,113(0,5);2,107(0,5);2,100(0,4);1,963(1,9);1,957(4,5);1,952(24,5);1,945(44,5);1,939(59,8);1,933(40,8);1,927(20,7);1,768(0,3);0,146(0,7);0,008(6,5);0,000(147,6);-0,009(6,1);-0,150(0,7) |
| I-A-102 | 1,61 | 1,79 | ¹H-NMR(400,0 MHz, CD3CN): δ= 8,474(0,7);8,471(0,7);8,462(0,7);8,459(0,8);8,406(1,0);8,400(1.0);7,726(0,4);7,722(0,5);7,720(0,5);7,716(0,4);7,706(0,4);7,702(0,5);7,700(0,5);7,696(0,4);7,390(0,6);7,378(0,6);7,369(0,5);7,357(0,5);6,428(1,2);5,849(2,9);5,447(3,7);3,845(8,1);3,737(16,0);3,411(8,1);2,158(11,4);1,958(0,5);1,952(3,0);1,946(5,4);1,940(7,3);1,934(5,0);1,927(2,6);0,008(0,6);0,000(14,9);-0,008(0,6) |
| I-A-103 | 1,23 | | ¹H-NMR(400,0 MHz, CD3CN): δ= 8,474(1,1);8,463(1,1);8,428(1,3);8,174(1,9);7,717(0,8);7,714(0,9);7,708(0,7);7,697(0,9);7,693(1,0);7,691(1,0);7,687(0,8);7,398(1,0);7,387(1,0);7,378(0.9);7,366(0.8);6.449(1,0);4,089(0,5);3,915(0,4);3,774(15,5);3,451(0,5);3,427(16,0);2,467(0,4);2,340(12,4);2,179(265,0);2,159(12,3);2,113(0,4);2,107(0,4);2,101(0,3);1,964(2,2);1,952(27,8);1,946(50,3);1,940(67,5);1,934(46,1);1,928(23,7);1,769(0,4);0,146(0,7);0,008(6,8);0,000(150,8);-0,008(6,8);-0,150(0,8) |
| I-A-104 | 0,97 | | ¹H-NMR(400,0 MHz, CD3CN): δ= 8,501(0,3);8,474(0,4);8,462(0,4);8,449(0,4);8,408(0,5);8,403(0,5);7,703(0,9);7,683(1,1);7,407(0,4);3,864(0,4);3,806(0,4);3,763(12,8);3,427(16,0);3,382(0,7);2,216(7,9);2,162(45,1);1,972(0,7);1,964(1,0);1,958(2,3);1,952(13,3);1,946(24,2);1,940(32,8);1,933(22,3);1,927(11,3);1,204(0,3);1,100(5,4);1,085(5,3);0,008(2,1);0,000(56,9);-0,009(2,0) |
| I-A-105 | 1,64 | 1,73 | ¹H-NMR(400,0 MHz, d₆-DMSO): δ= 9,087(6,0);9,085(6,0);8,529(2,0);8,523(2,1);8,500(1,6);8,497(1,7);8,488(1,7);8,485(1,7);7,865(0,8);7,861(0,9);7,858(0,9);7,855(0,8);7,844(0,9);7,841(1,0);7,838(1,0);7,834(0,9);7,488(1,1);7,476(1,1);7,468(1,0);7,456(1,0);6,522(1,2);5,754(9,2);3,770(16,0);3,417(15,1);3,321(12,8);2,520(0,5);2,511(8,3);2,507(17,5);2,502(23,5);2,498(16,9);2,493(8,1);0,008(1,2);0,000(36,7);-0,009(1,2) |
| I-A-106 | 1,20 | | ¹H-NMR(601,6 MHz, d₆-DMSO): δ= 8,512(1,5);8,508(1,6);8,490(1,2);8,483(1,2);8,337(3,1);8,328(3,1);7,881(0,8);7,879(1,0);7,877(0,9);7,875(0,8);7,868(0,9);7,865(1,1);7,864(1,0);7,861(0,9);7,466(1,0);7,458(1,0);7,453(1,0);7,445(0,9);6,726(3,3);6,716(3,3);6,467(1,1);3,900(2,4);3,791(15,7);3,697(16,0);3,401(14,9);3,358(1,0);3,328(672,2);3,304(2,7);3,282(0,5);3,174(1,0);3,165(1,0);2,617(0,6);2,614(0,8);2,611(0,5);2,523(1,4);2,520(1,8);2,517(2,0);2,508(46,4);2,505(93,3);2,502(127,2);2,499(96,7);2,496(49,0);2,389(0,6);2,386(0,8);2,383(0,6);1,909(0,5);0,000(0,9) |
| I-A-107 | 1,04 | 1,20 | ¹H-NMR(400,0 MHz, CD3CN): δ= 8,490(4,7);8,436(0,4);7,710(0,5);7,706(0,7);7,704(0,6);7,700(0,5);7,689(0,6);7,686(0,7);7,683(0,7);7,680(0,6);7,406(0,4);7,394(0,5);7,386(0,5);7,374(0,4);6,491(0,6);5,446(3,5);3,776(15,9);3,430(16,0);1,958(0,5);1,952(3,8);1,946(7,1);1,939(9,8);1,933(6,7);1,927(3,4);1,538(0,4);0,008(0,8);0,000(25,5);-0,009(0,9) |
| I-A-108 | 1,28 | 1,43 | ¹H-NMR(400,0 MHz, CD3CN): δ= 8,481(1,1);8,477(1,1);8,469(1,1);8,466(1,1);8,434(1,5);8,428(1,5);7,716(0,7);7,712(0,8);7,709(0,8);7,706(0,7);7,695(0,8);7,692(0,9);7,689(0,9);7,685(0,8);7,509(1,1);7,490(2,1);7,470(1,2);7,396(0,9);7,394(0,9);7,384(1,0);7,382(0,9);7,375(0,9);7,374(0,8);7,363(0,8);7,362(0,8);7,005(1,6);6,986(1,4);6,564(1,5);6,544(1,4);6,486(2,1);5,446(0,4);3,788(16,0);3,426(15,9);2,401(10,8);2,152(13,0);1,964(0,5);1,957(1,0);1,952(7,1);1,945(13,4);1,939(18, 5);1,933(12,7);1,927(6,5);1,270(0,4);0,008(1,3);0,000 (40,6);0,009(1,7) |
| I-A-109 | | | ¹H-NMR(400,0 MHz, CD3CN): δ= 8,483(0,7);8,473(0,7);8,429(0,8);8,202(0,6);7,705(0,6);7,701(0,7);7,698(0,7);7,694(0,6);7,684(0,7);7,681(0,8);7,678(0,8);7,674(0,7);7,463(0,6);7,459(0,6);7,443(0,7);7,439(0,6);7,397(0,7);7,385(0,7);7,376(0,7);7,364(0,6);6,767(0,7);6,747(0,7);6,482(0,5);5,447(1,3);3,767(15,6);3,421(16,0);2,249(7,3);2,158(11,1);1,958(0,6);1,952(4,5);1,945(8,5);1,939(11,8);1,933(8,0);1,927(4,1);0,008(1,1);0,000(33,1);-0,009(1,1) |
| I-A-110 | 1,77 | 1,93 | ¹H-NMR(400,0 MHz, CD3CN): δ= 8,475(1,1);8,472(1,2);8,463(1,2);8,460(1,4);8,442(1,1);8,423(1,6);8,417(1,6);8,034(1,1);8,015(1,1);7,718(0,7);7,714(0,8);7,711(0,8);7,708(0,7);7,697(0,8);7,693(1,0);7,691(1,0);7,687(0,8);7,402(1,0);7,390(1,1);7,381(1,0);7,369(0,9);7,327(0,8);7,315(0,8);7,307(0,8);7,295(0,7);6,490(1,8);5,447(3,1);3,763(0,6);3,756(0,6);3,734(16,0);3,429(15,9);3,388(1,1);2,190(31,2);1,958(0,5);1,952(3,9);1,946(7,3);1,940(10,1);1,934(6,8);1,928(3,5);0,007(0,6);0,000(17,0);-0,009(0,6) |
| I-A-111 | 1,73 | 1,78 | ¹H-NMR(400,0 MHz, d₆-DMSO): δ= 8,609(14,5);8,597(14,9);8,564(0,6);8,552(0,6);8,447(3,8);8,442(4,5);8,436(4,2);8,432(4,3);8,314(0,5);8,082(0,6);7,914(5,5);7,866(0,3);7,420(1,4);7,414(1,1);7,404(3,8);7,399(6,5);7,394(7,9);7,382(4,8);7,363(4,0);7,346(8,0);7,328(7,5);7,313(6,9);7,301(8,8);7,293(4,8);7,290(5,3);7,276(1,3);7,219(0,4);7,201(0,6);7,184(0,5);7,158(8,1);7,140(7,1);7,078(0,3);6,637(3,6);5,685(0,5);5,597(0,5);5,485(16,0);3,392(0,8);3,375(0,8);3,357(0,5);3,319(1 42,2);3,287(36,5);3,266(0,7);3,212(0,4);2,671 (1,1);2,506(140,0);2,502(186,0);2,498(152,0);2,329(1,1);1,3 55(0,4);1,109(0,7);1,091(1,5);1,074(0,7);0,146(0,8);0, 000(164,9);-0,150(0,8) |
| I-A-112 | 0,56 | 0,85 | ¹H-NMR(400,0 MHz, d₆-DMSO): δ= 8,740(5,4);8,728(5,6);8,485(3,5);8,480(3,7);8,472(1,7);8,469(1,7);7,842(0,8);7,838(0,9);7,835(0,9);7,831(0,9);7,821(0,9);7,817(1,1);7,815(1,1);7,811(0,9);7,458(1,2);7,446(1,2);7,438(1,1);7,425(1,1);7,410(1,4);7,398(2,6);7,385(1,3);6,224(3,6);4,469(9,4);4,098(0,6);4,085(0,6);3,642(16,0);3,357(15,2);3,321(16,5);3,176(2,7);3,163(2,7);2,525(0,4);2,511(10,4);2,507(21,6);2,502(29,1);2,498(21,8);2,493(11,3);0,008(0,5);0,000(16,5);-0,008(0,7) |
| I-A-113 | 0,66 | 0,93 | ¹H-NMR(400,0 MHz, d₆-DMSO): δ= 8,919(0,4);8,693(0,3);8,551(2,4);8,547(2,3);8,526(3,7);8,516(3,3);8,510(2,5);8,496(2,9);8,490(4,6);8,478(2,0);7,823(1,3);7,805(1,3);7,802(1,5);7,459(1,5);7,447(1,5);7,439(1,4);7,427(1,3);6,249(4,0);5,953(0,9);4,413(9,6);4,102(0,5);4,089(0,5);3,603(16,0);3,355(15,6);3,327(21,3);3,175(2,5);3,163(2,4);2,784(0,9);2,771(0,9);2,505(33,7);0,008(0,7);0,000(13,8) |
| I-A-114 | 0,20 | 0,85 | ¹H-NMR(400,0 MHz, d₆-DMSO): δ= 8,497(3,9);8,492(3,3);8,485(2,0);8,482(1,8);7,815(0,8);7,809(1,0);7,805(0,9);7,795(1,0);7,791(1,1);7,789(1,2);7,785(1,0);7,551(3,3);7,470(1,3);7,458(1,3);7,450(1,2);7,438(1,1);6,567(3,2);6,285(3,8);4,279(8,4);4,098(0,9);4,085(0,9);3,560(16,0);3,540(15,9);3,360(15,8);3,342(0,6);3,321(9,3);3,303(0,6);3,175(4,2);3,163(4,2);2,506(26,0);2,502(34,4);2,497(25,8);0,008(0,6);0,000(16,9);-0,008(0,7) |
| I-A-115 | 0,76 | 1,12 | ¹H-NMR(400,0 MHz, d₆-DMSO): δ= 8,496(2,7);8,490(3,8);8,481(1,8);8,478(1,6);7,854(0,9);7,850(1,1);7,847(1,0);7,843(0,9);7,833(1,0);7,829(1,2);7,827(1,1);7,823(0,9);7,557(2,6);7,552(2,5);7,468(1,3);7,456(1,3);7,448(1,2);7,436(1,2);6,278(3,8);5,939(2,8);5,934(2,7);4,174(8,9);4,101(0,5);4,088(0,5);3,763(16,0);3,599(15,8);3,364(15,1);3,322(13,8);3,176(2,2);3,162(2,1);2,524(0,5);2,506(20,9);2,502(26,4);2,498(18,9);0,008(0,4);0,000(11,8);-0,009(0,4) |
| I-A-116 | 0,16 | 1,11 | ¹H-NMR(400,0 MHz, d₆-DMSO): δ= 8,500(1,6);8,497(1,8);8,488(1,8);8,485(1,8);8,472(2,6);8,464(4,8);8,460(2,9);8,448(4,3);8,439(0,5);8,435(0,5);7,843(0,8);7,837(1,1);7,833(0,9);7,823(1,0);7,817(1,2);7,813(1,0);7,467(1,3);7,455(1,3);7,447(1,3);7,435(1,2);7,237(0,4);7,222(0,4);7,194(3,6);7,179(3,6);6,269(3,8);4,246(8,1);3,681(0,4);3,600(0,4);3,580(16,0);3,385(0,5);3,353(15,5);3,321(33,9);2,506(38,1);2,502(50,4);2,498(38,7);2,329(0,3);1,236(0,4);0,007(1,0);0,000(25,1) |
| I-A-117 | 0,63 | 1,16 | ¹H-NMR(400,0 MHz, d₆-DMSO): δ= 8,495(1,8);8,492(2,7);8,484(2,7);8,477(4,1);8,472(3,1);7,846(0,8);7,843(1,0);7,840(1,0);7,836(0,9);7,826(0,9);7,822(1,1);7,820(1,1);7,816(0,9);7,728(0,8);7,723(0,8);7,708(1,6);7,704(1,6);7,689(0,9);7,685(0,9);7,561(0,6);7,467(1,3);7,456(1,3);7,447(1,2);7,435(1,1);7,282(1,1);7,270(1,2);7,266(1,1);7,251(1,0);7.231(1,9);7,211(1,7);6,257(3,8);4,416(1,1);4,345(9,1);4,249(0,3);3,644(1,8);3,638(0,7);3,580(16,0);3,383(0,6);3,356(15,4);3,319(53,6);2,708(0,8);2,697(0,8);2,671(0,5);2,666(0,4);2,506(62,1);2,502(81,5);2,497(61,2);2,333(0,4);2,328(0,5);2,324(0,4);1,236(0,8);0,008(1,3);0,000(34,7);-0,008(1,6) |
| I-A-118 | | 0,89 | ¹H-NMR(400,0 MHz, d₆-DMSO): δ= 8,495(2,2);8,489(3,7);8,480(1,6);8,476(1,7);7,839(0,8);7,835(0,9);7,832(1,0);7,829(0,9);7,818(0,9);7,815(1,0);7,812(1,1);7,808(0,9);7,464(1,1);7,452(1,1);7,444(1,0);7,432(1,0);7,074(2,9);7,072(3,2);6,787(2,9);6,784(3,1);6,283(3,5);4,307(9,3);3,574(15,6);3,480(16,0);3,362(14,8);3,315(3,7);3,173(1,1);3,163(1,1);2,524(0,7);2,511(15,9);2,506(33,8);2,502(46,0);2,497(34,9);2,493(18,1);0,008(0,7);0,000(22,2);-0,008(1,0) |
| I-A-119 | 0,39 | 1,15 | ¹H-NMR(400,0 MHz, d₆-DMSO): δ= 8,500(1,5);8,496(1,8);8,491(2,5);8,485(3,8);8,444(1,3);8,440(1,4);8,432(1,4);8,428(1,5);8,413(1,9);8,408(2,0);7,831(0,8);7,827(1,0);7,825(0,9);7,821(0,8);7,811(0,9);7,807(1,0);7,804(1,0);7,801(0,9);7,596(0,7);7,592(1,1);7,587(0,7);7,577(0,8);7,572(1,2);7,567(0,8);7,470(1,2);7,457(1,2);7,450(1,1);7,437(1,1);7,314(1,1);7,303(1,1);7,295(1,0);7,283(0,9);6,281(3,7);4,269(7,6);3,553(16,0);3,355(15,2);3,317(44,0);2,677(0,5);2,672(0,6);2,668(0,5);2,525(1,8);2,512(38,7);2,508(79,5);2,503(106,6);2,499(79,6);2,334(0,5);2,330(0,7);2,325(0,5);0,008(0,7);0,000(22,6);-0,008(1,0) |
| I-A-120 | 1,34 | 1,55 | ¹H-NMR(400,0 MHz, d₆-DMSO): δ= 8,491(1,9);8,479(1,9);8,468(2,6);8,461(2,7);8,132(0,7);7,830(1,2);7,826(1,1);7,809(1,4);7,805(1,1);7,574(2,7);7,571(2,9);7,569(2,9);7,467(1,4);7,455(1,4);7,446(1,4);7,435(1,3);6,361(1,5);6,354(2,1);6,349(1,8);6,297(4,1);6,060(2,3);6,052(2,3);5,751(2,0);4,269(9,4);3,570(16,0);3,360(15,6);3,310(7,8);2,501(26,9);2,497(21,9);-0,001(1,7) |
| I-A-121 | 2,51 | 2,62 | ¹H-NMR(400,0 MHz, d₆-DMSO): δ= 8,496(1,6);8,492(1,7);8,484(3,9);8,480(3,3);8,136(0,8);7,842(0,8);7,838(1,0);7,836(1,0);7,832(0,9);7,822(0,9);7,818(1,1);7,816(1,1);7,812(0,9);7,553(2,6);7,548(2,7);7,536(2,7);7,516(2,9);7,460(1,2);7,448(1,2);7,440(1,2);7,428(1,1);7,178(1,5);7,173(1,5);7,157(1,3);7,152(1,3);6,280(3,8);5,751(2,6);4,258(7,7);3,600(16,0);3,358(15,4);3,310(8,4);2,524(0,6);2,506(30,2);2,501(39,9);2,497(29,9);0,000(3,0) |
| I-A-122 | 1,37 | 1,58 | ¹H-NMR(400,0 MHz, CD3CN): δ= 8,481(1,7);8,472(1,9);8,469(1,8);8,394(2,3);8,388(2,6);8,172(2,0);8,166(2,3);7,654(1,2);7,651(1,1);7,640(1,1);7,634(1,4);7,630(1,2);7,577(1,2);7,570(1,4);7,556(1,4);7,550(1,5);7,394(1,3);7,382(1,5);7,374(1,4);7,362(1,2);7,310(2,3);7,290(2,0);6,268(3,7);4,186(8,5);3,701(0,4);3,584(15, 6);3,370(16,0);2,501(1,2);2,163(40,1);2,113(0,5);2,106(0,4);2,100(0,4);1,951(13,0);1,945(23,4);1,939(32,0);1,933(25,9);1,927(16,2);1,270(0,4);0,000(24,1);-0,008(5,9) |
| I-A-123 | 1,69 | 1,92 | ¹H-NMR(400,0 MHz, CD3CN): δ= 8,487(1,3);8,484(1,3);8,475(1,3);8,472(1,3);8,391(1,7);8,386(1,7);7,675(0,7);7,671(0,8);7,668(0,8);7,664(0,7);7,654(0,9);7,650(1,0);7,648(0,9);7,644(0,8);7,395(1,0);7,393(1,0);7,383(1,0);7,381(1,0);7,375(0,9);7,373(0,9);7,363(0,9);7,361(0,8);7,331(0,6);7,327(0,6);7,314(0,4);7,310(1,2);7,306(0,4);7,293(0,6);7,289(0,7);7,272(0,3);6,956(1,6);6,936(2,6);6,927(0,3);6,916(1,5);6,264(3,2);5,447(2,6);4,156(5,1);3,628(15,2);3,376(16,0);2,178(21,3);1 ,958(0,5);1,952(3,5);1,946(6,5);1,940(8,9);1,934(6,1);1,927(3,1);0,008(0,6);0,000(16,4);-0,009(0,6) |
| I-A-124 | 1,72 | 2,03 | ¹H-NMR(400,0 MHz, CD3CN): δ= 8,479(1,3);8,476(1,3);8,467(1,3);8,464(1,2);8,406(1,5);8,404(1,6);8,399(1,5);8,398(1,4);7,682(0,8);7,678(0,9);7,675(0,8);7,671(0,8);7,661(1,0);7,658(1,0);7,655(0,9);7,651(0,8);7,391(1,0);7,389(1,0);7,379(1,0);7,377(1.0);7,371(0,9);7,369(0,9);7,359(0,8);7,357(0,8);7,286(0,5);7,269(0,7);7,265(1,2);7,248(1,2);7,244(0,7);7,227(0,6);6,770(1,3);6,749(1,1);6,712(0,7);6,689(1,3);6,668(0,6);6,666(0,6);6,268(3,0);5,451(0,4);5,447(5,1);4,150(4,4);4,147(4,2);3,754(14,4);3,619(15,8);3,379(16,0);2,170(42,1);1,964(0,4);1,958(0,9);1,952(4,1);1,946(7,2);1,940(9,5);1,933(6,4);1,927(3,2);0,008(1,0);0,004(1,6);0,00 0(15,8);-0,009(0,6) |
| I-A-125 | 2,09 | 2,28 | ¹H-NMR(400,0 MHz, CD3CN): δ= 8,484(1,2);8,481(1,3);8,472(1,3);8,469(1,3);8,428(1,6);8,422(1,7);7,696(0,7);7,693(0,8);7,690(0,7);7,686(0,7);7,676(0,8);7,672(0,9);7,670(0.9);7.666(0,8);7,396(1,0);7,394(0,9);7,384(1,0);7,382(0,9);7,375(1,1);7,3704(2,7);7,3698(2,7);7,364(1,0);7,350(4,5);7,259(1,7);7,240(1,4);7,237(1,2);7,219(0,9);6,271(3,1);5,448(5,4);4,362(8,2);3,754(0,3);3,633(16,0);3,382(16,0);2,181(32,5);1,964(0,3);1,958(0,7);1,952(5,1);1,946(9,5);1,940(13,1);1,934(8,9);1,928(4,6);0,008(0,7);0,000(22,2);-0,009(0,7) |
| I-A-126 | 2,47 | 2,54 | ¹H-NMR(400,0 MHz, CD3CN): δ= 8,485(1,2);8,482(1,3);8,474(1,2);8,470(1,3);8,430(1,6);8,424(1,6);7,709(1,1);7,690(2,8);7,688(2,7);7,670(2,0);7,664(0,8);7,481(0,7);7,461(1,2);7,441(0,5);7,393(0,9);7,391(0,9);7,381(0,9);7,380(0,9);7,373(0,8);7,371(0,8);7,361(0,8);7,359(0,8);6,295(3,3);5,446(5,7);4,461(4,7);3,756(0,6);3,659(16,0);3,388(15,9);3,274(0,6);2,141(8,8);1,958(0,7);1,952(5,1);1,946(9,5);1,939(13,1);1,933(8,9);1,927(4,5);0,008(0,8);0,000(24,l);-0,009(0,7) |
| I-A-127 | 1,89 | 2,09 | ¹H-NMR(400,0 MHz, CD3CN): δ= 8,487(1,4);8,483(1,5);8,475(1,4);8,472(1,5);8,404(2,0);8,397(2,0);7,685(0,8);7,681(0,9);7,679(1,0);7,675(0,9);7,665(0,9);7,661(1,0);7,658(1,1);7,655(0,9);7,395(1,2);7,383(1,2);7,375(1,1);7,363(1,0);7,314(0,5);7,299(0,5);7,293(1,2);7,279(1,2);7,273(1,1);7,259(1,2);7,245(2,1);7,226(0,8);7,072(0,9);7,052(1,4);7,032(0,6);7,028(0,6);6,267(3,5);5,448(6,5);4,238(5,1);4,235(5,2);3,625(16,0);3,378(16,0);2,189(25,7);1,953(3,9);1,947(6,8);1,940(9,0);1,934(6,1);1,928(3,1);0,008(0,8);0,000(13,5);-0,009(0,5) |
| I-A-128 | 1,41 | 1,68 | ¹H-NMR(400,0 MHz, CD3CN): δ= 8,465(0,6);8,461(0,6);8,453(0,6);8,449(0,6);8,402(0,8);8,396(0,8);7,676(0,4);7,673(0,4);7,670(0,4);7,666(0,4);7,656(0,4);7,652(0,4);7,650(0,4);7,646(0,4);7,374(0,5);7,372(0,5);7,362(0,5);7,360(0,5);7,354(0,4);7,352(0,4);7,342(0,4);7,340(0,4);6,257(1,7);5,958(2,3);5,447(0,5);4,217(4,4);3,813(16,0);3,696(8,0);3,689(0,4);3,375(8,0);2,169(14,8);1,958(0,4);1,952(2,7);1,946(5,0);1,940(6,9);1,934(4,7);1,928(2,4);0,000(9,7);-0,009(0,4) |
| I-A-129 | 2,32 | 2,44 | ¹H-NMR(400,0 MHz, CD3CN): δ= 8,488(1,2);8,484(1,3);8,476(1,3);8,472(1,3);8,411(1,6);8,405(1,7);7,696(0,7);7,692(0,8);7,689(0,8);7,686(0,7);7,675(0,8);7,672(0,9);7,669(0,9);7,665(0,7);7,402(1,0);7,400(0,9);7,390(1,0);7,388(0,9);7,381(0,9);7,380(0,8);7,370(0,8);7,368(0,8);7,100(0,5);7,084(0,9);7,078(1,0);7,063(1,8);7,031(3,3);7,012(1,2);6,306(3,5);5,446(6,4);4,259(7,8);3,764(0,6);3,552(16,0);3,376(15,8);3,268(0,6);2,416(0,3);2,340(0,3);2,328(0,4);2,255(20,5);2,169(23,9);1,972(0,7);1,963(0,3);1,957(0,7);1,952(4,9);1,945(9,1);1,939(12,6);1,933(8,6);1,927(4,4);0,008(0,5);0,000(16,5);-0,009(0,6) |
| I-A-130 | 1,18 | | ¹H-NMR(400,0 MHz, CD3CN): δ= 8,514(1,8);8,511(1,7);8,503(1,9);8,384(2,5);8,378(2,2);7,715(1,1);7,711(1,2);7,695(1,3);7,691(1,4);7,427(1,2);7,414(1,4);7,406(1,2);7,395(1,1);7,362(5,2);7,356(5,1);7,351(3,6);7,349(3,7);7,056(2,4);7,051(2,5);7,041(2,6);6,448(2,3);4,422(8,2);3,544(16,0);3,387(16,0);2,134(175,3);2,125(19,2);2,106(1,4);2,100(0,9);1,951(85,8);1,945(141,2);1,939(167,4);1,933(116,4);1,927(58,6);1,773(0,8);1,767(0,9);1,761(0,7);1,286(0,4);1,273(0,5);0,146(0,4);0,080(0,8);0,000(68,9);-0,009(7,5);-0,149(0,4);-0,383(6,2) |
| I-A-131 | 1,28 | 1,40 | ¹H-NMR(400,0 MHz, d₆-DMSO): δ= 8,501(2,5);8,494(2,8);8,488(1,9);8,480(1,7);8,476(1,8);7,831(0,8);7,827(1,0);7,825(1,0);7,821(0,9);7,810(0,9);7,804(1,2);7,800(1,0);7,455(1,4);7,443(1,4);7,434(1,3);7,422(1,2);6,819(3,8);6,355(3,1);5,667(8,2);4,109(0,6);4,096(1,7);4,083(1,7);4,070(0,6);3,544(16,0);3,361(15,8);3,321(18,5);3,176(8,0);3,163(7,7);2,525(0,5);2,507(20,5);2,502(27,6);2,498(20,8);2,003(12,4);0,008(0,5);0,000(14,7);-0,008(0,7) |
| I-A-132 | 0,59 | 0,94 | ¹H-NMR(601,6 MHz, d₆-DMSO): δ= 8,504(2,0);8,500(2,0);8,480(1,5);8,478(1,5);8,473(1,6);8,470(1,5);8,165(0,8);8,160(0,8);7,842(0,9);7,839(1,0);7,838(1,0);7,835(0,9);7,828(1,0);7,826(1,1);7,824(1,1);7,822(0,9);7,449(1,1);7,448(1,1);7,441(1,1);7,440(1,1);7,436(1,1);7,435(1,0);7,428(1,0);7,427(1,0);6,218(2,3);3,710(0,6);3,699(16,0);3,694(9,2);3,361(14,9);3,315(18,1);3,173(0,5);3,164(0,5);2,578(0,4);2,572(0,6);2,566(0,9);2,560(0,9);2,554(0,6);2,548(0,4);2,507(6,8);2,504(14,0);2,501(18,8);2,498(13,9);2,495(6,6);0,596(0,6);0,587(1,8);0,584(2,3);0,576(2,3);0,572(1,9);0,564(0,7);0,337(0,7);0,329(2,0);0,325(2,1);0,322(1,9);0,319(2,0);0,311(0,6);0,000(0,5) |
| I-A-133 | 1,06 | 1,22 | ¹H-NMR(400,0 MHz, CD3CN): δ= 8,464(0,9);8,425(0,4);7,897(0,4);7,719(0,6);7,701(0,9);7,385(0,6);6,340(1,0);6,245(0,7);3,982(0,4);3,843(2,2);3,739(10,6);3,640(4,0);3,625(4,7);3,610(0,7);3,567(9,3);3,389(8,2);3,380(4,0);2,308(16,0);1,964(1,1);1,952(13,8);1,946(25,4);1,940(34,5);1,933(24,6);1,927(13,0);1,849(0,5);1,832(0,4);1,774(0,5);1,768(0,5);1,762(0,5);1,756(0,5);1,305(15,6);0,146(0,3);0,008(3,6);0,000(78,1);-0,150(0,4) |
| I-A-134 | 1,27 | 1,56 | ¹H-NMR(400,0 MHz, CD3CN): δ= 8,438(1,2);8,434(1,2);8,426(1,3);8,422(1,5);8,419(1,7);8,412(1,6);7,685(0,7);7,681(0,8);7,679(0,8);7,675(0,7);7,665(0,8);7,661(0,9);7,658(0,9);7,654(0,8);7,559(1,2);7,539(1,3);7,434(1,0);7,414(1,4);7,357(0,9);7,356(0,9);7,345(0,9);7,344(0,9);7,337(0,9);7,335(0,8);7,325(0,8);7,324(0,8);7,307(0,6);7,305(0,7);7,289(1,2);7,287(1,3);7,269(0,9);7,266(0,8);7,243(1,0);7,240(1,0);7,222(1,2);7,220(1,0);7,205(0,5);7,202(0,5);6,392(1,8);5,447(3,0);3,855(15,7);3,764(16,0);3,404(15,8);3,388(0,4);2,184(70,5);1,972(0,9);1,964(0,6);1,958(1,2);1,952(7,8);1,946(14,5);1,940(19,8);1,934(13,5);1,927(6,9);1,269(0,5);1,204(0,5);0,008(0,5);0,000(16,6);-0,009(0,6) |
| I-A-135 | 0,62 | 0,89 | ¹H-NMR(400,0 MHz, CD3CN): δ= 8,470(0,5);8,407(0,5);7,699(0,6);7,696(0,7);7,693(0,7);7,690(0,6);7,679(0,7);7,675(0,8);7,673(0,8);7,669(0,7);7,386(0,6);7,374(0,6);7,366(0,5);7,354(0,5);6,435(1,1);5,447(1,5);3,880(15,2);3,847(0,7);3,411(15,1);2,649(0,7);2,436(16,0);2,163(23,5);1,964(0,4);1,958(0,7);1,952(4,6);1,946(8,5);1,940(11,8);1,934(8,1);1,928(4,1);0,008(0,5);0,000(13,5);-0,009(0,5) |
| I-A-136 | | | ¹H-NMR(400,0 MHz, CD3CN): δ= 8,637(1,7);8,635(1,6);8,473(0,8);8,463(0,8);8,440(0,9);8,158(1,4);7,718(0,6);7,714(0,7);7,711(0,7);7,708(0,6);7,697(0,7);7,694(0,9);7,691(0,9);7,687(0,8);7,395(0,7);7,383(0,8);7,374(0,8);7,363(0,7);6,481(0,5);6,222(0,5);5,447(5,0);3,849(15,1);3,808(14,9);3,763(3,8);3,427(16,0);3,388(3,9);2,171(32,3);1,965(0,5);1,959(1,0);1,953(6,7);1,947(12,4);1,940(17,2);1,934(11,7);1,928(5,9);0,008(0,6);0,000(18,6);-0,009(0,6) |
| I-A-137 | 1,41 | 1,46 | ¹H-NMR(400,0 MHz, CD3CN): δ= 8,453(0,6);8,442(0,7);8,396(0,8);7,673(0,7);7,670(0,8);7,667(0,7);7,663(0,6);7,653(0,7);7,649(0,8);7,647(0,8);7,643(0,7);7,356(0,8);7,345(0,7);7,336(0,7);7,324(0,6);6,357(1,3);5,446(4,8);4,263(0,7);4,245(2,2);4,227(2,2);4,208(0,7);3,926(16,0);3,756(1,9);3,393(15,7);3,388(2,5);2,142(12,3);1,963(0,5);1,957(1,0);1,952(5,6);1,945(10,4);1,939(14,4);1,933(9,9);1,927(5,0);1,295(2,7);1,277(5,6);1,259(2,7);0,000(0,6) |
| I-A-138 | 1,12 | 1,26 | ¹H-NMR(400,0 MHz, CD3CN): δ= 8,455(0,9);8,444(0,9);8,395(1,1);7,676(0,7);7,672(0,9);7,670(0,9);7,666(0,7);7,656(0,8);7,652(1,0);7,650(1,0);7,646(0,8);7,362(0,9);7,350(1,0);7,341(0,9);7,330(0,8);6,339(1,8);5,446(3,6);3,902(16,0);3,733(10,1);3,390(16,0);3,021(0,3);2,141(17,9);1,963(0,5);1,957(1,0);1,951(5,7);1,945(10,6);1,939(14,5);1,933(10,1);1,927(5,1);0,000(0,5) |
| I-A-139 | 0,87 | 1,00 | ¹H-NMR(400,0 MHz, CD3CN): δ= 8,452(1,2);8,441(1,2);8,395(1,6);8,390(1,5);7,673(0,8);7,668(1,0);7,664(0,8);7,652(0,9);7,647(1,2);7,643(0,9);7,359(1,1);7,347(1,1);7,339(1,0);7,327(1,0);7,103(1,3);6,973(2,7);6,843(1,3);6,321(2,2);5,447(4,0);4,103(0,7);3,923(0,7);3,894(16,0);3,718(13,2);3,388(15,8);3,021(1,7);2,162(23,5);1,964(0,7);1,958(1,7);1,952(8,0);1,946(14,1);1,940(18,8);1,934(12,8);1,928(6,4);1,271(0,4);0,882(0,6) |
| I-A-140 | 1,92 | 1,96 | ¹H-NMR(400,0 MHz, CD3CN): δ= 8,482(0,9);8,473(0,9);8,470(0,9);8,411(1,1);8,406(1,0);7,931(0,4);7,924(2,0);7,920(2,1);7,916(1,0);7,915(1,0);7,910(0,9);7,904(2,0);7,900(2,2);7,727(0,8);7,723(0,9);7,720(0,8);7,716(0,8);7,706(0,9);7,703(0,9);7,700(0,9);7,696(0,8);7,572(0,4);7,568(0,3);7,554(0,9);7,552(1,0);7,545(0,8);7,541(1,6);7,536(2,9);7,535(2,8);7,530(1,6);7,524(0,9);7,520(1,3);7,516(2,2);7,509(0,4);7,505(0,5);7,501(0,7);7,494(0,5);7,394(0,8);7,392(0,9);7,382(0,9);7,380(0,9);7,372(0,8);7,361(0,7);7,360(0,7);6,530(1,6);5,447(2,7);3,886(16,0);3,870(0,7);3,425(15,8);3,410(0,7);2,156(9,3);1,964(1,3);1,958(1,9);1,952(7,0);1,946(11,8);1,940(15,9);1,934(11,1);1,927(6,1);0,000(2,5) |
| I-A-141 | 1,39 | 1,84 | ¹H-NMR(400,0 MHz, CD3CN): δ= 8,438(1,4);8,390(1,2);7,687(0,4);7,666(1,4);7,646(1,3);7,489(2,5);7,472(2,0);7,393(3,4);7,353(1,3);7,344(1,4);5,446(5,4);4,057(0,4);3,861(2,4);3,805(2,7);3,763(0,5);3,602(2,4);3,433(0,5);3,387(16,0);2,156(30,0);1,963(0,8);1,951(8,5);1,945(15,3);1,939(20,4);1,933(14,6);1,927(7,7);1,269(0,7);0,823(0,3);0,000(1,9) |
| I-A-142 | 1,31 | 1,47 | ¹H-NMR(400,0 MHz, CD3CN): δ= 8,464(1,0);8,454(1,0);8,405(1,3);8,399(1,2);7,688(0,8);7,684(0,9);7,682(0,9);7,678(0,8);7,668(0,8);7,664(0,9);7,662(0,9);7,658(0,8);7,375(1,0);7,363(1,0);7,355(0,9);7,343(0,8);6,391(1,3);5,447(6,3);3,845(16,0);3,763(0,3);3,405(16,0);3,388(0,5);2,174(22,0);1,983(7,9);1,982(8,1);1,964(0,7);1,958(1,1);1,952(4,0);1,946(6,8);1,940(9,0);1,934(6,0);1,928(3,0);1,270(0,4);0,000(0,5) |
| I-A-143 | 1,12 | 1,27 | ¹H-NMR(400,0 MHz, CD3CN): δ= 8,450(1,0);8,447(1,0);8,438(1,0);8,435(1,0);8,392(1,3);8,386(1,3);7,666(0,7);7,662(0,8);7,660(0,8);7,656(0,6);7,646(0,8);7,642(0,9);7,639(0,9);7,636(0,7);7,611(0,7);7,358(0,9);7,347(0,9);7,338(0,8);7,326(0,7);6,325(0,5);5,447(5,2);3,863(10,4);3,830(8,8);3,810(0,5);3,799(16,0);3,763(0,8);3,386(15,7);2,164(26,8);1,964(0,6);1,958(1,2);1,952(6,7);1,946(12,4);1,940(17,0);1,934(11,6);1,928(5,8);1,270(0,4);0,000(1,5) |
| I-A-144 | | 0,86 | ¹H-NMR(400,0 MHz, CD3CN): δ= 8,447(1,4);8,443(1,5);8,435(1,4);8,432(1,5);8,386(2,0);8,379(2,0);7,659(0,7);7,655(0,9);7,650(0,8);7,639(0,8);7,634(1.0);7,630(0,8);7,351(1,1);7,339(1,1);7,331(1,0);7,319(1,0);7,129(2,9);6,962(2,8);6,259(2,8);5,447(4,1);3,853(16,0);3,806(1,4);3,647(15,2);3,386(1,9);3,375(15,7);2,170(4,9);1,964(0,5);1,958(1,0);1,952(4,8);1,946(8,7);1,939(11,8);1,933(8,3);1,927(4,3);0,000(1,0) |
| I-A-145 | 0,31 | 0,85 | ¹H-NMR(600,1 MHz, d₆-DMSO): δ= 8,587(5,5);8,585(5,8);8,580(6,0);8,577(6,0);8,571(7,7);8,568(7,8);7,894(3,4);7,891(4,0);7,890(3,9);7,887(3,5);7,880(3,8);7,878(4,2);7,876(4,3);7,874(3,7);7,5254(4,5);7,5245(4,4);7,5174(4,5);7,5166(4,4);7,512(4,4);7,511(4,4);7,504(4,2);7,503(4,1);5,718(5,0);4,449(8,6);4,437(15,3);4,424(9,6);3,948(10,1);3,935(16,0);3,923(9,0);3,790(0,4);3,686(0,5);3,494(0,5);3,459(0,6);3,450(0,7);3,370(4,4);3,346(7,9);3,335(73,5);3,216(0,6);2,794(0,4);2,787(0,4);2,720(2,5);2,614(0,5);2,611(0,3);2,523(0,7);2,520(0,9);2,517(0,8);2,508(23,1);2,505(50,2);2,502(69,7);2,499(51,5);2,496(25,3);2,389(0,3);2,386(0,5);2,383(0,3);1,194(0,4);1,182(0,7);1,170(0,4);0,000(2,5) |
| I-A-146 | 1,11 | 1,27 | ¹H-NMR(400,0 MHz, d₆-DMSO): δ= 8,632(15,5);8,620(16,0);8,505(3,7);8,502(4,1);8,493(4,1);8,490(4,6);8,485(5,6);8,479(5,4);8,313(0,5);7,842(1,9);7,838(2,3);7,835(2,3);7,831(2,1);7,821(2,2);7,817(2,5);7,815(2,7);7,811(2,2);7,492(2,9);7,480(2,9);7,471(2,7);7,459(2,6);7,329(4,1);7,317(7,8);7,304(4,0);6,535(4,3);5,754(1,0);4,306(1,7);4,288(5,9);4,271(6,0);4,253(1,8);3,417(36,1);3,317(141,0);2,675(1,1);2,670(1,4);2,666(1,0);2,524(3,5);2,510(74,8);2,506(159,0);2,501(224,8);2,497(171,3);2,492(84,5);2,333(0,9);2,328(1,3);2,324(1,0);1,310(6,5);1,292(14,9);1,274(6,4);0,146(0,6);0,008(4,5);0,000(133,6);-0,008(5,1);-0,150(0,6) |

### Biologische Beispiele

**Meloidogyne incognita- Test**

| | |
|---|---|
| Lösungsmittel: | 125,0 Gewichtsteile Aceton |

Zur Herstellung einer zweckmäßigen Wirkstoffzubereitung vermischt man 1 Gewichtsteil Wirkstoff mit der angegebenen Menge Lösungsmittel und verdünnt das Konzentrat mit Wasser auf die gewünschte Konzentration.

Gefäße werden mit Sand, Wirkstofflösung, einer Ei-Larven-Suspension des südlichen Wurzelgallenälchens (Meloidogyne incognita) und Salatsamen gefüllt. Die Salatsamen keimen und die Pflänzchen entwickeln sich. An den Wurzeln entwickeln sich die Gallen.

Nach 14 Tagen wird die nematizide Wirkung anhand der Gallenbildung in % bestimmt. Dabei bedeutet 100 %, dass keine Gallen gefunden wurden; 0 % bedeutet, dass die Zahl der Gallen an den behandelten Pflanzen der unbehandelten Kontrolle entspricht.

Bei diesem Test zeigen z. B. die folgenden Verbindungen der Herstellungsbeispiele Wirkung von 90% bei einer Aufwandmenge von 20ppm: I-A-7; I-A-35

**Myzus persicae - Sprühtest**

| | |
|---|---|
| Lösungsmittel: | 78 Gewichtsteile Aceton |
| Emulgator: | 1,5 Gewichtsteile Dimethylformamid Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (Brassica pekinensis), die von allen Stadien der Grünen Pfirsichblattlaus (Myzus persicae) befallen sind, werden mit einer Wirkstoffzubereitung der gewünschten Konzentration gespritzt.

Nach 5 oder 6 Tagen wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele Wirkung von 100% bei einer Aufwandmenge von 500 g/ha: I-A-1, I-A-2, I-A-3, I-A-8, I-A-10, ; I-A-11, I-A-18, I-A-19, I-A-21, I-A-22, I-A-26, I-A-37, I-A-40, I-A-45, I-A-46, I-A-47, I-A-48, I-A-49, I-A-50, I-A-51, I-A-52, I-A-53, I-A-54, I-A-65, I-A-68, I-A-69, I-A-70, I-A-71, I-A-94, I-A-95, I-A-96, I-A-101, I-A-102, I-A-103, I-A-104, I-A-105, I-A-106, I-A-108, I-A-110, I-A-112, I-A-116, I-A-122, I-A-126, I-A-134, I-A-135, I-A-136, I-A-138, I-A-139, I-A-140, I-A-141, I-A-144, I-A-145, I-B-1
Bei diesem Test zeigen z. B. die folgenden Verbindungen der Herstellungsbeispiele Wirkung von 90% bei einer Aufwandmenge von 500 g/ha: I-A-5, I-A-6, I-A-7, I-A-12, I-A-14, I-A-16, I-A-23, I-A-24, I-A-25, I-A-27, I-A-28, I-A-30, I-A-31, I-A-36, I-A-38, I-A-39, I-A-41, I-A-55, I-A-58, I-A-59, I-A-60, I-A-61, I-A-62, I-A-64, I-A-67, I-A-73, I-A-74, I-A-75, I-A-77, I-A-79, I-A-82, I-A-83, I-A-84, I-A-86, I-A-87, I-A-97, I-A-98, I-A-100, 1-A-107, I-A-109, I-A-113, I-A-117, I-A-119, I-A-120, I-A-123, I-A-124, I-A-125, I-A-127, I-A-128, I-A-130, I-A-131, I-A-132, I-A-137, I-A-142
Bei diesem Test zeigen z. B. die folgenden Verbindungen der Herstellungsbeispiele Wirkung von 90% bei einer Aufwandmenge von 100 g/ha: I-A-78, I-A-146
Bei diesem Test zeigen z. B. die folgenden Verbindungen der Herstellungsbeispiele Wirkung von 90% bei einer Aufwandmenge von 20 g/ha: I-A-9

**T etranvchus urticae - Sprühtest, OP-resistent**

| | |
|---|---|
| Lösungsmittel: | 78,0 GewichtsteileAceton |
| Emulgator: | 1,5 Gewichtsteile Dimethylformamid Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben (Phaseolus vulgaris), die von allen Stadien der Gemeinen Spinnmilbe (Tetranychus urticae) befallen sind, werden mit einer Wirkstoffzubereitung der gewünschten Konzentration gespritzt.

Nach 6 Tagen wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele eine Wirkung von 90% bei einer Aufwandmenge von 500g/ha: I-A-6, I-A-78, I-A-105

**Phaedon cochleariae - Sprühtest**

| | |
|---|---|
| Lösungsmittel: | 78,0 Gewichtsteile Aceton |
| Emulgator: | 1,5 Gewichtsteile Dimethylformamid Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (Brassica pekinensis) werden mit einer Wirkstoffzubereitung der gewünschten Konzentration gespritzt und nach dem Abtrocknen mit Larven des Meerrettichblattkäfers (Phaedon cochleariae) besetzt.

Nach 7 Tagen wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele Wirkung von 100% bei einer Aufwandmenge von 500g/ha: I-A-79, I-A-80
Bei diesem Test zeigen z. B. die folgenden Verbindungen der Herstellungsbeispiele Wirkung von 83% bei einer Aufwandmenge von 500g/ha: I-A-15

**Myzus persicae - Sprühtest**

| | |
|---|---|
| Lösungsmittel: | 14 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt. Bei erforderlicher Zugabe von Ammoniumsalzen oder/und Penetrationsförderern werden diese jeweils in einer Konzentration von 1000 ppm der Präparatelösung zugefügt.

Paprikapflanzen (Capsicum annuum), die stark von der Grünen Pfirsichblattlaus (Myzus persicae) befallen sind, werden durch Sprühen mit der Wirkstoffzubereitung in der gewünschten Konzentration behandelt.

Nach 6 Tagen wird die Abtötung in % bestimmt. Dabei bedeutet 100 %, dass alle Läuse abgetötet wurden; 0 % bedeutet, dass keine Läuse abgetötet wurden.

Bei diesem Test zeigen z. B. die folgenden Verbindungen der Herstellungsbeispiele Wirkung von 99% bei einer Aufwandmenge von 100ppm: I-A-66
Bei diesem Test zeigen z. B. die folgenden Verbindungen der Herstellungsbeispiele Wirkung von 100% bei einer Aufwandmenge von 20ppm: I-A-44, I-A-99
Bei diesem Test zeigen z. B. die folgenden Verbindungen der Herstellungsbeispiele Wirkung von 99% bei einer Aufwandmenge von 20ppm: I-A-4, I-A-20, I-A-57, I-A-89
Bei diesem Test zeigen z. B. die folgenden Verbindungen der Herstellungsbeispiele Wirkung von 95% bei einer Aufwandmenge von 20ppm: I-A-93

### Absetzbeispiele

**Myzus persicae - Sprühtest (MYZUPE)**

| | |
|---|---|
| Lösungsmittel: | 78,0 Gewichtsteile Aceton |
| Emulgator: | 1,5 Gewichtsteile Dimethylformamid Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (Brassica pekinensis), die von allen Stadien der Grünen Pfirsichblattlaus (Myzus persicae) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Blattläuse abgetötet wurden; 0 % bedeutet, dass keine Blattläuse abgetötet wurden.

Bei diesem Test zeigen z. B. die folgenden Verbindungen der Herstellungsbeispiele überlegene Wirksamkeit gegenüber dem Stand der Technik: siehe Tabelle

**Tetranychus urticae - Sprühtest ; OP-resistent (TETRUR)**

| | |
|---|---|
| Lösungsmittel: | 78,0 Gewichtsteile Aceton |
| Emulgator: | 1,5 Gewichtsteile Dimethylformamid Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Bohnenblattscheiben (Phaseolus vulgaris), die von allen Stadien der Gemeinen Spinnmilbe (Tetranychus urticae) befallen sind, werden mit einer Wirkstoffzubereitung der gewünschten Konzentration gespritzt.

Nach der gewünschten Zeit wird die Wirkung in % bestimmt. Dabei bedeutet 100 %, dass alle Spinnmilben abgetötet wurden; 0 % bedeutet, dass keine Spinnmilben abgetötet wurden.

Bei diesem Test zeigen z. B. die folgenden Verbindungen der Herstellungsbeispiele überlegene Wirksamkeit gegenüber dem Stand der Technik: siehe Tabelle

**Phaedon cochleariae - Sprühtest (PHAECO)**

| | |
|---|---|
| Lösungsmittel: | 78,0 Gewichtsteile Aceton |
| | 1,5 Gewichtsteile Dimethylformamid |
| Emulgator: | Alkylarylpolyglykolether |

Zur Herstellung einer zweckmäßigen Wirkstoffzubereitung löst man 1 Gewichtsteil Wirkstoff mit den angegebenen Gewichtsteilen Lösungsmittel und füllt mit Wasser, welches eine Emulgatorkonzentration von 1000 ppm enthält, bis zum Erreichen der gewünschten Konzentration auf. Zur Herstellung weiterer Testkonzentrationen wird mit emulgatorhaltigem Wasser verdünnt.

Chinakohlblattscheiben (Brassica pekinensis) werden mit einer Wirkstoffzubereitung der gewünschten Konzentration gespritzt und nach dem Abtrocknen mit Larven des Meerrettichblattkäfers (Phaedon cochleariae) besetzt.

Nach der gewünschten Zeit wird die Wirkung in % bestimmt. Dabei bedeutet 100 %, dass alle Käferlarven abgetötet wurden; 0 % bedeutet, dass keine Käferlarven abgetötet wurden.

Bei diesem Test zeigen z. B. die folgenden Verbindungen der Herstellungsbeispiele überlegene Wirksamkeit gegenüber dem Stand der Technik: siehe Tabelle

| Substanz | Struktur | Tierart | Konzentration | % Wirkung dat | |
|---|---|---|---|---|---|
| Bsp.Nr. 13 | 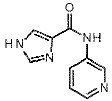 | PHAECO | 500 g ai/ha | 0 | 7 dat |
| Stand der Technik WO2011/009804 | | TETRUR | 500 g ai/ha | 0 | 6 dat |
| | | MYZUPE | 100 g ai/ha | 0 | 6 dat |
| Bsp.Nr. 14 | 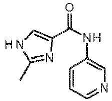 | PHAECO | 500 g ai/ha | 0 | 7 dat |
| Stand der Technik WO2011/009804 | | TETRUR | 500 g ai/ha | 0 | 6 dat |
| | | MYZUPE | 100 g ai/ha | 0 | 6 dat |
| Bsp.Nr. I-A-79 | 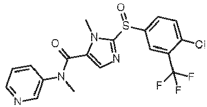 | PHAECO | 500 g ai/ha | 100 | 7 dat |
| Erfindungsgemäß | | | | | |
| Bsp.Nr. I-A-80 | 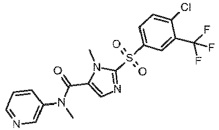 | PHAECO | 500 g ai/ha | 100 | 7 dat |
| Erfindungsgemäß | | | | | |
| Bsp.Nr. I-A-15 | 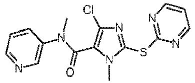 | PHAECO | 500 g ai/ha | 83 | 7 dat |
| Erfindungsgemäß | | | | | |
| Bsp.Nr. I-A-6 | 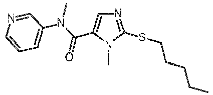 | TETRUR | 500 g ai/ha | 90 | 6 dat |
| Erfindungsgemäß | | MYZUPE | 100 g ai/ha | 90 | 6 dat |
| Bsp.Nr. I-A-78 | 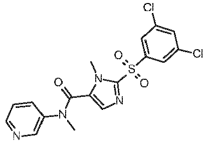 | TETRUR | 500 g ai/ha | 90 | 6 dat |
| Erfindungsgemäß | | MYZUPE | 100 g ai/ha | 90 | 6 dat |
| Bsp.Nr. I-A-105 | 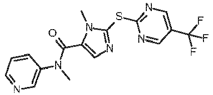 | TETRUR | 500 g ai/ha | 90 | 6 dat |
| Erfindungsgemäß | | MYZUPE | 100 g ai/ha | 100 | 6 dat |
| Bsp.Nr. I-A-39 Erfindungsgemäß | 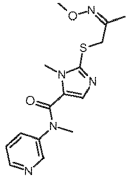 | TETRUR | 500 g ai/ha | 70 | 6 dat |
| Bsp.Nr. I-A-107 | 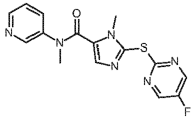 | TETRUR | 500 g ai/ha | 70 | 6 dat |
| Erfindungsgemäß | | MYZUPE | 100 g ai/ha | 90 | 6 dat |
| Bsp.Nr. I-A-110 | 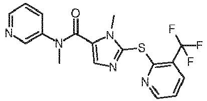 | TETRUR | 500 g ai/ha | 70 | 6 dat |
| Erfindungsgemäß | | MYZUPE | 100 g ai/ha | 100 | 6 dat |
| Bsp.Nr. I-A-125 | 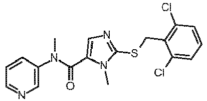 | TETRUR | 500 g ai/ha | 70 | 6 dat |
| Erfindungsgemäß | | MYZUPE | 100 g ai/ha | 70 | 6 dat |
| Bsp.Nr. I-A-1 | 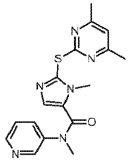 | MYZUPE | 100 g ai/ha | 100 | 6 dat |
| Erfindungsgemäß | | | | | |
| Bsp.Nr. I-A-5 | 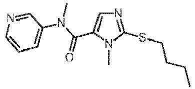 | MYZUPE | 100 g ai/ha | 100 | 6 dat |
| Erfindungsgemäß | | | | | |
| Bsp.Nr. I-A-10 | 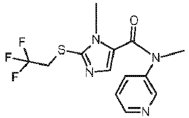 | MYZUPE | 100 g ai/ha | 100 | 6 dat |
| Erfindungsgemäß | | | | | |
| Bsp.Nr. I-A-21 | 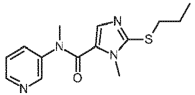 | MYZUPE | 100 g ai/ha | 100 | 6 dat |
| Erfindungsgemäß | | | | | |
| Bsp.Nr. I-A-37 | 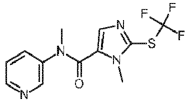 | MYZUPE | 100 g ai/ha | 100 | 6 dat |
| Erfindungsgemäß | | | | | |
| Bsp.Nr. I-A-50 | 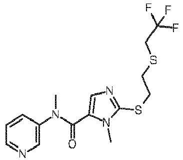 | MYZUPE | 100 g ai/ha | 100 | 5 dat |
| Erfindungsgemäß | | | | | |
| Bsp.Nr. I-A-51 | 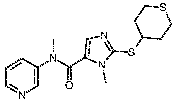 | MYZUPE | 100 g ai/ha | 100 | 5 dat |
| Erfindungsgemäß | | | | | |
| Bsp.Nr. I-A-52 | 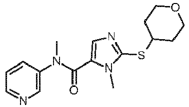 | MYZUPE | 100 g ai/ha | 100 | 5 dat |
| Erfindungsgemäß | | | | | |
| Bsp.Nr. I-A-53 | 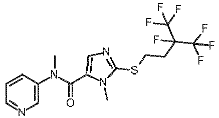 | MYZUPE | 100 g ai/ha | 100 | 5 dat |
| Erfindungsgemäß | | | | | |
| Bsp.Nr. I-A-65 | 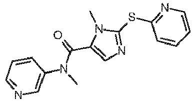 | MYZUPE | 100 g ai/ha | 100 | 6 dat |
| Erfindungsgemäß | | | | | |
| Bsp.Nr. I-A-134 | 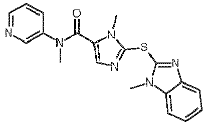 | MYZUPE | 100 g ai/ha | 100 | 5 dat |
| Erfindungsgemäß | | | | | |
| Bsp.Nr. I-A-2 | 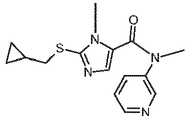 | MYZUPE | 100 g ai/ha | 90 | 6 dat |
| Erfindungsgemäß | | | | | |
| Bsp.Nr. I-A-3 | 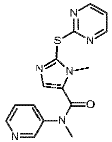 | MYZUPE | 100 g ai/ha | 90 | 6 dat |
| Erfindungsgemäß | | | | | |
| Bsp.Nr. I-A-8 | 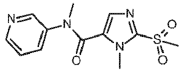 | MYZUPE | 100 g ai/ha | 90 | 6 dat |
| Erfindungsgemäß | | | | | |
| Bsp.Nr. I-A-11 | 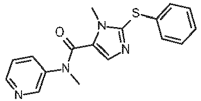 | MYZUPE | 100 g ai/ha | 90 | 6 dat |
| Erfindungsgemäß | | | | | |
| Bsp.Nr. I-A-14 | 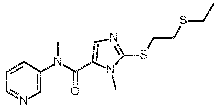 | MYZUPE | 100 g ai/ha | 90 | 6 dat |
| Erfindungsgemäß | | | | | |
| Bsp.Nr. I-A-18 | 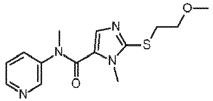 | MYZUPE | 100 g ai/ha | 90 | 6 dat |
| Erfindungsgemäß | | | | | |
| Bsp.Nr. I-A-19 | 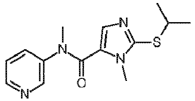 | MYZUPE | 100 g ai/ha | 90 | 6 dat |
| Erfindungsgemäß | | | | | |
| Bsp.Nr. I-A-22 | 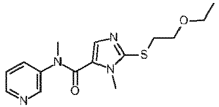 | MYZUPE | 100 g ai/ha | 90 | 6 dat |
| Erfindungsgemäß | | | | | |
| Bsp.Nr. I-A-30 | 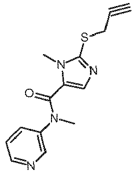 | MYZUPE | 100 g ai/ha | 90 | 6 dat |
| Erfindungsgemäß | | | | | |
| Bsp.Nr. I-A-36 | 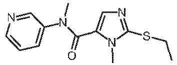 | MYZUPE | 100 g ai/ha | 90 | 6 dat |
| Erfindungs gemäß | | | | | |
| Bsp.Nr. I-A-40 | 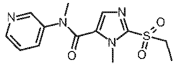 | MYZUPE | 100 g ai/ha | 90 | 6 dat |
| Erfindungsgemäß | | | | | |
| Bsp.Nr. I-A-45 | 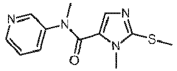 | MYZUPE | 100 g ai/ha | 90 | 6 dat |
| Erfindungsgemäß | | | | | |
| Bsp.Nr. I-A-46 | 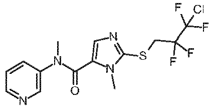 | MYZUPE | 100 g ai/ha | 90 | 5 dat |
| Erfindungsgemäß | | | | | |
| Bsp.Nr. I-A-47 | 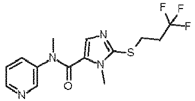 | MYZUPE | 100 g ai/ha | 90 | 5 dat |
| Erfindungsgemäß | | | | | |
| Bsp.Nr. I-A-48 | 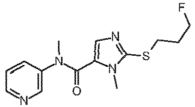 | MYZUPE | 100 g ai/ha | 90 | 5 dat |
| Erfindungsgemäß | | | | | |
| Bsp.Nr. I-A-49 | 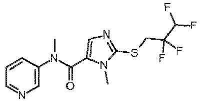 | MYZUPE | 100 g ai/ha | 90 | 5 dat |
| Erfindungsgemäß | | | | | |
| Bsp.Nr. I-A-54 | 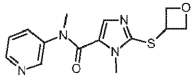 | MYZUPE | 100 g ai/ha | 90 | 5 dat |
| Erfindungsgemäß | | | | | |
| Bsp.Nr. I-A-55 | 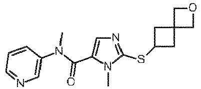 | MYZUPE | 100 g ai/ha | 90 | 5 dat |
| Erfindungsgemäß | | | | | |
| Bsp.Nr. I-A-59 | 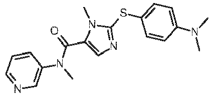 | MYZUPE | 100 g ai/ha | 90 | 6 dat |
| Erfindungsgemäß | | | | | |
| Bsp.Nr. I-A-61 | 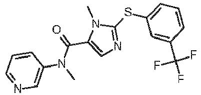 | MYZUPE | 100 g ai/ha | 90 | 6 dat |
| Erfindungsgemäß | | | | | |
| Bsp.Nr. I-A-82 | 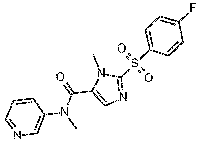 | MYZUPE | 100 g ai/ha | 90 | 6 dat |
| Erfindungsgemäß | | | | | |
| Bsp.Nr. I-A-86 | 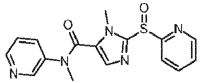 | MYZUPE | 100 g ai/ha | 90 | 6 dat |
| Erfindungsgemäß | | | | | |
| Bsp.Nr. I-A-87 | 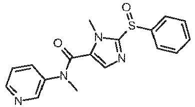 | MYZUPE | 100 g ai/ha | 90 | 6 dat |
| Erfindungsgemäß | | | | | |
| Bsp.Nr. I-A-94 | 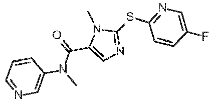 | MYZUPE | 100 g ai/ha | 90 | 6 dat |
| Erfindungsgemäß | | | | | |
| Bsp.Nr. I-A-95 | 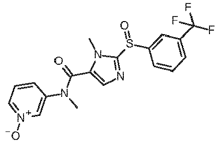 | MYZUPE | 100 g ai/ha | 90 | 5 dat |
| Erfindungsgemäß | | | | | |
| Bsp.Nr. I-A-96 | 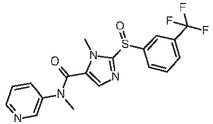 | MYZUPE | 100 g ai/ha | 90 | 5 dat |
| Erfindungsgemäß | | | | | |
| Bsp.Nr. I-A-100 | 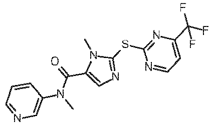 | MYZUPE | 100 g ai/ha | 90 | 6 dat |
| Erfindungsgemäß | | | | | |
| Bsp.Nr. I-A-101 | 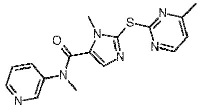 | MYZUPE | 100 g ai/ha | 90 | 6 dat |
| Erfindungsgemäß | | | | | |
| Bsp.Nr. I-A-102 | 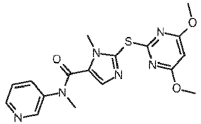 | MYZUPE | 100 g ai/ha | 90 | 6 dat |
| Erfindungsgemäß | | | | | |
| Bsp.Nr. I-A-104 | 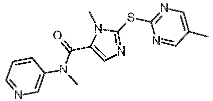 | MYZUPE | 100 g ai/ha | 90 | 6 dat |
| Erfindungsgemäß | | | | | |
| Bsp.Nr. I-A-106 | 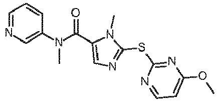 | MYZUPE | 100 g ai/ha | 90 | 6 dat |
| Erfindungsgemäß | | | | | |
| Bsp.Nr. I-A-112 | 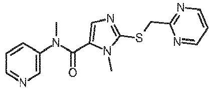 | MYZUPE | 100 g ai/ha | 90 | 6 dat |
| Erfindungsgemäß | | | | | |
| Bsp.Nr. I-A-113 | 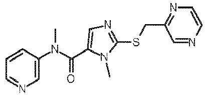 | MYZUPE | 100 g ai/ha | 90 | 6 dat |
| Erfindungsgemäß | | | | | |
| Bsp.Nr. I-A-116 | 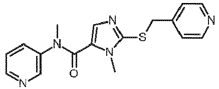 | MYZUPE | 100 g ai/ha | 90 | 6 dat |
| Erfindungsgemäß | | | | | |
| Bsp.Nr. I-A-117 | 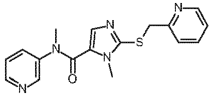 | MYZUPE | 100 g ai/ha | 90 | 6 dat |
| Erfindungsgemäß | | | | | |
| Bsp.Nr. I-A-122 | 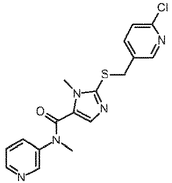 | MYZUPE | 100 g ai/ha | 90 | 6 dat |
| Erfindungsgemäß | | | | | |
| Bsp.Nr. I-A-128 | 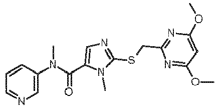 | MYZUPE | 100 g ai/ha | 90 | 6 dat |
| Erfindungsgemäß | | | | | |
| Bsp.Nr. I-A-130 | 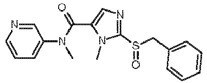 | MYZUPE | 100 g ai/ha | 90 | 5 dat |
| Erfindungsgemäß | | | | | |
| Bsp.Nr. I-A-135 | 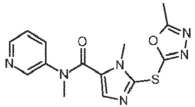 | MYZUPE | 100 g ai/ha | 90 | 5 dat |
| Erfindungsgemäß | | | | | |
| Bsp.Nr. I-A-136 | 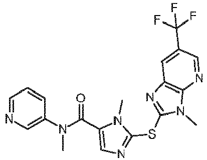 | MYZUPE | 100 g ai/ha | 90 | 5 dat |
| Erfindungsgemäß | | | | | |
| Bsp.Nr. I-A-137 | 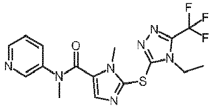 | MYZUPE | 100 g ai/ha | 90 | 5 dat |
| Erfindungsgemäß | | | | | |
| Bsp.Nr. I-A-138 | 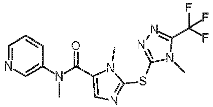 | MYZUPE | 100 g ai/ha | 90 | 5 dat |
| Erfindungsgemäß | | | | | |
| Bsp.Nr. I-A-139 | 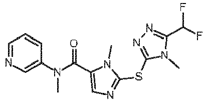 | MYZUPE | 100 g ai/ha | 90 | 5 dat |
| Erfindungsgemäß | | | | | |
| Bsp.Nr. I-A-142 | 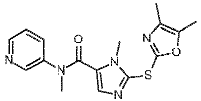 | MYZUPE | 100 g ai/ha | 90 | 5 dat |
| Erfindungsgemäß | | | | | |
| Bsp.Nr. I-A-144 | 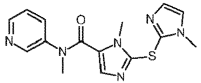 | MYZUPE | 100 g ai/ha | 90 | 5 dat |
| Erfindungsgemäß | | | | | |
| Bsp.Nr. I-B-1 | 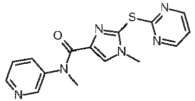 | MYZUPE | 100 g ai/ha | 90 | 6 dat |
| Erfindungsgemäß | | | | | |
| Bsp.Nr. I-A-4 | 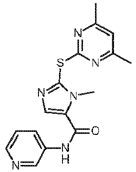 | MYZUPE | 100 g ai/ha | 70 | 6 dat |
| Erfindungsgemäß | | | | | |
| Bsp.Nr. I-A-7 | 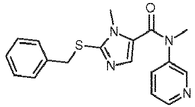 | MYZUPE | 100 g ai/ha | 70 | 6 dat |
| Erfindungsgemäß | | | | | |
| Bsp.Nr. I-A-9 | 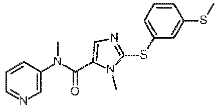 | MYZUPE | 100 g ai/ha | 70 | 6 dat |
| Erfindungsgemäß | | | | | |
| Bsp.Nr. I-A-12 | 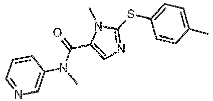 | MYZUPE | 100 g ai/ha | 70 | 6 dat |
| Erfindungsgemäß | | | | | |
| Bsp.Nr. I-A-26 | 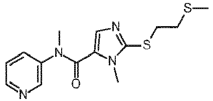 | MYZUPE | 100 g ai/ha | 70 | 6 dat |
| Erfindungsgemäß | | | | | |
| Bsp.Nr. I-A-27 | 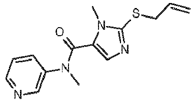 | MYZUPE | 100 g ai/ha | 70 | 6 dat |
| Erfindungsgemäß | | | | | |
| Bsp.Nr. I-A-38 | 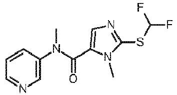 | MYZUPE | 100 g ai/ha | 70 | 6 dat |
| Erfindungsgemäß | | | | | |
| Bsp.Nr. I-A-43 | 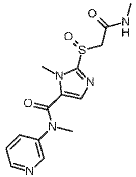 | MYZUPE | 100 g ai/ha | 70 | 6 dat |
| Erfindungsgemäß | | | | | |
| Bsp.Nr. I-A-56 | 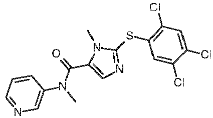 | MYZUPE | 100 g ai/ha | 70 | 6 dat |
| Erfindungsgemäß | | | | | |
| Bsp.Nr. I-A-58 | 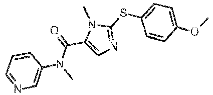 | MYZUPE | 100 g ai/ha | 70 | 6 dat |
| Erfindungsgemäß | | | | | |
| Bsp.Nr. I-A-60 | 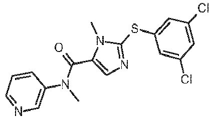 | MYZUPE | 100 g ai/ha | 70 | 6 dat |
| Erfindungsgemäß | | | | | |
| Bsp.Nr. I-A-62 | 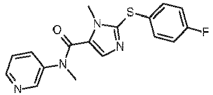 | MYZUPE | 100 g ai/ha | 70 | 6 dat |
| Erfindungsgemäß | | | | | |
| Bsp.Nr. I-A-64 | 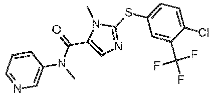 | MYZUPE | 100 g ai/ha | 70 | 6 dat |
| Erfindungsgemäß | | | | | |
| Bsp.Nr. I-A-69 | 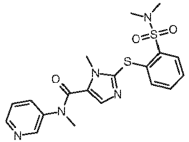 | MYZUPE | 100 g ai/ha | 70 | 6 dat |
| Erfindungsgemäß | | | | | |
| Bsp.Nr. I-A-70 | 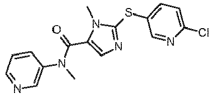 | MYZUPE | 100 g ai/ha | 70 | 6 dat |
| Erfindungsgemäß | | | | | |
| Bsp.Nr. I-A-71 | 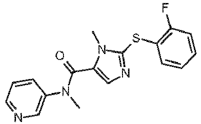 | MYZUPE | 100 g ai/ha | 70 | 6 dat |
| Erfindungsgemäß | | | | | |
| Bsp.Nr. I-A-77 | 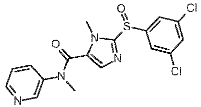 | MYZUPE | 100 g ai/ha | 70 | 6 dat |
| Erfindungsgemäß | | | | | |
| Bsp.Nr. I-A-84 | 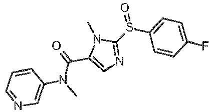 | MYZUPE | 100 g ai/ha | 70 | 6 dat |
| Erfindungsgemäß | | | | | |
| Bsp.Nr. I-A-89 | 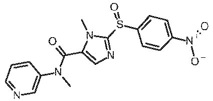 | MYZUPE | 100 g ai/ha | 70 | 6 dat |
| Erfindungsgemäß | | | | | |
| Bsp.Nr. I-A-93 | 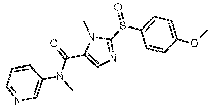 | MYZUPE | 100 g ai/ha | 70 | 6 dat |
| Erfindungsgemäß | | | | | |
| Bsp.Nr. I-A-97 | 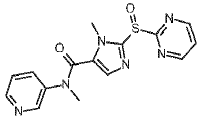 | MYZUPE | 100 g ai/ha | 70 | 6 dat |
| Erfindungsgemäß | | | | | |
| Bsp.Nr. I-A-99 | 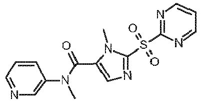 | MYZUPE | 100 g ai/ha | 70 | 6 dat |
| Erfindungsgemäß | | | | | |
| Bsp.Nr. I-A-108 | 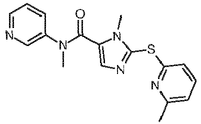 | MYZUPE | 100 g ai/ha | 70 | 6 dat |
| Erfindungsgemäß | | | | | |
| Bsp.Nr. I-A-109 | 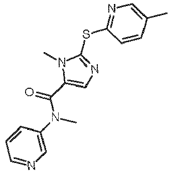 | MYZUPE | 100 g ai/ha | 70 | 6 dat |
| Erfindungsgemäß | | | | | |
| Bsp.Nr. I-A-126 | 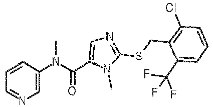 | MYZUPE | 100 g ai/ha | 70 | 6 dat |
| Erfindungsgemäß | | | | | |
| Bsp.Nr. I-A-140 | 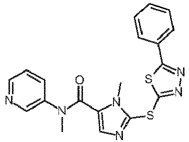 | MYZUPE | 100 g ai/ha | 70 | 5 dat |
| Erfindungsgemäß | | | | | |
| Bsp.Nr. I-A-141 | 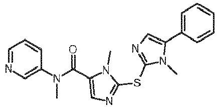 | MYZUPE | 100 g ai/ha | 70 | 5 dat |
| Erfindungsgemäß | | | | | |
| Bsp.Nr. I-A-145 | 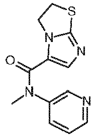 | MYZUPE | 100 g ai/ha | 70 | 5 dat |
| Erfindungsgemäß | | | | | |
| Bsp.Nr. I-A-146 | 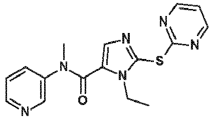 | MYZUPE | 100 g ai/ha | 90 | 5 dat |
| Erfindungsgemäß | | | | | |

## Patentansprüche

1. Verbindungen der Formel (I) 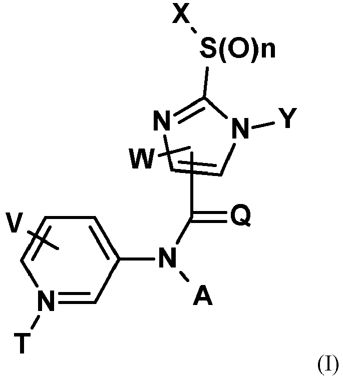 in welcher
Q für Sauerstoff oder Schwefel steht,
V für einen Rest aus der Reihe Wasserstoff, Halogen, Alkyl, Halogenalkyl, Alkoxy, Halogenalkoxy und Cyano steht,
W für einen Rest aus der Reihe Wasserstoff, Halogen, Alkyl, Halogenalkyl, Alkoxy, Halogenalkoxy und Cyano steht,
X für einen Rest aus der Reihe gegebenenfalls substituiertes Alkyl, Alkenyl, Alkinyl, gegebenenfalls durch Heteroatome unterbrochenes und gegebenenfalls substituiertes, gesättigtes oder ungesättigtes Cycloalkyl, gegebenenfalls durch Heteroatome unterbrochenes und gegebenenfalls substituiertes, gesättigtes oder ungesättigtes Cycloalkylalkyl, gegebenenfalls substituiertes Aryl, Hetaryl, gegebenenfalls substituiertes Arylalkyl, Hetarylalkyl und Cyano steht,
Y für einen Rest aus der Reihe Wasserstoff, gegebenenfalls substituiertes Alkyl, Alkenyl, Alkinyl, gegebenenfalls durch Heteroatome unterbrochenes und gegebenenfalls substituiertes Cycloalkyl, gegebenenfalls durch Heteroatome unterbrochenes und gegebenenfalls substituiertes Cycloalkylalkyl, Arylalkyl, Hetarylalkyl und Cyano steht,
n für eine Zahl 0,1 oder 2 steht,
A für einen Rest aus der Reihe Wasserstoff, gegebenenfalls substituiertes Alkyl, Alkenyl, Alkinyl und gegebenenfalls durch Heteroatome unterbrochenes und gegebenenfalls substituiertes Cycloalkyl und Cycloalkyl-alkyl steht,
T für Sauerstoff oder ein Elektronenpaar steht,
und deren Salze.

2. Verbindungen gemäß Anspruch 1, wobei
Q für Sauerstoff oder Schwefel steht,
V für einen Rest aus der Reihe Wasserstoff, Halogen, C₁-C₄-Alkyl, C₁-C₄-Halogenalkyl, C₁-C₄-Alkoxy, C₁-C₄-Halogenalkoxy und Cyano steht,
W für einen Rest aus der Reihe Wasserstoff, Halogen, C₁-C₄-Alkyl, C₁-C₄-Halogenalkyl, C₁-C₄-Alkoxy, C₁-C₄-Halogenalkoxy und Cyano steht,
X für einen Rest aus der Reihe gegebenenfalls einfach bis mehrfach unabhängig voneinander durch Halogen, C₁-C₄-Alkoxy, C₁-C₄-Alkyl-S(O)ₘ-, Cyano, C(O)OR², CONR²R³, C(G)R² substituiertes C₁-C₈-Alkyl, C₃-C₈-Alkenyl, C₃-C₈-Alkinyl, gegebenenfalls einfach bis zweifach unabhängig voneinander durch O, S(O)ₘ, C(G)R², NR⁴ unterbrochenes und gegebenenfalls einfach bis vierfach unabhängig voneinander durch Halogen, C₁-C₄-Alkyl, C₁-C₄-Halogenalkyl, C₁-C₄-Alkoxy, Cyano substituiertes C₃-C₈-Cycloalkyl oder C₅-C₈-Cycloalkenyl, gegebenenfalls einfach bis zweifach unabhängig voneinander durch O, S(O)ₘ, C(G)R², NR⁴ unterbrochenes und gegebenenfalls einfach bis vierfach unabhängig voneinander durch Halogen, C₁-C₄-Alkyl, C₁-C₄-Halogenalkyl, C₁-C₄-Alkoxy, Cyano substituiertes, geradkettiges oder verzweigtes, C₃-C₈-Cycloalkyl-C₁-C₄-Alkyl oder C₅-C₈-Cycloalkenyl-C₁-C₄-Alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₘ-, C₁-C₄-Halogenalkoxy, C₁-C₄-Halogenalkyl-S(O)ₘ-, Nitro und Cyano substituiertes Aryl oder Hetaryl, welches gegebenenfalls durch einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₘ-, C₁-C₄-Halogenalkoxy, C₁-C₄-Halogenalkyl-S(O)ₘ-, Nitro und Cyano substituiertes Aryl oder Hetaryl substituiert sein kann, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₘ- C₁-C₄-Halogenalkoxy, C₁-C₄-Halogenalkyl-S(O)ₘ-, Nitro und Cyano substituiertes, geradkettiges oder verzweigtes Aryl-C₁-C₄-alkyl, Hetaryl-C₁-C₄-alkyl steht,
G für O, N-CN, N-OR² steht,
Y für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₘ-, Cyano substituiertes C₁-C₆-Alkyl, C₃-C₆-Alkenyl, C₃-C₆-Alkinyl, gegebenenfalls einfach bis zweifach unabhängig voneinander durch Halogen, C₁-C₄-Alkyl, C₁-C₄-Halogenalkyl, C₁-C₄-Alkoxy, Cyano substituiertes C₃-C₈-Cycloalkyl, gegebenenfalls einfach bis zweifach unabhängig voneinander durch O, S(O)ₘ, CO, NR⁴ unterbrochenes und gegebenenfalls einfach bis vierfach unabhängig voneinander durch Halogen, C₁-C₄-Alkyl, C₁-C₄-Halogenalkyl, C₁-C₄-Alkoxy, Cyano substituiertes, geradkettiges oder verzweigtes C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₘ-, C₁-C₄-Halogenalkoxy, C₁-C₄-Halogenalkyl-S(O)ₘ-, Nitro und Cyano substituiertes Arylalkyl oder Hetarylalkyl und Cyano steht,
m für eine Zahl 0,1 oder 2 steht,
n für eine Zahl 0,1 oder 2 steht,
A für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₘ-, Cyano substituiertes C₁-C₆-Alkyl, C₃-C₆-Alkenyl, C₃-C₆-Alkinyl und gegebenenfalls einfach bis zweifach unabhängig voneinander durch Halogen, C₁-C₄-Alkyl, C₁-C₄-Halogenalkyl, C₁-C₄-Alkoxy, Cyano substituiertes C₃-C₆-Cycloalkyl und gegebenenfalls einfach bis zweifach unabhängig voneinander durch Halogen, C₁-C₄-Alkyl, C₁-C₄-Halogenalkyl, C₁-C₄-Alkoxy, Cyano substituiertes, geradkettiges oder verzweigtes C₃-C₈-Cycloalkyl-C₁-C₄-alkyl steht,
R² für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₘ-, substituiertes C₁-C₈-Alkyl, C₃-C₈-Alkenyl, C₃-C₈-Alkinyl, gegebenenfalls einfach durch O, S(O)ₘ, unterbrochenes und gegebenenfalls einfach bis zweifach unabhängig voneinander durch Halogen, C₁-C₄-Alkyl, C₁-C₄-Halogenalkyl, C₁-C₄-Alkoxy, Cyano substituiertes C₃-C₈-Cycloalkyl, gegebenenfalls einfach durch O, S(O)ₙ, unterbrochenes und gegebenenfalls einfach bis zweifach unabhängig voneinander durch Halogen, C₁-C₄-Alkyl, C₁-C₄-Halogenalkyl, C₁-C₄-Alkoxy, Cyano substituiertes, geradkettiges oder verzweigtes C₃-C₈-Cycloalkyl-C₁-C₄-alkyl, gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₘ-, C₁-C₄-Halogenalkoxy, C₁-C₄-Halogenalkyl-S(O)ₘ-, Nitro und Cyano substituiertes Aryl, Hetaryl und gegebenenfalls einfach bis dreifach unabhängig voneinander durch Halogen, C₁-C₄-Alkyl, C₁-C₄-Halogenalkyl, C₁-C₄-Alkoxy, C₁-C₄-Alkyl-S(O)ₘ-, C₁-C₄-Halogenalkoxy, C₁-C₄-Halogenalkyl-S(O)ₘ-, Nitro und Cyano substituiertes, geradkettiges oder verzweigtes Aryl-C₁-C₄-alkyl, Hetaryl-C₁-C₄-alkyl steht,
R³ für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₘ-, Cyano substituiertes C₁-C₆-Alkyl, C₃-C₆-Alkenyl, C₃-C₆-Alkinyl steht,
R⁴ für einen Rest aus der Reihe Wasserstoff, gegebenenfalls einfach bis mehrfach unabhängig voneinander durch Halogen, C₁-C₄-Alkoxy, C₁-C₄-Alkyl-S(O)ₘ-, Cyano substituiertes C₁-C₆-Alkyl, C₃-C₆-Alkenyl, C₃-C₆-Alkinyl und für die Reste CONR²R³ und COR² steht, wobei für R² und R³ die vorstehenden Definitionen des Vorzugbereichs (1) gelten,
T für Sauerstoff oder ein Elektronenpaar steht
und deren Salze.

3. Verbindungen gemäß Anspruch 1, wobei
Q für Sauerstoff steht
V für einen Rest aus der Reihe Wasserstoff, Fluor, Chlor, Brom, Methyl, Ethyl, Difluormethyl, Trifluormethyl, Methoxy, Ethoxy, Difluormethoxy, Trifluormethoxy und Cyano steht,
W für einen Rest aus der Reihe Wasserstoff, Fluor, Chlor, Brom, Methyl, Ethyl, Difluormethyl, Trifluormethyl, Methoxy, Ethoxy, Difluormethoxy, Trifluormethoxy und Cyano steht,
X für einen Rest aus der Reihe gegebenenfalls einfach bis siebenfach unabhängig voneinander durch Fluor, Chlor, Brom, Methoxy, Ethoxy, Methyl-S(O)ₘ-, Ethyl-S(O)ₘ-, Cyano, C(O)OR², CONR²R³, C(G)R² substituiertes C₁-C₆-Alkyl, C₃-C₆-Alkenyl, C₃-C₆-Alkinyl, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Brom, Methyl, Ethyl, Trifluormethyl, Methoxy, Ethoxy, Cyano substituiertes C₃-C₆-Cycloalkyl, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Brom, Methyl, Ethyl, Trifluormethyl, Methoxy, Ethoxy, Cyano substituiertes, geradkettiges oder verzweigtes C₃-C₆-Cycloalkyl-C₁-C₂-alkyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₙ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes Phenyl, Naphtyl Pyridyl, Pyrimidyl, Pyridazinyl, Pyrazinyl, Triazinyl, Furanyl, Thienyl, Oxazolyl, Isoxazolyl, Thiazolyl, Isothiazolyl, Pyrazolyl, Imidazolyl, Oxdiazolyl, Thiadiazolyl, Triazolyl, Benzimidazolyl, Imidazopyridinyl, welches gegebenenfalls durch einfach bis dreifach unabhängig voneinander durch Fluor, Chlor, Methyl, Ethyl, Difluormethyl, Trifluormethyl, Methoxy, Ethoxy, Methyl-S(O)m-, Ethyl-S(O)n-, Difluormethoxy, Trifluormethoxy, Trifluormethyl-S(O)m-, Difluorethyl-S(O)m-, Trifluorethyl-S(O)m-, Nitro und Cyano substituiertes Phenyl substituiert sein kann, gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₘ-, Et-S(O)ₘ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes, geradkettiges oder verzweigtes Phenyl-C₁-C₂-alkyl, Pyridyl-C₁-C₂-alkyl, Pyrimidyl-C₁-C₂-alkyl, Thiazolyl-C₁-C₂-alkyl, Pyrazolyl-C₁-C₂-alkyl und Cyano steht,
G für O, N-OR² steht,
Y für einen Rest aus der Reihe Wasserstoff, gegebenenfalls einfach bis fünffach unabhängig voneinander durch Fluor, Chlor, Brom, Methoxy, Ethoxy, Methyl-S(O)ₘ-, Ethyl-S(O)ₘ-,Cyano substituiertes C₁-C₄-Alkyl, C₃-C₄-Alkenyl, C₃-C₄-Alkinyl, gegebenenfalls einfach bis zweifach unabhängig voneinander durch Fluor, Chlor, Brom, Methyl, Ethyl, Trifluormethyl, Methoxy, Ethoxy, Cyano substituiertes C₃-C₆-Cycloalkyl, gegebenenfalls einfach bis zweifach unabhängig voneinander durch O, S(O)ₘ, CO, NR⁴ unterbrochenes und gegebenenfalls einfach bis vierfach unabhängig voneinander durch Fluor, Chlor, Brom, Methyl, Ethyl, Trifluormethyl, Methoxy, Ethoxy, Cyano substituiertes, geradkettiges oder verzweigtes C₃-C₆-Cycloalkyl-C₁-C₂-alkyl, gegebenenfalls einfach bis zweifach unabhängig voneinander durch Fluor, Chlor, Brom, Methyl, Ethyl, C₁-C₄-Halogenalkyl, Trifluormethyl, Methoxy, Ethoxy, Cyano Nitro und Cyano substituiertes Aryl- C₁-C₂-alkyl oder Hetaryl- C₁-C₂-alkyl und Cyano steht,
m für eine Zahl 0,1 oder 2 steht,
n für eine Zahl 0,1 oder 2 steht,
A für einen Rest aus der Reihe Wasserstoff, gegebenenfalls einfach bis fünffach unabhängig voneinander durch Fluor, Chlor, Brom, Methoxy, Ethoxy, Methyl-S(O)ₘ-, Ethyl-S(O)ₘ-, Cyano substituiertes C₁-C₆-Alkyl, C₃-C₆-Alkenyl, C₃-C₆-Alkinyl und gegebenenfalls einfach bis zweifach unabhängig voneinander durch Fluor, Chlor, Brom, Methyl, Ethyl, Trifluormethyl, Methoxy, Ethoxy, Cyano substituiertes C₃-C₆-Cycloalkyl und gegebenenfalls einfach bis zweifach unabhängig voneinander durch Fluor, Chlor, Brom, Methyl, Ethyl, Trifluormethyl, Methoxy, Ethoxy, Cyano substituiertes, geradkettiges oder verzweigtes C₃-C₆-Cycloalkyl-C₁-C₂-alkyl steht,
R² für einen Rest aus der Reihe Wasserstoff, gegebenenfalls einfach bis fünffach unabhängig voneinander durch Fluor, Chlor, Brom, Methoxy, Ethoxy, Methyl-S(O)ₘ-, Ethyl-S(O)ₘ-, substituiertes C₁-C₆-Alkyl, C₃-C₆-Alkenyl, C₃-C₆-Alkinyl, gegebenenfalls einfach durch O, S(O)ₘ, unterbrochenes und gegebenenfalls einfach bis zweifach unabhängig voneinander durch Fluor, Chlor, Brom, Methyl, Ethyl, Trifluormethyl, Methoxy, Ethoxy, Cyano substituiertes C₃-C₆-Cycloalkyl, gegebenenfalls einfach durch O, S(O)ₘ, unterbrochenes und gegebenenfalls einfach bis zweifach unabhängig voneinander durch Fluor, Chlor, Brom, Methyl, Ethyl, Trifluormethyl, Methoxy, Ethoxy, Cyano substituiertes, geradkettiges oder verzweigtes C₃-C₆-Cycloalkyl-C₁-C₂-alkyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₘ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes Phenyl oder Pyridyl und gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₘ-, Difluormethoxy, Trifluormethoxy, Trifluormethyl-S(O)ₙ-, Difluorethyl-S(O)ₙ-, Trifluorethyl-S(O)ₙ-, Nitro und Cyano substituiertes, geradkettiges oder verzweigtes Phenyl-C₁-C₂-alkyl, Pyridyl-C₁-C₂-alkyl, Pyrimidyl-C₁-C₂-alkyl, Thiazolyl-C₁-C₂-alkyl steht,
R³ für einen Rest aus der Reihe Wasserstoff, gegebenenfalls einfach bis fünffach unabhängig voneinander durch Fluor, Chlor, Brom, Methoxy, Ethoxy, Methyl-S(O)ₘ-, Ethyl-S(O)ₘ-, Cyano substituiertes C₁-C₄-Alkyl, C₃-C₄-Alkenyl, C₃-C₄-Alkinyl steht,
R⁴ für einen Rest aus der Reihe Wasserstoff, gegebenenfalls einfach bis fünffach unabhängig voneinander durch Fluor, Chlor, Brom, Methoxy, Ethoxy, Methyl-S(O)ₘ-, Ethyl-S(O)ₘ-, Cyano substituiertes C₁-C₄-Alkyl, C₃-C₄-Alkenyl, C₃-C₄-Alkinyl und für die Reste CONR²R³ und COR² steht, wobei für R² und R³ die vorstehenden Definitionen des Vorzugbereichs (2) gelten,
T für Sauerstoff oder ein Elektronenpaar steht
und deren Salze.

4. Verbindungen der Formel (I-A) 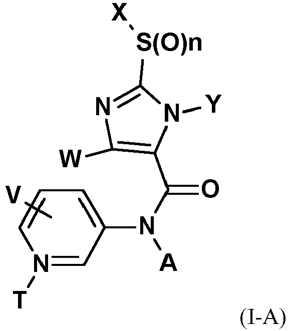 gemäß einem der Ansprüche 1 bis 3 und deren Salze.

5. Verbindungen der Formel (I-B) 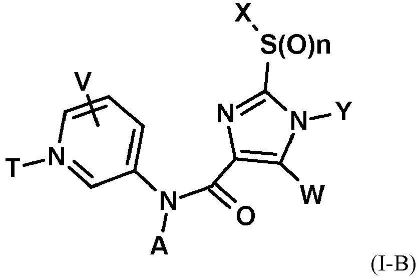 gemäß einem der Ansprüche 1 bis 3 und deren Salze.

6. Verbindungen gemäß Anspruch 4 oder 5, wobei
V für einen Rest aus der Reihe Wasserstoff, Fluor, Chlor, Methyl und Cyano steht,
W für einen Rest aus der Reihe Wasserstoff, Fluor, Chlor, Brom. Methyl, Ethyl und Cyano steht,
X für einen Rest aus der Reihe gegebenenfalls einfach, zweifach, dreifach, vierfach, fünffach unabhängig voneinander durch Fluor, Chlor, Brom, Methoxy, Ethoxy, Methyl-S(O)ₘ-, Ethyl-S(O)ₘ-, Cyano substituiertes C₁-C₆-Alkyl, C₃-C₄-Alkenyl, C₃-C₄-Alkinyl, die gegebenenfalls einfach durch die Gruppen C(O)OR², CONR²R³, C(G)R² substituiert sein können, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Methyl, Ethyl, Trifluormethyl, Methoxy, Cyano substituiertes C₃-C₆-Cycloalkyl, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Methyl, Ethyl, Trifluormethyl, Methoxy, Cyano substituiertes C₃-C₆-Cycloalkyl-methyl, gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₘ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes Phenyl, Pyridyl, Pyrimidyl, Thienyl, Thiazolyl, Oxazolyl, Imidazolyl, Oxdiazolyl, Thiadiazolyl, Triazolyl, Benzimidazolyl, Imidazopyridinyl, welches gegebenenfalls durch einfach bis zweifach unabhängig voneinander durch Fluor, Chlor, Methyl, Ethyl, Difluormethyl, Trifluormethyl, Methoxy, Ethoxy, Methyl-S(O)m-, Ethyl-S(O)n-, Difluormethoxy, Trifluormethoxy, Trifluormethyl-S(O)m-, Difluorethyl-S(O)m-, Trifluorethyl-S(O)m-, Nitro und Cyano substituiertes Phenyl substituiert sein kann, gegebenenfalls einfach bis zweifach unabhängig voneinander durch Fluor, Chlor, Brom, Methyl, Ethyl, Difluormethyl, Trifluormethyl, Methoxy, Ethoxy, Me-S(O)ₘ-, Et-S(O)ₘ-, Difluormethoxy, Trifluormethoxy, Trifluormethyl-S(O)ₙ-, Difluorethyl-S(O)ₘ-, Trifluorethyl-S(O)ₘ-, Nitro und Cyano substituiertes Benzyl, Pyridyl-methyl, Pyrimidyl-methyl, Thiazolyl-methyl, Pyrazolyl-C₁-C₂-alkyl und Cyano steht,
G für O, N-OR² steht,
Y für einen Rest aus der Reihe Wasserstoff, gegebenenfalls einfach bis dreifach unabhängig voneinander durch Fluor, Methoxy, Ethoxy, Cyano substituiertes Methyl, Ethyl, Propyl, Allyl Propargyl und Benzyl steht,
m für eine Zahl 0,1 oder 2 steht,
n für eine Zahl 0,1 oder 2 steht,
A für einen Rest aus der Reihe Wasserstoff, gegebenenfalls einfach bis dreifach unabhängig voneinander durch Fluor, Methoxy, Ethoxy, Cyano substituiertes Methyl, Ethyl, Propyl, Allyl, Propargyl, Cylopropyl oder Cyclopropylmethyl steht,
R² für einen Rest aus der Reihe Wasserstoff, gegebenenfalls einfach bis dreifach unabhängig voneinander durch Fluor, Chlor, Methoxy, Ethoxy, Methyl-S(O)ₘ-, Ethyl-S(O)ₘ-, substituiertes C₁-C₆-Alkyl, C₃-C₆-Alkenyl, C₃-C₆-Alkinyl, gegebenenfalls einfach durch O, S(O)ₘ, unterbrochenes und gegebenenfalls einfach bis zweifach unabhängig voneinander durch Fluor, Chlor, Methyl, Ethyl, Trifluormethyl, Methoxy, Cyano substituiertes C₃-C₆-Cycloalkyl, gegebenenfalls einfach durch O, S(O)ₘ, unterbrochenes und gegebenenfalls einfach bis zweifach unabhängig voneinander durch Fluor, Chlor, Methyl, Ethyl, Trifluormethyl, Methoxy, Cyano substituiertes C₃-C₆-Cycloalkyl-methyl gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₘ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes Phenyl oder Pyridyl und gegebenenfalls einfach bis zweifach unabhängig voneinander durch Fluor, Chlor, Brom, Methyl, Ethyl, Difluormethyl, Trifluormethyl, Methoxy, Ethoxy, Methyl-S(O)ₘ-, Ethyl-S(O)ₘ-, Difluormethoxy, Trifluormethoxy, Trifluormethyl-S(O)ₘ-, Difluorethyl-S(O)ₘ-, Trifluorethyl-S(O)ₘ-, Nitro und Cyano substituiertes Benzyl, Pyridyl-methyl, Pyrimidyl-methyl, Thiazolyl-methyl steht,
R³ für einen Rest aus der Reihe Wasserstoff, gegebenenfalls einfach bis dreifach unabhängig voneinander durch Fluor, Chlor, Brom, Methoxy, Ethoxy, Methyl-S(O)ₘ-, Ethyl-S(O)ₘ-, Cyano substituiertes C₁-C₄-Alkyl, C₃-C₄-Alkenyl, C₃-C₄-Alkinyl steht,
R⁴ für einen Rest aus der Reihe Wasserstoff, gegebenenfalls einfach bis dreifach unabhängig voneinander durch Fluor, Chlor, Brom, Methoxy, Ethoxy, Methyl-S(O)ₘ-, Ethyl-S(O)ₘ-, Cyano substituiertes C₁-C₄-Alkyl, C₃-C₄-Alkenyl, C₃-C₄-Alkinyl und für die Reste CONR²R³ und COR² steht, wobei für R² und R³ die vorstehenden Definitionen des Vorzugbereichs (3A) gelten,
T für Sauerstoff oder ein Elektronenpaar steht
und deren Salze.

7. Verbindungen gemäß einem der Ansprüche 4 oder 5, wobei
V für Wasserstoff und Fluor steht,
W für einen Rest aus der Reihe Wasserstoff, Chlor, Brom und Methyl steht,
X für einen Rest aus der Reihe gegebenenfalls einfach bis dreifach unabhängig voneinander durch Fluor, Chlor, Brom, Methoxy, Ethoxy, Methylsulfanyl, Ethylsulfanyl, Methylsulfinyl, Ethylsulfinyl, Methylsulfonyl, Ethylsulfonyl, substituiertes Methyl, Ethyl, Propyl, Isopropyl, Butyl, iso-Butyl, sec.-Butyl, tert.-Butyl, Pentyl, Isopentyl, 2.2-dimethyl-propyl, Hexyl, neo-Hexyl, Allyl, Methallyl, 2-Butenyl, Propargyl, 2-Butinyl, die gegebenenfalls einfach durch die Gruppen C(O)OR², CONR²R³, C(G)R² substituiert sein können, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Methyl, Ethyl, Trifluormethyl, Methoxy, Cyano substituiertes Cyclopropyl, Cyclobutyl, Cyclopentyl, Cyclohexyl, gegebenenfalls einfach bis zweifach unabhängig voneinander durch O, S(O)ₘ, CO, NR⁴ unterbrochenes und gegebenenfalls einfach bis zweifach unabhängig voneinander durch Fluor, Chlor, Methyl, Ethyl, Trifluormethyl, Methoxy, Cyano substituiertes Cyclopropylmethyl, Cyclobutylmethyl, Cyclopentylmethyl, Cyclohexylmethyl, gegebenenfalls einfach bis zweifach unabhängig voneinander durch Fluor, Chlor, Brom, Methyl, Ethyl, Difluormethyl, Trifluormethyl, Methoxy, Ethoxy, Methylsulfanyl, Ethylsulfanyl, Methylsulfinyl, Ethylsulfinyl, Methylsulfonyl, Ethylsulfonyl, Difluormethoxy, Trifluormethoxy, Trifluormethylsulfanyl, Trifluormethylsulfinyl, Trifluormethylsulfonyl, Difluormethylsulfanyl, Difluormethylsulfinyl, Difluormethylsulfonyl, Trifluorethylsulfanyl, Trifluorethylsulfinyl Trifluorethylsulfonyl, Nitro und Cyano substituiertes Phenyl, Pyridyl, Pyrimidyl, Thienyl, Thiazolyl, Oxazolyl, Imidazolyl, Oxdiazolyl, Thiadiazolyl, Triazolyl, Benzimidazolyl, Imidazopyridinyl, welches gegebenenfalls durch Phenyl substituiert sein kann, gegebenenfalls einfach bis zweifach unabhängig voneinander durch Fluor, Chlor, Brom, Methyl, Ethyl, Difluormethyl, Trifluormethyl, Methoxy, Ethoxy, Methylsulfanyl, Ethylsulfanyl, Methylsulfinyl, Ethylsulfinyl, Methylsulfonyl, Ethylsulfonyl, Difluormethoxy, Trifluormethoxy, Trifluormethylsulfanyl, Trifluormethylsulfinyl, Trifluormethylsulfonyl, Difluormethylsulfanyl, Difluormethylsulfinyl, Difluormethylsulfonyl, Trifluorethylsulfanyl, Trifluorethylsulfinyl Trifluorethylsulfonyl, Nitro und Cyano substituiertes Benzyl, Pyridylmethyl, Pyrimidylmethyl, Thiazolylmethyl, Pyrazolyl-C₁-C₂-alkyl und Cyano steht,
G für O, N-OR² steht,
Y für einen Rest aus der Reihe Wasserstoff, Methyl, Ethyl, Propyl, Difluorethyl, Trifluorethyl, Methoxymethyl, Ethoxymethyl, Cyanomethyl und Benzyl steht,
m für eine Zahl 0,1 oder 2 steht,
n für eine Zahl 0,1 oder 2 steht,
A für einen Rest aus der Reihe Wasserstoff, Methyl, Ethyl, Propyl, Difluorethyl, Trifluorethyl, Methoxymethyl, Ethoxymethyl, Cyanomethyl, Allyl, Propargyl, Cylopropyl oder Cyclopropyl-methyl steht,
R² für einen Rest aus der Reihe Wasserstoff, gegebenenfalls einfach bis dreifach unabhängig voneinander durch Fluor, Chlor, Methoxy, Ethoxy substituiertes Methyl, Ethyl, Propyl, Isopropyl, n-Butyl, iso-Butyl, sec.-Butyl, tert.-Butyl, Allyl, Methallyl, 2-Butenyl, Propargyl, 2-Butinyl gegebenenfalls einfach durch O, S(O)ₘ, unterbrochenes und gegebenenfalls einfach bis zweifach unabhängig voneinander durch Fluor, Chlor, Methyl, Ethyl, Trifluormethyl, Methoxy, Cyano substituiertes Cyclopropyl, Cyclobutyl, Cyclopentyl, Cyclohexyl,, gegebenenfalls einfach durch O, S(O)ₘ, unterbrochenes und gegebenenfalls einfach bis zweifach unabhängig voneinander durch Fluor, Chlor, Methyl, Ethyl, Trifluormethyl, Methoxy, Cyano substituiertes Cyclopropyl-methyl, Cyclobutyl-methyl, Cyclopentyl-methyl, Cyclohexyl-methyl, gegebenenfalls einfach bis zweifach unabhängig voneinander durch Fluor, Chlor, Brom, Methyl, Ethyl, Difluormethyl, Trifluormethyl, Methoxy, Ethoxy, Methylsulfanyl, Ethylsulfanyl, Methylsulfinyl, Ethylsulfinyl, Methylsulfonyl, Ethylsulfonyl, Difluormethoxy, Trifluormethoxy, Trifluormethylsulfanyl, Trifluormethylsulfinyl, Trifluormethylsulfonyl, Difluormethylsulfanyl, Difluormethylsulfinyl, Difluormethylsulfonyl, Trifluorethylsulfanyl, Trifluorethylsulfinyl Trifluorethylsulfonyl, Nitro und Cyano substituiertes Phenyl oder Pyridyl und gegebenenfalls einfach bis zweifach unabhängig voneinander durch Fluor, Chlor, Brom, Methyl, Ethyl, Difluormethyl, Trifluormethyl, Methoxy, Ethoxy, Difluormethoxy, Trifluormethoxyl, Nitro und Cyano substituiertes Benzyl, Pyridyl-methyl, Pyrimidylmethyl, Thiazolylmethyl steht,
R³ für einen Rest aus der Reihe Wasserstoff, Methyl, Ethyl, Propyl, Isopropyl, Butyl, iso-Butyl, und Allyl steht,
R⁴ für einen Rest aus der Reihe Wasserstoff, gegebenenfalls einfach bis dreifach unabhängig voneinander durch Fluor, Methoxy, Ethoxy oder Cyano substituiertes Methyl, Ethyl, Propyl, Isopropyl, Butyl, iso-Butyl, und Allyl und für die Reste CONR²R³ und COR² steht, wobei für R² und R³ vorstehenden Definitionen des Vorzugbereichs (4A) gelten,
T für Sauerstoff oder einem Elektronenpaar steht
und deren Salze.

8. Verbindungen der Formel (I-A) 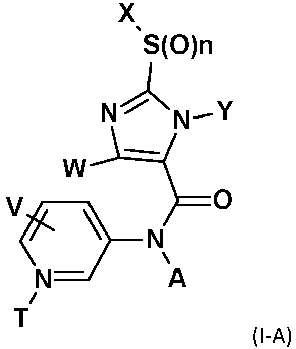 gemäß Anspruch 1, wobei
V für Wasserstoff steht,
W für einen Rest aus der Reihe Wasserstoff, Chlor und Brom und bevorzugt für Wasserstoff steht,
X für einen Rest aus der Reihe, Methyl, Ethyl,n-Butyl, n-Pentyl, n-Propyl, iso-Propyl, Allyl, 3,3-Dimethylallyl, Propargyl, Cyclohexyl, Tetrahydro-pyranyl, Tetrahydro-thiopyranyl, 3-Oxetanyl, 5-Oxa-[3.3.0]-bicyclohepatanyl,, Methoxyethyl, Methoxypropyl, Ethoxyethyl, Ethylthioethyl, Methyltioethyl, Difluormethyl, Trifluormethyl, 2,2,2-Trifluorethyl, 3,3,3-Trifluorpropyl, 3-Chlor-2,2,3,3-tetra-fluorpropyl, 3-Fluorpropyl, 3,3-Difluorpropyl, 2,2,2-Trifluorethylthio-ethyl, Methylcarbonyl-methyl, c-Propylcarbonyl-methyl, tert.-Butylcarbonyl-methyl, Methoxycarbonyl-methyl, Ethoxycarbonyl-methyl, Hydroxycarbonyl-methyl, Carbamoyl-methyl, N-Methylcarbamoyl-methyl, N-c-Propylcarbamoyl-methyl, N,N-Dimethylcarbamoyl-methyl, 2-Methoximinopropyl, Cyclopropylmethyl, Phenyl, 4-Methylphenyl, 2-Nitrophenyl, 3-Methylthiophenyl, 4-Chlor-phenyl, 4-Fluor-phenyl, 4-tert.-Butyl-phenyl, 4-Methoxy-phenyl, 4-Nitrophenyl, 4-Diemethylamino-phenyl, 2-Fluor-phenyl, 2-Methoxy-phenyl, 2-Dimethylaminosulfonyl-phenyl, 2-Dimethylaminocarbamoyl-phenyl, 3-Nitrophenyl, 3-Trifluormethyl-phenyl, 3-Chlor-phenyl, 2,5-Dichlor-phenyl, 3,5-Dichlor-phenyl, 4-Chlor-3-trifluormethyl-phenyl, 2,4,5-Trichlor-phenyl, 2-Pyridyl, 5-(2-Chlor)pyridyl, 2-(5-Methyl)-pyridyl, 2-(6-Methyl)-pyridyl, 2-(3-Trifluormethyl)-pyridyl, 2-Pyrimidyl, 2-(4-Methyl)-pyrimidyl, 2-(5-Methyl)-pyrimidyl, 2-(4-Methoxy)-pyrimidyl, 2-(5-Fluor)-pyrimidyl, 2-(4-Trifluormethyl)-pyrimidyl, 2-(5-Trifluormethyl)-pyrimidyl, 2-(4,6-Dimethyl)-pyrimidyl, 2-(4,5-Dimethyl)-pyrimidyl, 2-(4,6-Dimethoxy)-pyrimidyl, -CH₂-2-pyrimidyl,-CH₂-2-pyrazinyl,-CH₂-5-(1-methyl)-imidazolyl,-CH₂-3-(1-methyl)-pyrazolyl, -CH₂-4-pyridyl,-CH₂-2-pyridyl, -CH₂-2-(1-methyl)-imidazolyl, -CH₂-3-pyridyl, -CH₂-2-furanyl, -CH₂-5-(2-chlor)-pyridyl, Benzyl, 3,4-Dichlor-benzyl, 2,6-Difluor-benzyl, 2-Fluor-6-methoxy-benzyl, 2,6-Dichlor-benzyl, 2-Chlor-6-trifluormethyl-benzyl, 2-Chlor-6-fluor-benzyl, -CH2-2-(4,6-dimethoxy)-pyrimidyl, 2,6-Dimethyl-benzyl, -CH₂-1-(3-nitro-5-methyl)-pyrazolyl, 2-(1-Methyl)-benzimidazolyl, 2-(5-Methyl)-oxdiazolyl, 2-[3-Methyl-6-(trifluormethyl)-imidazo[4.5]-pyridinyl, 3-[4-Ethyl-5-(trifluormethyl)]-1,2,4-triazolyl, 3-[4-Methyl-5-(trifluormethyl)]--1,2,4-triazolyl, 3-[4-Methyl-5-(difluormethyl)]--1,2,4-triazolyl, 2-(5-Phenyl)-1.3.4-thiadiazolyl, 2-(1-Methyl-5-phenyl)-imidazolyl, 2-(4,5-Dimethyl)-oxazolyl, 2-(1-Methyl-5-methoxycarbonyl)-imidazolyl, 2-(1-Methyl)-imidazolyl, 1,2-Ethandiyl- steht,
Y für Methyl, Ethyl und Benzyl steht,
n für eine Zahl 0 oder 2 und bevorzugt für 0 steht,
A für einen Rest aus der Reihe Wasserstoff und Methyl und bevorzugt für Methyl steht,
T für ein Elektronenpaar steht und deren Salze
und
Verbindungen der Formel (I-B) 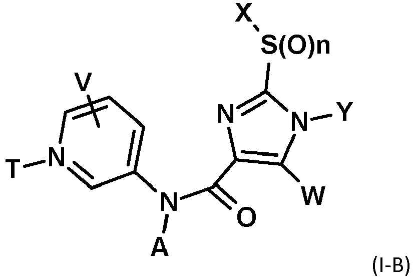 gemäß Anspruch 1, wobei
V für Wasserstoff steht,
W für einen Rest aus der Reihe Wasserstoff, Chlor und Brom und bevorzugt für Wasserstoff steht,
X für einen Rest aus der Reihe 4,6-Dimethylpyrimidyl, n-Butyl, n-Pentyl, Benzyl, Methyl, 3-Methylthiophenyl, 2,2,2-Trifluorethyl, Phenyl, 4-Methylphenyl, Pyrimidyl, Ethylthioethyl, 2-Nitrophenyl, Cyclopropylmethyl und bevorzugt für einen Pyrimidyl- Rest steht,
Y für Methyl steht,
n für eine Zahl 0 oder 2 und bevorzugt für 0 steht,
A für einen Rest aus der Reihe Wasserstoff und Methyl und bevorzugt für Methyl steht,
T für ein Elektronenpaar steht
und deren Salze.

9. Mittel, mit einem Gehalt von mindestens einer Verbindung gemäß einem der Ansprüche 1 bis 8 und üblichen Streckmitteln und/oder oberflächenaktiven Substanzen insbesondere zur Bekämpfung von tierischen Schädlingen.

10. Verfahren zur Bekämpfung von tierischen Schädlingen, bei dem man wenigstens eine Verbindung gemäß einem der Ansprüche 1 bis 8 oder ein Mittel gemäß Anspruch 9 auf die tierischen Schädlinge und/oder ihren Lebensraum einwirken lässt, wobei die chirurgische, therapeutische und diagnostische Behandlung des menschlichen oder tierischen Körpers ausgeschlossen ist..

11. Verwendung wenigstens einer Verbindung gemäß einem der Ansprüche 1 bis 8 oder eines Mittels gemäß Anspruch 9 zur Bekämpfung von tierischen Schädlingen, die chirurgische, therapeutische und diagnostische Behandlung des menschlichen oder tierischen Körpers ausgeschlossen ist.

12. Verwendung wenigstens einer Verbindung gemäß einem der Ansprüche 1 bis 8 zum Schutz des Vermehrungsmaterials von Pflanzen.

13. Agrochemische Formulierung enthaltend wenigstens eine Verbindung gemäß einem der Ansprüche 1 bis 8 in biologisch wirksamen Gehalten von zwischen 0,00000001 und 98 Gew.-%, bezogen auf das Gewicht der agrochemischen Formulierung, sowie Streckmittel und/oder oberflächenaktive Stoffe.

14. Agrochemische Formulierung gemäß Anspruch 13 zusätzlich enthaltend einen weiteren agrochemischen Wirkstoff.

15. Zwischenprodukte der Formeln (XI) und (XXI) 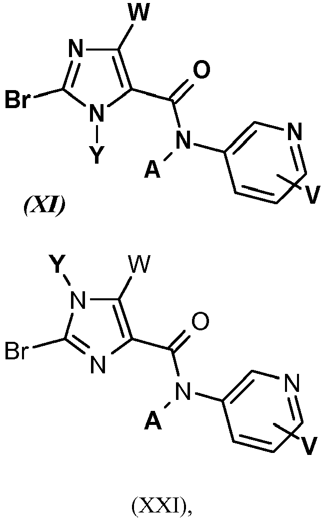 wobei die Reste A, Y, V und W die Bedeutungen gemäß einem der Ansprüche 1 bis 3 oder 6 bis 8 und besonders bevorzugt die Bedutung gemäß Anspruch 8 haben.

16. Zwischenprodukte der Formeln (XV) und (XXII) 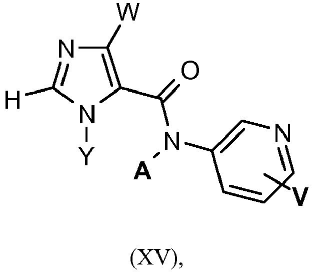 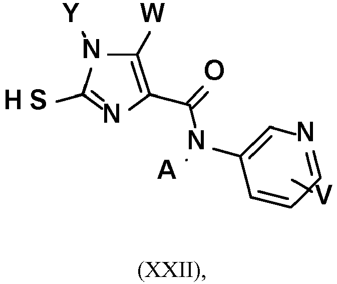 wobei
für die Zwischenprodukte der Formel (XV)
V für Wasserstoff steht,
W für einen Rest aus der Reihe Wasserstoff, Chlor und Brom und bevorzugt für Wasserstoff steht,
Y für Methyl, Ethyl oder Benzyl steht und
A für einen Rest aus der Reihe Wasserstoff und Methyl und bevorzugt für Methyl steht
und für die Zwischenprodukte der Formel (XXII)
V für Wasserstoff steht,
W für einen Rest aus der Reihe Wasserstoff, Chlor und Brom und bevorzugt für Wasserstoff steht,
Y für Methyl steht und
A für einen Rest aus der Reihe Wasserstoff und Methyl und bevorzugt für Methyl steht.

## Claims

1. Compounds of the formula (I) 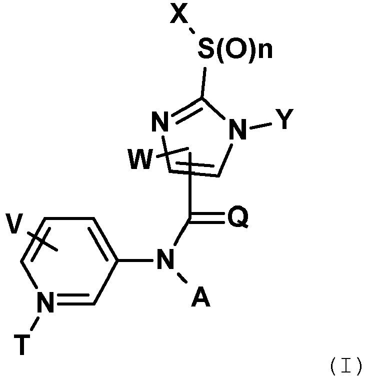 in which
Q represents oxygen or sulphur,
V represents a radical from the series hydrogen, halogen, alkyl, haloalkyl, alkoxy, haloalkoxy and cyano,
W represents a radical from the series hydrogen, halogen, alkyl, haloalkyl, alkoxy, haloalkoxy and cyano,
X represents a radical from the series optionally substituted alkyl, alkenyl, alkynyl, optionally substituted cycloalkyl which is saturated or unsaturated and optionally interrupted by heteroatoms, optionally substituted cycloalkylalkyl which is saturated or unsaturated and optionally interrupted by heteroatoms, optionally substituted aryl, hetaryl, optionally substituted arylalkyl, hetarylalkyl and cyano,
Y represents a radical from the series hydrogen, optionally substituted alkyl, alkenyl, alkynyl, optionally substituted cycloalkyl which is optionally interrupted by heteroatoms, optionally substituted cycloalkylalkyl which is optionally interrupted by heteroatoms, arylalkyl, hetarylalkyl and cyano,
n represents a number 0, 1 or 2,
A represents a radical from the series hydrogen, optionally substituted alkyl, alkenyl, alkynyl and optionally substituted cycloalkyl and cycloalkylalkyl which are optionally interrupted by heteroatoms,
T represents oxygen or an electron pair,
and salts thereof.

2. Compounds according to Claim 1, where
Q represents oxygen or sulphur,
V represents a radical from the series hydrogen, halogen, C₁-C₄-alkyl, C₁-C₄-haloalkyl, C₁-C₄-alkoxy, C₁-C₄-haloalkoxy and cyano,
W represents a radical from the series hydrogen, halogen, C₁-C₄-alkyl, C₁-C₄-haloalkyl, C₁-C₄-alkoxy, C₁-C₄-haloalkoxy and cyano,
X represents a radical from the series C₁-C₈-alkyl, C₃-C₈-alkenyl, C₃-C₈-alkynyl, optionally mono- or polysubstituted independently of one another by halogen, C₁-C₄-alkoxy, C₁-C₄-alkyl-S(O)ₘ-, cyano, C(O)OR², CONR²R³, C(G)R²; C₃-C₈-cycloalkyl or C₅-C₈-cycloalkenyl, optionally interrupted once or twice independently of one another by O, S(O)ₘ, C(G)R², NR⁴ and optionally mono- to tetrasubstituted independently of one another by halogen, C₁-C₄-alkyl, C₁-C₄-haloalkyl, C₁-C₄-alkoxy, cyano; straight-chain or branched C₃-C₈-cycloalkyl-C₁-C₄-alkyl or C₅-C₈-cycloalkenyl-C₁-C₄-alkyl, optionally interrupted once or twice independently of one another by O, S(O)ₘ, C(G)R², NR⁴ and optionally mono- to tetrasubstituted independently of one another by halogen, C₁-C₄-alkyl, C₁-C₄-haloalkyl, C₁-C₄-alkoxy, cyano; aryl, optionally mono- to trisubstituted independently of one another by halogen, C₁-C₄-alkyl, C₁-C₄-haloalkyl, C₁-C₄-alkoxy, C₁-C₄-alkyl-S(O)ₘ-, C₁-C₄-haloalkoxy, C₁-C₄-haloalkyl-S(O)ₘ-, nitro and cyano; or hetaryl, which can be optionally mono- to trisubstituted independently of one another by halogen, C₁-C₄-alkyl, C₁-C₄-haloalkyl, C₁-C₄-alkoxy, C₁-C₄-alkyl-S(O)ₘ-, C₁-C₄-haloalkoxy, C₁-C₄-haloalkyl-S(O)ₘ-, nitro and cyano; straight-chain or branched aryl-C₁-C₄-alkyl, hetaryl-C₁-C₄-alkyl, optionally mono- to trisubstituted independently of one another by halogen, C₁-C₄-alkyl, C₁-C₄-haloalkyl, C₁-C₄-alkoxy, C₁-C₄-alkyl-S(O)ₘ-, C₁-C₄-haloalkoxy, C₁-C₄-haloalkyl-S(O)ₘ-, nitro and cyano,
G represents O, N-CN, N-OR²,
Y represents a radical from the series hydrogen; C₁-C₆-alkyl, C₃-C₆-alkenyl, C₃-C₆-alkynyl, optionally mono- or polysubstituted independently of one another by halogen, C₁-C₄-alkoxy, C₁-C₄-alkyl-S(O)ₘ-, cyano; C₃-C₈-cycloalkyl, optionally mono- or disubstituted independently of one another by halogen, C₁-C₄-alkyl, C₁-C₄-haloalkyl, C₁-C₄-alkoxy, cyano; straight-chain or branched C₃-C₈-cycloalkyl-C₁-C₄-alkyl, optionally interrupted once or twice independently of one another by O, S(O)ₘ, CO, NR⁴ and optionally mono- to tetrasubstituted independently of one another by halogen, C₁-C₄-alkyl, C₁-C₄-haloalkyl, C₁-C₄-alkoxy, cyano; arylalkyl or hetarylalkyl, optionally mono- to trisubstituted independently of one another by halogen, C₁-C₄-alkyl, C₁-C₄-haloalkyl, C₁-C₄-alkoxy, C₁-C₄-alkyl-S(O)ₘ-, C₁-C₄-haloalkoxy, C₁-C₄-haloalkyl-S(O)ₘ-, nitro and cyano; and cyano,
m represents a number 0, 1 or 2,
n represents a number 0, 1 or 2,
A represents a radical from the series hydrogen; C₁-C₆-alkyl, C₃-C₆-alkenyl, C₃-C₆-alkynyl, optionally mono- or polysubstituted independently of one another by halogen, C₁-C₄-alkoxy, C₁-C₄-alkyl-S(O)ₘ-, cyano; C₃-C₆-cycloalkyl, optionally mono- or disubstituted independently of one another by halogen, C₁-C₄-alkyl, C₁-C₄-haloalkyl, C₁-C₄-alkoxy, cyano; and straight-chain or branched C₃-C₈-cycloalkyl-C₁-C₄-alkyl, optionally mono- or disubstituted independently of one another by halogen, C₁-C₄-alkyl, C₁-C₄-haloalkyl, C₁-C₄-alkoxy, cyano,
R² represents a radical from the series hydrogen; C₁-C₈-alkyl, C₃-C₈-alkenyl, C₃-C₈-alkynyl, optionally mono- or polysubstituted independently of one another by halogen, C₁-C₄-alkoxy, C₁-C₄-alkyl-S(O)ₘ-; C₃-C₈-cycloalkyl, optionally interrupted once by O, S(O)ₘ and optionally mono- or disubstituted independently of one another by halogen, C₁-C₄-alkyl, C₁-C₄-haloalkyl, C₁-C₄-alkoxy, cyano; straight-chain or branched C₃-C₈-cycloalkyl-C₁-C₄-alkyl, optionally interrupted once by O, S(O)ₙ and optionally mono- or disubstituted independently of one another by halogen, C₁-C₄-alkyl, C₁-C₄-haloalkyl, C₁-C₄-alkoxy, cyano; aryl, hetaryl, optionally mono- to trisubstituted independently of one another by halogen, C₁-C₄-alkyl, C₁-C₄-haloalkyl, C₁-C₄-alkoxy, C₁-C₄-alkyl-S(O)ₘ-, C₁-C₄-haloalkoxy, C₁-C₄-haloalkyl-S(O)ₘ-, nitro and cyano; and straight-chain or branched aryl-C₁-C₄-alkyl, hetaryl-C₁-C₄-alkyl, optionally mono- to trisubstituted independently of one another by halogen, C₁-C₄-alkyl, C₁-C₄-haloalkyl, C₁-C₄-alkoxy, C₁-C₄-alkyl-S(O)ₘ-, C₁-C₄-haloalkoxy, C₁-C₄-haloalkyl-S(O)ₘ-, nitro and cyano,
R³ represents a radical from the series hydrogen; C₁-C₆-alkyl, C₃-C₆-alkenyl, C₃-C₆-alkynyl, optionally mono- or polysubstituted independently of one another by halogen, C₁-C₄-alkoxy, C₁-C₄-alkyl-S(O)ₘ-, cyano,
R⁴ represents a radical from the series hydrogen; C₁-C₆-alkyl, C₃-C₆-alkenyl, C₃-C₆-alkynyl, optionally mono- or polysubstituted independently of one another by halogen, C₁-C₄-alkoxy, C₁-C₄-alkyl-S(O)ₘ-, cyano, and represents the radicals CONR²R³ and COR², where the above definitions of the preferred range (1) apply to R² and R³,
T represents oxygen or an electron pair,
and salts thereof.

3. Compounds according to Claim 1, where
Q represents oxygen,
Y represents a radical from the series hydrogen, fluorine, chlorine, bromine, methyl, ethyl, difluoromethyl, trifluoromethyl, methoxy, ethoxy, difluoromethoxy, trifluoromethoxy and cyano,
W represents a radical from the series hydrogen, fluorine, chlorine, bromine, methyl, ethyl, difluoromethyl, trifluoromethyl, methoxy, ethoxy, difluoromethoxy, trifluoromethoxy and cyano,
X represents a radical from the series C₁-C₆-alkyl, C₃-C₆-alkenyl, C₃-C₆-alkynyl, optionally mono- to heptasubstituted independently of one another by fluorine, chlorine, bromine, methoxy, ethoxy, methyl-S(O)ₘ-, ethyl-S(O)ₘ-, cyano, C(O)OR², CONR²R³, C(G)R²; C₃-C₆-cycloalkyl, optionally interrupted once or twice independently of one another by O, S(O)ₘ, CO, NR⁴ and optionally mono- or disubstituted independently of one another by fluorine, chlorine, bromine, methyl, ethyl, trifluoromethyl, methoxy, ethoxy, cyano; straight-chain or branched C₃-C₆-cycloalkyl-C₁-C₂-alkyl, optionally interrupted once or twice independently of one another by O, S(O)ₘ, CO, NR⁴ and optionally mono- or disubstituted independently of one another by fluorine, chlorine, bromine, methyl, ethyl, trifluoromethyl, methoxy, ethoxy, cyano; phenyl, naphthyl, pyridyl, pyrimidyl, pyridazinyl, pyrazinyl, triazinyl, furanyl, thienyl, oxazolyl, isoxazolyl, thiazolyl, isothiazolyl, pyrazolyl, imidazolyl, oxadiazolyl, thiadiazolyl, triazolyl, benzimidazolyl, imidazopyridinyl, which may optionally be substituted by phenyl which is mono- to trisubstituted independently of one another by fluorine, chlorine, methyl, ethyl, difluoromethyl, trifluoromethyl, methoxy, ethoxy, methyl-S(O)m-, ethyl-S(O)m-, difluoromethoxy, trifluoromethoxy, trifluoromethyl-S(O)m-, difluoroethyl-S(O)m-, trifluoromethyl-S(O)m-, nitro and cyano, optionally mono- or disubstituted independently of one another by fluorine, chlorine, bromine, methyl, ethyl, difluoromethyl, trifluoromethyl, methoxy, ethoxy, methyl-S(O)ₘ-, ethyl-S(O)ₙ-, difluoromethoxy, trifluoromethoxy, trifluoromethyl-S(O)ₘ-, difluoroethyl-S(O)ₘ-, trifluoroethyl-S(O)ₘ-, nitro and cyano; straight-chain or branched phenyl-C₁-C₂-alkyl, pyridyl-C₁-C₂-alkyl, pyrimidyl-C₁-C₂-alkyl, thiazolyl-C₁-C₂-alkyl, pyrazolyl-C₁-C₂-alkyl, optionally mono- or disubstituted independently of one another by fluorine, chlorine, bromine, methyl, ethyl, difluoromethyl, trifluoromethyl, methoxy, ethoxy, Me-S(O)ₘ-, Et-S(O)ₘ-, difluoromethoxy, trifluoromethoxy, trifluoromethyl-S(O)ₘ-, difluoroethyl-S(O)ₘ-, trifluoroethyl-S(O)ₘ-, nitro and cyano; and cyano,
G represents O, N-OR²,
Y represents a radical from the series hydrogen; C₁-C₄-alkyl, C₃-C₄-alkenyl, C₃-C₄-alkynyl, optionally mono- to pentasubstituted independently of one another by fluorine, chlorine, bromine, methoxy, ethoxy, methyl-S(O)ₘ-, ethyl-S(O)ₘ-, cyano; C₃-C₆-cycloalkyl, optionally mono- or disubstituted independently of one another by fluorine, chlorine, bromine, methyl, ethyl, trifluoromethyl, methoxy, ethoxy, cyano; straight-chain or branched C₃-C₆-cycloalkyl-C₁-C₂-alkyl, optionally interrupted once or twice independently of one another by O, S(O)ₘ, CO, NR⁴ and optionally mono- to tetrasubstituted independently of one another by fluorine, chlorine, bromine, methyl, ethyl, trifluoromethyl, methoxy, ethoxy, cyano; aryl-C₁-C₂-alkyl or hetaryl-C₁-C₂-alkyl, optionally mono- or disubstituted independently of one another by fluorine, chlorine, bromine, methyl, ethyl, C₁-C₄-haloalkyl, trifluoromethyl, methoxy, ethoxy, cyano, nitro and cyano; and cyano,
m represents a number 0, 1 or 2,
n represents a number 0, 1 or 2,
A represents a radical from the series hydrogen; C₁-C₆-alkyl, C₃-C₆-alkenyl, C₃-C₆-alkynyl, optionally mono- to pentasubstituted independently of one another by fluorine, chlorine, bromine, methoxy, ethoxy, methyl-S(O)ₘ-, ethyl-S(O)ₘ-, cyano; C₃-C₆-cycloalkyl, optionally mono- or disubstituted independently of one another by fluorine, chlorine, bromine, methyl, ethyl, trifluoromethyl, methoxy, ethoxy, cyano; straight-chain or branched C₃-C₆-cycloalkyl-C₁-C₂-alkyl, optionally mono- or disubstituted independently of one another by fluorine, chlorine, bromine, methyl, ethyl, trifluoromethyl, methoxy, ethoxy, cyano,
R² represents a radical from the series hydrogen; C₁-C₆-alkyl, C₃-C₆-alkenyl, C₃-C₆-alkynyl, optionally mono- to pentasubstituted independently of one another by fluorine, chlorine, bromine, methoxy, ethoxy, methyl-S(O)ₘ-, ethyl-S(O)ₘ-; C₃-C₆-cycloalkyl, optionally interrupted once by O, S(O)ₘ, and optionally mono- or disubstituted independently of one another by fluorine, chlorine, bromine, methyl, ethyl, trifluoromethyl, methoxy, ethoxy, cyano; straight-chain or branched C₃-C₆-cycloalkyl-C₁-C₂-alkyl, optionally interrupted once by O, S(O)ₘ and optionally mono- or disubstituted independently of one another by fluorine, chlorine, bromine, methyl, ethyl, trifluoromethyl, methoxy, ethoxy, cyano; phenyl or pyridyl, optionally mono- or disubstituted independently of one another by fluorine, chlorine, bromine, methyl, ethyl, difluoromethyl, trifluoromethyl, methoxy, ethoxy, methyl-S(O)ₘ-, ethyl-S(O)ₘ-, difluoromethoxy, trifluoromethoxy, trifluoromethyl-S(O)ₘ-, difluoroethyl-S(O)ₘ-, trifluoroethyl-S(O)ₘ-, nitro and cyano; and straight-chain or branched phenyl-C₁-C₂-alkyl, pyridyl-C₁-C₂-alkyl, pyrimidyl-C₁-C₂-alkyl, thiazolyl-C₁-C₂-alkyl, optionally mono- or disubstituted independently of one another by fluorine, chlorine, bromine, methyl, ethyl, difluoromethyl, trifluoromethyl, methoxy, ethoxy, methyl-S (O)ₘ-, ethyl-S (O)ₘ-, difluoromethoxy, trifluoromethoxy, trifluoromethyl-S(O)ₙ-, difluoroethyl-S(O)ₙ-, trifluoroethyl-S(O)ₙ-, nitro and cyano,
R³ represents a radical from the series hydrogen; C₁-C₄-alkyl, C₃-C₄-alkenyl, C₃-C₄-alkynyl, optionally mono- to pentasubstituted independently of one another by fluorine, chlorine, bromine, methoxy, ethoxy, methyl-S(O)ₘ-, ethyl-S(O)ₘ-, cyano,
R⁴ represents a radical from the series hydrogen; C₁-C₄-alkyl, C₃-C₄-alkenyl, C₃-C₄-alkynyl, optionally mono- to pentasubstituted independently of one another by fluorine, chlorine, bromine, methoxy, ethoxy, methyl-S(O)ₘ-, ethyl-S(O)ₘ-, cyano, and represents the radicals CONR²R³ and COR², where the above definitions of the preferred range (2) apply to R² and R³,
T represents oxygen or an electron pair,
and salts thereof.

4. Compounds of the formula (I-A) 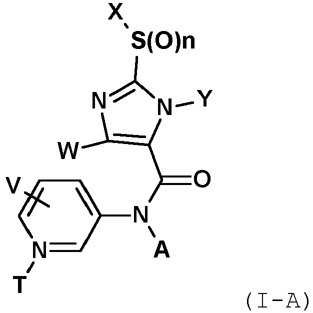 according to any of Claims 1 to 3, and salts thereof.

5. Compounds of the formula (I-B) 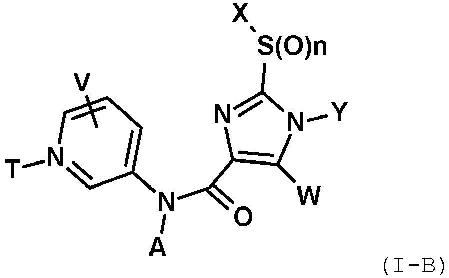 according to any of Claims 1 to 3, and salts thereof.

6. Compounds according to Claim 4 or 5 where
V represents a radical from the series hydrogen, fluorine, chlorine, methyl and cyano,
W represents a radical from the series hydrogen, fluorine, chlorine, bromine, methyl, ethyl and cyano.
X represents a radical from the series C₁-C₆-alkyl, C₃-C₄-alkenyl, C₃-C₄-alkynyl, which are optionally mono-, di-, tri-, tetra-, pentasubstituted independently of one another by fluorine, chlorine, bromine, methoxy, ethoxy, methyl-S(O)ₘ-, ethyl-S(O)ₘ-, cyano and may optionally be monosubstituted by the groups C(O)OR², CONR²R³, C(G)R²; C₃-C₆-cycloalkyl, optionally interrupted once or twice independently of one another by O, S(O)ₘ, CO, NR⁴ and optionally mono- or disubstituted independently of one another by fluorine, chlorine, methyl, ethyl, trifluoromethyl, methoxy, cyano; C₃-C₆-cycloalkyl-methyl, optionally interrupted once or twice independently of one another by O, S(O)ₘ, CO, NR⁴ and optionally mono- or disubstituted independently of one another by fluorine, chlorine, methyl, ethyl, trifluoromethyl, methoxy, cyano; phenyl, pyridyl, pyrimidyl, thienyl, thiazolyl, oxazolyl, imidazolyl, oxadiazolyl, thiadiazolyl, triazolyl, benzimidazolyl, imidazopyridinyl, which may optionally be substituted by phenyl which is mono- or disubstituted independently of one another by fluorine, chlorine, methyl, ethyl, difluoromethyl, trifluoromethyl, methoxy, ethoxy, methyl-S(O)m-, ethyl-S(O)n-, difluoromethoxy, trifluoromethoxy, trifluoromethyl-S(O)m-, difluoroethyl-S(O)m-, trifluoroethyl-S(O)m-, nitro and cyano, optionally mono- or disubstituted independently of one another by fluorine, chlorine, bromine, methyl, ethyl, difluoromethyl, trifluoromethyl, methoxy, ethoxy, methyl-S(O)ₘ-, ethyl-S(O)ₘ-, difluoromethoxy, trifluoromethoxy, trifluoromethyl-S(O)ₘ-, difluoroethyl-S(O)ₘ-, trifluoroethyl-S(O)ₘ-, nitro and cyano; benzyl, pyridylmethyl, pyrimidylmethyl, thiazolylmethyl, pyrazolyl-C₁-C₂-alkyl, optionally mono- or disubstituted independently of one another by fluorine, chlorine, bromine, methyl, ethyl, difluoromethyl, trifluoromethyl, methoxy, ethoxy, Me-S(O)ₘ-, Et-S(O)ₘ-, difluoromethoxy, trifluoromethoxy, trifluoromethyl-S(O)ₙ-, difluoroethyl-S(O)ₘ-, trifluoroethyl-S(O)ₘ-, nitro and cyano; and cyano,
G represents O, N-OR²,
Y represents a radical from the series hydrogen; methyl, ethyl, propyl, allyl, propargyl and benzyl, optionally mono- to trisubstituted independently of one another by fluorine, methoxy, ethoxy, cyano,
m represents a number 0, 1 or 2,
n represents a number 0, 1 or 2,
A represents a radical from the series hydrogen; methyl, ethyl, propyl, allyl, propargyl, cyclopropyl or cyclopropylmethyl, optionally mono- to trisubstituted independently of one another by fluorine, methoxy, ethoxy, cyano,
R² represents a radical from the series hydrogen; C₁-C₆-alkyl, C₃-C₆-alkenyl, C₃-C₆-alkynyl, optionally mono- to trisubstituted independently of one another by fluorine, chlorine, methoxy, ethoxy, methyl-S(O)ₘ-, ethyl-S(O)ₘ-; C₃-C₆-cycloalkyl, optionally interrupted once by O, S(O)ₘ and optionally mono- or disubstituted independently of one another by fluorine, chlorine, methyl, ethyl, trifluoromethyl, methoxy, cyano; C₃-C₆-cycloalkylmethyl, optionally interrupted once by O, S(O)ₘ and optionally mono- or disubstituted independently of one another by fluorine, chlorine, methyl, ethyl, trifluoromethyl, methoxy, cyano; phenyl or pyridyl, optionally mono- or disubstituted independently of one another by fluorine, chlorine, bromine, methyl, ethyl, difluoromethyl, trifluoromethyl, methoxy, ethoxy, methyl-S(O)ₘ-, ethyl-S(O)ₘ-, difluoromethoxy, trifluoromethoxy, trifluoromethyl-S(O)ₘ-, difluoroethyl-S(O)ₘ-, trifluoroethyl-S(O)ₘ-, nitro and cyano; and benzyl, pyridylmethyl, pyrimidylmethyl, thiazolylmethyl, optionally mono- or disubstituted independently of one another by fluorine, chlorine, bromine, methyl, ethyl, difluoromethyl, trifluoromethyl, methoxy, ethoxy, methyl-S(O)ₘ-, ethyl-S(O)ₘ-, difluoromethoxy, trifluoromethoxy, trifluoromethyl-S(O)ₘ-, difluoroethyl-S(O)ₘ-, trifluoroethyl-S(O)ₘ-, nitro and cyano,
R³ represents a radical from the series hydrogen; C₁-C₄-alkyl, C₃-C₄-alkenyl, C₃-C₄-alkynyl, optionally mono- to trisubstituted independently of one another by fluorine, chlorine, bromine, methoxy, ethoxy, methyl-S(O)ₘ-, ethyl-S(O)ₘ-, cyano,
R⁴ represents a radical from the series hydrogen; C₁-C₄-alkyl, C₃-C₄-alkenyl, C₃-C₄-alkynyl, optionally mono- to trisubstituted independently of one another by fluorine, chlorine, bromine, methoxy, ethoxy, methyl-S(O)ₘ-, ethyl-S(O)ₘ-, cyano, and represents the radicals CONR²R³ and COR², where the above definitions of the preferred range (3A) apply to R² and R³,
T represents oxygen or an electron pair,
and salts thereof.

7. Compounds according to Claim 4 or 5 where
V represents hydrogen or fluorine,
W represents a radical from the series hydrogen, chlorine, bromine and methyl,
X represents a radical from the series methyl, ethyl, propyl, isopropyl, butyl, isobutyl, sec-butyl, tert-butyl, pentyl, isopentyl, 2,2-dimethylpropyl, hexyl, neohexyl, allyl, methallyl, 2-butenyl, propargyl, 2-butynyl, which are optionally mono- to trisubstituted independently of one another by fluorine, chlorine, bromine, methoxy, ethoxy, methylsulphanyl, ethylsulphanyl, methylsulphinyl, ethylsulphinyl, methylsulphonyl, ethylsulphonyl and may optionally be monosubstituted by the groups C(O)OR², CONR²R³, C(G)R²; cyclopropyl, cyclobutyl, cyclopentyl, cyclohexyl, optionally interrupted once or twice independently of one another by O, S(O)ₘ, CO, NR⁴ and optionally mono- or disubstituted independently of one another by fluorine, chlorine, methyl, ethyl, trifluoromethyl, methoxy, cyano; cyclopropylmethyl, cyclobutylmethyl, cyclopentylmethyl, cyclohexylmethyl, optionally interrupted once or twice independently of one another by O, S(O)ₘ, CO, NR⁴ and optionally mono- or disubstituted independently of one another by fluorine, chlorine, methyl, ethyl, trifluoromethyl, methoxy, cyano; phenyl, pyridyl, pyrimidyl, thienyl, thiazolyl, oxazolyl, imidazolyl, oxadiazolyl, thiadiazolyl, triazolyl, benzimidazolyl, imidazopyridinyl which may optionally be substituted by phenyl, optionally mono- or disubstituted independently of one another by fluorine, chlorine, bromine, methyl, ethyl, difluoromethyl, trifluoromethyl, methoxy, ethoxy, methylsulphanyl, ethylsulphanyl, methylsulphinyl, ethylsulphinyl, methylsulphonyl, ethylsulphonyl, difluoromethoxy, trifluoromethoxy, trifluoromethylsulphanyl, trifluoromethylsulphinyl, trifluoromethylsulphonyl, difluoromethylsulphanyl, difluoromethylsulphinyl, difluoromethylsulphonyl, trifluoroethylsulphanyl, trifluoroethylsulphinyl trifluoroethylsulphonyl, nitro and cyano; benzyl, pyridylmethyl, pyrimidylmethyl, thiazolylmethyl, pyrazolyl-C₁-C₂-alkyl, optionally mono- or disubstituted independently of one another by fluorine, chlorine, bromine, methyl, ethyl, difluoromethyl, trifluoromethyl, methoxy, ethoxy, methylsulphanyl, ethylsulphanyl, methylsulphinyl, ethylsulphinyl, methylsulphonyl, ethylsulphonyl, difluoromethoxy, trifluoromethoxy, trifluoromethylsulphanyl, trifluoromethylsulphinyl, trifluoromethylsulphonyl, difluoromethylsulphanyl, difluoromethylsulphinyl, difluoromethylsulphonyl, trifluoroethylsulphanyl, trifluoroethylsulphinyl trifluoroethylsulphonyl, nitro and cyano; and cyano,
G represents O, N-OR²,
Y represents a radical from the series hydrogen, methyl, ethyl, propyl, difluoroethyl, trifluoroethyl, methoxymethyl, ethoxymethyl, cyanomethyl and benzyl,
m represents a number 0, 1 or 2,
n represents a number 0, 1 or 2,
A represents a radical from the series hydrogen, methyl, ethyl, propyl, difluoroethyl, trifluoroethyl, methoxymethyl, ethoxymethyl, cyanomethyl, allyl, propargyl, cylopropyl or cyclopropylmethyl,
R² represents a radical from the series hydrogen; methyl, ethyl, propyl, isopropyl, n-butyl, isobutyl, sec-butyl, tert-butyl, allyl, methallyl, 2-butenyl, propargyl, 2-butynyl, optionally mono- to trisubstituted independently of one another by fluorine, chlorine, methoxy, ethoxy; cyclopropyl, cyclobutyl, cyclopentyl, cyclohexyl, optionally interrupted once by O, S(O)ₘ and optionally mono- or disubstituted independently of one another by fluorine, chlorine, methyl, ethyl, trifluoromethyl, methoxy, cyano; cyclopropylmethyl, cyclobutylmethyl, cyclopentylmethyl, cyclohexylmethyl, optionally interrupted once by O, S(O)ₘ and optionally mono- or disubstituted independently of one another by fluorine, chlorine, methyl, ethyl, trifluoromethyl, methoxy, cyano; phenyl or pyridyl, optionally mono- or disubstituted independently of one another by fluorine, chlorine, bromine, methyl, ethyl, difluoromethyl, trifluoromethyl, methoxy, ethoxy, methylsulphanyl, ethylsulphanyl, methylsulphinyl, ethylsulphinyl, methylsulphonyl, ethylsulphonyl, difluoromethoxy, trifluoromethoxy, trifluoromethylsulfanyl, trifluoromethylsulphinyl, trifluoromethylsulphonyl, difluoromethylsulphanyl, difluoromethylsulphinyl, difluoromethylsulphonyl, trifluoroethylsulphanyl, trifluoroethylsulphinyl, trifluoroethylsulphonyl, nitro and cyano; and benzyl, pyridylmethyl, pyrimidylmethyl, thiazolylmethyl, optionally mono- or disubstituted independently of one another by fluorine, chlorine, bromine, methyl, ethyl, difluoromethyl, trifluoromethyl, methoxy, ethoxy, difluoromethoxy, trifluoromethoxy, nitro and cyano,
R³ represents a radical from the series hydrogen, methyl, ethyl, propyl, isopropyl, butyl, isobutyl and allyl,
R⁴ represents a radical from the series hydrogen, optionally once to three times independently of one another by fluorine, methoxy, ethoxy or cyano substituted methyl, ethyl, propyl, isopropyl, butyl, isobutyl, and allyl and represents the radicals CONR²R³ and COR², where the above definitions of the preferred range (4A) apply to R² and R³,
T represents oxygen or an electron pair,
and salts thereof.

8. Compounds of the formula (I-A) 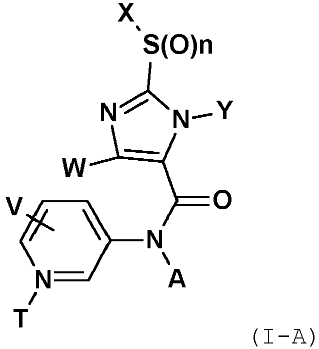 according to Claim 1, where
V represents hydrogen,
W represents a radical from the series hydrogen, chlorine and bromine and preferably represents hydrogen,
X represents a radical from the series methyl, ethyl, n-butyl, n-pentyl, n-propyl, isopropyl, allyl, 3,3-dimethylallyl, propargyl, cyclohexyl, tetrahydropyranyl, tetrahydrothiopyranyl, 3-oxetanyl, 5-oxa-[3.3.0]-bicycloheptanyl, methoxyethyl, methoxypropyl, ethoxyethyl, ethylthioethyl, methylthioethyl, difluoromethyl, trifluoromethyl, 2,2,2-trifluoroethyl, 3,3,3-trifluoropropyl, 3-chloro-2,2,3,3-tetrafluoropropyl, 3-fluoropropyl, 3,3-difluoropropyl, 2,2,2-trifluoroethylthioethyl, methylcarbonylmethyl, cyclopropylcarbonylmethyl, tert-butylcarbonylmethyl, methoxycarbonylmethyl, ethoxycarbonylmethyl, hydroxycarbonylmethyl, carbamoylmethyl, N-methylcarbamoylmethyl, N-cyclopropylcarbamoylmethyl, N,N-dimethylcarbamoylmethyl, 2-methoximinopropyl, cyclopropylmethyl, phenyl, 4-methylphenyl, 2-nitrophenyl, 3-methylthiophenyl, 4-chlorophenyl, 4-fluorophenyl, 4-tert-butylphenyl, 4-methoxyphenyl, 4-nitrophenyl, 4-diemethylaminophenyl, 2-fluorophenyl, 2-methoxyphenyl, 2-dimethylaminosulphonylphenyl, 2-dimethylaminocarbamoylphenyl, 3-nitrophenyl, 3-trifluoromethylphenyl, 3-chlorophenyl, 2,5-dichlorophenyl, 3,5-dichlorophenyl, 4-chloro-3-trifluoromethylphenyl, 2,4,5-trichlorophenyl, 2-pyridyl, 5-(2-chloro)pyridyl, 2-(5-methyl)pyridyl, 2-(6-methyl)pyridyl, 2-(3-trifluoromethyl)pyridyl, 2-pyrimidyl, 2-(4-methyl)pyrimidyl, 2-(5-methyl)pyrimidyl, 2-(4-methoxy)pyrimidyl, 2-(5-fluoro)pyrimidyl, 2-(4-trifluoromethyl)pyrimidyl, 2-(5-trifluoromethyl)pyrimidyl, 2-(4,6-dimethyl)pyrimidyl, 2-(4,5-dimethyl)pyrimidyl, 2-(4,6-dimethoxy)pyrimidyl, -CH₂-2-pyrimidyl, -CH₂-2-pyrazinyl, -CH₂-5-(1-methyl)imidazolyl,-CH₂-3-(1-methyl)pyrazolyl, -CH₂-4-pyridyl, -CH₂-2-pyridyl, -CH₂-2-(1-methyl)imidazolyl, -CH₂-3-pyridyl, -CH₂-2-furanyl, -CH₂-5-(2-chloro)pyridyl, benzyl, 3,4-dichlorobenzyl, 2,6-difluorobenzyl, 2-fluoro-6-methoxybenzyl, 2,6-dichlorobenzyl, 2-chloro-6-trifluoromethylbenzyl, 2-chloro-6-fluorobenzyl, -CH₂-2-(4,6-dimethoxy)pyrimidyl, 2,6-dimethylbenzyl,-CH₂-1-(3-nitro-5-methyl)pyrazolyl, 2-(1-methyl)benzimidazolyl, 2-(5-methyl)oxadiazolyl, 2-[3-methyl-6-(trifluoromethyl)imidazo[4.5]pyridinyl, 3-[4-ethyl-5-(trifluoromethyl)]-1,2,4-triazolyl, 3-[4-methyl-5-(trifluoromethyl)]-1,2,4-triazolyl, 3-[4-methyl-5-(difluoromethyl)]-1,2,4-triazolyl, 2-(5-phenyl)-1,3,4-thiadiazolyl, 2-(1-methyl-5-phenyl)imidazolyl, 2-(4,5-dimethyl)oxazolyl, 2-(1-methyl-5-methoxycarbonyl)imidazolyl, 2-(1-methyl)imidazolyl, 1,2-ethanediyl,
Y represents methyl, ethyl or benzyl,
n represents a number 0 or 2 and preferably represents 0,
A represents a radical from the series hydrogen and methyl and preferably represents methyl,
T represents an electron pair and salts thereof
and
compounds of the formula (I-B) 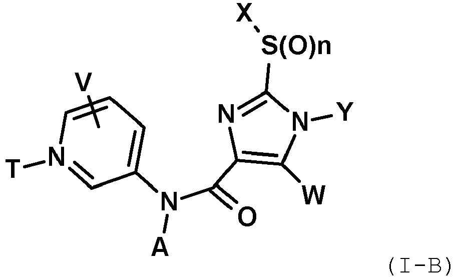 according to Claim 1, where
V represents hydrogen,
W represents a radical from the series hydrogen, chlorine and bromine and preferably represents hydrogen,
X represents a radical from the series 4,6-dimethylpyrimidyl, n-butyl, n-pentyl, benzyl, methyl, 3-methylthiophenyl, 2,2,2-trifluoroethyl, phenyl, 4-methylphenyl, pyrimidyl, ethylthioethyl, 2-nitrophenyl, cyclopropylmethyl and preferably represents a pyrimidyl radical,
Y represents methyl,
n represents a number 0 or 2 and preferably represents 0,
A represents a radical from the series hydrogen and methyl and preferably represents methyl,
T represents an electron pair
and salts thereof.

9. Composition, comprising at least one compound according to any of Claims 1 to 8 and customary extenders and/or surfactants in particular for controlling animal pests.

10. Method for controlling animal pests, where at least one compound according to any of Claims 1 to 8 or a composition according to Claim 9 is allowed to act on the animal pests and/or their habitat, where the surgical, therapeutic and diagnostic treatment of the human or animal body is excluded.

11. Use of at least one compound according to any of Claims 1 to 8 or of a composition according to Claim 9 for controlling animal pests, where the surgical, therapeutic and diagnostic treatment of the human or animal body is excluded.

12. Use of at least one compound according to any of Claims 1 to 8 for protecting the propargation material of plants.

13. Agrochemical formulation comprising at least one compound according to any of Claims 1 to 8 in biologically effective amounts of from 0.00000001 to 98% by weight based on the weight of the agrochemical formulation, and also extenders and/or surfactants.

14. Agrochemical formulation according to Claim 13, additionally comprising a further agrochemically active compound.

15. Intermediates of the formulae (XI) and (XXI) 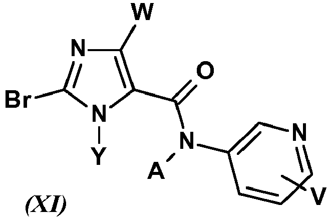 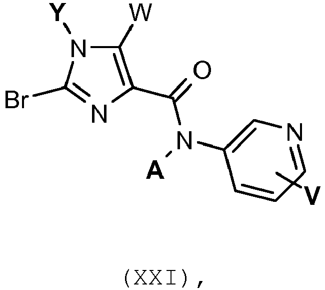 where the radicals A, Y, V and W have the meanings according to any of Claims 1 to 3 or 6 to 8 and particularly preferably the meaning according to Claim 8.

16. Intermediates of the formulae (XV) and (XXII) 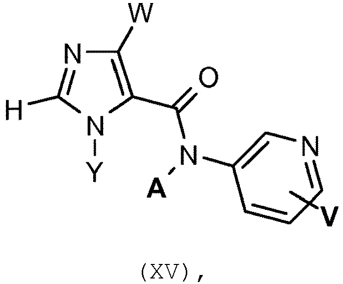 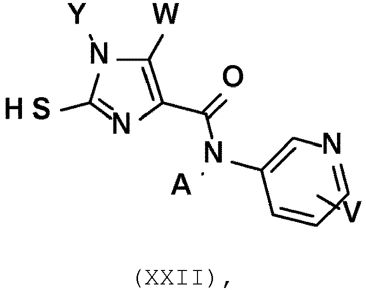 where
for the intermediates of the formula (XV)
V represents hydrogen,
W represents a radical from the series hydrogen, chlorine and bromine and preferably represents hydrogen,
Y represents methyl, ethyl or benzyl and
A represents a radical from the series hydrogen and methyl and preferably represents methyl
and for the intermediates of the formula (XXII)
V represents hydrogen,
W represents a radical from the series hydrogen, chlorine and bromine and preferably represents hydrogen,
Y represents methyl and
A represents a radical from the series hydrogen and methyl and preferably represents methyl.

## Revendications

1. Composés de formule (I) 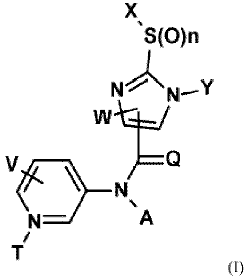 dans laquelle
Q représente un atome d'oxygène ou de soufre,
V représente un radical de la série constituée par les atomes d'hydrogène et d'halogène, les groupes alkyle, halogénoalkyle, alcoxy, halogénoalcoxy et cyano,
W représente un radical de la série constituée par les atomes d'hydrogène et d'halogène, les groupes alkyle, halogénoalkyle, alcoxy, halogénoalcoxy et cyano,
X représente un radical de la série constituée par un groupe alkyle, alcényle, alcynyle, éventuellement substitué, un groupe cycloalkyle saturé ou insaturé, éventuellement interrompu par des hétéroatomes et éventuellement substitué, un groupe cycloalkylalkyle saturé ou insaturé, éventuellement interrompu par des hétéroatomes et éventuellement substitué, un groupe aryle, hétéroaryle, éventuellement substitué, un groupe arylalkyle, hétéroarylalkyle, éventuellement substitué et un groupe cyano,
Y représente un radical de la série constituée par un atome d'hydrogène, un groupe alkyle, alcényle, alcynyle, éventuellement substitué, un groupe cycloalkyle éventuellement interrompu par des hétéroatomes et éventuellement substitué, un groupe cycloalkylalkyle, arylalkyle, hétéroarylalkyle, éventuellement interrompu par des hétéroatomes et éventuellement substitué, et un groupe cyano,
n représente un nombre 0, 1 ou 2,
A représente un radical de la série constituée par un atome d'hydrogène, un groupe alkyle, alcényle, alcynyle, éventuellement substitué et un groupe cycloalkyle ou cycloalkyl-alkyle éventuellement interrompu par des hétéroatomes et éventuellement substitué,
T représente un atome d'oxygène ou une paire d'électrons,
et leurs sels.

2. Composés selon la revendication 1, dans lesquels
Q représente un atome d'oxygène ou de soufre,
V représente un radical de la série constituée par les atomes d'hydrogène et d'halogène, les groupes alkyle en C₁-C₄, halogénoalkyle(C₁-C₄), alcoxy en C₁-C₄, halogénoalcoxy(C₁-C₄) et cyano,
W représente un radical de la série constituée par les atomes d'hydrogène et d'halogène, les groupes alkyle en C₁-C₄, halogénoalkyle(C₁-C₄), alcoxy en C₁-C₄, halogénoalcoxy(C₁-C₄) et cyano,
X représente un radical de la série constituée par un groupe alkyle en C₁-C₈, alcényle en C₃-C₈, alcynyle en C₃-C₈, éventuellement une à plusieurs fois substitué, chaque fois indépendamment, par halogéno, alcoxy en C₁-C₄, alkyl (C₁-C₄)-S(O)ₘ-, cyano, C(O)OR², CONR²R³, C(G)R², un groupe cycloalkyle en C₃-C₈ ou cycloalcényle en C₅-C₈, éventuellement interrompu une ou deux fois, chaque fois indépendamment, par O, S(O)ₘ, C(G)R², NR⁴ et éventuellement une à quatre fois substitué, chaque fois indépendamment, par halogéno, alkyle en C₁-C₄, halogénoalkyle(C₁-C₄), alcoxy en C₁-C₄, cyano, un groupe cycloalkyl (C₃-C₈)-alkyle (C₁-C₄) ou cycloalcényl (C₅-C₈)-alkyle (C₁-C₄) à chaîne droite ou ramifié, éventuellement interrompu une ou deux fois, chaque fois indépendamment, par O, S(O)ₘ, C(G)R², NR⁴ et éventuellement une à quatre fois substitué, chaque fois indépendamment, par halogéno, alkyle en C₁-C₄, halogénoalkyle(C₁-C₄), alcoxy en C₁-C₄, cyano, un groupe aryle ou hétéroaryle éventuellement une à trois fois substitué, chaque fois indépendamment, par halogéno, alkyle en C₁-C₄, halogénoalkyle(C₁-C₄), alcoxy en C₁-C₄, alkyl (C₁-C₄)-S(O)ₘ-, halogénoalcoxy(C₁-C₄), halogénoalkyl(C₁-C₄)-S(O)ₘ-, nitro et cyano, qui peut éventuellement être substitué par aryle ou hétéroaryle une à trois fois substitué, chaque fois indépendamment, par halogéno, alkyle en C₁-C₄, halogénoalkyle(C₁-C₄), alcoxy en C₁-C₄, alkyl (C₁-C₄)-S(O)ₘ-, halogénoalcoxy (C₁-C₄), halogénoalkyl (C₁-C₄)-S(O)ₘ-, nitro et cyano, un groupe aryl-alkyle(C₁-C₄), hétéroaryl-alkyle(C₁-C₄), à chaîne droite ou ramifié, éventuellement une à trois fois substitué, chaque fois indépendamment, par halogéno, alkyle en C₁-C₄, halogénoalkyle(C₁-C₄), alcoxy en C₁-C₄, alkyl(C₁-C₄)-S(O)ₘ-, halogénoalcoxy(C₁-C₄), halogénoalkyl(C₁-C₄)-S(O)ₘ-, nitro et cyano.
G représente O, N-CN, N-OR²,
Y représente un radical de la série constituée par un atome d'hydrogène, un groupe alkyle en C₁-C₆, alcényle en C₃-C₆, alcynyle en C₃-C₆, éventuellement une à plusieurs fois substitué, chaque fois indépendamment, par halogéno, alcoxy en C₁-C₄, alkyl (C₁-C₄)-S(O)ₘ-, cyano, un groupe cycloalkyle en C₃-C₈ éventuellement une ou deux fois substitué, chaque fois indépendamment, par halogéno, alkyle en C₁-C₄, halogénoalkyle (C₁-C₄), alcoxy en C₁-C₄, cyano, un groupe cycloalkyl (C₃-C₈)-alkyle (C₁-C₄) à chaîne droite ou ramifié, éventuellement interrompu une ou deux fois, chaque fois indépendamment, par O, S(O)ₘ, CO, NR⁴ et éventuellement une à quatre fois substitué, chaque fois indépendamment, par halogéno, alkyle en C₁-C₄, halogénoalkyle (C₁-C₄), alcoxy en C₁-C₄, cyano, un groupe arylalkyle ou hétéroarylalkyle, une à trois fois substitué, chaque fois indépendamment, par halogéno, alkyle en C₁-C₄, halogénoalkyle(C₁-C₄), alcoxy en C₁-C₄, alkyl (C₁-C₄)-S(O)ₘ-, halogénoalcoxy (C₁-C₄), halogénoalkyl(C₁-C₄)-S(O)ₘ-, nitro et cyano, et un groupe cyano,
m représente un nombre 0, 1 ou 2,
n représente un nombre 0, 1 ou 2,
A représente un radical de la série constituée par un atome d'hydrogène, un groupe alkyle en C₁-C₆, alcényle en C₃-C₆, alcynyle en C₃-C₆, éventuellement une à plusieurs fois substitué, chaque fois indépendamment, par halogéno, alcoxy en C₁-C₄, alkyl(C₁-C₄)-S(O)ₘ-, cyano et un groupe cycloalkyle en C₃-C₆ éventuellement une ou deux fois substitué, chaque fois indépendamment, par halogéno, alkyle en C₁-C₄, halogénoalkyle (C₁-C₄), alcoxy en C₁-C₄, cyano et un groupe cycloalkyl (C₃-C₈)-alkyle (C₁-C₄) à chaîne droite ou ramifié, éventuellement une ou deux fois substitué, chaque fois indépendamment, par halogéno, alkyle en C₁-C₄, halogénoalkyle (C₁-C₄), alcoxy en C₁-C₄, cyano,
R² représente un radical de la série constituée par un atome d'hydrogène, un groupe alkyle en C₁-C₈, alcényle en C₃-C₈, alcynyle en C₃-C₈, éventuellement une à plusieurs fois substitué, chaque fois indépendamment, par halogéno, alcoxy en C₁-C₄, alkyl(C₁-C₄)-S(O)ₙ-, un groupe cycloalkyle en C₃-C₈ éventuellement interrompu une fois par O, S(O)ₘ et éventuellement une ou deux fois substitué, chaque fois indépendamment, par halogéno, alkyle en C₁-C₄, halogénoalkyle(C₁-C₄), alcoxy en C₁-C₄, cyano, un groupe cycloalkyl (C₃-C₈)-alkyle (C₁-C₄) à chaîne droite ou ramifié, éventuellement interrompu une fois par O, S(O)ₘ et éventuellement une ou deux fois substitué, chaque fois indépendamment, par halogéno, alkyle en C₁-C₄, halogénoalkyle(C₁-C₄), alcoxy en C₁-C₄, cyano, un groupe aryle, hétéroaryle, éventuellement une à trois fois substitué, chaque fois indépendamment, par halogéno, alkyle en C₁-C₄, halogénoalkyle(C₁-C₄), alcoxy en C₁-C₄, alkyl (C₁-C₄)-S(O)ₘ-, halogénoalcoxy(C₁-C₄), halogénoalkyl(C₁-C₄)-S(O)ₘ-, nitro et cyano et un groupe aryl-alkyle(C₁-C₄), hétéroarylalkyle (C₁-C₄) à chaîne droite ou ramifié, éventuellement une à trois fois substitué, chaque fois indépendamment, par halogéno, alkyle en C₁-C₄, halogénoalkyle (C₁-C₄), alcoxy en C₁-C₄, alkyl(C₁-C₄)-S(O)ₘ-, halogénoalcoxy(C₁-C₄), halogénoalkyl(C₁-C₄)-S(O)ₘ-, nitro et cyano,
R³ représente un radical de la série constituée par un atome d'hydrogène, un groupe alkyle en C₁-C₆, alcényle en C₃-C₆, alcynyle en C₃-C₆, éventuellement une à plusieurs fois substitué, chaque fois indépendamment, par halogéno, alcoxy en C₁-C₄, alkyl(C₁-C₄)-S(O)ₘ-, cyano,
R⁴ représente un radical de la série constituée par un atome d'hydrogène, un groupe alkyle en C₁-C₆, alcényle en C₃-C₆, alcynyle en C₃-C₆, éventuellement une à plusieurs fois substitué, chaque fois indépendamment, par halogéno, alcoxy en C₁-C₄, alkyl(C₁-C₄)-S(O)ₘ-, cyano, et représente les radicaux CONR²R³ et COR², les définitions précédentes de l'ensemble de préférences (1) s'appliquant à R² et R³,
T représente un atome d'oxygène ou une paire d'électrons,
et leurs sels.

3. Composés selon la revendication 1, dans lesquels
Q représente un atome d'oxygène,
V représente un radical de la série constituée par les atomes d'hydrogène, de fluor, chlore, brome, les groupes méthyle, éthyle, difluorométhyle, trifluorométhyle, méthoxy, éthoxy, difluorométhoxy, trifluorométhoxy et cyano,
W représente un radical de la série constituée par les atomes d'hydrogène, de fluor, chlore, brome, les groupes méthyle, éthyle, difluorométhyle, trifluorométhyle, méthoxy, éthoxy, difluorométhoxy, trifluorométhoxy et cyano,
X représente un radical de la série constituée par un groupe alkyle en C₁-C₆, alcényle en C₃-C₆, alcynyle en C₃-C₆, éventuellement une à sept fois substitué, chaque fois indépendamment, par fluoro, chloro, bromo, méthoxy, éthoxy, méthyl-S(O)ₘ-, éthyl-S(O)ₘ-, cyano, C(O)OR², CONR²R³, C(G)R², un groupe cycloalkyle en C₃-C₆ éventuellement interrompu une ou deux fois, chaque fois indépendamment, par O, S(O)ₘ, CO, NR⁴ et éventuellement une ou deux fois substitué, chaque fois indépendamment, par fluoro, chloro, bromo, méthyle, éthyle, trifluorométhyle, méthoxy, éthoxy, cyano, un groupe cycloalkyl(C₃-C₆)-alkyle(C₁-C₂) à chaîne droite ou ramifié, éventuellement interrompu une ou deux fois, chaque fois indépendamment, par O, S(O)ₘ, CO, NR⁴ et éventuellement une ou deux fois substitué, chaque fois indépendamment, par fluoro, chloro, bromo, méthyle, éthyle, trifluorométhyle, méthoxy, éthoxy, cyano, un groupe phényle, naphtyle, pyridyle, pyrimidyle, pyridazinyle, pyrazinyle, triazinyle, furanyle, thiényle, oxazolyle, isoxazolyle, thiazolyle, isothiazolyle, pyrazolyle, imidazolyle, oxadiazolyle, thiadiazolyle, triazolyle, benzimidazolyle, imidazopyridinyle, éventuellement une ou deux fois substitué, chaque fois indépendamment, par fluoro, chloro, bromo, méthyle, éthyle, difluorométhyle, trifluorométhyle, méthoxy, éthoxy, méthyl-S(O)ₘ-, éthyl-S(O)ₙ-, difluorométhoxy, trifluorométhoxy, trifluorométhyl-S(O)ₘ-, difluoroéthyl-S(O)ₘ-, trifluoroéthyl-S(O)ₘ-, nitro et cyano, qui peut éventuellement être substitué par phényle une à trois fois substitué, chaque fois indépendamment, par fluoro, chloro, méthyle, éthyle, difluorométhyle, trifluorométhyle, méthoxy, éthoxy, méthyl-S(O)ₘ-, éthyl-S(O)ₙ-, difluorométhoxy, trifluorométhoxy, trifluorométhyl-S(O)ₘ-, difluoroéthyl-S(O)ₘ-, trifluoroéthyl-S(O)ₘ-, nitro et cyano, un groupe phényl-alkyle(C₁-C₂), pyridylalkyle(C₁-C₂), pyrimidyl-alkyle(C₁-C₂), thiazolylalkyle(C₁-C₂), pyrazolyl-alkyle(C₁-C₂), à chaîne droite ou ramifié, éventuellement une ou deux fois substitué, chaque fois indépendamment, par fluoro, chloro, bromo, méthyle, éthyle, difluorométhyle, trifluorométhyle, méthoxy, éthoxy, Me-S(O)ₘ-, Et-S(O)ₘ-, difluorométhoxy, trifluorométhoxy, trifluorométhyl-S(O)ₘ-, difluoroéthyl-S(O)ₘ-, trifluoroéthyl-S(O)ₘ-, nitro et cyano, et un groupe cyano,
G représente O, N-OR²,
Y représente un radical de la série constituée par un atome d'hydrogène, un groupe alkyle en C₁-C₄, alcényle en C₃-C₄, alcynyle en C₃-C₄, éventuellement une à cinq fois substitué, chaque fois indépendamment, par fluoro, chloro, bromo, méthoxy, éthoxy, méthyl-S(O)ₘ-, éthyl-S(O)ₘ-, cyano, un groupe cycloalkyle en C₃-C₆ éventuellement une ou deux fois substitué, chaque fois indépendamment, par fluoro, chloro, bromo, méthyle, éthyle, trifluorométhyle, méthoxy, éthoxy, cyano, un groupe cycloalkyl(C₃-C₆)-alkyle(C₁-C₂) à chaîne droite ou ramifié, éventuellement interrompu une ou deux fois, chaque fois indépendamment, par O, S(O)ₘ, CO, NR⁴ et éventuellement une à quatre fois substitué, chaque fois indépendamment, par fluoro, chloro, bromo, méthyle, éthyle, trifluorométhyle, méthoxy, éthoxy, cyano, un groupe aryl-alkyle(C₁-C₂) ou hétéroaryl-alkyle(C₁-C₂) éventuellement une ou deux fois substitué, chaque fois indépendamment, par fluoro, chloro, bromo, méthyle, éthyle, halogénoalkyle(C₁-C₄), trifluorométhyle, méthoxy, éthoxy, cyano, nitro, et un groupe cyano,
m représente un nombre 0, 1 ou 2,
n représente un nombre 0, 1 ou 2,
A représente un radical de la série constituée par un atome d'hydrogène, un groupe alkyle en C₁-C₆, alcényle en C₃-C₆, alcynyle en C₃-C₆, éventuellement une à cinq fois substitué, chaque fois indépendamment, par fluoro, chloro, bromo, méthoxy, éthoxy, méthyl-S(O)ₘ-, éthyl-S(O)ₘ-, cyano et un groupe cycloalkyle en C₃-C₆ éventuellement une ou deux fois substitué, chaque fois indépendamment, par fluoro, chloro, bromo, méthyle, éthyle, trifluorométhyle, méthoxy, éthoxy, cyano et un groupe cycloalkyl(C₃-C₆)-alkyle(C₁-C₂) à chaîne droite ou ramifié, éventuellement une ou deux fois substitué, chaque fois indépendamment, par fluoro, chloro, bromo, méthyle, éthyle, trifluorométhyle, méthoxy, éthoxy, cyano,
R² représente un radical de la série constituée par un atome d'hydrogène, un groupe alkyle en C₁-C₆, alcényle en C₃-C₆, alcynyle en C₃-C₆, éventuellement une à cinq fois substitué, chaque fois indépendamment, par fluoro, chloro, bromo, méthoxy, éthoxy, méthyl-S(O)ₘ-, éthyl-S(O)ₘ-, un groupe cycloalkyle en C₃-C₆ éventuellement interrompu une fois par O, S(O)ₘ et éventuellement une ou deux fois substitué, chaque fois indépendamment, par fluoro, chloro, bromo, méthyle, éthyle, trifluorométhyle, méthoxy, éthoxy, cyano, un groupe cycloalkyl(C₃-C₆)-alkyle (C₁-C₂) à chaîne droite ou ramifié, éventuellement interrompu une fois par O, S(O)ₘ et éventuellement une ou deux fois substitué, chaque fois indépendamment, par fluoro, chloro, bromo, méthyle, éthyle, trifluorométhyle, méthoxy, éthoxy, cyano, un groupe phényle ou pyridyle éventuellement une ou deux fois substitué, chaque fois indépendamment, par fluoro, chloro, bromo, méthyle, éthyle, difluorométhyle, trifluorométhyle, méthoxy, éthoxy, méthyl-S(O)ₘ-, éthyl-S(O)ₘ-, difluorométhoxy, trifluorométhoxy, trifluorométhyl-S(O)ₘ-, difluoroéthyl-S(O)ₘ-, trifluoroéthyl-S(O)ₘ-, nitro et cyano et un groupe phényl-alkyle(C₁-C₂), pyridyl-alkyle(C₁-C₂), pyrimidyl-alkyle(C₁-C₂), thiazolyl-alkyle(C₁-C₂), à chaîne droite ou ramifié, éventuellement une ou deux fois substitué, chaque fois indépendamment, par fluoro, chloro, bromo, méthyle, éthyle, difluorométhyle, trifluorométhyle, méthoxy, éthoxy, méthyl-S(O)ₘ-, éthyl-S(O)ₘ-, difluorométhoxy, trifluorométhoxy, trifluorométhyl-S(O)ₙ-, difluoroéthyl-S(O)ₙ-, trifluoroéthyl-S(O)ₙ-, nitro et cyano,
R³ représente un radical de la série constituée par un atome d'hydrogène, un groupe alkyle en C₁-C₄, alcényle en C₃-C₄, alcynyle en C₃-C₄, éventuellement une à cinq fois substitué, chaque fois indépendamment, par fluoro, chloro, bromo, méthoxy, éthoxy, méthyl-S(O)ₘ-, éthyl-S(O)ₘ-, cyano,
R⁴ représente un radical de la série constituée par un atome d'hydrogène, un groupe alkyle en C₁-C₄, alcényle en C₃-C₄, alcynyle en C₃-C₄, éventuellement une à cinq fois substitué, chaque fois indépendamment, par fluoro, chloro, bromo, méthoxy, éthoxy, méthyl-S(O)ₘ-, éthyl-S(O)ₘ-, cyano, et représente les radicaux CONR²R³ et COR², les définitions précédentes de l'ensemble de préférences (2) s'appliquant à R² et R³,
T représente un atome d'oxygène ou une paire d'électrons,
et leurs sels.

4. Composés de formule (I-A) 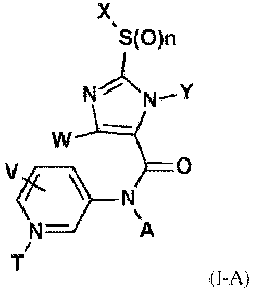 selon l'une quelconque des revendications 1 à 3 et leurs sels.

5. Composés de formule (I-B) 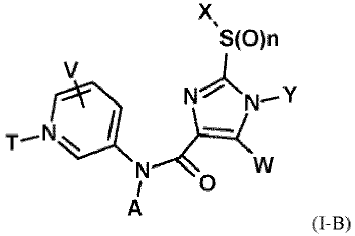 selon l'une quelconque des revendications 1 à 3 et leurs sels.

6. Composés selon la revendication 4 ou 5, dans lesquels
V représente un radical de la série constituée par les atomes d'hydrogène, de fluor, de chlore, les groupes méthyle, éthyle et cyano,
W représente un radical de la série constituée par les atomes d'hydrogène, de fluor, de chlore, de brome, les groupes méthyle, éthyle et cyano,
X représente un radical de la série constituée par un groupe alkyle en C₁-C₆, alcényle en C₃-C₄, alcynyle en C₃-C₄, éventuellement une fois, deux fois, trois fois, quatre fois, cinq fois substitué, chaque fois indépendamment, par fluoro, chloro, bromo, méthoxy, éthoxy, méthyl-S(O)ₘ-, éthyl-S(O)ₘ-, cyano, qui peut éventuellement être une fois substitué par les groupes C(O)OR², CONR²R³, C(G)R², un groupe cycloalkyle en C₃-C₆ éventuellement interrompu une ou deux fois, chaque fois indépendamment, par O, S(O)ₘ, CO, NR⁴ et éventuellement une ou deux fois substitué, chaque fois indépendamment, par fluoro, chloro, méthyle, éthyle, trifluorométhyle, méthoxy, cyano, un groupe cycloalkyl(C₃-C₆)-méthyle éventuellement interrompu une ou deux fois, chaque fois indépendamment, par O, S(O)ₘ, CO, NR⁴ et éventuellement une ou deux fois substitué, chaque fois indépendamment, par fluoro, chloro, méthyle, éthyle, trifluorométhyle, méthoxy, cyano, un groupe phényle, pyridyle, pyrimidyle, thiényle, thiazolyle, oxazolyle, imidazolyle, oxadiazolyle, thiadiazolyle, triazolyle, benzimidazolyle, imidazopyridinyle, éventuellement une ou deux fois substitué, chaque fois indépendamment, par fluoro, chloro, bromo, méthyle, éthyle, difluorométhyle, trifluorométhyle, méthoxy, éthoxy, méthyl-S(O)ₘ-, éthyl-S(O)ₘ-, difluorométhoxy, trifluorométhoxy, trifluorométhyl-S(O)ₘ-, difluoroéthyl-S(O)ₘ-, trifluoroéthyl-S(O)ₘ-, nitro et cyano, qui peut éventuellement être substitué par phényle une ou deux fois substitué, chaque fois indépendamment, par fluoro, chloro, méthyle, éthyle, difluorométhyle, trifluorométhyle, méthoxy, éthoxy, méthyl-S(O)ₘ-, éthyl-S(O)ₙ-, difluorométhoxy, trifluorométhoxy, trifluorométhyl-S(O)ₘ-, difluoroéthyl-S(O)ₘ-, trifluoroéthyl-S(O)ₘ-, nitro et cyano, un groupe benzyle, pyridyl-méthyle, pyrimidyl-méthyle, thiazolyl-méthyle, pyrazolyl-alkyle(C₁-C₂), éventuellement une ou deux fois substitué, chaque fois indépendamment, par fluoro, chloro, bromo, méthyle, éthyle, difluorométhyle, trifluorométhyle, méthoxy, éthoxy, Me-S(O)ₘ-, Et-S(O)ₘ-, difluorométhoxy, trifluorométhoxy, trifluorométhyl-S(O)ₙ-, difluoroéthyl-S(O)ₘ-, trifluoroéthyl-S(O)ₘ-, nitro et cyano, et un groupe cyano,
G représente O, N-OR²,
Y représente un radical de la série constituée par un atome d'hydrogène, un groupe méthyle, éthyle, propyle, allyle, propargyle ou benzyle, éventuellement une à trois fois substitué, chaque fois indépendamment, par fluoro, méthoxy, éthoxy, cyano,
m représente un nombre 0, 1 ou 2,
n représente un nombre 0, 1 ou 2,
A représente un radical de la série constituée par un atome d'hydrogène, un groupe méthyle, éthyle, propyle, allyle, propargyle, cyclopropyle ou cyclopropylméthyle, éventuellement une à trois fois substitué, chaque fois indépendamment, par fluoro, méthoxy, éthoxy, cyano,
R² représente un radical de la série constituée par un atome d'hydrogène, un groupe alkyle en C₁-C₆, alcényle en C₃-C₆, alcynyle en C₃-C₆, éventuellement une à trois fois substitué, chaque fois indépendamment, par fluoro, chloro, méthoxy, éthoxy, méthyl-S(O)ₘ-, éthyl-S(O)ₘ-, un groupe cycloalkyle en C₃-C₆ éventuellement interrompu une fois par O, S(O)ₘ et éventuellement une ou deux fois substitué, chaque fois indépendamment, par fluoro, chloro, méthyle, éthyle, trifluorométhyle, méthoxy, cyano, un groupe cycloalkyl(C₃-C₆)-méthyle éventuellement interrompu une fois par O, S(O)ₘ et éventuellement une ou deux fois substitué, chaque fois indépendamment, par fluoro, chloro, méthyle, éthyle, trifluorométhyle, méthoxy, cyano, un groupe phényle ou pyridyle éventuellement une ou deux fois substitué, chaque fois indépendamment, par fluoro, chloro, bromo, méthyle, éthyle, difluorométhyle, trifluorométhyle, méthoxy, éthoxy, méthyl-S(O)ₘ-, éthyl-S(O)ₘ-, difluorométhoxy, trifluorométhoxy, trifluorométhyl-S(O)ₘ-, difluoroéthyl-S(O)ₘ-, trifluoroéthyl-S(O)ₘ-, nitro et cyano, et un groupe benzyle, pyridyl-méthyle, pyrimidyl-méthyle, thiazolyl-méthyle, éventuellement une ou deux fois substitué, chaque fois indépendamment, par fluoro, chloro, bromo, méthyle, éthyle, difluorométhyle, trifluorométhyle, méthoxy, éthoxy, méthyl-S(O)ₘ-, éthyl-S(O)ₘ-, difluorométhoxy, trifluorométhoxy, trifluorométhyl-S(O)ₘ-, difluoroéthyl-S(O)ₘ-, trifluoroéthyl-S(O)ₘ-, nitro et cyano,
R³ représente un radical de la série constituée par un atome d'hydrogène, un groupe alkyle en C₁-C₄, alcényle en C₃-C₄, alcynyle en C₃-C₄, éventuellement une à trois fois substitué, chaque fois indépendamment, par fluoro, chloro, bromo, méthoxy, éthoxy, méthyl-S(O)ₘ-, éthyl-S(O)ₘ-, cyano,
R⁴ représente un radical de la série constituée par un atome d'hydrogène, un groupe alkyle en C₁-C₄, alcényle en C₃-C₄, alcynyle en C₃-C₄, éventuellement une à trois fois substitué, chaque fois indépendamment, par fluoro, chloro, bromo, méthoxy, éthoxy, méthyl-S(O)ₘ-, éthyl-S(O)ₘ-, cyano, et représente les radicaux CONR²R³ et COR², les définitions précédentes de l'ensemble de préférences (3A) s'appliquant à R² et R³,
T représente un atome d'oxygène ou une paire d'électrons,
et leurs sels.

7. Composés selon l'une quelconque des revendications 4 et 5, dans lesquels
V représente un atome d'hydrogène ou de fluor,
W représente un radical de la série constituée par les atomes d'hydrogène, de chlore, brome, et le groupe méthyle,
X représente un radical de la série constituée par un groupe méthyle, éthyle, propyle, isopropyle, butyle, isobutyle, sec-butyle, tert-butyle, pentyle, isopentyle, 2, 2-diméthyl-propyle, hexyle, néo-hexyle, allyle, méthallyle, 2-butényle, propargyle, 2-butynyle, éventuellement une à trois fois substitué, chaque fois indépendamment, par fluoro, chloro, bromo, méthoxy, éthoxy, méthylsulfanyle, éthylsulfanyle, méthylsulfinyle, éthylsulfinyle, méthylsulfonyle, éthylsulfonyle, qui peut éventuellement être une fois substitué par les groupes C(O)OR², CONR²R³, C(G)R², un groupe cyclopropyle, cyclobutyle, cyclopentyle, cyclohexyle, éventuellement interrompu une ou deux fois, chaque fois indépendamment, par O, S(O)ₘ, CO, NR⁴ et éventuellement une ou deux fois substitué, chaque fois indépendamment, par fluoro, chloro, méthyle, éthyle, trifluorométhyle, méthoxy, cyano, un groupe cyclopropylméthyle, cyclobutylméthyle, cyclopentylméthyle, cyclohexylméthyle, éventuellement interrompu une ou deux fois, chaque fois indépendamment, par O, S(O)ₘ, CO, NR⁴ et éventuellement une ou deux fois substitué, chaque fois indépendamment, par fluoro, chloro, méthyle, éthyle, trifluorométhyle, méthoxy, cyano, un groupe phényle, pyridyle, pyrimidyle, thiényle, thiazolyle, oxazolyle, imidazolyle, oxadiazolyle, thiadiazolyle, triazolyle, benzimidazolyle, imidazopyridinyle, éventuellement une ou deux fois substitué, chaque fois indépendamment, par fluoro, chloro, bromo, méthyle, éthyle, difluorométhyle, trifluorométhyle, méthoxy, éthoxy, méthylsulfanyle, éthylsulfanyle, méthylsulfinyle, éthylsulfinyle, méthylsulfonyle, éthylsulfonyle, difluorométhoxy, trifluorométhoxy, trifluorométhylsulfanyle, trifluorométhylsulfinyle, trifluorométhylsulfonyle, difluorométhylsulfanyle, difluorométhylsulfinyle, difluorométhylsulfonyle, trifluoroéthylsulfanyle, trifluoroéthylsulfinyle, trifluoroéthylsulfonyle, nitro et cyano, qui peut être éventuellement substitué par phényle, un groupe benzyle, pyridylméthyle, pyrimidylméthyle, thiazolylméthyle, pyrazolyl-alkyle(C₁-C₂), éventuellement une ou deux fois substitué, chaque fois indépendamment, par fluoro, chloro, bromo, méthyle, éthyle, difluorométhyle, trifluorométhyle, méthoxy, éthoxy, méthylsulfanyle, éthylsulfanyle, méthylsulfinyle, éthylsulfinyle, méthylsulfonyle, éthylsulfonyle, difluorométhoxy, trifluorométhoxy, trifluorométhylsulfanyle, trifluorométhylsulfinyle, trifluorométhylsulfonyle, difluorométhylsulfanyle, difluorométhylsulfinyle, difluorométhylsulfonyle, trifluoroéthylsulfanyle, trifluoroéthylsulfinyle, trifluoroéthylsulfonyle, nitro et cyano, et un groupe cyano,
G représente O, N-OR²,
Y représente un radical de la série constituée par un atome d'hydrogène, les groupes méthyle, éthyle, propyle, difluoroéthyle, trifluoroéthyle, méthoxyméthyle, éthoxyméthyle, cyanométhyle et benzyle,
m représente un nombre 0, 1 ou 2,
n représente un nombre 0, 1 ou 2,
A représente un radical de la série constituée par un atome d'hydrogène, un groupe méthyle, éthyle, propyle, difluoroéthyle, trifluoroéthyle, méthoxyméthyle, éthoxyméthyle, cyanométhyle, allyle, propargyle, cyclopropyle ou cyclopropylméthyle,
R² représente un radical de la série constituée par un atome d'hydrogène, un groupe méthyle, éthyle, propyle, isopropyle, n-butyle, isobutyle, sec-butyle, tert-butyle, allyle, méthallyle, 2-butényle, propargyle, 2-butynyle, éventuellement une à trois fois substitué, chaque fois indépendamment, par fluoro, chloro, méthoxy, éthoxy, un groupe cyclopropyle, cyclobutyle, cyclopentyle, cyclohexyle éventuellement interrompu une fois par O, S(O)ₘ et éventuellement une ou deux fois substitué, chaque fois indépendamment, par fluoro, chloro, méthyle, éthyle, trifluorométhyle, méthoxy, cyano, un groupe cyclopropylméthyle, cyclobutylméthyle, cyclopentylméthyle, cyclohexylméthyle, éventuellement interrompu une fois par O, S(O)ₘ et éventuellement une ou deux fois substitué, chaque fois indépendamment, par fluoro, chloro, méthyle, éthyle, trifluorométhyle, méthoxy, cyano, un groupe phényle ou pyridyle éventuellement une ou deux fois substitué, chaque fois indépendamment, par fluoro, chloro, bromo, méthyle, éthyle, difluorométhyle, trifluorométhyle, méthoxy, éthoxy, méthylsulfanyle, éthylsulfanyle, méthylsulfinyle, éthylsulfinyle, méthylsulfonyle, éthylsulfonyle, difluorométhoxy, trifluorométhoxy, trifluorométhylsulfanyle, trifluorométhylsulfinyle, trifluorométhylsulfonyle, difluorométhylsulfanyle, difluorométhylsulfinyle, difluorométhylsulfonyle, trifluoroéthylsulfanyle, trifluoroéthylsulfinyle, trifluoroéthylsulfonyle, nitro et cyano, et un groupe benzyle, pyridylméthyle, pyrimidyl-méthyle, thiazolyle-méthyle, éventuellement une ou deux fois substitué, chaque fois indépendamment, par fluoro, chloro, bromo, méthyle, éthyle, difluorométhyle, trifluorométhyle, méthoxy, éthoxy, difluorométhoxy, trifluorométhoxy, nitro et cyano,
R³ représente un radical de la série constituée par un atome d'hydrogène, les groupes méthyle, éthyle, propyle, isopropyle, butyle, isobutyle et allyle,
R⁴ représente un radical de la série constituée par un atome d'hydrogène, un groupe méthyle, éthyle, propyle, isopropyle, butyle, isobutyle ou allyle, éventuellement une à trois fois substitué, chaque fois indépendamment, par fluoro, méthoxy, éthoxy ou cyano, et représente les radicaux CONR²R³ et COR², les définitions précédentes de l'ensemble de préférences (4A) s'appliquant à R² et R³,
T représente un atome d'oxygène ou une paire d'électrons,
et leurs sels.

8. Composés de formule (I-A) 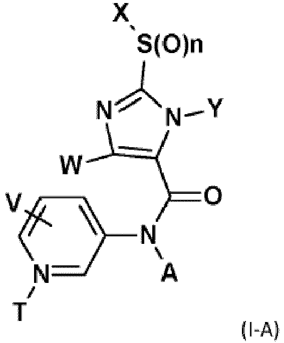 selon la revendication 1, dans laquelle
V représente un atome d'hydrogène,
W représente un radical de la série constituée par les atomes d'hydrogène, de chlore et de brome, et de préférence un atome d'hydrogène,
X représente un radical de la série constituée par les groupes méthyle, éthyle, n-butyle, n-pentyle, n-propyle, isopropyle, allyle, 3,3-diméthylallyle, propargyle, cyclohexyle, tétrahydropyranyle, tétrahydrothiopyranyle, 3-oxétanyle, 5-oxa-[3.3.0]-bicyclohépatanyle, méthoxyéthyle, méthoxypropyle, éthoxyéthyle, éthylthioéthyle, méthylthioéthyle, difluorométhyle, trifluorométhyle, 2,2,2-trifluoroéthyle, 3,3,3-trifluoropropyle, 3-chloro-2,2,3,3-tétrafluoropropyle, 3-fluoropropyle, 3,3-difluoropropyle, 2,2,2-trifluoroéthylthio-éthyle, méthylcarbonyl-méthyle, c-propylcarbonyl-méthyle, tert-butylcarbonylméthyle, méthoxycarbonyl-méthyle, éthoxycarbonyl-méthyle, hydroxycarbonyl-méthyle, carbamoyl-méthyle, n-méthylcarbamoyl-méthyle, N-c-propylcarbamoyl-méthyle, N,N-diméthylcarbamoyl-méthyle, 2-méthoximino-propyle, cyclopropylméthyle, phényle, 4-méthylphényle, 2-nitrophényle, 3-méthylthiophényle, 4-chloro-phényle, 4-fluoro-phényle, 4-tert-butyl-phényle, 4-méthoxy-phényle, 4-nitro-phényle, 4-diméthylamino-phényle, 2-fluoro-phényle, 2-méthoxy-phényle, 2-diméthylamino-sulfonyl-phényle, 2-diméthylaminocarbamoyl-phényle, 3-nitrophényle, 3-trifluorométhyl-phényle, 3-chlorophényle, 2,5-dichloro-phényle, 3,5-dichlorophényle, 4-chloro-3-trifluorométhyl-phényle, 2,4,5-trichloro-phényle, 2-pyridyle, 5-(2-chloro)pyridyle, 2-(5-méthyl)-pyridyle, 2-(6-méthyl)pyridyle, 2-(3-trifluorométhyl)-pyridyle, 2-pyrimidyle, 2-(4-méthyl)-pyrimidyle, 2-(5-méthyl)-pyrimidyle, 2-(4-méthoxy)-pyrimidyle, 2-(5-fluoro)-pyrimidyle, 2-(4-trifluorométhyl)-pyrimidyle, 2-(5-trifluorométhyl)-pyrimidyle, 2-(4,6-diméthyl)-pyrimidyle, 2-(4,5-diméthyl)-pyrimidyle, 2-(4,6-diméthoxy)-pyrimidyle, -CH₂-2-pyrimidyle, -CH₂-2-pyrazinyle, -CH₂-5-(1-méthyl)-imidazolyle, -CH₂-3-(1-méthyl)-pyrazolyle,-CH₂-4-pyridyle, -CH₂-2-pyridyle, -CH₂-2-(1-méthyl)-imidazolyle, -CH₂-3-pyridyle, -CH₂-2-furanyle, -CH₂-5-(2-chloro)-pyridyle, benzyle, 3,4-dichloro-benzyle, 2,6-difluorobenzyle, 2-fluoro-6-méthoxybenzyle, 2,6-dichloro-benzyle, 2-chloro-6-trifluorométhyl-benzyle, 2-chloro-6-fluoro-benzyle, -CH₂-2-(4,6-diméthoxy)-pyrimidyle, 2,6-diméthyl-benzyle, -CH₂-1-(3-nitro-5-méthyl)-pyrazolyle, 2-(1-méthyl)-benzimidazolyle, 2-(5-méthyl)-oxadiazolyle, 2-[3-méthyl-6-(trifluoro-méthyl)-imidazo[4.5]-pyridinyle, 3-[4-éthyl-5-(trifluorométhyl)]-1,2,4-triazolyle, 3-[4-méthyl-5-(trifluorométhyl)]-1,2,4-triazolyle, 3-[4-méthyl-5-(difluorométhyl)]-1,2,4-triazolyle, 2-(5-phényl)-1.3.4-thiadiazolyle, 2-(1-méthyl-5-phényl)-imidazolyle, 2-(4,5-diméthyl)-oxazolyle, 2-(1-méthyl-5-méthoxycarbonyl)-imidazolyle, 2-(1-méthyl)-imidazolyle, 1,2-éthanediyle,
Y représente un groupe méthyle, éthyle ou benzyle,
n représente un nombre 0 ou 2 et de préférence 0,
A représente un radical de la série constituée par un atome d'hydrogène et un groupe méthyle, et de préférence un groupe méthyle,
T représente une paire d'électrons,
et leurs sels
et
composés de formule (I-B) 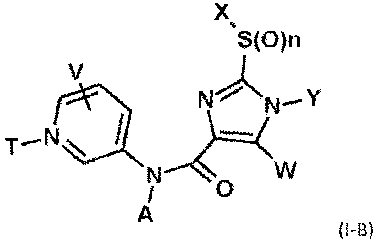 selon la revendication 1, dans laquelle
V représente un atome d'hydrogène,
W représente un radical de la série constituée par les atomes d'hydrogène, de chlore et de brome, et de préférence un atome d'hydrogène,
X représente un radical de la série constituée par les groupes 4,6-diméthylpyrimidyle, n-butyle, n-pentyle, benzyle, méthyle, 3-méthylthiophényle, 2,2,2-trifluoroéthyle, phényle, 4-méthylphényle, pyrimidyle, éthylthioéthyle, 2-nitrophényle, cyclopropylméthyle et de préférence un radical pyrimidyle,
Y représente un groupe méthyle,
n représente un nombre 0 ou 2 et de préférence 0,
A représente un radical de la série constituée par un atome d'hydrogène et un groupe méthyle, et de préférence un groupe méthyle,
T représente une paire d'électrons,
et leurs sels.

9. Agent, ayant une teneur en au moins un composé selon l'une quelconque des revendications 1 à 8 et des excipients usuels et/ou des substances tensioactives usuelles, en particulier destiné à la lutte contre des organismes nuisibles animaux.

10. Procédé pour la lutte contre des organismes nuisibles animaux, dans lequel on fait agir sur les organismes nuisibles animaux et/ou leur habitat au moins un composé selon l'une quelconque des revendications 1 à 8 ou un agent selon la revendication 9, à l'exclusion du traitement chirurgical, thérapeutique et diagnostique du corps humain ou animal.

11. Utilisation d'au moins un composé selon l'une quelconque des revendications 1 à 8 ou un agent selon la revendication 9, pour la lutte contre des organismes nuisibles animaux, à l'exclusion du traitement chirurgical, thérapeutique et diagnostique du corps humain ou animal.

12. Utilisation d'au moins un composé selon l'une quelconque des revendications 1 à 8 pour la protection du matériel de multiplication de plantes.

13. Composition agrochimique contenant au moins un composé selon l'une quelconque des revendications 1 à 8 à des concentrations biologiquement efficaces comprises entre 0,00000001 et 98 % en poids, par rapport au poids de la composition agrochimique, ainsi que des excipients et/ou des substances tensioactives.

14. Composition agrochimique selon la revendication 13, contenant en outre une autre substance active agrochimique.

15. Produits intermédiaires de formules (XI) et (XXI) 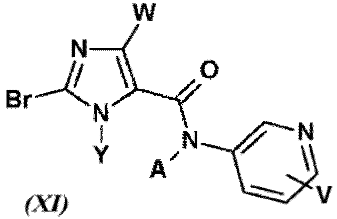 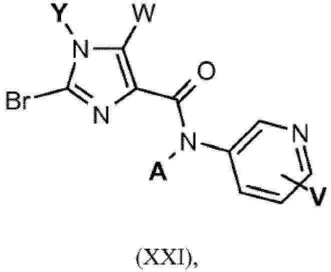 dans lesquelles les radicaux A, Y, V et W ont les significations selon l'une quelconque des revendications 1 à 3 ou 6 à 8 en de façon particulièrement préférée la signification selon la revendication 8.

16. Produits intermédiaires de formules (XV) et (XXII) 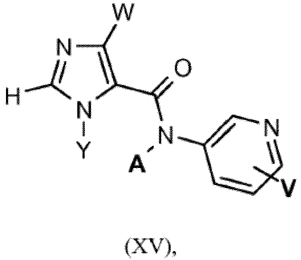 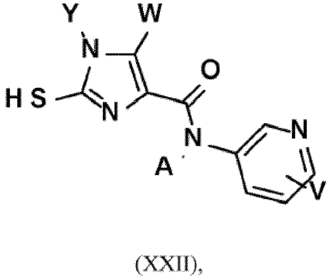 dans lesquelles
pour les produits intermédiaires de formule (XV)
V représente un atome d'hydrogène,
W représente un radical de la série constituée par les atomes d'hydrogène, de chlore et de brome et de préférence représente un atome d'hydrogène,
Y représente un groupe méthyle, éthyle ou benzyle et
A représente un radical de la série constituée par un atome d'hydrogène et un groupe méthyle, et de préférence représente un groupe méthyle
et pour les produits intermédiaires de formule (XXII)
V représente un atome d'hydrogène,
W représente un radical de la série constituée par les atomes d'hydrogène, de chlore et de brome et de préférence représente un atome d'hydrogène,
Y représente un groupe méthyle et
A représente un radical de la série constituée par un atome d'hydrogène et un groupe méthyle, et de préférence représente un groupe méthyle.
